(19) Europäisches Patentamt / European Patent Office / Office européen des brevets

(11) EP 4 076 440 B1

(12) EUROPEAN PATENT SPECIFICATION

(45) Date of publication and mention of the grant of the patent:
**08.07.2026 Bulletin 2026/28**

(21) Application number: **20903540.1**

(22) Date of filing: **17.12.2020**

(51) International Patent Classification (IPC):
***C07D 471/04*** (2006.01) ***C07D 487/04*** (2006.01)
***C07D 519/00*** (2006.01) ***A61K 31/437*** (2006.01)
***A61K 31/444*** (2006.01) ***A61K 31/4709*** (2006.01)
***A61K 31/4725*** (2006.01) ***A61K 31/498*** (2006.01)
***A61K 31/501*** (2006.01) ***A61K 31/5025*** (2006.01)
***A61K 31/506*** (2006.01) ***A61K 31/517*** (2006.01)
***A61K 31/538*** (2006.01) ***A61K 45/06*** (2006.01)
***A61P 35/00*** (2006.01) ***A61P 35/02*** (2006.01)

(52) Cooperative Patent Classification (CPC):
(C-Sets available)
**A61K 45/06; A61K 31/437; A61K 31/444; A61K 31/4709; A61K 31/4725; A61K 31/498; A61K 31/501; A61K 31/5025; A61K 31/506; A61K 31/517; A61K 31/538; A61P 35/00; A61P 35/02; C07D 471/04; C07D 487/04;** (Cont.)

(86) International application number:
**PCT/US2020/065593**

(87) International publication number:
**WO 2021/127166 (24.06.2021 Gazette 2021/25)**

(54) **1H-PYRROLO[3,2-C]PYRIDINE AND 1H-PYRROLO[3,2-B]PYRIDINE DERIVATIVES AS ENL/AF9 YEATS INHIBITORS FOR THE TREATMENT OF LEUKEMIA**

1H-PYRROLO[3,2-C]PYRIDIN- UND 1H-PYRROLO[3,2-B]PYRIDIN-DERIVATE ALS ENL/AF9 YEATS INHIBITOREN ZUR BEHANDLUNG VON LEUKÄMIE

DÉRIVÉS DE 1H-PYRROLO[3,2-C]PYRIDINE ET 1H-PYRROLO[3,2-B]PYRIDINE EN TANT QU'INHIBITEURS DE YEATS ENL/AF9 POUR LE TRAITEMENT DE LEUCÉMIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2019 US 201962949160 P**

(43) Date of publication of application:
**26.10.2022 Bulletin 2022/43**

(73) Proprietors:
- **The Rockefeller University**
  **New York, NY 10065 (US)**
- **Bridge Medicines, LLC**
  **New York, NY 10021 (US)**
- **Fukase, Yoshiyuki**
  **Edgwater, New Jersey 07020 (US)**
- **Michino, Mayako**
  **New York, NY 10044 (US)**
- **Stamford, Andrew W.**
  **Chatham, NJ 07928 (US)**
- **Miller, Michael W.**
  **Scotch Plains, NJ 07076 (US)**
- **Huggins, David**
  **New York, NY 10021 (US)**
- **Meinke, Peter**
  **Scotch Plains, New York 07076 (US)**

(72) Inventors:
- **ALLIS, C. David**
  **New York, New York 10065-6399 (US)**
- **WAN, Liling**
  **New York, New York 10065-6399 (US)**
- **VACCA, Joseph**
  **Telford, PA 18969 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **LADDUWAHETTY, Tammy**
  **London N21 3QA (GB)**
- **KHAN, Tanweer A.**
  **Bridgewater, New Jersey 08807 (US)**
- **LIVERTON, Nigel**
  **Harleysville, Pennsylvania 19438 (US)**
- **FUKASE, Yoshiyuki**
  **Edgewater, New Jersey 07020 (US)**
- **MICHINO, Mayako**
  **New York, New York 10044 (US)**
- **STAMFORD, Andrew W.**
  **Chatham, New Jersey 07928 (US)**
- **MILLER, Michael W.**
  **Scotch Plains, New Jersey 07076 (US)**
- **HUGGINS, David**
  **New York, New York 10021 (US)**
- **MEINKE, Peter**
  **Scotch Plains, New Jersey 07076 (US)**

(74) Representative: **Arnason Faktor**
**Intellectual Property Consulting**
**Gudridarstig 2-4**
**113 Reykjavik (IS)**



(56) References cited:
**WO-A1-2014/207260** **US-A1- 2011 015 225**
**US-A1- 2013 018 073** **US-B2- 7 541 366**

- **HEIDENREICH DAVID ET AL: "Structure-Based Approach toward Identification of Inhibitory Fragments for Eleven-Nineteen-Leukemia Protein (ENL)", JOURNAL OF MEDICINAL CHEMISTRY, vol. 61, no. 23, 8 November 2018 (2018-11-08), US, pages 10929 - 10934, XP055955713, ISSN: 0022-2623, DOI: 10.1021/acs.jmedchem.8b01457**
- **CHRISTOTT THOMAS, JAMES BENNETT, CARMEN COXON, OCTOVIA MONTEIRO, CHARLINE GIROUD, VIKTOR BEKE, SUET LING FELCE, VICKI GAMBLE, CARI: "Discovery of a Selective Inhibitor for the YEATS Domains of ENL/AF9", SLAS DISCOVERY: ADVANCING LIFE SCIENCES R&D, MARY ANN LIEBERT, vol. 24, no. 2, 1 February 2019 (2019-02-01), pages 133 - 141, XP055837858, ISSN: 2472-5552**



(52) Cooperative Patent Classification (CPC): (Cont.)
**C07D 519/00**

C-Sets
**A61K 31/437, A61K 2300/00;**
**A61K 31/444, A61K 2300/00;**
**A61K 31/4709, A61K 2300/00;**
**A61K 31/4725, A61K 2300/00;**
**A61K 31/498, A61K 2300/00;**
**A61K 31/501, A61K 2300/00;**
**A61K 31/5025, A61K 2300/00;**
**A61K 31/506, A61K 2300/00;**
**A61K 31/517, A61K 2300/00;**
**A61K 31/538, A61K 2300/00**

## Description

FIELD OF THE INVENTION

The present application relates generally to compounds that inhibit ENL/AF9 YEATS and are therefore useful for treating leukemia. The compounds are acylated 6-aminoindoles and acylated 6-aminopyrrolopyridines, more specifically derivatives of 1H-pyrrolo[3,2-b]pyridine and 1 H-pyrrolo[3,2-c]pyridine.

BACKGROUND OF THE INVENTION

Cancer cells are characterized by aberrant epigenetic landscapes and often exploit chromatin machinery to activate oncogenic gene expression programs. Recognition of modified histones by "reader" proteins constitutes a key mechanism underlying these processes; therefore, targeting such pathways holds clinical promise, as exemplified by the development of BET (bromo- and extra-terminal domain) bromodomain inhibitors. The YEATS domain is an acetyllysine-binding module. It has recently been shown [Wan et al. Nature 543, 265-269 (2017)] that the YEATS domain-containing protein ENL is required for disease maintenance in acute myeloid leukemia (AML). Depletion of ENL led to antileukemic effects, including increased terminal myeloid differentiation and suppression of leukemia growth. ENL binds to acetylated histone H3, and colocalizes with H3K27ac and H3K9ac on the promoters of actively transcribed genes that are essential for leukemias. Disrupting the interaction between the YEATS domain and histone acetylation via structure-based mutagenesis reduced RNA polymerase II recruitment to ENL target genes, leading to suppression of oncogenic gene expression programs. Importantly, disruption of ENL's functionality further sensitized leukemia cells to BET inhibitors. Thus, ENL inhibitors-either alone or in combination with BET inhibitors-represent promising therapy for AML.

Many BET inhibitors are currently or recently in clinical trials. For example, Birabresib (OTX015), (CAS No. 202590-98-5), an oral BET inhibitor, is now in clinical trial in patients with acute leukemia and in patients with selected advanced solid tumors. BET-d246, (CAS No. 2140289-17-2), is reported to exhibit strong anti-tumor activity in preclinical studies in mice. Mivebresib (ABBV-075) (CAS No. 1445993-26-9), in a 2-part phase 1 study in patients with solid tumors (part 1) and acute myeloid leukemia (AML; part 2) was well tolerated and showed antileukemic effects in patients with relapsed and refractory AML. I-BET 151 (CAS No. 1300031-49-5) in mice reduced myeloma tumors; treatment with I-BET *151 in vivo* impairs the leukemic engraftment of patient-derived primary samples and lowers the disease burden in mice. RO6870810/TEN-010 (CAS No. 1349719-98-7) is in clinical trial in patients with AML and Myelodysplastic Syndrome. CPI-0610 (CAS No. 1380087-89-7) is in clinical trial in patients with progressive lymphoma and with multiple myeloma. Molibresib (I-BET-762) (CAS No. 1260907-17-2) is in clinical trials for NUTcarcinoma. CPI 203 (CAS No. 1446144-04-2) is in phase 2 clinical trials for patients with myelofibrosis. PFI-1 (CAS No. 1403764-72-6) was in phase 1 clinical trial for solid tumors. JQ1 (CAS No. 1268524-70-4) is a potent inhibitor of the BET family of bromodomain proteins. While widely used in laboratory applications, JQ1 has a short half-life which precludes its use as a human therapeutic.

Christott et al. (2019) (Christott Thomas et al. "Discovery of a Selective Inhibitor for the YEATS Domains of ENL/AF9", SLAS Discovery: Advancing Life Sciences R&D, Vol. 24, Issue 2, 2019, Pages 133-141, ISSN 2472-5552, https://doi.org/10.1177/2472555218809904) describe how they developed a peptide displacement assay (histone 3 tail with acylated lysine) and screened a small-molecule library totaling more than 24,000 compounds for their propensity to disrupt the YEATS domain-histone peptide binding. Among these, they identified a dual inhibitor of ENL (Kd = 745 $\pm$ 45 nM) and its paralog AF9 (Kd = 523 $\pm$ 53 nM) and performed "SAR by catalog" with the aim of starting the development of a chemical probe for ENL.

Hedeinreich et al. (2018) (Heidenreich David et al., "Structure-Based Approach toward Identification of Inhibitory Fragments for Eleven-Nineteen-Leukemia Protein (ENL)". J Med Chem. 2018 Dec 13;61(23):10929-10934. doi: 10.1021/acs.jmedchem.8b01457. Epub 2018 Nov 26. PMID: 30407816) disclose a crystallography-based strategy and the discovery of fragments binding to the ENL YEATS domain. Crystal structures combined with synthetic efforts led to the identification of a submicromolar binder, providing first starting points for the development of chemical probes for this reader domain family.

US 2013/018073 A1 discloses an indole derivative having a melanin-concentrating hormone receptor antagonistic action, which is useful as an agent for the prophylaxis or treatment of obesity and the like. Specifically, the disclosure relates to a compound represented by the formula:

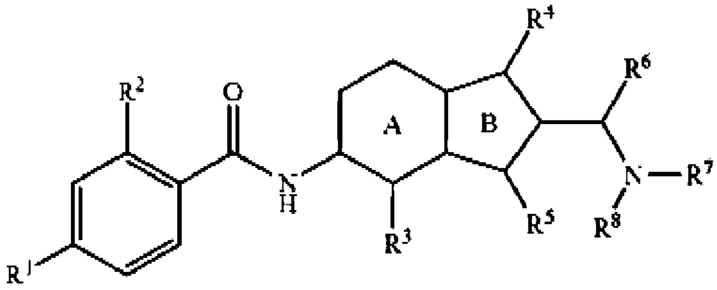

but these do not encompass the compounds provided by the present patent claims.

SUMMARY OF THE INVENTION

The invention is directed to compounds, pharmaceutical compositions, in vitro methods for inhibiting YEATS/ENL and compounds for use in methods for treating leukemia.

In one aspect, the invention relates to a compound as defined in claim 1, specifically a compound selected from

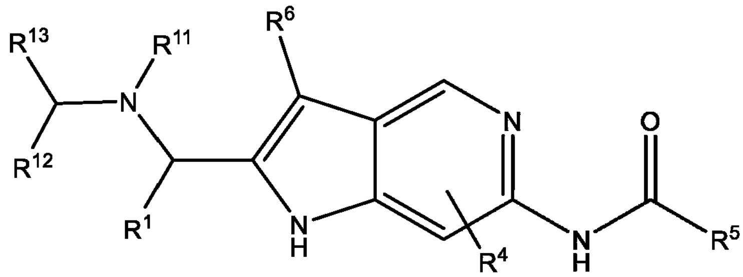

and

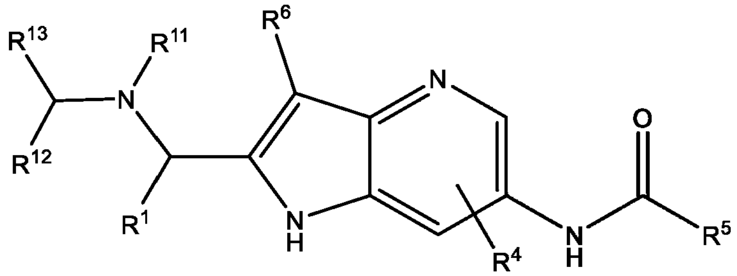

wherein:

$R^1$ is chosen from: H, $(C_{1-6})$alkyl, aryl$(C_{1-6})$alkyl, $(C_{3-12})$cycloalkyl$(C_{1-6})$alkyl, heterocyclyl, heterocyclyl$(C_{1-6})$alkyl, $(C_{1-6})$alkylamino$(C_{1-6})$alkyl, heterocyclylamino$(C_{1-6})$alkyl, heterocyclyl$(C_{1-6})$alkylamino$(C_{1-6})$alkyl, $(C_{3-12})$cycloalkylamino$(C_{1-6})$alkyl, $(C_{3-12})$cycloalkyl$(C_{1-6})$alkylamino$(C_{1-6})$alkyl, arylamino$(C_{1-6})$alkyl, and aryl$(C_{1-6})$alkylamino$(C_{1-6})$alkyl;

$R^4$ is chosen from H, $CH_3$, and Cl;

$R^5$ is chosen from 5- or 6- member carbocycle or heterocycle; bicyclic 5:6 carbocycle or heterocycle and bicyclic 6:6 carbocycle or heterocycle, not attached at a nitrogen of said heterocycle, wherein said carbocycle or heterocycle may be optionally substituted with one or more groups chosen from $(C_{1-8})$hydrocarbyl, $(C_{1-10})$alkoxy, halogen, $(C_{1-6})$haloalkyl, $-SO_2(C_{1-6})$alkyl, $-SO_2NH(C_{0-3}H_{1-7})$, $-CONH(C_{0-3}H_{1-7})$, $-SO_2NH(C_{1-6})$oxaalkyl, -CN, $CH_2CN$, $CH_2NH_2$, $-NH_2$, $NR^{14}$ where $R^{14}$ is independently chosen from hydrogen, $(C_{1-6})$fluoroalkyl, and $(C_{1-3})$alkoxy, $-CH_2OH$, benzyloxy, $-C(=NH)-NH_2$, -A-Het and additionally, when $R^5$ is a 5- or 6-member heterocycle, bicyclic 5:6 heterocycle, quinoline, isoquinolin, quinazolin, or benzo[*b*][1,4]oxazine, =O; wherein A is chosen from direct bond, $-(C_{1-6})$alkyl-, $-(C_{1-10})$alkoxy-, $-CO(C_{1-6})$alkyl-, $-SO_2(C_{1-6})$alkyl-, $-SO_2NH(C_{1-6})$alkyl-, and $-CONH(C_{1-6})$alkyl-;

and Het is heterocyclyl optionally substituted with $(C_{1-6})$hydrocarbyl, $(C_{1-10})$alkoxy, $(C_{1-10})$alkoxy(C=O)-, hydroxy, =O, or halogen and

$R^6$ is selected from: H, Me, F, and Cl;

$R^{11}$, $R^{12}$ and $R^{13}$ are chosen from the following three groups:

(a) $R^{11}$ is H or $CH_3$;

$R^{12}$ is chosen from H, $(C_1-C_6)$hydrocarbyl, hydroxy$(C_1-C_6)$hydrocarbyl, and 5- or 6-membered monocyclic heterocyclyl, wherein said heterocycle may be optionally substituted with $(C_1-C_6)$hydrocarbyl, hydroxyl, or hydroxy$(C_1-C_6)$hydrocarbyl; and
$R^{13}$ is hydrogen or methyl; or

(b) $R^{11}$ and $R^{12}$ taken together form an optionally substituted nitrogenous heterocycle attached via nitrogen, said nitrogenous heterocycle chosen from (a) a monocyclic aliphatic nitrogenous heterocycle, (b) a 5:5 or 5:6 bicyclic aliphatic nitrogenous heterocycle, (c) a spirobicyclic aliphatic nitrogenous heterocycle, and (d) an 8-azabicyclo [3.2.1]octane, wherein said optional substituents are independently chosen from $(C_{1-10})$hydrocarbyl, halo$(C_{1-10})$hydrocarbyl, halo$(C_{1-10})$hydrocarbyloxy, -$(C_{1-10})$alkoxy, COOH, -$SO_2(C_{1-6})$alkyl, =O, =S, and =NH; and
$R^{13}$ is chosen from H, $(C_{1-10})$hydrocarbyl, halo$(C_{1-10})$hydrocarbyl, halo$(C_{1-10})$hydrocarbyloxy, -$(C_{1-10})$alkoxy, -$SO_2(C_{1-6})$alkyl, =O, =S, and =NH; or

(c) $R^{11}$ is H; and
$R^{12}$ and $R^{13}$ taken together form a 3- to 7-membered aliphatic carbocycle, wherein said carbocycle may be optionally substituted with $(C_1-C_6)$hydrocarbyl, hydroxyl, or hydroxy$(C_1-C_6)$hydrocarbyl.

In an embodiment $R^{11}$ and $R^{12}$ taken together form an optionally substituted nitrogenous heterocycle, Q, chosen from (a) a monocyclic aliphatic nitrogenous heterocycle, (b) a 5:5 or 5:6 bicyclic aliphatic nitrogenous heterocycle, (c) a spirobicyclic aliphatic nitrogenous heterocycle, and (d) an 8-azabicyclo[3.2.1]octane, wherein said optional substituents are independently chosen from $(C_{1-10})$hydrocarbyl, halo$(C_{1-10})$hydrocarbyl, halo$(C_{1-10})$hydrocarbyloxy, -$(C_{1-10})$alkoxy, COOH, -$SO_2(C_{1-6})$alkyl, =O, =S, and =NH;
$R^1$ is chosen from: H, $(C_{1-6})$alkyl, aryl$(C_{1-6})$alkyl, $(C_{3-12})$cycloalkyl$(C_{1-6})$alkyl, heterocyclyl, heterocyclyl$(C_{1-6})$alkyl, $(C_{1-6})$alkylamino$(C_{1-6})$alkyl, heterocyclylamino$(C_{1-6})$alkyl, heterocyclyl$(C_{1-6})$alkylamino$(C_{1-6})$alkyl, $(C_{3-12})$cycloalkylamino$(C_{1-6})$alkyl, $(C_{3-12})$cycloalkyl$(C_{1-6})$alkylamino$(C_{1-6})$alkyl, arylamino$(C_{1-6})$alkyl, and aryl$(C_{1-6})$alkylamino$(C_{1-6})$alkyl.
In another aspect, the invention relates to an in vitro method of suppressing oncogene expression in a cell comprising exposing the cell to a compound as described herein.
In another aspect, the invention relates to a compound as claimed for use in method for treating a patient with leukemia.

DETAILED DESCRIPTION OF THE INVENTION

The present invention provides cyclic compounds having the structure as described above, which are useful for treating leukemia.
In a first subgenus, the compounds are pyrrolo[3,2-c]pyridines of formula II:

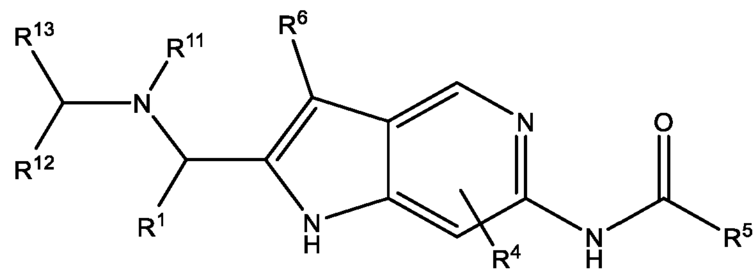

II

In a second subgenus, the compounds are pyrrolo[3,2-b]pyridines of formula III:

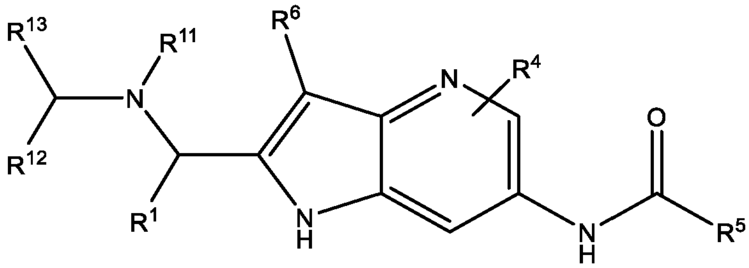

III

In some embodiments $R^{11}$ and $R^{12}$ taken together form an optionally substituted nitrogenous heterocycle, selected from be (a) a monocyclic aliphatic nitrogenous heterocycle, (b) a 5:5 or 5:6 bicyclic aliphatic nitrogenous heterocycle, (c) a spirobicyclic aliphatic nitrogenous heterocycle, and (d) an 8-azabicyclo[3.2.1]octane, optionally substituted with $(C_{1-10})$hydrocarbyl, halo$(C_{1-10})$hydrocarbyl, halo$(C_{1-10})$hydrocarbyloxy, -$(C_{1-10})$alkoxy, COOH, -$SO_2$ $(C_{1-6})$alkyl, =O, =S, and =NH.

In some embodiments $R^{11}$ and $R^{12}$ taken together form a monocyclic aliphatic nitrogenous heterocycle optionally substituted with one or more groups chosen from $(C_{1-10})$hydrocarbyl, halo$(C_{1-10})$hydrocarbyl, halo$(C_{1-10})$hydrocarbyloxy, -$(C_{1-10})$alkoxy, COOH, -$SO_2(C_{1-6})$alkyl, =O, =S, and =NH.

In some embodiments $R^{11}$ and $R^{12}$ taken together form a 5:5 or 5:6 bicyclic aliphatic nitrogenous heterocycle optionally substituted with one or more groups chosen from $(C_{1-10})$hydrocarbyl, halo$(C_{1-10})$hydrocarbyl, halo$(C_{1-10})$ hydrocarbyloxy, -$(C_{1-10})$alkoxy, COOH, -$SO_2(C_{1-6})$alkyl, =O, =S, and =NH.

In some embodiments $R^{11}$ and $R^{12}$ taken together form a spirobicyclic aliphatic nitrogenous heterocycle.

$R^5$ may be a carbocycle or heterocycle chosen from phenyl, indazole, pyridine, imidazolopyridine, pyrazolopyrimidine, imidazolopyrazine, triazolopyridine, and benzoxazine, or reduced forms thereof. The carbocycle or heterocycle $R^5$ may be optionally substituted, although, as noted above, when all of $X^1$, $X^2$ and $X^3$ are carbon (i.e. formula V), and $R^5$ is phenyl, the phenyl must be substituted with a heterocycle. In a particular embodiment, $R^5$ is indazole substituted with methyl.

In some embodiments, $R^{11}$ is hydrogen. In some embodiments, $R^{12}$ and $R^{13}$ taken together form a 3- to 7-membered aliphatic carbocycle, wherein said carbocycle may be optionally substituted with $(C_1\text{-}C_6)$hydrocarbyl, hydroxyl, or hydroxy$(C_1\text{-}C_6)$hydrocarbyl.

In some embodiments, $R^{11}$ and $R^{13}$ are hydrogen, methyl, or a combination thereof. In some embodiments, $R^{12}$ is hydrogen, $(C_1\text{-}C_6)$hydrocarbyl, hydroxy$(C_1\text{-}C_6)$hydrocarbyl, and 5- or 6- membered monocyclic heterocyclyl, wherein said heterocycle may be optionally substituted with $(C_1\text{-}C_6)$hydrocarbyl, hydroxyl, or hydroxy$(C_1\text{-}C_6)$hydrocarbyl.

In some embodiments $R^1$ may be hydrogen. In some embodiments n may be 2. In some embodiments, $R^4$ is hydrogen. In some embodiments, $R^6$ is hydrogen or methyl; in other embodiments, $R^6$ is chloro. In some embodiments, $R^5$ is pyridine substituted with an optionally substituted heterocycle, -CN or -$SO_2NHCH_3$.

In some embodiments, $R^5$ is phenyl substituted with one or more groups chosen from $(C_{1-6})$hydrocarbyl, $(C_{1-6})$ alkoxy, halogen, $(C_{1-6})$haloalkyl, -$SO_2(C_{1-6})$alkyl, -$SO_2NH(C_{0-3}H_{1-7})$, -CN, -$NH_2$, -$CH_2OH$, benzyloxy, and heterocyclyl optionally substituted with methyl, hydroxy, =O, or halogen. In particular, the phenyl may be substituted with fluoro and/or an optionally substituted heterocycle.

In one aspect, the invention relates to compounds described above as compounds for use for treating a leukemic patient with said compound. The method may additionally comprise administering a BET inhibitor. BET inhibitors are a well-known class of compounds, examples of which include OTX015, BET-d246, ABBV-075, I-BET 151, RO6870810, TEN-010, CPI-0610, I-BET-762, CPI 203, PFI-1, and JQ1. Administration may be concomitant, which, as used herein, refers to administration of two separate medicaments within a period of time that is insufficient for partial or complete elimination of at least one of the medicaments. In some embodiments, concomitant administration encompasses administration of a second medicament within the t½ of a first-administered drug. The term "combination treatment," as used herein, encompasses administration of two or more agents to a subject so that both agents and/or their active metabolites are present in the subject at the same time. Combination treatment can include simultaneous administration in separate compositions, administration at different times in separate compositions, or administration in a composition in which both agents are present.

It is to be understood that pharmaceutical compositions of compounds of the present inventions comprise one or more pharmaceutically acceptable excipients, including, but not limited to, one or more binders, bulking agents, buffers, stabilizing agents, surfactants, wetting agents, lubricating agents, diluents, disintegrants, viscosity enhancing or reducing agents, emulsifiers, suspending agents, preservatives, antioxidants, opacifying agents, glidants, processing aids, colorants, sweeteners, taste-masking agents, perfuming agents, flavoring agents, diluents, polishing agents, polymer matrix systems, plasticizers and other known additives to provide an elegant presentation of the drug or aid in the manufacturing

of a medicament or pharmaceutical product comprising a composition of the present inventions. Examples of carriers and excipients well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005.

In various embodiments, non-limiting examples of excipients include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (e.g., Nos. 2208, 2906, 2910), hydroxypropyl cellulose, titanium dioxide, talc, calcium carbonate (e.g., granules or powder), microcrystalline cellulose, powdered cellulose, dextrates, kaolin, silicic acid, sorbitol, starch, pre-gelatinized starch, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, a syloid silica gel (AEROSIL200, manufactured by W.R. Grace Co. of Baltimore, MD), a coagulated aerosol of synthetic silica (marketed by Degussa Co. of Plano, TX), CAB-O-SII, (a pyrogenic silicon dioxide product sold by Cabot Co. of Boston, MA), colorants and mixtures thereof.

The terms "subject" or "subject in need thereof" are used interchangeably herein. These terms refer to a patient who has been diagnosed with the underlying disorder to be treated. The subject may currently be experiencing symptoms associated with the disorder or may have experienced symptoms in the past. Additionally, a "subject in need thereof" may be a patient at risk of developing a particular disease, or to a patient reporting one or more of the physiological systems of a disease, even though a diagnosis of this disease may not have been made.

As used herein, the terms "treatment" or "treating" are used interchangeably. These terms refer to an approach for obtaining beneficial or desired results including, but not limited to, therapeutic benefit. Therapeutic benefit includes eradication or amelioration of the underlying disorder being treated; it also includes the eradication or amelioration of one or more of the symptoms associated with the underlying disorder such that an improvement is observed in the patient, notwithstanding that the patient may still be afflicted with the underlying disorder.

As used herein, the term "optionally substituted" may be used interchangeably with "unsubstituted or substituted". The term "substituted" refers to the replacement of one or more hydrogen atoms in a specified group with a specified radical. For example, substituted aryl, heterocyclyl etc. refer to aryl or heterocyclyl wherein one or more H atoms in each residue are replaced with halogen, haloalkyl, alkyl, acyl, alkoxyalkyl, hydroxyloweralkyl, carbonyl, phenyl, heteroaryl, benzenesulfonyl, hydroxy, loweralkoxy, haloalkoxy, alkoxy, carboxy, alkoxycarbonyl [-C(=O)O-alkyl], carboxamido [-C(=O)$NH_2$], alkylaminocarbonyl [-C(=O)NH-alkyl], cyano, acetoxy, nitro, amino, alkylamino, dialkylamino, dialkylaminoalkyl, dialkylaminoalkoxy, heterocyclylalkoxy, arylalkyl, (cycloalkyl)alkyl, heterocyclyl, heterocyclylalkyl, alkylaminoalkyl, heterocyclylaminoalkyl, heterocyclylalkylaminoalkyl, cycloalkylaminoalkyl, cycloalkylalkylaminoalkyl, arylaminoalkyl, and arylalkylaminoalkyl, mercapto, alkylthio, alkylsulfinyl, benzyl, heterocyclyl, phenoxy, benzyloxy, heteroaryloxy, aminosulfonyl, amidino, guanidino, and ureido. ($C_{1-6}$)hydrocarbyl, -$SO_2$alkyl, -$SO_2NH_2$, or -$SO_2$NHalkyl.

Unless otherwise specified, alkyl is intended to include linear or branched hydrocarbon structures. Lower alkyl refers to alkyl groups of from 1 to 6 carbon atoms. Examples of lower alkyl groups include methyl, ethyl, propyl, isopropyl, butyl, s-and t-butyl and the like. Preferred alkyl groups are those of $C_{20}$ or below.

$C_1$ to $C_{20}$ hydrocarbon or hydrocarbyl (as a substituent) includes alkyl, cycloalkyl, polycycloalkyl, alkenyl, alkynyl, aryl and combinations thereof. Examples include cyclopropylmethyl, benzyl, phenethyl, cyclohexylmethyl, camphoryl and naphthylethyl. Hydrocarbon refers to any substituent comprised of hydrogen and carbon as the only elemental constituents. Cycloalkyl is a subset of hydrocarbyl and includes cyclic hydrocarbon groups of from 3 to 8 carbon atoms. Examples of cycloalkyl groups include c-propyl, c-butyl, c-pentyl, norbornyl and the like.

Unless otherwise specified, the term "carbocycle" is intended to include ring systems in which the ring atoms are all carbon but of any oxidation state. Thus ($C_3$-$C_8$) carbocycle refers to both non-aromatic and aromatic systems, including such systems as cyclopropane, benzene and cyclohexene; ($C_8$-$C_{12}$) carbopolycycle refers to such systems as norbornane, decalin, indane and naphthalene. Carbocycle, if not otherwise limited, refers to monocycles, bicycles and polycycles.

Alkoxy or alkoxyl refers to groups of from 1 to 8 carbon atoms of a straight, or branched configuration and combinations thereof attached to the parent structure through an oxygen. Examples include methoxy, ethoxy, propoxy, isopropoxy, and the like. Lower-alkoxy refers to groups containing one to four carbons. For the purpose of this application, alkoxy and lower alkoxy include methylenedioxy and ethylenedioxy.

Thiaalkyl and azaalkyl refer to alkyl residues in which one or more carbons has been replaced by sulfur or nitrogen, respectively. Examples include ethylaminoethyl and methylthiopropyl.

Unless otherwise specified, acyl refers to formyl and to groups of 1, 2, 3, 4, 5, 6, 7 and 8 carbon atoms of a straight, branched, cyclic configuration, saturated, unsaturated and aromatic and combinations thereof, attached to the parent structure through a carbonyl functionality. One or more carbons in the acyl residue may be replaced by nitrogen, oxygen or sulfur as long as the point of attachment to the parent remains at the carbonyl. Examples include acetyl, benzoyl, propionyl, isobutyryl, t-butoxycarbonyl, benzyloxycarbonyl and the like. Lower-acyl refers to groups containing one to four carbons. The double bonded oxygen, when referred to as a substituent itself is called "oxo".

Aryl and heteroaryl mean (i) a phenyl group (or benzene) or a monocyclic 5- or 6-membered heteroaromatic ring containing 1-4 heteroatoms selected from O, N, or S; (ii) a bicyclic 9- or 10-membered aromatic or heteroaromatic ring system containing 0-4 heteroatoms selected from O, N, or S; or (iii) a tricyclic 13- or 14-membered aromatic or heteroaromatic ring system containing 0-5 heteroatoms selected from O, N, or S. The aromatic 6- to 14-membered carbocyclic rings include, e.g., benzene, naphthalene, indane, tetralin, and fluorene and the 5- to 10-membered aromatic heterocyclic rings include, e.g., imidazole, pyridine, indole, thiophene, benzopyranone, thiazole, furan, benzimidazole, quinoline, isoquinoline, quinoxaline, pyrimidine, pyrazine, tetrazole and pyrazole. As used herein aryl and heteroaryl refer to residues in which one or more rings are aromatic, but not all need be.

Arylalkyl refers to a substituent in which an aryl residue is attached to the parent structure through alkyl. Examples are benzyl, phenethyl and the like. Heteroarylalkyl refers to a substituent in which a heteroaryl residue is attached to the parent structure through alkyl. In one embodiment, the alkyl group of an arylalkyl or a heteroarylalkyl is an alkyl group of from 1 to 6 carbons. Examples include, e.g., pyridinylmethyl, pyrimidinylethyl and the like.

Heterocycle means a cycloalkyl or aryl carbocycle residue in which from one to four carbons is replaced by a heteroatom selected from the group consisting of N, O and S. The nitrogen and sulfur heteroatoms may optionally be oxidized, and the nitrogen heteroatom may optionally be quaternized. Unless otherwise specified, a heterocycle may be non-aromatic or aromatic. Examples of heterocycles that fall within the scope of the invention include pyrrolidine, pyrazole, pyrrole, indole, quinoline, isoquinoline, tetrahydroisoquinoline, benzofuran, benzodioxan, benzodioxole (commonly referred to as methylenedioxyphenyl, when occurring as a substituent), tetrazole, morpholine, thiazole, pyridine, pyridazine, pyrimidine, thiophene, furan, oxazole, oxazoline, isoxazole, dioxane, tetrahydrofuran and the like. It is to be noted that heteroaryl is a subset of heterocycle in which the heterocycle is aromatic. Examples of heteroaromatic rings include: furan, benzofuran, isobenzofuran, pyrrole, indole, isoindole, thiophene, benzothiophene, imidazole, benzimidazole, purine, pyrazole, indazole, oxazole, benzoxazole, isoxazole, benzisoxazole, thiazole, benzothiazole, triazole, tetrazole, pyridine, quinoline, isoquinoline, pyrazine, quinoxaline, acridine, pyrimidine, quinazoline, pyridazine, cinnoline, phthalazine, and triazine. Examples of heterocyclyl residues additionally include piperazinyl, 2-oxopiperazinyl, 2-oxopiperidinyl, 2-oxo-pyrrolidinyl, 2-oxoazepinyl, azepinyl, 4-piperidinyl, pyrazolidinyl, imidazolyl, imidazolinyl, imidazolidinyl, pyrazinyl, oxazolidinyl, isoxazolidinyl, thiazolidinyl, isothiazolyl, quinuclidinyl, isothiazolidinyl, benzimidazolyl, thiadiazolyl, benzopyranyl, benzothiazolyl, tetrahydrofuryl, tetrahydropyranyl, thienyl, benzothienyl, thiamorpholinyl, thiamorpholinylsulfoxide, thiamorpholinylsulfone, oxadiazolyl, triazolyl and tetrahydroquinolinyl.

An oxygen heterocycle is a heterocycle containing at least one oxygen in the ring; it may contain additional oxygens, as well as other heteroatoms. A sulphur heterocycle is a heterocycle containing at least one sulphur in the ring; it may contain additional sulphurs, as well as other heteroatoms. Oxygen heteroaryl is a subset of oxygen heterocycle; examples include furan and oxazole. Sulphur heteroaryl is a subset of sulphur heterocycle; examples include thiophene and thiazine. A nitrogen heterocycle is a heterocycle containing at least one nitrogen in the ring; it may contain additional nitrogens, as well as other heteroatoms. Examples include piperidine, piperazine, morpholine, pyrrolidine and thiomorpholine. Nitrogen heteroaryl is a subset of nitrogen heterocycle; examples include pyridine, pyrrole and thiazole.

Bicyclic nitrogenous heterocycles include (1) fused bicycles such as octahydrocyclopenta[c]pyrrole; (2) azaspirohexanes, heptanes and octanes, such as 6-oxa-2-azaspiro[3.4]octane, 2,6-diazaspiro[3.4]octane, 2-azaspiro[3.3]heptane, 2-oxa-6-azaspiro[3.3]heptane, and 7-oxa-2-azaspiro[3.5]nonane; and (3) an azabicycloalkane: 8-azabicyclo[3.2.1]octane. In the compounds described herein, these bicyclic nitrogenous heterocycles may be attached to the carbon bearing $R^1$ in formula I via nitrogen.

As used herein, and as would be understood by the person of skill in the art, the recitation of "a compound" - unless expressly further limited - is intended to include salts of that compound. Thus, for example, the recitation "a compound of formula Ia" as depicted

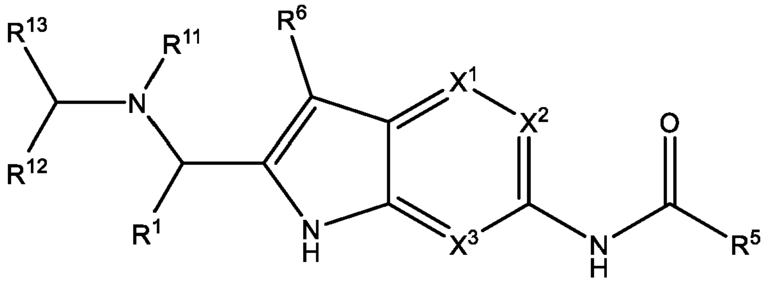

Ia

would include salts:

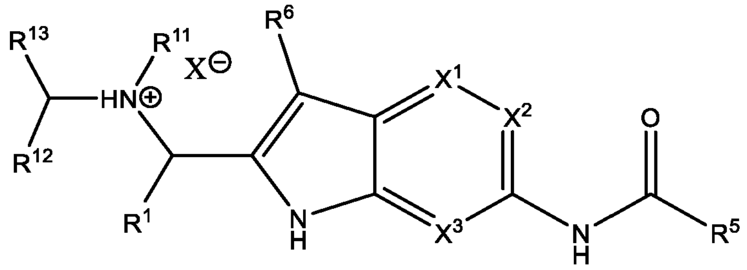

in which X is any counterion. In a particular embodiment, the term "compound of formula I" refers to the compound or a pharmaceutically acceptable salt thereof. The term "pharmaceutically acceptable salt" refers to salts prepared from pharmaceutically acceptable non-toxic acids or bases including inorganic acids and bases and organic acids and bases. When the compounds of the present invention are basic, as they usually would be, salts may be prepared from pharmaceutically acceptable non-toxic acids including inorganic and organic acids. Suitable pharmaceutically acceptable acid addition salts for the compounds of the present invention include acetic, adipic, alginic, ascorbic, aspartic, benzenesulfonic (besylate), benzoic, boric, butyric, camphoric, camphorsulfonic, carbonic, citric, ethanedisulfonic, ethanesulfonic, ethylenediaminetetraacetic, formic, fumaric, glucoheptonic, gluconic, glutamic, hydrobromic, hydrochloric, hydroiodic, hydroxynaphthoic, isethionic, lactic, lactobionic, laurylsulfonic, maleic, malic, mandelic, methanesulfonic, mucic, naphthylenesulfonic, nitric, oleic, pamoic, pantothenic, phosphoric, pivalic, polygalacturonic, salicylic, stearic, succinic, sulfuric, tannic, tartaric acid, teoclatic, p-toluenesulfonic, and the like. When the compounds contain an acidic side chain, for example when $R^3$ is CHCOOH, suitable pharmaceutically acceptable base addition salts for the compounds of the present invention include, but are not limited to, metallic salts made from aluminum, calcium, lithium, magnesium, potassium, sodium and zinc or organic salts made from lysine, arginine, N,N'-dibenzylethylenediamine, chloroprocaine, choline, diethanolamine, ethylenediamine, meglumine (N-methylglucamine) and procaine. Further pharmaceutically acceptable salts include, when appropriate, nontoxic ammonium cations and carboxylate, sulfonate and phosphonate anions attached to alkyl having from 1 to 20 carbon atoms.

It is to be understood that pharmaceutical compositions with compounds of the present inventions comprise one or more pharmaceutically acceptable excipients, including, but not limited to, one or more binders, bulking agents, buffers, stabilizing agents, surfactants, wetting agents, lubricating agents, diluents, disintegrants, viscosity enhancing or reducing agents, emulsifiers, suspending agents, preservatives, antioxidants, opaquing agents, glidants, processing aids, colorants, sweeteners, taste-masking agents, perfuming agents, flavoring agents, diluents, polishing agents, polymer matrix systems, plasticizers and other known additives to provide an elegant presentation of the drug or aid in the manufacturing of a medicament or pharmaceutical product comprising a composition of the present inventions. Examples of carriers and excipients well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005.

Unless otherwise stated or depicted, structures depicted herein are also meant to include all stereoisomeric (e.g., enantiomeric, diastereomeric, and cis-trans isomeric) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and cis-trans isomeric (or conformational) mixtures of the present compounds are within the scope of the invention. Unless otherwise stated, all tautomeric forms of the compounds of the invention are within the scope of the invention. Additionally, unless otherwise stated, structures depicted

herein are also meant to include compounds that differ only in the presence of one or more isotopically enriched atoms. For example, compounds having the present structures except for the replacement of hydrogen by deuterium or tritium, or the replacement of a carbon by a $^{13}C$- or $^{14}C$-enriched carbon are within the scope of this invention. Such compounds are useful, for example, as analytical tools or probes in biological assays.

The term "solvate" refers to a compound of Formula I in the solid state, wherein molecules of a suitable solvent are incorporated in the crystal lattice along with the compound of formula I. A suitable solvent for therapeutic administration is physiologically tolerable at the dosage administered. Examples of suitable solvents for therapeutic administration are ethanol and water. When water is the solvent, the solvate is referred to as a hydrate. In general, solvates are formed by dissolving the compound in the appropriate solvent and isolating the solvate by cooling or using an antisolvent. The solvate is typically dried or azeotroped under ambient conditions.

During the chemical syntheses, various protecting groups may be employed and subsequently removed in order to generate the compounds of the present invention. Exemplary protecting groups and conditions for their removal are described in Greene's Protecting Groups in Organic Synthesis P. G. M. Wuts, T. W. Greene, Fourth Edition, Wiley, New York, 2006.

EXAMPLES

The following compounds have been prepared, isolated and characterized using the methods disclosed herein. They demonstrate examples of the invention. (examples 18 and 19 are reference examples for comparative purposes)

**General Synthetic Schemes**

The compounds of the present invention were prepared by methods well known in the art of synthetic organic chemistry. During synthetic sequences it was sometimes necessary or desirable to protect sensitive or reactive groups on any of the molecules concerned. This was achieved by means of conventional protecting groups, such as those described in T. W. Greene and P. G. M. Wuts Greene's Protective Groups in Organic Synthesis, Fourth edition, John Wiley and Sons, 2006. The protecting groups were removed at a convenient subsequent stage using methods well known in the art.

In general, compounds of the present invention can be prepared by the methods illustrated in the general reaction schemes described below, or by modifications thereof, using readily available starting materials, reagents and conventional synthetic procedures. However, those skilled in the art will recognize that other methods may also be suitable. Also, in these reactions, it is possible to make use of variants that are in themselves known, but are not mentioned here.

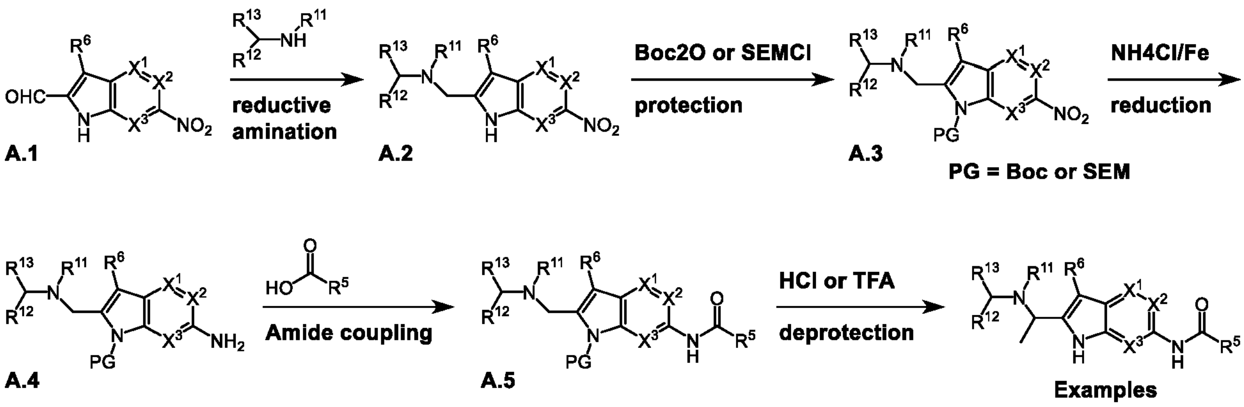

A.1 A.2 A.3 A.4 A.5 Examples

Scheme A

The aldehyde **A.1** can be converted into amine **A.2** using reductive amination conditions (e.g., $Na(AcO)_3BH$ or $NaCNBH_4$ with appropriate amine). The amine **A.2** can be protected with an appropriate functional group such as BOC or SEM to produce the protected intermediate **A.3.** The nitro intermediate **A.3** can be reduced to the amine **A.4** ($NH_4Cl/Fe$). The amine **A.4** can be functionalized via typical amide coupling conditions (e.g., HATU/base/$R^5CO_2H$) to produce the intermediate **A.5**. The protecting group in **A.5** can be removed using acid conditions (e.g., HCl or TFA) to produce representative examples illustrated in the specification.

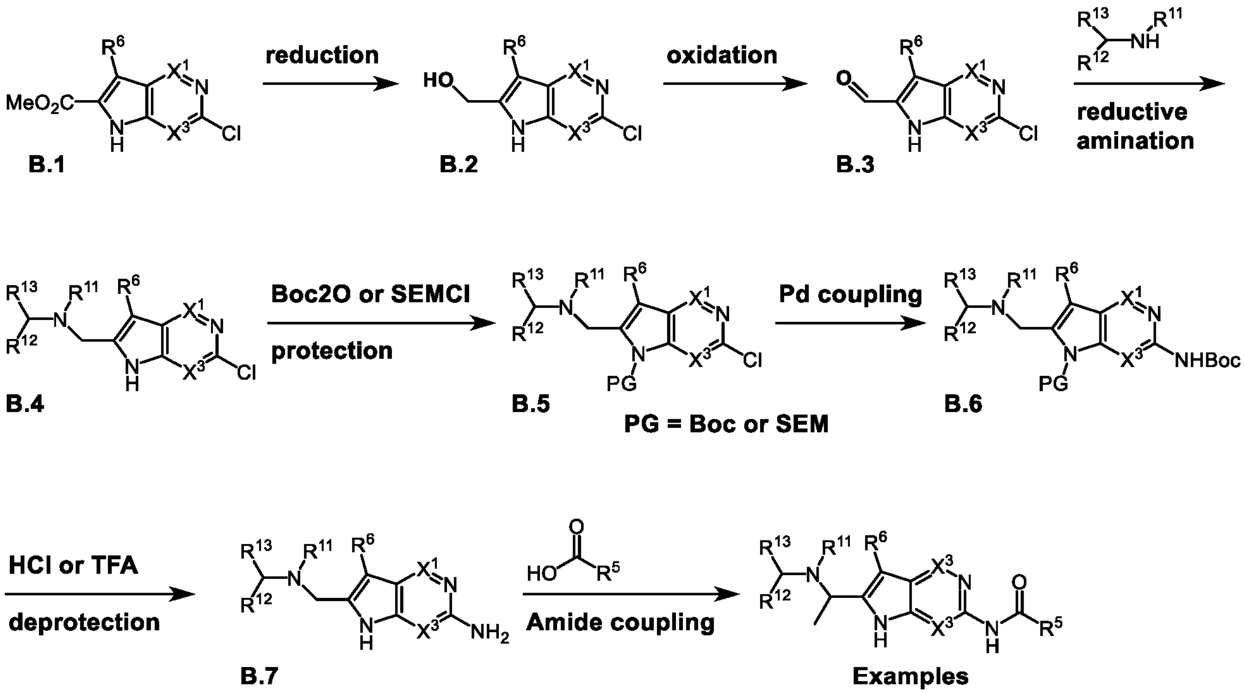


Scheme B

The methyl ester **B.1** can be reduced to the alcohol **B.2** using standard conditions to those versed in the art (e.g., $LiAlH_4$). The alcohol **B.2** can be oxidized to the aldehyde **B.3** with reagents such as Dess-Martin periodinane. The aldehyde **B.3** can be converted into the amine **B.4** using reductive amination conditions (e.g., $Na(AcO)_3BH$ or $NaCNBH_4$ with the appropriate amine). The NH in **B.4** can be protected with an appropriate group (e.g., Boc or SEM) to furnish the protected intermediate **B.5.** The chlorine in **B.5** can be transformed into the BOC protected intermediate **B.6** via appropriate Pd(0) catalyzed conditions (e.g., Pd(0)/ligand/$NH_2BOC$). The protecting groups in **B.6** can be removed under acid conditions (e.g., HCl or TFA) to furnish the amine **B.7.** The amine **B.7** can be converted into representative examples via standard amide coupling conditions (e.g., HATU/base/$R^5CO_2H$).

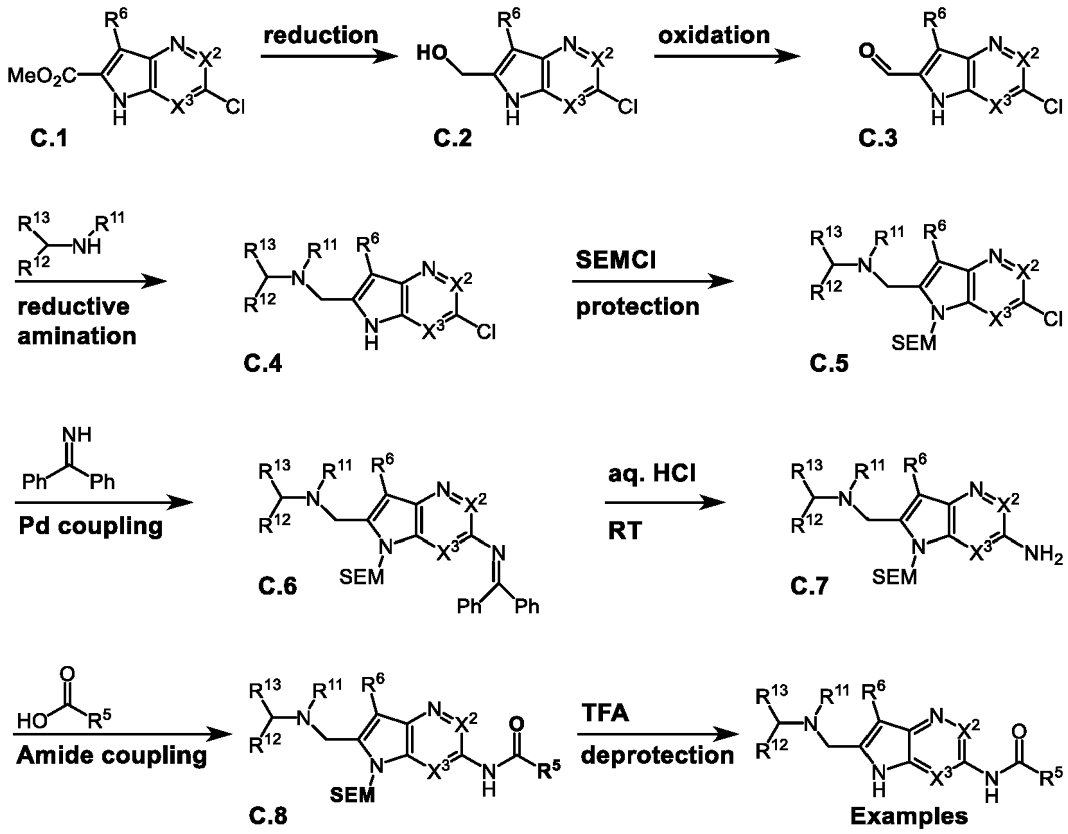


Scheme C

The ester **C.1** can be converted into the alcohol **C.2** using appropriate reducing reagents (e.g., $LiAlH_4$). The alcohol **C.2** can be converted into the aldehyde **C.3** using the appropriate oxidation conditions (e.g., Dess-Martin periodinane). The aldehyde **C.3** can be converted into the amine **C.4** using reductive amination conditions (e.g., $Na(AcO)_3BH$ or $NaCNBH_4$ with appropriate amine). The amine **C.4** can be protected with an appropriate protecting group to furnish **C.5** (e.g., SEM). The chlorine intermediate **C.5** can be converted into the imine intermediate **C.6** using Pd(0) catalysis with benzophenone imine and appropriate ligand. The imine in **C.6** can be hydrolyzed to the amine **C.7** (e.g., aqueous HCl). The amine **C.7** can be converted into the SEM protected **C.8** using standard amide coupling conditions (e.g., HATU/base/$R^5CO_2H$). The SEM group in **C.8** can be removed to furnish representative examples using acidic conditions such as TFA.

The following abbreviations are used in the synthetic routes: DCE (1,2-dichloroethane), THF (tetrahydrofuran), MeOH (methanol), DCM (dicholoromethane), HBTU ((2-(1*H*-benzotriazol-1-yl)-1,1,3,3-tetramethyluronium hexafluorophosphate), Dess Martin periodinane (3-oxo-1,3-dihydro-1$\lambda^5$,2-benziodoxole-1,1,1-triyl triacetate), DMF (*N,N*-dimethylformamide), BINAP ((2,2'-bis(diphenylphosphino)-1,1'-binaphthyl)), ACN (acetonitrile), TEA (triethylamine), AcOH (acetic acid), EtOH (ethanol), EtOAc (ethyl acetate), DMAP (*N,N*-dimethylpyridin-4-amine), XPhos (2-dicyclohexylphosphino-2',4',6'-triisopropylbiphenyl), TFA (trifluoroacetic acid), HATU (1-[bis(dimethylamino)methylene]-1H-1,2,3-triazolo[4,5-b]pyridinium 3-oxide hexafluorophosphate), SEMCl ((2-chloromethoxyethyl)trimethylsilane), dba ((1*E*, 4*E*)-1,5-diphenylpenta-1,4-dien-3-one), and $PPh_3$ (triphenylphosphine).

Preparative HPLC purification refers to the use of a water/acetonitrile gradient with or without the use of additives such as HCl, formic acid, TFA, or $NH_4HCO_3$ using an appropriate hydrophobic stationary phase.

The following acids used are depicted in **Table A.** For acids that are known, the CAS number is shown in the Reference column. Synthetic schemes for all other acids are depicted in the specification.

Table A

| Acid | Structure | Reference |
|---|---|---|
| A | 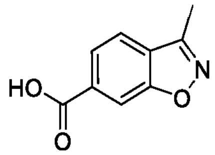 | 478169-72-1 |
| B | 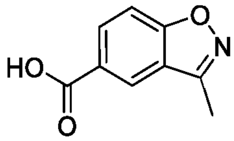 | Prepared |
| C | 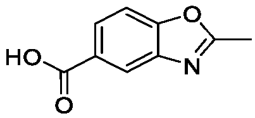 | 90322-32-0 |
| D | 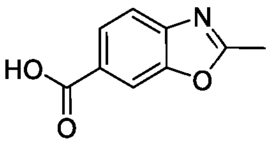 | 13452-14-7 |
| E | 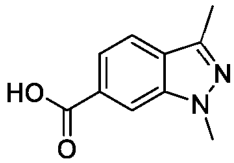 | Prepared |
| F | 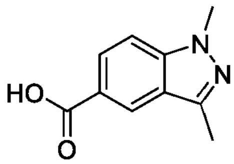 | Prepared |
| G | 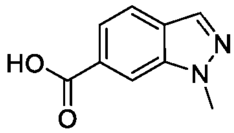 | 1031417-77-2 |
| H | 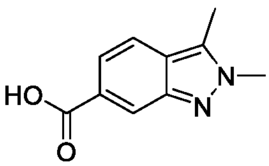 | Prepared |
| I | 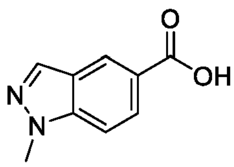 | 1176754-31-6 |
| J | 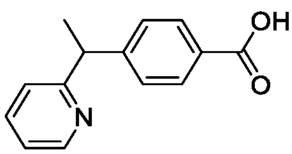 | Prepared |

(continued)

| Acid | Structure | Reference |
|---|---|---|
| K | 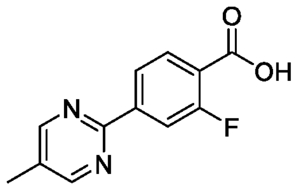 | Prepared |
| L | 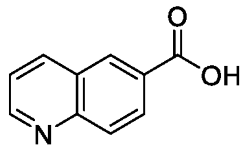 | 10349-57-2 |
| M | 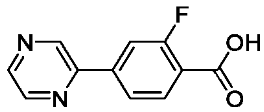 | 1344154-19-3 |
| O | 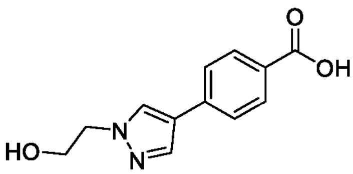 | Prepared |
| P | 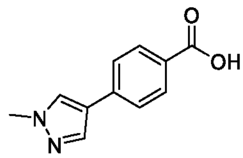 | Prepared |
| Q | 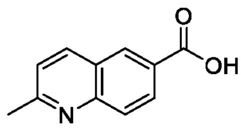 | 635-80-3 |
| R | 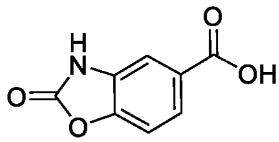 | Prepared |
| S | 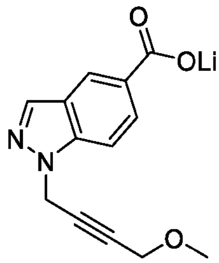 | Prepared |

(continued)

| Acid | Structure | Reference |
|---|---|---|
| T | 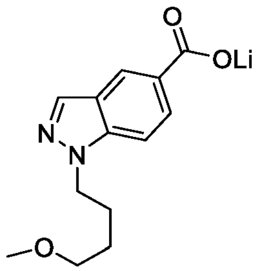 | Prepared |
| U | 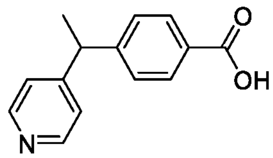 | Prepared |
| V | 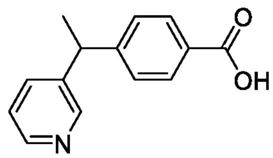 | Prepared |
| W | 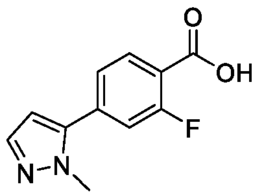 | Prepared |
| X | 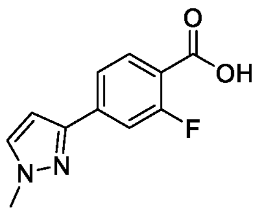 | Prepared |
| Y | 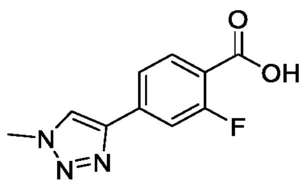 | Prepared |
| Z | 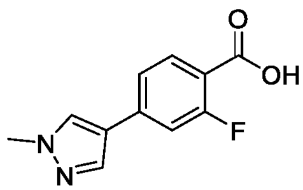 | Prepared |
| AA | 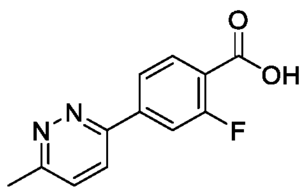 | Prepared |

(continued)

| Acid | Structure | Reference |
|---|---|---|
| AB | 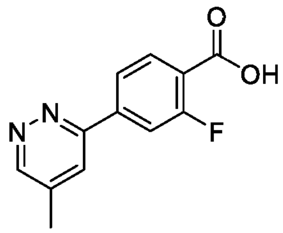 | Prepared |
| AC | 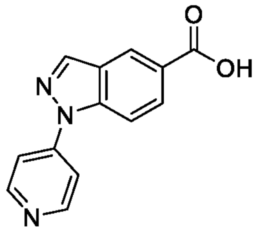 | Prepared |
| AD | 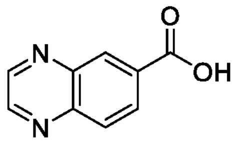 | 6925-00-4 |
| AE | 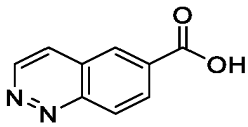 | Prepared |
| AF | 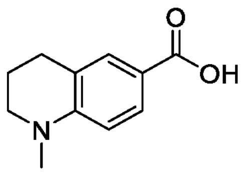 | Prepared |
| AG | 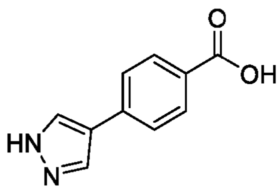 | Prepared |
| AH | 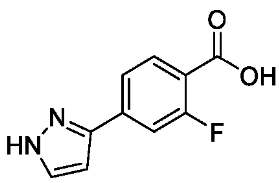 | Prepared |
| AI | 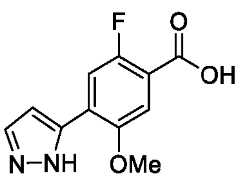 | Prepared |

(continued)

| Acid | Structure | Reference |
|---|---|---|
| AJ | 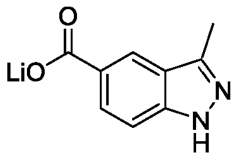 | Prepared |
| AK | 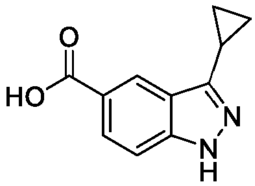 | Prepared |
| AL | 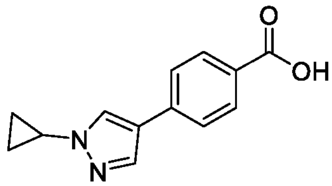 | Prepared |
| AM | 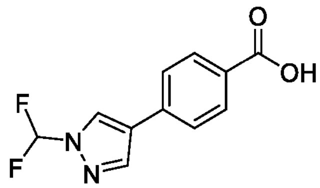 | Prepared |
| AN | 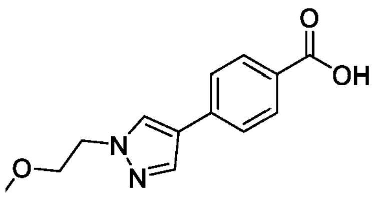 | Prepared |
| AO | 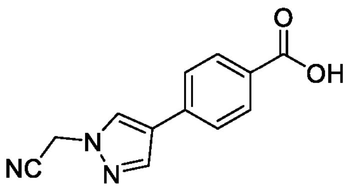 | Prepared |
| AP | 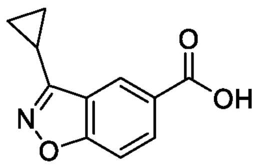 | Prepared |
| AQ | 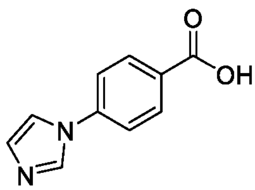 | 17616-04-5 |

(continued)

| Acid | Structure | Reference |
|---|---|---|
| AR | 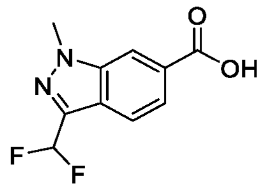 | Prepared |
| AS | 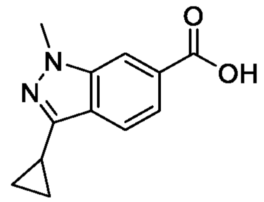 | Prepared |
| AT | 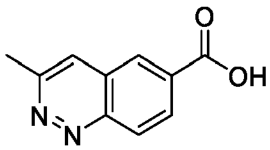 | Prepared |
| AU | 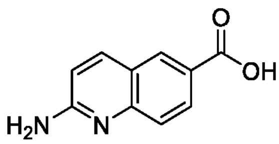 | Prepared |
| AV | 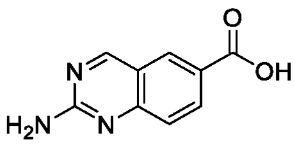 | Prepared |
| AW | 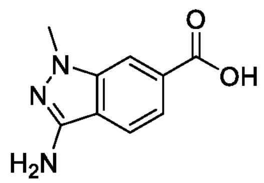 | Prepared |
| AX | 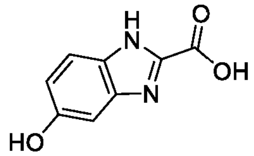 | Prepared |
| AY | 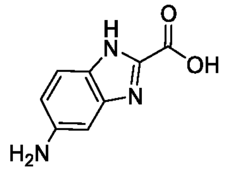 | Prepared |
| AZ | 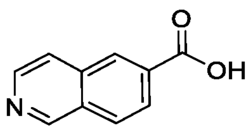 | 106778-43-2 |

(continued)

| Acid | Structure | Reference |
|---|---|---|
| BA | 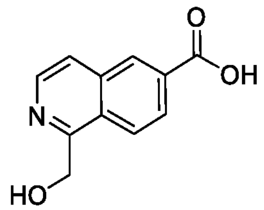 | Prepared |
| BB | 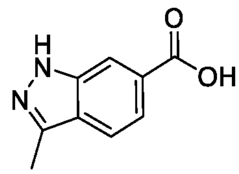 | Prepared |
| BC | 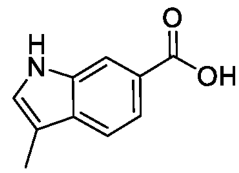 | Prepared |
| BD | 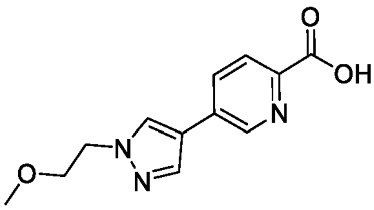 | Prepared |
| BE | 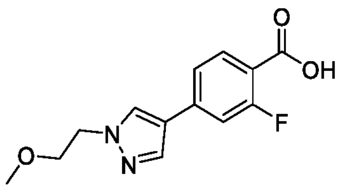 | Prepared |
| BF | 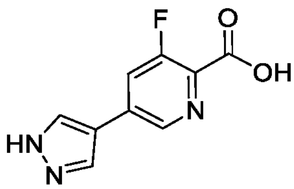 | Prepared |
| BG | 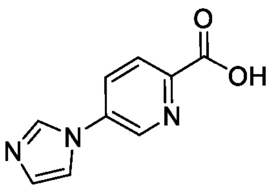 | Prepared |
| BH | 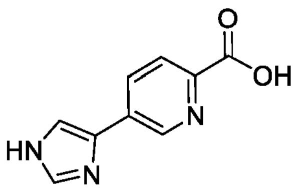 | Prepared |

(continued)

| Acid | Structure | Reference |
|---|---|---|
| BI | 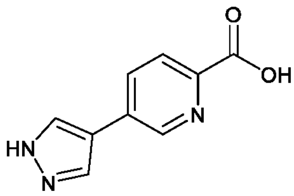 | Prepared |
| BJ | 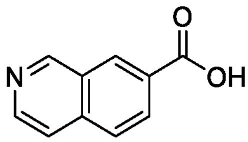 | 221050-96-0 |
| BK | 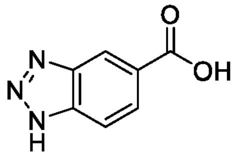 | 23814-12-2 |
| BL | 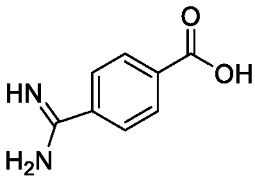 | 15535-95-2 |
| BM | 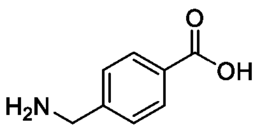 | 56-91-7 |
| BN | 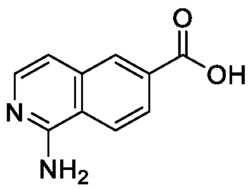 | Prepared |
| BO | 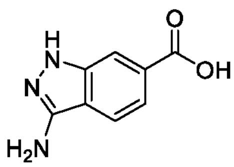 | Prepared |
| BP | 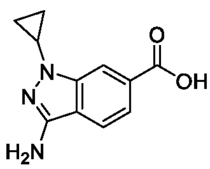 | Prepared |
| BQ | 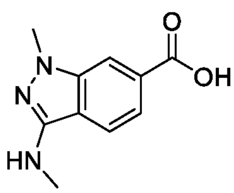 | Prepared |

(continued)

| Acid | Structure | Reference |
|---|---|---|
| BR | 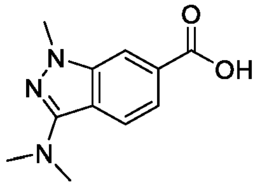 | Prepared |
| BS | 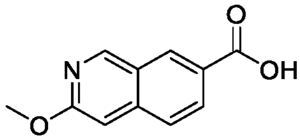 | 1374258-69-1 |
| BT | 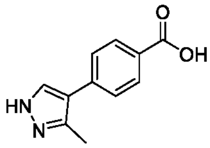 | Prepared |
| BU | 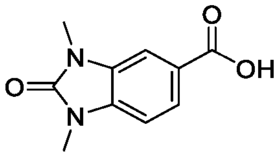 | Prepared |
| BV | 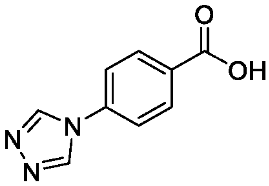 | 157069-48-2 |
| BW | 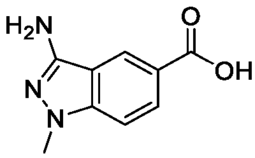 | Prepared |
| BX | 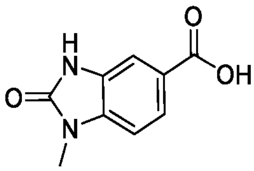 | Prepared |
| BY | 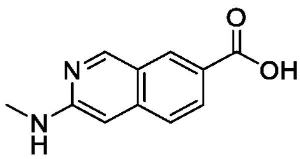 | Prepared |
| BZ | 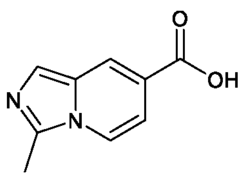 | 1330750-70-3 |

(continued)

| Acid | Structure | Reference |
|---|---|---|
| CA | 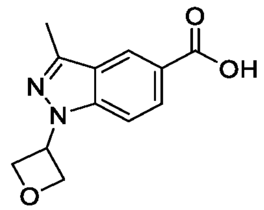 | Prepared |
| CB | 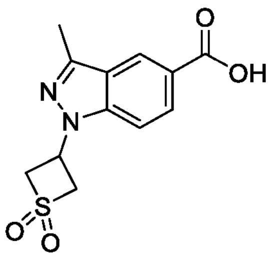 | Prepared |
| CC | 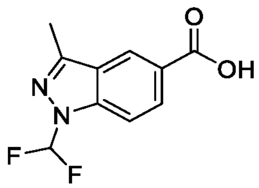 | Prepared |
| CE | 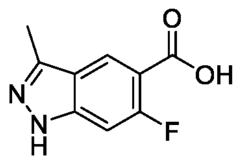 | Prepared |
| CF | 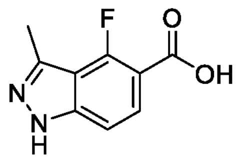 | Prepared |
| CG | 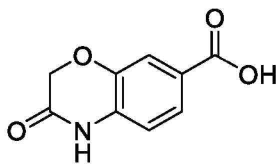 | 214848-62-1 |
| CH | 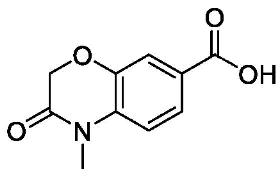 | Prepared |
| CI | 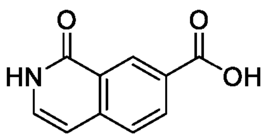 | Prepared |

(continued)

| Acid | Structure | Reference |
|---|---|---|
| **CJ** | 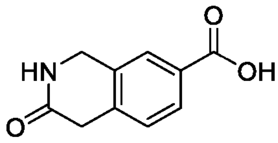 | **Prepared** |
| **CK** | 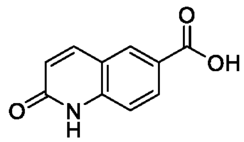 | **Prepared** |
| **CL** | 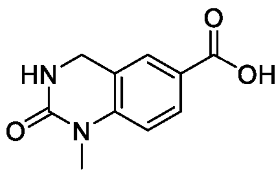 | **Prepared** |
| **CM** | 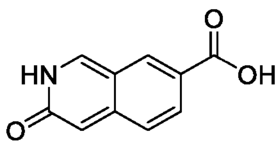 | **Prepared** |
| **CN** | 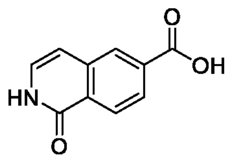 | **Prepared** |

Preparation of Acid B

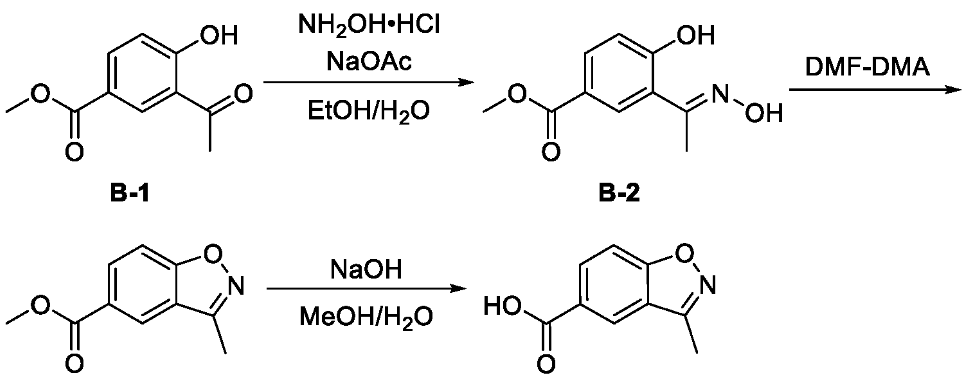


**B-1** **B-2**

**B-3** **Acid B**

Methyl 4-hydroxy-3-[(1E)-1-(hydroxyimino)ethyl]benzoate (B-2)

Into a 250-mL 3-necked round-bottom flask purged and maintained under an inert atmosphere of nitrogen, was placed methyl 3-acetyl-4-hydroxybenzoate **(B-1,** 4.80 g, 24.7 mmol, 1.0 equiv), EtOH (50.4 mL), $H_2O$ (21.6mL), hydroxylamine hydrochloride (3.4 g, 49.504 mmol, 2.00 equiv), sodium acetate (5.1 g, 61.804 mmol, 2.50 equiv). The resulting solution was stirred for 2 hr at 80 °C. The reaction mixture was cooled and the resulting solution was diluted with 100 mL of $H_2O$. The solids were collected by filtration. **LCMS:** $[M+H]^+$=210.

Methyl 3-methylbenzo[d]isoxazole-5-carboxylate as a white solid (B-3)

Into a 100-mL 3-necked round-bottom flask purged and maintained under an inert atmosphere of nitrogen, was placed methyl (E)-4-hydroxy-3-(1-(hydroxyimino)ethyl)benzoate **(B-2,** 3.5 g, 16.8 mmol, 1.00 equiv), dioxane (19.3 mL), DMF-DMA (9.2 mL). The resulting solution was stirred for 2 hr at 85 °C. The resulting mixture was concentrated. The residue was applied onto a silica gel column and eluted with ethyl acetate/petroleum ether (1:3). **LCMS:** $[M+H]^+$=192.

3-methyl-1,2-benzoxazole-5-carboxylic acid as a white solid **(Acid B)**

Into a 50-mL round-bottom flask, was placed methyl 3-methyl-1,2-benzoxazole-5-carboxylate **(1-3,** 1.0 g, 5.23 mmol, 1.0 equiv), MeOH (10.0 mL), $H_2O$ (3.0mL), sodium hydroxide (1.05 g, 26.3 mmol, 5.02 equiv). The resulting solution was stirred for 10 hr at room temperature. The resulting mixture was concentrated. The resulting solution was diluted with 20 mL of $H_2O$ and extracted with 2x20 mL of ethyl acetate and the aqueous layers combined. The pH value of the solution was adjusted to pH 3 and the resulting solids were collected by filtration. **$^1$H -NMR:** (300 MHz, DMSO-$d_6$, ppm) $\delta$ 13.17 (s, 1H), 8.47 (dd, $J$ = 1.8, 0.7 Hz, 1H), 8.21 (dd, $J$ = 8.8, 1.7 Hz, 1H), 7.79 (dd, $J$ = 8.7, 0.7 Hz, 1H), 2.61 (s, 3H).

Preparation of Acid E

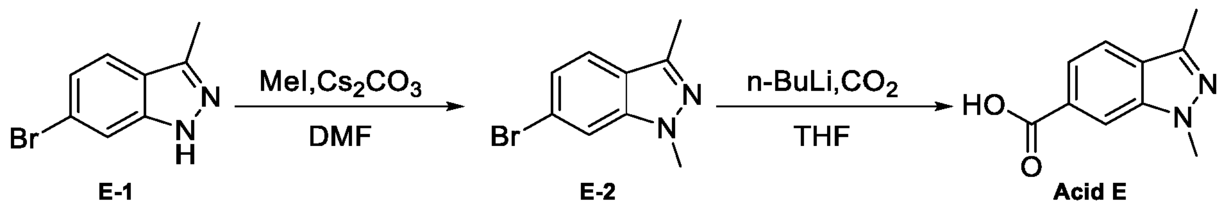


**E-1** **E-2** **Acid E**

6-bromo-1,3-dimethylindazole (E-2)

Into a 100 mL 3-necked round-bottom flask were added 6-bromo-3-methyl-1H-indazole **(E-1,** 2.90 g, 13.74 mmol, 1.00 equiv) and DMF (60 mL), $Cs_2CO_3$ (8.95 g, 27.48 mmol, 2.0 equiv) at room temperature. To the above mixture was added MeI (2.34 g, 16.49 mmol, 1.2 equiv) dropwise at room temperature. The resulting mixture was stirred for additional 2 h at room temperature. The resulting mixture was diluted with EtOAc (200 mL) and the resulting mixture was washed with 3x100 mL of brine. The resulting mixture was dried over anhydrous $Na_2SO_4$. After filtration, the filtrate was concentrated under reduced pressure. The residue was purified by silica gel column chromatography, eluted with PE/EtOAc (3: 1) to afford 6-bromo-1,3-dimethylindazole **(E-2,** 2.25 g, 72.75%) as a white solid, and 6-bromo-2,3-dimethylindazole (0.8 g, 25.9%) as a white solid. **$^1$H-NMR:** (300 MHz, Methanol-$d_4$, ppm) $\delta$ 7.72 (m, 1H), 7.60 (dd, $J$ = 8.7, 1.7 Hz, 1H), 7.23 (dd, $J$ = 8.6, 1.7 Hz, 1H), 3.95 (d, $J$ = 1.1 Hz, 3H), 2.52 (s, 3H).

1,3-dimethylindazole-6-carboxylic acid (Acid E)

Into a 250-mL 3-necked round-bottom flask purged and maintained with an inert atmosphere of nitrogen, was placed 6-bromo-1,3-dimethylindazole **(E-2)** under Ar at -78 °C, n-BuLi (5.86 mL, 2 M soln, 1.5equiv) was added dropwise. The resulting solution was stirred for 30 min at -78 °C. Followed by the slow addition of the Dry Ice (20.0 g). The resulting solution was stirred for 30 min at -78 °C. The resulting solution was allowed to react, with stirring, for an additional 2 hr at -78 °C. The reaction was then quenched by the addition of 100 mL of water/ice. The resulting solution was extracted with 2x30 mL of ethyl acetate and the aqueous layers combined. HCl (3 mol/L) was employed to adjust the pH to 3. The resulting solution was extracted with 3x30 mL of ethyl acetate dried over anhydrous sodium sulfate and concentrated under vacuum. This resulted in 1.4 g (75.31%) of 1,3-dimethylindazole-6-carboxylic acid **(Acid E)** as a white solid.

Preparation of Acid F

Into a 250-mL 3-necked round-bottom flask purged and maintained with an inert atmosphere of nitrogen, was placed 5-bromo-1,3-dimethylindazole (1.90 g, 8.48 mmol, 1.0 equiv), THF (57 mL), under Ar at -78 °C, n-BuLi (8.5 mL, 2.5 M soln, 1.5 equiv) was added dropwise. The resulting solution was stirred for 30 min at -78 °C. Followed by the slow addition of the Dry Ice (20.00 g). The resulting solution was allowed to react, with stirring, for an additional 2 hr at -78 °C. The reaction was then quenched by the addition of water/ice and extracted with 2x30 mL of ethyl acetate and the aqueous

layers combined. The pH value of the solution was adjusted to 3 with HCl (3M). The solids were collected by filtration. This resulted in 1 g (62.5%) of 1,3-dimethylindazole-5-carboxylic acid **(Acid F)** as a white solid. **LCMS:** $[M+H]^+$=191.

Preparation of Acid H

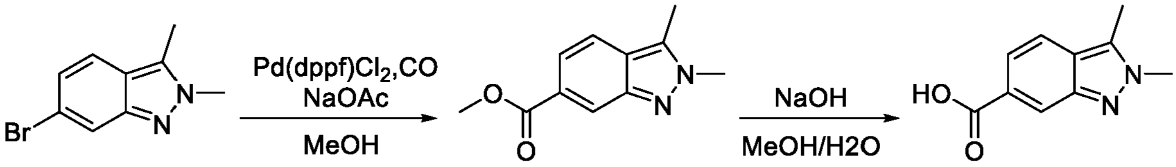


**H-1** **H-2** **Acid H**

Methyl 2,3-dimethylindazole-6-carboxylate (H-2)

Into a 50-mL pressure tank reactor, was placed 6-bromo-2,3-dimethylindazole **(H-1,** 650.0 mg, 2.89 mmol, 1.0 equiv), $CH_3OH$ (6.5 mL), $Pd(dppf)Cl_2CH_2Cl_2$ (117.9 mg, 0.14 mmol, 0.05 equiv), NaOAc (710.68 mg, 8.66 mmol, 3.0 equiv), CO(10 atm). The resulting solution was stirred for 24h at 95 °C. The resulting solution was diluted with 20 mL of $H_2O$. The resulting solution was extracted with 3x30 mL of ethyl acetate and the organic layers combined. The resulting mixture was washed with 2 x30 mL of Brine. The mixture was dried over anhydrous sodium sulfate and concentrated. **LCMS:** $[M+H]^+$=205.

2,3-dimethylindazole-6-carboxylic acid (Acid H)

Into a 100-mL round-bottom flask, was placed methyl 2,3-dimethylindazole-6-carboxylate **(H-2,** 700.0 mg, 3.43 mmol, 1.0 equiv), MeOH (21mL), $H_2O$ (7 mL), sodium hydroxide (685.5 mg, 17.14 mmol, 5.0equiv). The resulting solution was stirred for 10h at room temperature. The resulting mixture was concentrated and extracted with 2x50 mL of ethyl acetate, then the aqueous layers combined. The pH value of the solution was adjusted to 3 with HCl (3M). The solids were collected by filtration. This resulted in 500 mg (76.7%) of 2,3-dimethylindazole-6-carboxylic acid **(Acid H)** as a white solid. **$^1$H-NMR:** (300 MHz, DMSO-$d_6$, ppm) δ 12.81 (s, 1H), 8.17 (t, $J$ = 1.2 Hz, 1H), 7.74 (dd, $J$ = 8.7, 0.9 Hz, 1H), 7.49 (dd, $J$ = 8.7, 1.4 Hz, 1H), 4.10 (s, 3H), 2.63 (s, 3H).

Preparation of Acid J

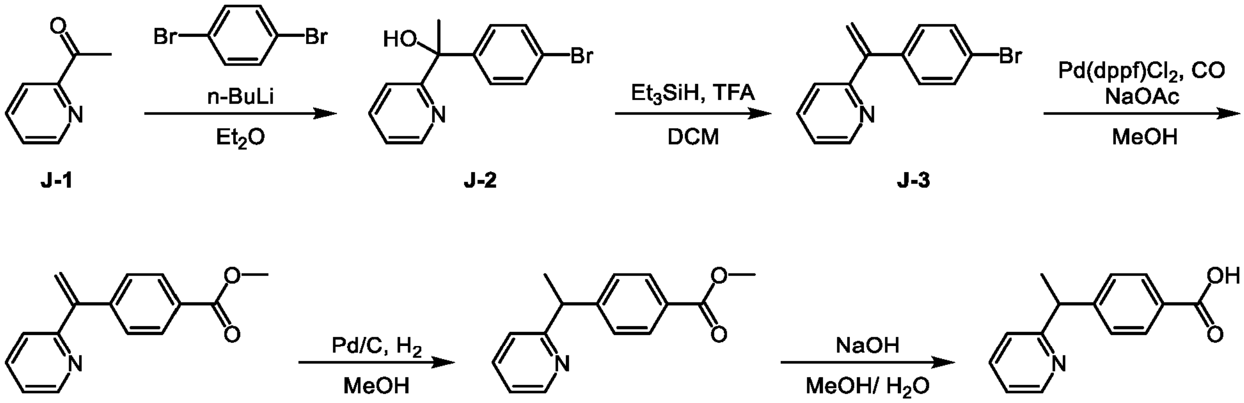


**J-1** **J-2** **J-3**

**J-4** **J-5** **Acid J**

1-(4-bromophenyl)-1-(pyridin-2-yl)ethanol (J-2)

To a stirred solution of dibromobenzene (10.0 g, 42.39 mmol, 1.0 equiv) in ether (200 mL) was added n-BuLi (16.96 mL, 42.39 mmol, 1.00 equiv) dropwise at -78 °C under nitrogen atmosphere. The resulting mixture was stirred for 30

min at -78 °C under nitrogen atmosphere. To the above mixture was added a mixture of 2-acetylpyridine **(J-1,** 5.65 g, 46.63 mmol, 1.10 equiv) in 50 mL of ether dropwise at -78 °C. The resulting mixture was stirred for additional 30 min at -78 °C. Then it was allowed to warm to room temperature slowly. The reaction was quenched by the addition of sat. NH4Cl (aq.) (100 mL) at 5 °C. The resulting mixture was extracted with EtOAc (3 x 50 mL). The combined organic layers were washed with brine (1x100 mL), dried over anhydrous $Na_2SO_4$. After filtration, the filtrate was concentrated under reduced pressure. The residue was purified by silica gel column chromatography, eluted with PE/EtOAc (2:1) to afford 1-(4-bromophenyl)-1-(pyridin-2-yl)ethanol **(J-2,** 4.5 g, 38.2%) as a yellow solid. **LCMS:** $[M+1]^+$=278.

2-[1-(4-bromophenyl)ethenyl]pyridine (J-3)

Into a 100 mL 3-necked round-bottom flask were added 1-(4-bromophenyl)-1-(pyridin-2-yl)ethanol **(J-2,** 1.10 g, 3.96 mmol, 1.00 equiv), DCM, triethylsilane (4.60 g, 39.55 mmol, 10.00 equiv), and TFA (9.02 g, 79.1 mmol, 20.0 equiv) at room temperature. The resulting mixture was stirred for 48h at room temperature under nitrogen atmosphere. The resulting mixture was concentrated under vacuum. The resulting mixture was diluted with $CH_2Cl_2$ (50mL). The resulting mixture was washed with 3x30 mL of $NaHCO_3$(sat.) The resulting organic layer was dried over anhydrous $Na_2SO_4$. After filtration, the filtrate was concentrated under reduced pressure. The residue was purified by silica gel column chromatography, eluted with PE/EtOAc (1:1) to afford 2-[1-(4-bromophenyl)ethenyl]pyridine **(J-3,** 900 mg, 78.74%) as a light yellow oil. **LCMS:** $[M+1]^+$=260.

Methyl 4-[1-(pyridin-2-yl)ethenyl]benzoate (J-4)

Into a 30 mL pressure tank reactor were added 2-[1-(4-bromophenyl)ethenyl]pyridine **(J-3,** 900 mg, 3.46mmol, 1.00 equiv), MeOH (20 mL), NaOAc (851 mg, 10.38 mmol, 3.00 equiv) and Pd(dppf)$Cl_2$ (253 mg, 0.346 mmol, 0.10 equiv) at room temperature. The resulting mixture was stirred for overnight at 90 °C under CO (20 atm) atmosphere. The resulting mixture was concentrated under reduced pressure. The residue was dissolved in EtOAc (100 mL). The resulting mixture was washed with 1x30 mL of water. The organic layer was, dried over anhydrous $Na_2SO_4$. After filtration, the filtrate was concentrated under reduced pressure. The residue was purified by silica gel column chromatography, eluted with PE/EtOAc (1:1) to afford methyl 4-[1-(pyridin-2-yl)ethenyl]benzoate **(J-4,** 660 mg, 74.94%) as a yellow solid. **LCMS:** $[M+1]^+$=240

Methyl 4-[1-(pyridin-2-yl)ethyl]benzoate (J-5)

Into a 50 mL round-bottom flask were added methyl 4-[1-(pyridin-2-yl)ethenyl]benzoate **(J-4,** 660.0 mg, 2.76 mmol, 1.00 equiv), MeOH (20 mL) and Pd/C (530.0 mg, 10%) at room temperature. The reaction was stirred for 24h at room temperature under 1atm of $H_2$(g). The resulting mixture was filtrated, the filter cake was washed with MeOH (2x20 mL). The filtrate was concentrated under reduced pressure. This resulted in methyl 4-[1-(pyridin-2-yl)ethyl]benzoate **(J-5,** 600 mg, 90%) as a dark oil. **LCMS:** $[M+1]^+$=242.

4-[1-(pyridin-2-yl)ethyl]benzoic acid (Acid J)

Into a 50 mL round-bottom flask were added methyl 4-[1-(pyridin-2-yl)ethyl]benzoate **(J-5,** 600.0 mg, 2.49 mmol, 1.00 equiv) and MeOH (10 mL) at room temperature. To the above mixture was added NaOH (198.92 mg, 4.97 mmol, 2.00 equiv) in $H_2O$ (5 mL) at room temperature. The resulting mixture was stirred for 16h at room temperature. The resulting mixture was extracted with EtOAc (1 x 50 mL). The aqueous layer was acidified to pH 4 with 2M HCl. The resulting mixture was concentrated under reduced pressure. This resulted in 4-[1-(pyridin-2-yl)ethyl]benzoic acid **(Acid J,** 700 mg, 86.71%) as a brown solid. **LCMS:** $[M+1]^+$=228.

Preparation of Acid K

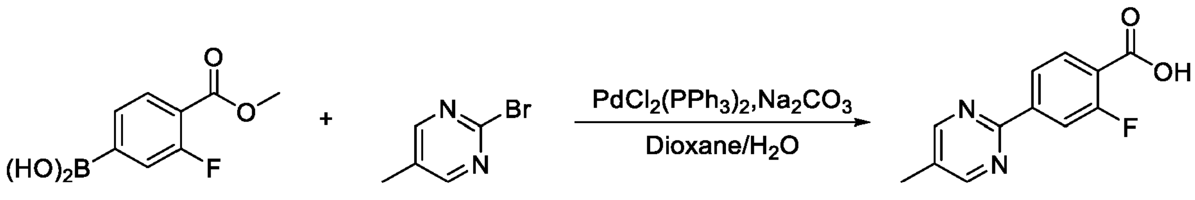


K-1 K-2 Acid K

Into a 40-mL vial purged and maintained with an inert atmosphere of nitrogen, was placed 3-fluoro-4-(methoxycarbonyl)phenylboronic acid **(K-1,** 0.97 g, 4.9 mmol, 1.00 equiv), dioxane (10 mL), $H_2O$ (10 mL), 2-bromo-5-methylpyrimidine **(K-2,** 0.85 g, 4.9 mmol, 1.00 equiv), palladium chloride; bis(triphenylphosphine) (0.34 g, 0.484 mmol, 0.10 equiv), sodium carbonate (1.58 g, 14.77 mmol, 3.0 equiv). The resulting solution was stirred for 16 h at 80 °C in an oil bath. The reaction mixture was cooled. The resulting mixture was concentrated under vacuum. The resulting solution was diluted with 30 mL of $H_2O$. The resulting solution was extracted with 2x30 mL of ethyl acetate and the aqueous layers combined. The pH value of the solution was adjusted to 3 with 3M HCl. The solids were collected by filtration. This resulted in 0.85 g (75%) of 2-fluoro-4-(5-methylpyrimidin-2-yl)benzoic acid **(Acid K)** as a brown solid. **LCMS:** $[M+H]^+$=233.

Preparation of Acid O

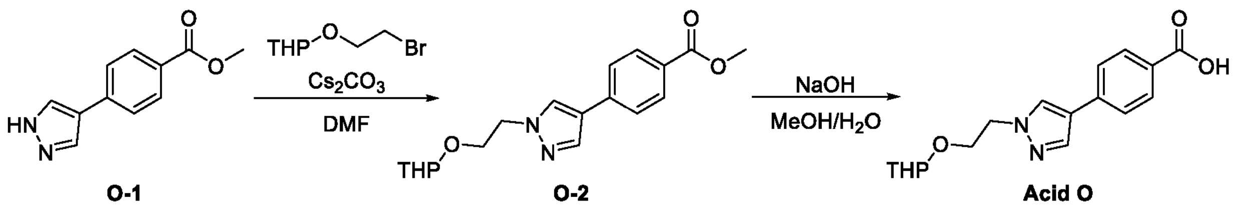


O-1 O-2 Acid O

Methyl 4-[1-[2-(oxan-2-yloxy)ethyl]pyrazol-4-yl]benzoate (O-2)

Into a 40-mL vial purged and maintained with an inert atmosphere of nitrogen, was placed a solution of methyl 4-(1H-pyrazol-4-yl)benzoate (**O-1**, 400.0 mg, 1.98 mmol, 1.00 equiv) in DMF (10 mL), 2-(2-bromoethoxy)oxane (537.7mg, 2.57 mmol, 1.30 equiv) and cesium carbonate (1.3g, 3.96 mmol, 2.00 equiv). The reaction was stirred for 12 h at room temperature. The resulting solution was diluted with 20 mL of $H_2O$ and extracted with 3x10 mL of ethyl acetate The resulting mixture was washed with 2x10 mL of brine, dried over anhydrous sodium sulfate and concentrated under vacuum. This resulted in 400 mg (61.21%) of methyl 4-[1-[2-(oxan-2-yloxy)ethyl]pyrazol-4-yl]benzoate (**O-2**) as a yellow solid. **LCMS:** $[M+H]^+$=331.

4-[1-[2-(oxan-2-yloxy)ethyl]pyrazol-4-yl]benzoic acid (Acid O)

Into a 20-mL vial, was placed a solution of methyl 4-[1-[2-(oxan-2-yloxy)ethyl]pyrazol-4-yl]benzoate (**O-2**, 400.0 mg, 1.21 mmol, 1.00 equiv) in MeOH (10 mL), sodium hydroxide (193.70 mg, 4.844 mmol, 4.00 equiv), $H_2O$ (5.00 mL). The resulting solution was stirred for 16 h at room temperature and concentrated under vacuum. The pH value of the solution was adjusted to 2 with 2M HCl . The solids were collected by filtration. This resulted in 200 mg (52.22%) of 4-[1-[2-(oxan-2-yloxy)ethyl]pyrazol-4-yl]benzoic acid (Acid O) as a white solid. **LCMS:** $[M+H]^+$=317.

Preparation of Acid P

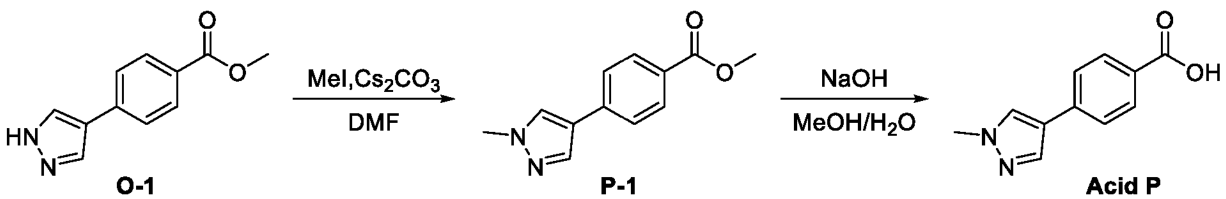


O-1 P-1 Acid P

Methyl 4-(1-methylpyrazol-4-yl)benzoate (P-1)

Into a 20-mL round-bottom flask purged and maintained with an inert atmosphere of nitrogen, was placed a solution of methyl 4-(1H-pyrazol-4-yl)benzoate (**O-1**, 400.0 mg, 1.978 mmol, 1.00 equiv) in DMF (10 mL), MeI (365.00 mg, 2.572 mmol, 1.30 equiv), $Cs_2CO_3$ (1.3g, 3.956 mmol, 2 equiv). The resulting solution was stirred for 12h at 60 °C. The resulting solution was diluted with 20 mL of $H_2O$. The resulting solution was extracted with 3x20 mL of ethyl acetate and the organic layers combined. The resulting mixture was washed with 3x15 mL of brine. The mixture was dried over anhydrous sodium sulfate and concentrated under vacuum. This resulted in 300 mg (70%) of methyl 4-(1-methylpyrazol-4-yl) benzoate **(P-1)** as a yellow solid. **LCMS:** $[M+H]^+$=217.

Into a 20-mL vial, was placed a solution of methyl 4-(1-methylpyrazol-4-yl)benzoate **(P-1,** 300.0 mg, 1.39 mmol, 1.00 equiv) in MeOH (10 mL), a solution of sodium hydroxide (221.96 mg, 5.549 mmol, 4.00 equiv) in $H_2O$ (5 mL). The resulting solution was stirred for 16h at room temperature. The resulting mixture was concentrated under vacuum. 2M HCl was added to adjust the pH to 2-3. The solids were collected by filtration. This resulted in 200 mg (71.3%) of 4-(1-methylpyrazol-4-yl)benzoic acid **(Acid P)** as a white solid.

Preparation of Acid R

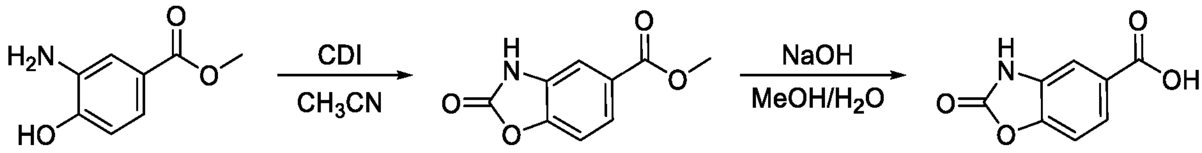


**R-1** **R-2** **Acid R**

Methyl 2-oxo-3H-1,3-benzoxazole-5-carboxylate (R-2).

Into a 20-mL vial purged and maintained with an inert atmosphere of nitrogen, was placed a solution of methyl 3-amino-4-hydroxybenzoate **(R-1,** 1g, 5.98 mmol, 1.00 equiv) in $CH_3CN$ (12 mL), CDI (3.0g, 17.95 mmol, 3.00 equiv). The resulting solution was stirred for 16h at 80 °C. The resulting mixture was concentrated under vacuum. The residue was applied onto a silica gel column with ethyl acetate/petroleum ether (1: 1). This resulted in 920 mg (79.62%) of methyl 2-oxo-3H-1,3-benzoxazole-5-carboxylate **(R-2)** as a white solid. **LCMS:** $[M+H]^+$=194.

2-oxo-3H-1,3-benzoxazole-5-carboxylic acid (Acid R)

Into a 12-mL vial, was placed a solution of methyl 2-oxo-3H-1,3-benzoxazole-5-carboxylate (200.00 mg, 1.035 mmol, 1.00 equiv) in MeOH (3 mL), a solution of sodium hydroxide (82.8 mg, 2.071 mmol, 2.00 equiv) in $H_2O$ (1 mL). The resulting solution was stirred for 10h at room temperature. The resulting mixture was concentrated under vacuum. 2M HCl(aq) was added to adjust the pH to 2. The solids were collected by filtration. This resulted in 150 mg (81%) of 2-oxo-3H-1,3-benzoxazole-5-carboxylic acid **(Acid R)** as a white solid. **LCMS:** $[M+H]^+$=180.

Preparation of Acid S

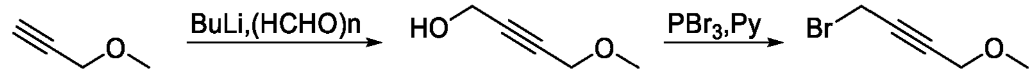


S-1 S-2 S-3

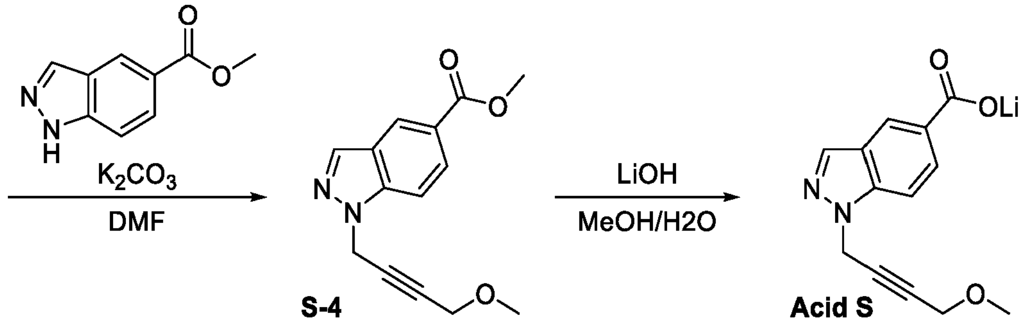


S-4 Acid S

4-methoxybut-2-yn-1-ol (S-2)

Into a 250-mL 3-necked round-bottom flask purged and maintained with an inert atmosphere of nitrogen, was placed 3-methoxy-propyne **(S-1,** 4.90 g, 69.909 mmol, 1.00 equiv). This was followed by the addition of THF (100.00 mL), in portions at -78 °C. To this was added n-BuLi (33.60 mL, 83.880 mmol, 1.2 equiv) dropwise with stirring at -78 °C in 20 min. The resulting solution was stirred for 1 hr at -78 °C. To the mixture was added Paraformaldehyde (10.08 g, 111.855 mmol, 1.60 equiv) at -78 °C. The resulting solution was stirred for 20 h at room temperature. The reaction was then quenched by the addition of 50 mL of $NH_4Cl$(aq). The resulting solution was extracted with 2x100 mL of ethyl acetate and the organic layers combined and concentrated. The residue was applied onto a silica gel column with ethyl acetate/petroleum ether (1:3). This resulted in 3.4 g (48.58%) of 4-methoxybut-2-yn-1-ol **(S-2)** as yellow oil. **GC-MS:** (ES, m/z): M =100.

1-bromo-4-methoxybut-2-yne (S-3)

Into a 100-mL 3-necked round-bottom flask purged and maintained with an inert atmosphere of nitrogen, was placed 4-methoxybut-2-yn-1-ol **(S-2,** 1.10 g, 10.987 mmol, 1.00 equiv), Et2O (20.00 mL, 192.791 mmol, 17.55 equiv), Pyridine (0.20 mL, 2.485 mmol, 0.23 equiv). This was followed by the addition of PBr3 (1.19 g, 4.395 mmol, 0.40 equiv) dropwise with stirring at 0 °C. The resulting solution was stirred for 1 h at 35 °C. The reaction was then quenched by the addition of 10 mL of NaCl(aq). The resulting solution was extracted with 2x30 mL of Et2O and the organic layers combined and concentrated. This resulted in 1.24 g (69.23%) of 1-bromo-4-methoxybut-2-yne **(S-3)** as light yellow oil. **GC-MS:** $[M+H]^+$=162.

Methyl 1-(4-methoxybut-2-yn-1-yl)indazole-5-carboxylate (S-4)

Into a 100-mL round-bottom flask purged and maintained with an inert atmosphere of nitrogen, was placed 1-bromo-4-methoxybut-2-yne **(S-3,** 1.20 g, 7.361 mmol, 1.34 equiv), methyl 1H-indazole-5-carboxylate (970.00 mg, 5.506 mmol, 1.00 equiv), KI (90.00 mg, 0.542 mmol, 0.10 equiv), $K_2CO_3$ (2.16 g, 15.629 mmol, 2.84 equiv), DMF (10.00 mL). The resulting solution was stirred for 8 h at 90 °C. The solids were filtered out. The resulting mixture was concentrated. The residue was applied onto a silica gel column with ethyl acetate/petroleum ether (1:1). This resulted in 418 mg (29.39%) of methyl 1-(4-methoxybut-2-yn-1-yl)indazole-5-carboxylate **(S-4)as** a solid. **LCMS:** $[M+H]^+$=259.

1-(4-methoxybut-2-yn-1-yl)indazole-5-carboxylate (Acid S)

Into a 50-mL round-bottom flask, was placed methyl 1-(4-methoxybut-2-yn-1-yl)indazole-5-carboxylate **(S-4,** 218.00 mg, 0.844 mmol, 1.00 equiv), LiOH (30.32 mg, 1.266 mmol, 1.50 equiv), MeOH (10.00 mL), $H_2O$ (2.00 mL). The resulting solution was stirred for 3 h at 50 °C. The resulting mixture was concentrated. This resulted in 207 mg (98.03%) of lithio 1-(4-methoxybut-2-yn-1-yl)indazole-5-carboxylate **(Acid S)** as a light yellow solid. **LCMS:** [M+2H-Li]=245.

Preparation of Acid T

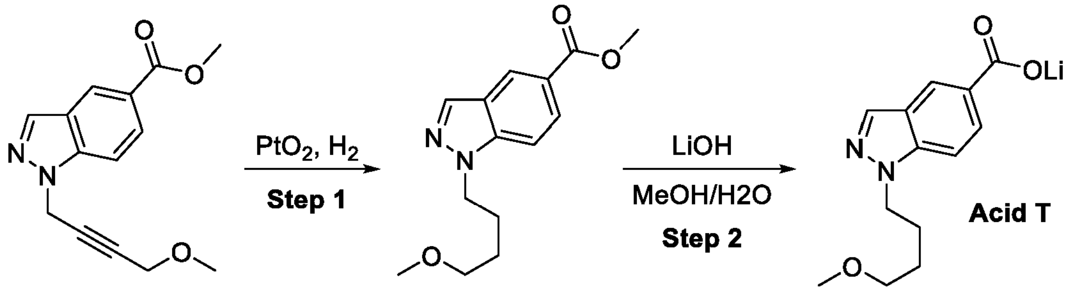


Step 1. Into a 50-mL round-bottom flask purged and maintained with an inert atmosphere of nitrogen, was placed methyl 1-(4-methoxybut-2-yn-1-yl)indazole-5-carboxylate **(Example** 27, 200.00 mg, 0.774 mmol, 1.00 equiv), MeOH (10.00 mL), $PtO_2$ (50.00 mg, 0.220 mmol, 0.28 equiv). The mixture was stirred 12h at 40 °C under an atmosphere of hydrogen (60psi). The catalyst was filtered and the resulting mixture was concentrated. This resulted in 157 mg (77.29%) of methyl 1-(4-methoxybutyl)indazole-5-carboxylate as a brown solid. **LCMS:** $[M+H]^+$=263.

Step 2. Into a 50-mL round-bottom flask, was placed methyl 1-(4-methoxybutyl)indazole-5-carboxylate (160.00 mg, 0.610 mmol, 1.00 equiv), LiOH (21.91 mg, 0.915 mmol, 1.50 equiv), MeOH (5.00 mL), $H_2O$ (1.00 mL). The resulting solution was stirred for 20 min at 50 °C. The resulting mixture was concentrated. This resulted in 150 mg (96.74%) of lithio 1-(4-methoxybutyl)indazole-5-carboxylate as a white solid. **LCMS:** [M+2H-Li]=249.

Preparation of Acid U

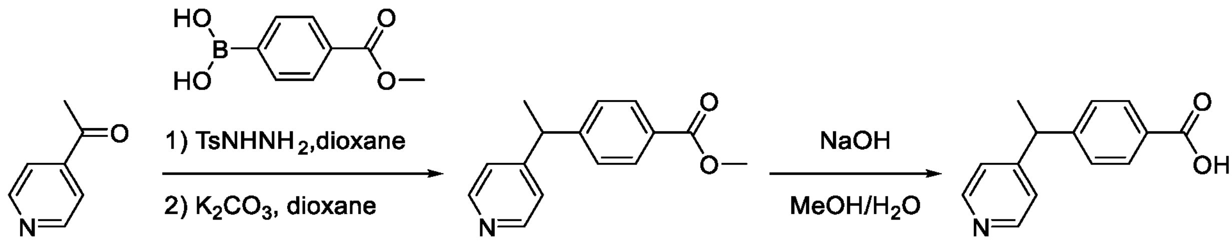


**U-1** **U-2** **Acid U**

Methyl 4-[1-(pyridin-4-yl)ethyl]benzoate (U-2)

Into a 100 mL round-bottom flask were added 4-acetylpyridine (U-1, 611.0 mg, 5.044 mmol, 1.00 equiv), dioxane (40.0 mL) and 4-toluenesulfonyl hydrazide (1409 mg, 7.566 mmol, 1.50 equiv) at room temperature. The resulting mixture was stirred for 3 h at 80 °C under nitrogen atmosphere. To the above mixture was added 4-(methoxycarbonyl) phenylboronic acid (1007.6 mg, 5.6 mmol, 1.11 equiv) and $K_2CO_3$ (2091.2 mg, 15.131 mmol, 3.00 equiv) at 80 °C. The resulting mixture was stirred for additional 4 h at 110 °C. The mixture was allowed to cool down to room temperature. The resulting mixture was diluted with EtOAc (60 mL). Then 60 mL of water was added. The mixture was separated and the aqueous phase was extracted with EtOAc (3x30 mL). The combined organic layers were washed with brine (1x50 mL), dried over anhydrous $Na_2SO_4$. After filtration, the filtrate was concentrated under reduced pressure. The residue was purified by silica gel column chromatography, eluted with PE/EtOAc (3:2) to afford methyl 4-[1-(pyridin-4-yl)ethyl]benzoate **(U-2,** 270 mg, 22.19%) as a yellow oil. **LCMS:** $[M+1]^+$=242.

4-[1-(pyridin-4-yl)ethyl]benzoic acid (Acid U)

Into a 8 mL vial were added methyl 4-[1-(pyridin-4-yl)ethyl]benzoate **(U-2,** 250.00 mg, 1.036 mmol, 1.00 equiv), MeOH (4 mL) and NaOH (124.32 mg, 3.108 mmol, 3.00 equiv) in $H_2O$ (2 mL) at room temperature. The resulting mixture was stirred for 4 h at room temperature. The resulting mixture was concentrated under reduced pressure. The residue was dissolved in water (20 mL). The aqueous layer was extracted with EtOAc (3x10 mL). The aqueous phase was collected, and acidified to pH 5 with HCl (aq. 1 M). The precipitated solids were collected by filtration and washed with water. The resulting solid was dried under infrared light. This resulted in 4-[1-(pyridin-4-yl)ethyl]benzoic acid **(Acid U,** 190 mg, 80.69%) as a yellow solid. **LCMS:** $[M+1]^+$=228.

Preparation of Acid V

Prepared in the same fashion as Acid U, except that **U-1** is replaced with 3-acetylpyridine (1.00 g, 8.255 mmol, 1.00 equiv).

Preparation of Acid W

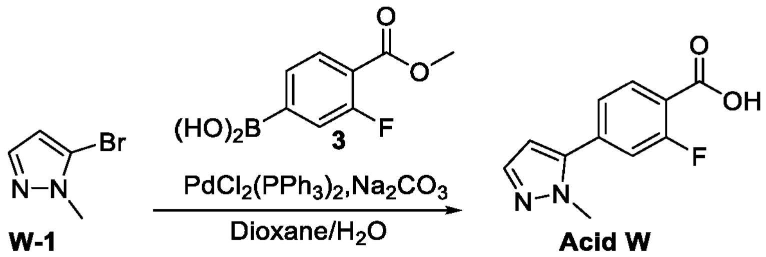


**W-1** **Acid W**

Into a 40-mL vial purged and maintained with an inert atmosphere of nitrogen, was placed 5-bromo-1-methylpyrazole (500.18 mg, 3.107 mmol, 1.00 equiv), dioxane (5.00 mL), $H_2O$ (5.00 mL), 3-fluoro-4-(methoxycarbonyl)phenylboronic acid (615.00 mg, 3.107 mmol, 1.00 equiv), $Na_2CO_3$ (987.81 mg, 9.320 mmol, 3.00 equiv), $Pd(PPh_3)_2Cl_2$ (218.06 mg, 0.311 mmol, 0.10 equiv). The resulting solution was stirred for 16 hr at 80 °C. The resulting mixture was concentrated under vacuum. The resulting solution was diluted with 50 mL of $H_2O$. The resulting solution was extracted with 2x30 mL of ethyl acetate and the aqueous layers combined. The pH value of the solution was adjusted to HCl with 3 (3 mol/L). The solids were collected by filtration. This resulted in 600 mg (87.71%) of 2-fluoro-4-(2-methylpyrazol-3-yl) benzoic acid as a brown solid. **LCMS:** $[M+H]^+$=221.

The following acids in **Table B** were prepared in the same fashion as Acid W, but with the indicated **W-1.**

Table B

| Acid | Structure | W-1 |
|---|---|---|
| **X** | 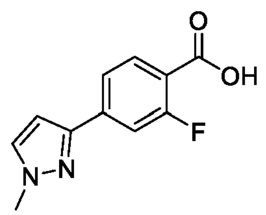 | 3-bromo-1-methylpyrazole |
| **Y** | 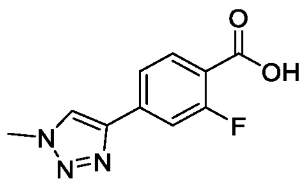 | 4-bromo-1-methyl-1,2,3-triazole |
| **Z** | 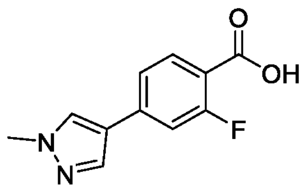 | 4-bromo-1-methylpyrazole |
| **AA** | 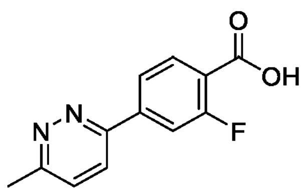 | 3-bromo-6-methylpyridazine |

(continued)

| Acid | Structure | W-1 |
|---|---|---|
| AB | 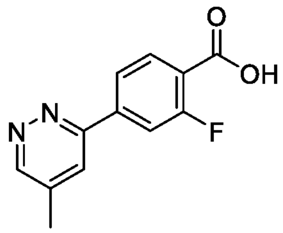 | 3-chloro-5-methylpyridazine |

Preparation of Acid AC

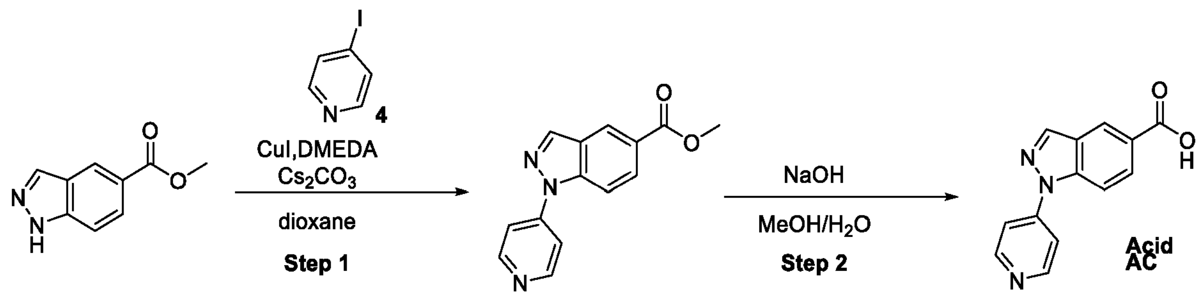


Step 1. Into a 250-mL 3-necked round-bottom flask purged and maintained with an inert atmosphere of nitrogen, was placed methyl 1H-indazole-5-carboxylate (2.0 g, 11.35 mmol, 1.00 equiv), dioxane (40.00 mL), 4-iodopyridine (2.33 g, 11.366 mmol, 1.00 equiv), CuI (2.16 g, 11.342 mmol, 1.00 equiv), DMEDA (0.20 g, 2.269 mmol, 0.20 equiv), $Cs_2CO_3$ (11.10 g, 34.068 mmol, 3.00 equiv). The resulting solution was stirred for 2 days at 100 °C in an oil bath. The resulting mixture was concentrated under vacuum. The resulting solution was diluted with 40 mL of $H_2O$. The resulting solution was extracted with 3x20 mL of ethyl acetate and the organic layers combined. The resulting mixture was washed with 2 x30 mL of Brine. The mixture was dried over anhydrous sodium sulfate and concentrated under vacuum. This resulted in 1.6 g (36.95%) of methyl 1-(pyridin-4-yl)indazole-5-carboxylate as an off-white solid. **LCMS:** $[M+H]^+$=254.

Step 2. Into a 100-mL vial, was placed methyl 1-(pyridin-4-yl)indazole-5-carboxylate (1.60 g, 6.318 mmol, 1.00 equiv), $CH_3OH$ (32.00 mL), $H_2O$ (10.00 mL), Sodium hydroxide (1.26 g, 31.590 mmol, 5.00 equiv). The resulting solution was stirred for 16 hr at room temperature. The resulting mixture was concentrated under vacuum. The resulting solution was diluted with 30 mL of $H_2O$. The resulting solution was extracted with 2x30 mL of ethyl acetate and the aqueous layers combined. The pH value of the solution was adjusted to 3 with HCl (3 mol/L). The solids were collected by filtration. This resulted in 350 mg (23.16%) of 1-(pyridin-4-yl)indazole-5-carboxylic acid as an off-white solid. **LCMS:** $[M+H]^+$=240.

Preparation of Acid AE

Into a 30-mL sealed tube, was placed 6-bromocinnoline (600.00 mg, 2.870 mmol, 1.00 equiv), MeOH (12.00 L), Pd(dppf)$Cl_2$ (210.01 mg, 0.287 mmol, 0.10 equiv), TEA (871.30 mg, 8.611 mmol, 3.00 equiv), carbon monoxide (10 atm). The resulting solution was stirred for overnight at 60 degrees C in an oil bath. The resulting mixture was concentrated under vacuum. The resulting solution was diluted with 30 mL of $H_2O$. The resulting solution was extracted with 3x20 mL of ethyl acetate and the organic layers combined and dried over anhydrous sodium sulfate and concentrated under vacuum. This resulted in 600 mg (95.34%) of methyl cinnoline-6-carboxylate as a red solid. **LCMS (PH-PUK):** $[M+H]^+$=189.

Preparation of Acid AF

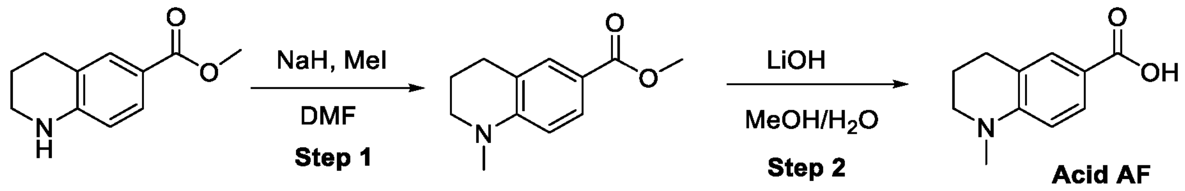


Step 1. Into a 100-mL 3-necked round-bottom flask purged and maintained with an inert atmosphere of nitrogen, was placed methyl 1,2,3,4-tetrahydroquinoline-6-carboxylate (350.0 mg, 1.830 mmol, 1.00 equiv), DMF (15.00 mL). This was followed by the addition of NaH (109.80 mg, 2.745 mmol, 1.50 equiv, 60%), in portions at 0 °C. To this was added $CH_3I$ (285.76 mg, 2.013 mmol, 1.10 equiv) dropwise with stirring at 0 °C. The resulting solution was stirred for 1 h at 0 °C to room temperature. The reaction was then quenched by the addition of 10 mL of water/ice. The resulting solution was extracted with 3x20 mL of ethyl acetate and the organic layers combined and concentrated. The residue was applied onto a silica gel column with ethyl acetate/petroleum ether (1: 1). This resulted in 260 mg (69.21%) of methyl 1-methyl-3,4-dihydro-2H-quinoline-6-carboxylate as a yellow solid. **LCMS:** $[M+H]^+$=206.

Step 2. Into a 50-mL round-bottom flask, was placed methyl 1-methyl-3,4-dihydro-2H-quinoline-6-carboxylate (260.0 mg, 1.27 mmol, 1.00 equiv), LiOH (60.7 mg, 2.53 mmol, 2.0 equiv), MeOH (10.0 mL), $H_2O$ (5.0 mL). The resulting solution was stirred for 12 h at 50 °C. The resulting mixture was concentrated. The reaction was then quenched by the addition of 20 mL of water. The pH value of the solution was adjusted to 4 with citric. acid. The solids were collected by filtration. This resulted in 207 mg (85.5%) of 1-methyl-3,4-dihydro-2H-quinoline-6-carboxylic acid as a light yellow solid. **LCMS:** $[M+H]^+$=192.

Preparation of Acid AG

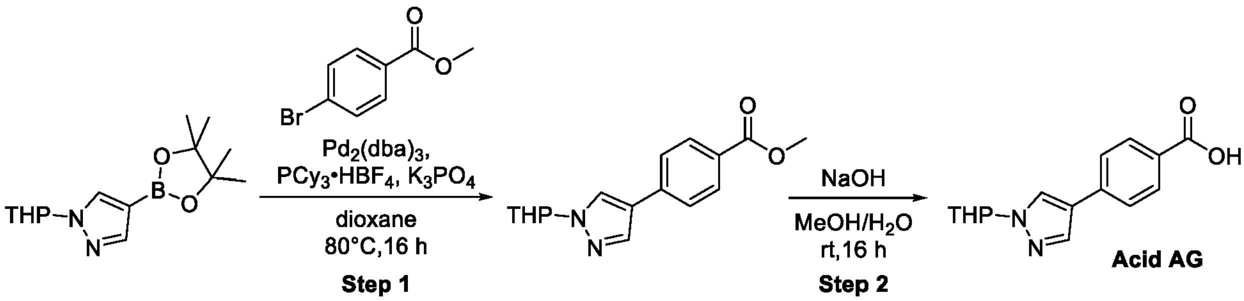


Step 1. Into a 250-mL 4-necked round-bottom flask purged and maintained with an inert atmosphere of nitrogen, was placed 1-(oxan-2-yl)-4-(4,4,5,5-tetramethyl-1,3,2-dioxaborolan-2-yl)pyrazole (10.0 g, 35.95 mmol, 1.00 equiv), dioxane (100.0 mL), $Pd_2(dba)_3$ (1646.03 mg, 1.8 mmol, 0.05 equiv), (phosphoperoxy)potassium; dipotassium (22893.12 mg, 107.852 mmol, 3.00 equiv), $PCy_3.HBF_4$ (661.92 mg, 1.798 mmol, 0.05 equiv), methyl 4-bromobenzoate (11596.53 mg, 53.926 mmol, 1.50 equiv). The resulting solution was stirred for 16 hr at 80 °C. The solids were filtered out. The resulting solution was diluted with 100 mL of $H_2O$. The resulting solution was extracted with 3x100 mL of ethyl acetate and the organic layers combined. This resulted in 6 g (58.29%) of methyl 4-[1-(oxan-2-yl)pyrazol-4-yl]benzoate as yellow oil. **LCMS:** $[M+1]^+$=287.

Step 2. Into a 50-mL 4-necked round-bottom flask purged and maintained with an inert atmosphere of nitrogen, was placed methyl 4-[1-(oxan-2-yl)pyrazol-4-yl]benzoate (400.00 mg, 1.397 mmol, 1.00 equiv), $H_2O$ (4.00 mL), MeOH (4.00 mL), sodium hydroxide (223.50 mg, 5.588 mmol, 4.00 equiv). The resulting solution was stirred for 16 hr at room temperature. The resulting mixture was concentrated. The pH value of the solution was adjusted to 2-3 with HCL (1 mol/L). The solids were collected by filtration. This resulted in 300 mg (78.9%) of 4-[1-(oxan-2-yl)pyrazol-4-yl]benzoic acid as a white solid. **LCMS:** $[M+1]^+$=273.

Preparation of Acid AH

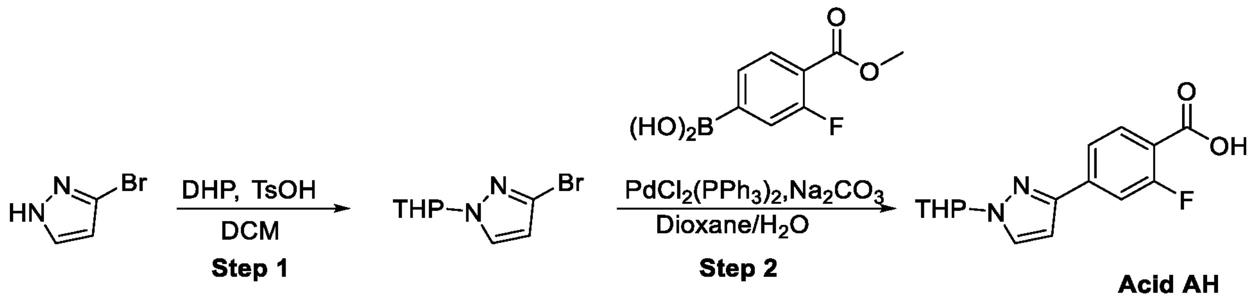


Acid AH (protected)

Step 1. Into a 100-mL round-bottom flask, was placed 3-bromo-1H-pyrazole (1.50 g, 10.206 mmol, 1.00 equiv), DCM (30.00 mL), dihydropyran (1.29 g, 15.336 mmol, 1.50 equiv), TsOH (0.05 g, 0.510 mmol, 0.05 equiv). The resulting solution was stirred for overnight at 80 °C in an oil bath. The resulting solution was diluted with 50 mL of $NaHCO_3$ (5%). The resulting solution was extracted with 3x30 mL of dichloromethane and the organic layers combined and dried over anhydrous sodium sulfate and concentrated under vacuum. This resulted in 2 g (84.8%) of 3-bromo-1-(oxan-2-yl)pyrazole as brown oil. **LCMS:** $[M+H]^+$=231.

Step 2. Into a 40-mL vial purged and maintained with an inert atmosphere of nitrogen, was placed 3-fluoro-4-(methoxycarbonyl)phenylboronic acid (0.86 g, 4.327 mmol, 1.00 equiv), dioxane (12.00 mL), $H_2O$(12.00 mL), 3-bromo-1-(oxan-2-yl)pyrazole (1.00 g, 4.327 mmol, 1.00 equiv), $Na_2CO_3$ (1.38 g, 13.02 mmol, 3.01 equiv), $Pd(PPh_3)_2Cl_2$ (0.30 g, 0.427 mmol, 0.10 equiv), BINAP (0.54 g, 0.867 mmol, 0.20 equiv). The resulting solution was stirred for 16 hr at 80 °C in an oil bath. The resulting mixture was concentrated under vacuum. The resulting solution was diluted with 30 mL of $H_2O$. The resulting solution was extracted with 2x20 mL of ethyl acetate and the aqueous layers combined. The pH value of the solution was adjusted to 3 with HCl (3 mol/L). The solids were collected by filtration. This resulted in 1 g (62.38%) of 2-fluoro-4-[1-(oxan-2-yl)pyrazol-3-yl]benzoic acid as a white solid. **LCMS:** $[M+H]^+$=291.

Preparation of Acid Al

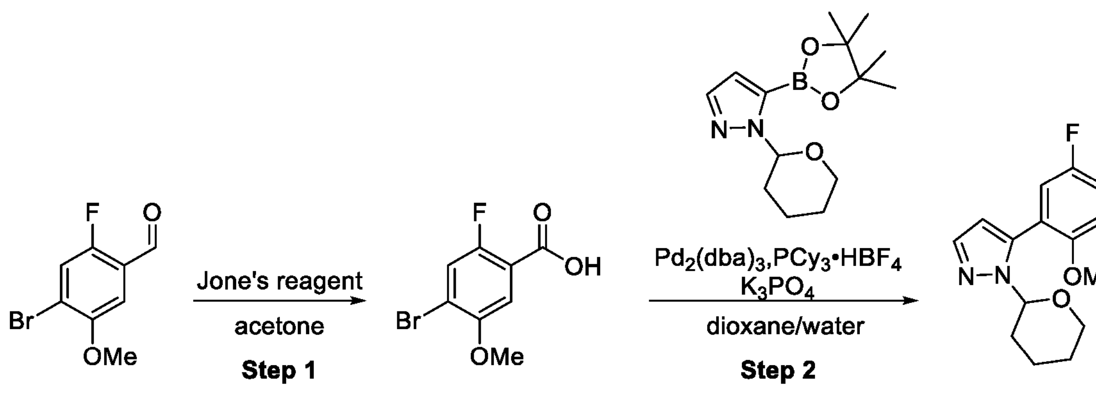


Acid Al (protected)

Step 1. Into a 100-mL round-bottom flask purged and maintained with an inert atmosphere of nitrogen, was placed 4-bromo-2-fluoro-5-methoxybenzaldehyde (1.00 g, 4.29 mmol, 1.00 equiv), acetone (43.00 mL), Jones reagent (10.3 mL). The resulting solution was stirred for 4 hr at 0 °C in an ice/salt bath. The resulting solution was diluted with 40 mL of $H_2O$. The resulting solution was extracted with 2x30 mL of ethyl acetate and the organic layers combined and dried over anhydrous sodium sulfate and concentrated under vacuum. This resulted in 0.7 g (65.5%) of 4-bromo-2-fluoro-5-methoxybenzoic acid as a brown solid. **LCMS:** $[M-H]^+$=247.

Step 2. Into a 40-mL vial purged and maintained with an inert atmosphere of nitrogen, was placed 4-bromo-2-fluoro-5-methoxybenzoic acid (0.70 g, 2.811 mmol, 1.00 equiv), dioxane (20.0 mL), 1-(oxan-2-yl)-5-(4,4,5,5-tetramethyl-1,3,2-dioxaborolan-2-yl)pyrazole (0.78 g, 2.804 mmol, 1.00 equiv), $Pd_2(dba)_3$ (0.13 g, 0.141 mmol, 0.05 equiv), $PCy_3 \cdot HBF_4$ (0.08 g, 0.217 mmol, 0.08 equiv), $K_3PO_4$ (1.79 g, 8.433 mmol, 3.00 equiv). The resulting solution was stirred for 16 hr at 80 °C in an oil bath. The resulting mixture was concentrated under vacuum. The resulting solution was diluted with 30 mL of $H_2O$. The resulting solution was extracted with 2 x 20 mL of ethyl acetate and the aqueous layers combined. The pH value of the solution was adjusted to 3 with HCl (3 mol/L). The solids were collected by filtration. This resulted in 600 mg (66.64%) of 2-fluoro-5-methoxy-4-[2-(oxan-2-yl)pyrazol-3-yl]benzoic acid as a brown solid. **LCMS:** $[M+H]^+$=321.

Preparation of Acid AJ

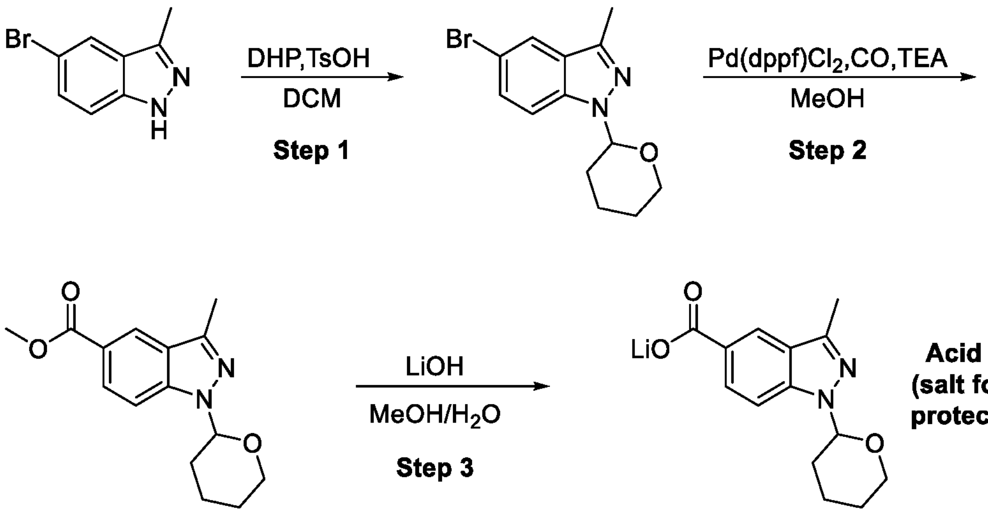


**Acid AJ (salt form, protected)**

Step 1. Into a 100-mL 3-necked round-bottom flask, was placed 5-bromo-3-methyl-1H-indazole (2.00 g, 9.48 mmol, 1.00 equiv), DHP (1.20 g, 14.214 mmol, 1.50 equiv), DCM (20.00 mL), TsOH (163.18 mg, 0.948 mmol, 0.10 equiv). The resulting solution was stirred for 5 h at room temperature. The reaction was then quenched by the addition of 50 mL of water. The resulting solution was extracted with 2x50 mL of ethyl acetate and the organic layers combined and concentrated. The residue was applied onto a silica gel column with ethyl acetate/petroleum ether (1:3). This resulted in 1.6 g (57.20%) of 5-bromo-3-methyl-1-(oxan-2-yl)indazole as a white solid. **LCMS:** $[M+H]^+$=295.

Step 2. Into a 100-mL pressure tank reactor, was placed 5-bromo-3-methyl-1-(oxan-2-yl)indazole (1.60 g, 5.420 mmol, 1.00 equiv), TEA (1.65 g, 16.260 mmol, 3.00 equiv), Pd(dppf)$Cl_2$ (793.22 mg, 1.084 mmol, 0.20 equiv), MeOH (20.00 mL). The flask was evacuated and flushed three times with nitrogen, followed by flushing with CO(gas). The mixture was stirred 6h at 60 °C under an atmosphere of CO(0.3MPa).The resulting mixture was concentrated. The residue was applied onto a silica gel column with ethyl acetate/petroleum ether (1:2). This resulted in 1.25 g (84.07%) of methyl 3-methyl-1-(oxan-2-yl)indazole-5-carboxylate as a white solid. **LCMS:** $[M+H]^+$=275.

Step 3. Into a 50-mL round-bottom flask, was placed methyl 3-methyl-1-(oxan-2-yl)indazole-5-carboxylate (600.0 mg, 2.187 mmol, 1.00 equiv), $H_2O$ (2.0 mL), MeOH (10.0 mL), LiOH (157.1mg, 6.56 mmol, 3.00 equiv). The resulting solution was stirred for 12 h at 40 °C. The resulting mixture was concentrated. This resulted in 510 mg (87.58%) of lithio 3-methyl-1-(oxan-2-yl)indazole-5-carboxylate as a white solid. LCMS: [M+2H-Li] =275.

Preparation of Acid AK

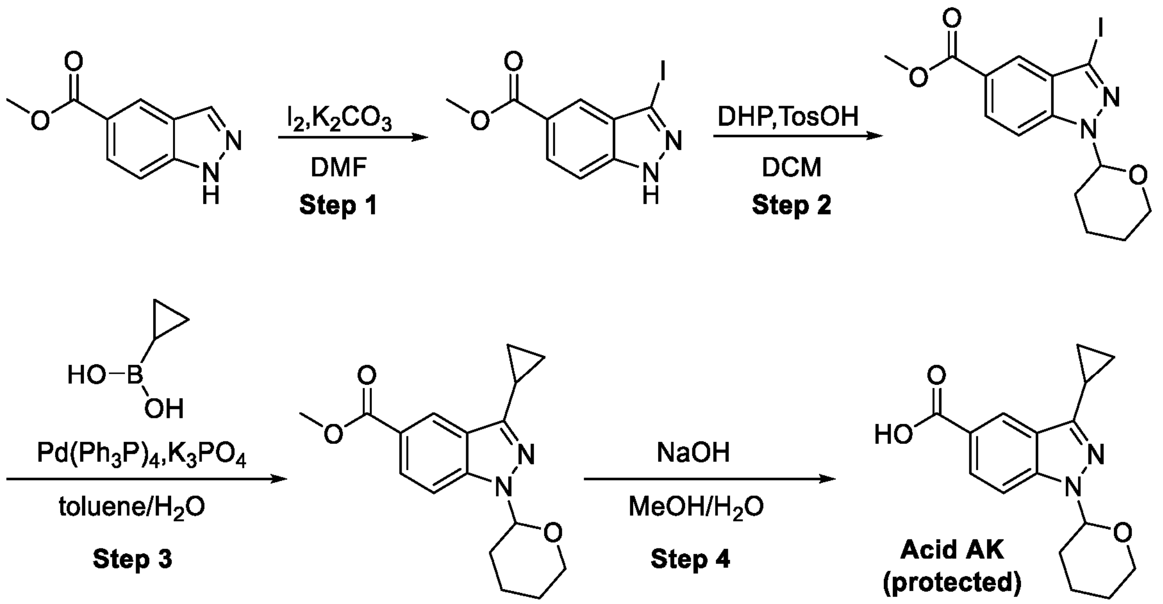


Into a 100-mL 3-necked round-bottom flask purged and maintained with an inert atmosphere of nitrogen, was placed methyl 1H-indazole-5-carboxylate (2 g, 11.352 mmol, 1.00 equiv), $K_2CO_3$ (4.71 g, 34.056 mmol, 3.00 equiv), DMF (60.00 mL). This was followed by the addition of $I_2$ (8.64 g, 34.056 mmol, 3.00 equiv), in portions at 0 °C. The resulting solution was stirred for 16 h at room temperature. The reaction was then quenched by the addition of 100 mL of $NaHSO_3$ (aq). The solids were collected by filtration. This resulted in 2.9 g (84.57%) of methyl 3-iodo-1H-indazole-5-carboxylate as a yellow solid. **LCMS:** $[M+H]^+$=387.

Step 2. Into a 250-mL 3-necked round-bottom flask, was placed methyl 3-iodo-1H-indazole-5-carboxylate (2.40 g, 7.945 mmol, 1.00 equiv), DHP (1.00 g, 11.918 mmol, 1.50 equiv), TsOH (273.63 mg, 1.589 mmol, 0.20 equiv), DCM (50.00 mL). The resulting solution was stirred for 5 h at room temperature. The reaction was then quenched by the addition of 50 mL of water. The resulting solution was extracted with 2 x 50 mL of ethyl acetate and the organic layers combined and concentrated. This resulted in 2.4 g (78.22%) of methyl 3-iodo-1-(oxan-2-yl)indazole-5-carboxylate as a solid. **LCMS:** $[M+H]^+$=387.

Step 3. Into a 100-mL 3-necked round-bottom flask purged and maintained with an inert atmosphere of nitrogen, was placed methyl 3-iodo-1-(oxan-2-yl)indazole-5-carboxylate (1.40 g, 3.625 mmol, 1.00 equiv), cyclopropylboronic acid (0.62 g, 7.250 mmol, 2.00 equiv), $Pd(PPh_3)_4$ (418.91 mg, 0.363 mmol, 0.10 equiv), $K_3PO_4$ (3.08 g, 14.500 mmol, 4.00 equiv), Toluene (36.00 mL), $H_2O$ (2.40 mL). The resulting solution was stirred for 16 h at 110 °C. The solids were filtered out. The resulting mixture was concentrated. The residue was applied onto a silica gel column with ethyl acetate/petroleum ether (1:2). This resulted in 910 mg (83.57%) of methyl 3-cyclopropyl-1-(oxan-2-yl)indazole-5-carboxylate as a light brown solid. **LCMS** $[M+H]^+$=301.

Step 4. Into a 50-mL round-bottom flask, was placed methyl 3-cyclopropyl-1-(oxan-2-yl)indazole-5-carboxylate (510.00 mg, 1.698 mmol, 1.00 equiv), NaOH (135.83 mg, 3.396 mmol, 2.00 equiv), MeOH (10.00 mL), $H_2O$ (2.00 mL). The resulting solution was stirred for 12 h at room temperature. The resulting mixture was concentrated. The pH value of the solution was adjusted to 4 with citric acid (aq). The solids were collected by filtration. This resulted in 400 mg (82.27%) of 3-cyclopropyl-1-(oxan-2-yl)indazole-5-carboxylic acid as a white solid. **LCMS:** [M+H]=287.

Preparation of Acid AL

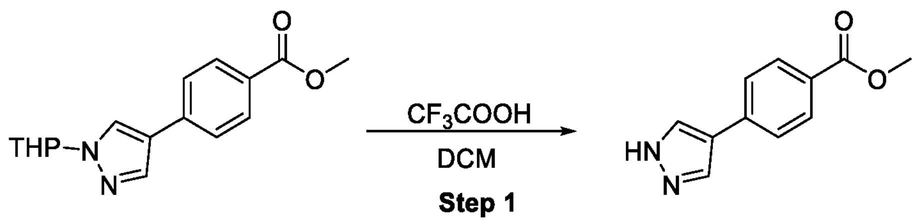


Step 1. Into a 250-mL 4-necked round-bottom flask purged and maintained with an inert atmosphere of nitrogen,

was placed methyl 4-[1-(oxan-2-yl)pyrazol-4-yl]benzoate (6.00 g, 20.955 mmol, 1.00 equiv), DCM (60.00 mL), trifluoroacetic acid (8216.28 mg, 83.819 mmol, 4.00 equiv). The resulting solution was stirred for 16 hr at room temperature. The resulting mixture was concentrated. This resulted in 4 g (94.40%) of methyl 4-(1H-pyrazol-4-yl)benzoate as a white solid. **LCMS:** $[M+1]^+$=203.

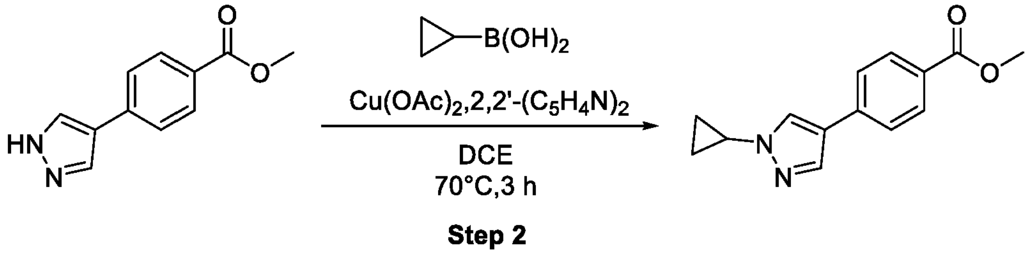


Step 2. Into a 40-mL round-bottom flask, was placed methyl 4-(1H-pyrazol-4-yl)benzoate (400.00 mg, 1.978 mmol, 1.00 equiv), DCE (10.00 mL), 2,2 (370.30 mg, 2.374 mmol, 1.20 equiv), $Cu(AcO)_2$ (431.15 mg, 2.374 mmol, 1.20 equiv), cyclopropylboronic acid (424.80 mg, 4.945 mmol, 2.50 equiv). The resulting solution was stirred for 3 hr at 70 °C. The resulting solution was diluted with 20 mL of $H_2O$. The resulting solution was extracted with 3x20 mL of dichloromethane and the organic layers combined and concentrated. This resulted in 300 mg (62.60%) of methyl 4-(1-cyclopropylpyrazol-4-yl)benzoate as yellow oil. **LCMS:** $[M+1]^+$=243.

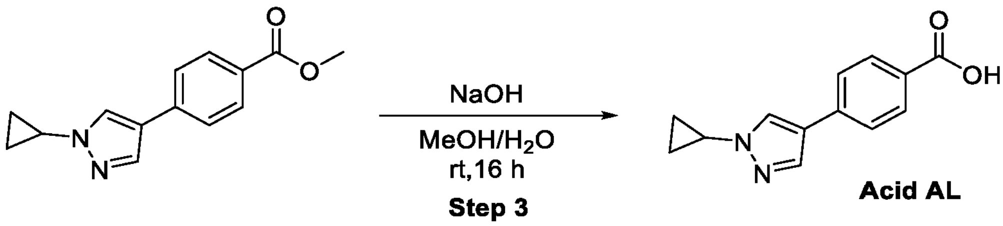


**Acid AL**

Step 3. Into a 50-mL 4-necked round-bottom flask purged and maintained with an inert atmosphere of nitrogen, was placed methyl 4-(1-cyclopropylpyrazol-4-yl)benzoate (300.00 mg, 1.238 mmol, 1.00 equiv), $H_2O$ (3.00 mL), MeOH (3.00 mL), sodium hydroxide (198.10 mg, 4.953 mmol, 4.00 equiv). The resulting solution was stirred for 16 hr at room temperature. The resulting mixture was concentrated. The pH value of the solution was adjusted to 2-3 with HCL (1M). The solids were collected by filtration. This resulted in 150 mg (53.07%) of 4-(1-cyclopropylpyrazol-4-yl)benzoic acid as a white solid. **LCMS:** $[M+1]^+$=229.

Preparation of Acid AM

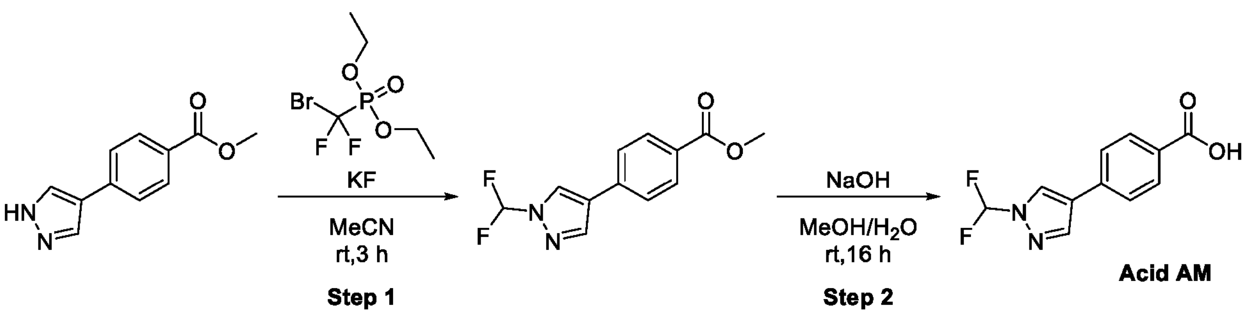


**Acid AM**

Step 1. Into a 20-mL round-bottom flask, was placed methyl 4-(1H-pyrazol-4-yl)benzoate (400.00 mg, 1.978 mmol, 1.00 equiv), $CH_3CN$ (4.00 mL), potassium fluoride (229.84 mg, 3.956 mmol, 2.00 equiv), diethyl bromodifluoromethylphosphonate (792.25 mg, 2.967 mmol, 1.50 equiv). The resulting solution was stirred for 3 hr at room temperature. The resulting solution was diluted with 10 mL of $H_2O$. This resulted in 400 mg (80.17%) of methyl 4-[1-(difluoromethyl)pyrazol-4-yl]benzoate as yellow oil. **LCMS:** $[M+1]^+$=253.

Step 2. Into a 40-mL round-bottom flask, was placed methyl 4-[1-(difluoromethyl)pyrazol-4-yl]benzoate (400.00 mg, 1.586 mmol, 1.00 equiv), $H_2O$ (4.00 mL), MeOH (4.00 mL), sodium hydroxide (253.73 mg, 6.344 mmol, 4.00 equiv).

The resulting solution was stirred for 16 hr at room temperature. The resulting mixture was concentrated. The pH value of the solution was adjusted to 2-3 with HCL (1M). The solids were collected by filtration. This resulted in 260 mg (68.83%) of 4-[1-(difluoromethyl)pyrazol-4-yl]benzoic acid as a white solid. **LCMS:** $[M+1]^+$=239.

Preparation of Acid AN

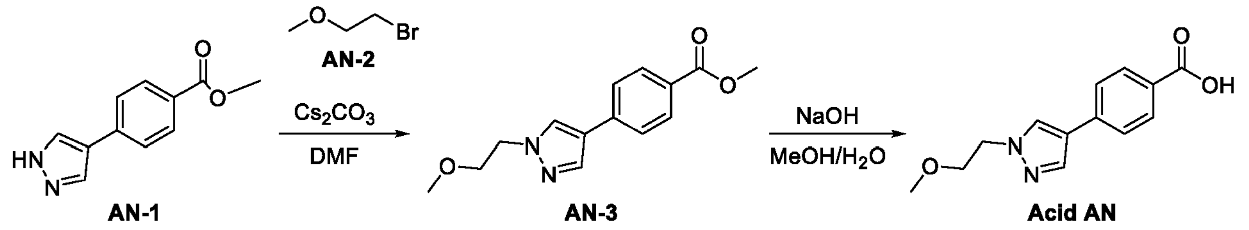

AN-1 AN-3 Acid AN

Methyl 4-[1-(2-methoxyethyl)pyrazol-4-yl]benzoate (AN-3)

Into a 40-mL round-bottom flask, was placed methyl 4-(1H-pyrazol-4-yl)benzoate (AN-1, 400.00 mg, 1.978 mmol, 1.00 equiv), cesium carbonate (1939.50 mg, 5.934 mmol, 3.0 equiv), DMF (10.00 mL), 2-bromoethyl methyl ether (**AN-2**, 412.41 mg, 2.967 mmol, 1.50 equiv). The resulting solution was stirred for 12 hr at room temperature. The resulting solution was diluted with 10 mL of $H_2O$. The resulting solution was extracted with 3x10 mL of ethyl acetate and the organic layers combined. The resulting mixture was washed with 3 x10 ml of brine. The resulting mixture was concentrated. This resulted in 400 mg (77.69%) of methyl 4-[1-(2-methoxyethyl)pyrazol-4-yl]benzoate (**AN-3**) as yellow oil. **LCMS:** $[M+1]^+$=261.

4-[1-(2-methoxyethyl)pyrazol-4-yl]benzoic acid (Acid AN)

Into a 40-mL round-bottom flask, was placed methyl 4-[1-(2-methoxyethyl)pyrazol-4-yl]benzoate (400.00 mg, 1.537 mmol, 1.00 equiv), MeOH (4.00 mL), $H_2O$ (4.00 mL), sodium hydroxide (245.86 mg, 6.147 mmol, 4.00 equiv). The resulting solution was stirred for 16 hr at room temperature. The resulting mixture was concentrated. The pH value of the solution was adjusted to 2-3 with HCL (1M). The solids were collected by filtration. This resulted in 260 mg (68.70%) of 4-[1-(2-methoxyethyl)pyrazol-4-yl]benzoic acid (Acid AN) as a white solid. **LCMS:** $[M+1]^+$=247.

Preparation of Acid AO

Prepared in the same fashion as Acid AN, except that **AN-2** is replaced with bromoacetonitrile.

Preparation of Acid AP

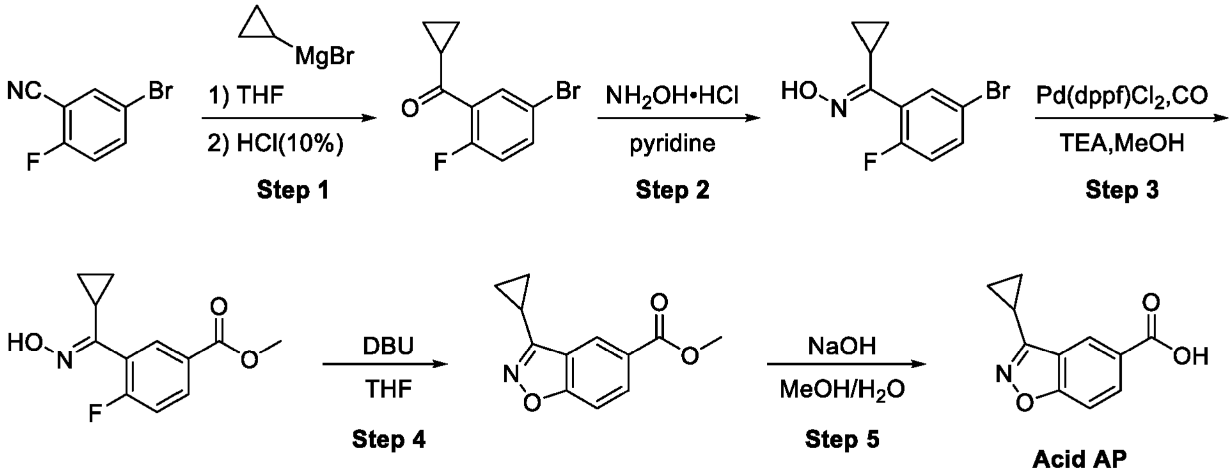

Acid AP

Step 1. Into a 500-mL round-bottom flask purged and maintained with an inert atmosphere of nitrogen, was placed 5-bromo-2-fluorobenzonitrile (5.5 g, 27.499 mmol, 1.00 equiv), tetrahydrofuran (110.00 mL). Bromo(cyclopropyl) magnesium(1M in THF) (68.75 mL, 68.748 mmol, 2.50 equiv) was added and the resulting solution was stirred for 2 h at -78 °C. The resulting solution was allowed to react, with stirring, for an additional 30 min at 25 °C.The reaction was then quenched by the addition of 100 mL of HCl (10%) and was stired for an additional 6 h. The resulting solution was extracted with 3 x 50 mL of ethyl acetate and the aqueous layers combined. The resulting mixture was washed with 3 x 50 mL of brine. The resulting mixture was concentrated. The residue was applied onto a silica gel column with ethyl acetate/hexane (1:10). This resulted in 3.3 g (49.37%) of (5-bromo-2-fluorophenyl)(cyclopropyl)methanone as a off-white solid. **LCMS:** $[M+1]^+$=243.

Step 2. Into a 50-mL round-bottom flask purged and maintained with an inert atmosphere of nitrogen, was placed (5-bromo-2-fluorophenyl)(cyclopropyl)methanone (1.50 g, 6.171 mmol, 1.00 equiv), $NH_2OH$.HCl (3001.77 mg, 0.042 mmol, 7.00 equiv), Pyridine (15.00 mL, 186.353 mmol, 30.20 equiv). The resulting solution was stirred for 3 h at 115 °C. The pH value of the solution was adjusted to 3 with HCl (1 mol/L). The resulting solution was extracted with 3x50 mL of ethyl acetate concentrated. This resulted in 1.25 g (78.49%) of (E)-N-[(5-bromo-2-fluorophenyl)(cyclopropyl)methylidene] hydroxylamine as a off-white solid. **LCMS:** $[M+1]^+$=258.

Step 3. Into a 50-mL pressure tank reactor, was placed (E)-N-[(5-bromo-2-fluorophenyl)(cyclopropyl)methylidene]hydroxylamine (1.25 g, 4.843 mmol, 1.00 equiv), TEA (1470.27 mg, 14.529 mmol, 3.00 equiv), Pd(dppf)$Cl_2$ (354.38 mg, 0.484 mmol, 0.10 equiv), MeOH (20.00 mL), CO (20 atm). The resulting solution was stirred for 12 h at 80 °C. The resulting mixture was concentrated. The residue was applied onto a silica gel column with ethyl acetate/hexane (1:2).This resulted in 850 mg (73.98%) of methyl 3-[(1E)-cyclopropyl(hydroxyimino)methyl]-4-fluorobenzoate as a brown solid. **LCMS:** $[M+1]^+$=238.

Step 4. Into a 20-mL vial purged and maintained with an inert atmosphere of nitrogen, was placed methyl 3-[(1E)-cyclopropyl(hydroxyimino)methyl]-4-fluorobenzoate (450.00 mg, 1.897 mmol, 1.00 equiv), tetrahydrofuran (8 mL), 1,8-Diazabicyclo[5.4.0]undec-7-ene (1433.25 mg, 5.691 mmol, 3.00 equiv). The resulting solution was stirred for 12 h at 75 °C.The reaction was then quenched by the addition of 20 mL of aq of citric acid (5%) .The resulting solution was extracted with 3 x 10 mL of ethyl acetate and the organic layers combined and concentrated. The residue was applied onto a silica gel column with ethyl acetate/hexane (1:2).This resulted in 140 mg (33.98%) of methyl 3-cyclopropyl-1,2-benzoxazole-5-carboxylate as a off-white solid. **LCMS:** $[M+1]^+$=218.

Step 5. Into a 20-mL vial purged and maintained with an inert atmosphere of nitrogen, was placed methyl 3-cyclopropyl-1,2-benzoxazole-5-carboxylate (140.00 mg, 0.644 mmol, 1.00 equiv), $H_2O$ (1.00 mL), methanol (4.00 mL), sodium hydroxide (51.56 mg, 1.289 mmol, 2 equiv). The resulting solution was stirred for 2 h at 25 °C. The pH value of the solution was adjusted to 3 with HCl (37 %). The resulting mixture was concentrated. This resulted in 220 mg (crude) of 3-cyclopropyl-1,2-benzoxazole-5-carboxylic acid as a off-white solid. **LCMS:** $[M+1]^+$=204.

Preparation of Acid AR

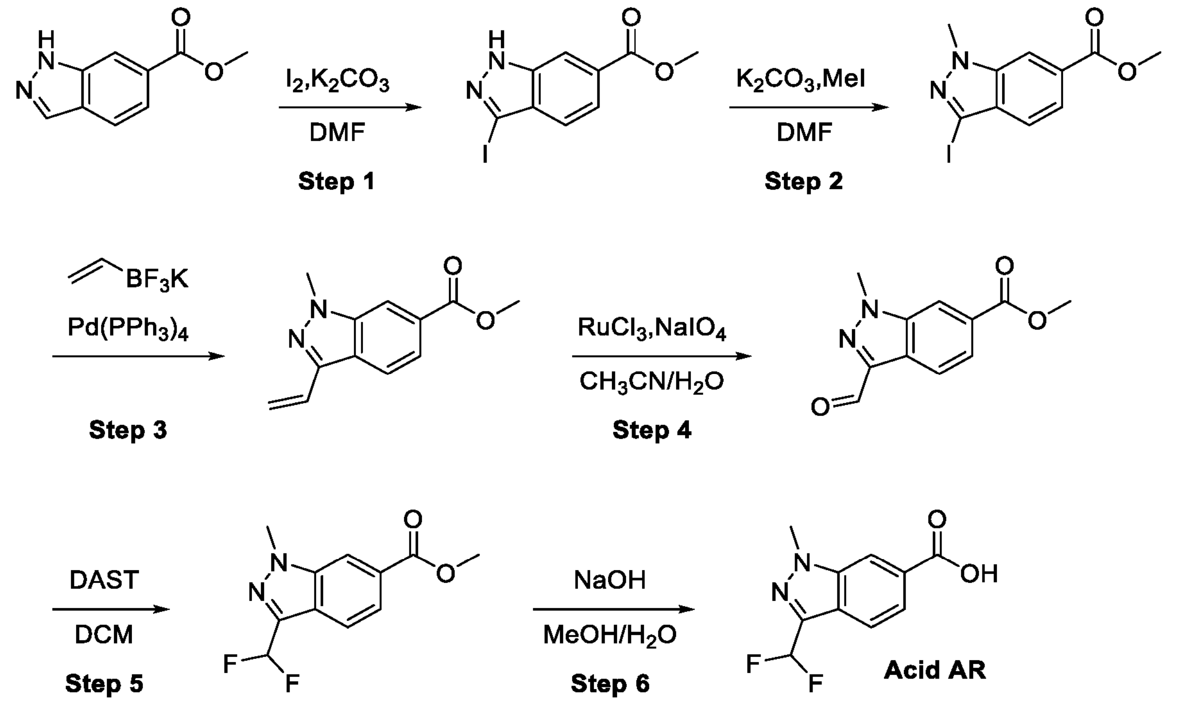


Into a 100-mL 3-necked round-bottom flask, was placed methyl 1H-indazole-6-carboxylate (4.00 g, 22.705 mmol, 1.00 equiv), $K_2CO_3$ (6275.83 mg, 45.410 mmol, 2.00 equiv), DMF (100.00 mL). This was followed by the addition of $I_2$ (8643.98 mg, 34.058 mmol, 1.50 equiv), in portions at 0 °C. The resulting solution was stirred for 16 h at room temperature. The reaction was then quenched by the addition of 100 mL of $Na_2S_2O_3$(aq). The resulting solution was extracted with 3x100 mL of ethyl acetate and the organic layers combined. This resulted in 7.4 g (crude) of methyl 3-iodo-1H-indazole-6-carboxylate as a yellow solid. **LCMS:** $[M+H]^+$=303.

Step 2. Into a 1-L 3-necked round-bottom flask, was placed methyl 3-iodo-1H-indazole-6-carboxylate (7.40 g, 24.498 mmol, 1.00 equiv), $K_2CO_3$ (10.16 g, 73.494 mmol, 3.00 equiv), DMF (740.00 mL). This was followed by the addition of $CH_3I$ (6.95 g, 48.996 mmol, 2.00 equiv) dropwise with stirring at 0 °C. The resulting solution was stirred for 10 h at room temperature. The solids were filtered out. The resulting mixture was concentrated. The residue was applied onto a silica gel column with ethyl acetate/petroleum ether (1:3). This resulted in 5.34 g (68.96%) of methyl 3-iodo-1-methylindazole-6-carboxylate as an off-white solid. **LCMS:** $[M+H]^+$=317.

Step 3. Into a 50-mL round-bottom flask purged and maintained with an inert atmosphere of nitrogen, was placed methyl 3-ethenyl-1-methylindazole-6-carboxylate (500.00 mg, 2.312 mmol, 1.00 equiv), trifluoro(vinyl)-14-borane, potassium salt (1238.90 mg, 0.000 mmol, 4.00 equiv), toluene (10.00 mL), $H_2O$ (2.00 mL), $K_3PO_4$ (1963.25 mg, 9.248 mmol, 4.00 equiv), $Pd(PPh_3)_4$ (267.19 mg, 0.231 mmol, 0.10 equiv). The resulting solution was stirred for 16 h at 110 °C in an oil bath. The solids were filtered out. The resulting mixture was concentrated. The residue was applied onto a silica gel column with ethyl acetate/petroleum ether (1:2). This resulted in 240 mg (32.8%) of methyl 3-iodo-1-methylindazole-6-carboxylate as a white solid. **LCMS:** $[M+H]^+$=217.

Step 4. Into a 100-mL 3-necked round-bottom flask, was placed methyl 3-ethenyl-1-methylindazole-6-carboxylate (750.00 mg, 3.468 mmol, 1.00 equiv), $H_2O$ (4.00 mL), $CH_3CN$ (20.00 mL), $NaIO_4$ (1483.71 mg, 6.936 mmol, 2.00 equiv), $RuCl_3.H_2O$ (78.19 mg, 0.347 mmol, 0.10 equiv). The resulting solution was stirred for 10 h at room temperature. The reaction was then quenched by the addition of 100 mL of $Na_2S_2O_3$(aq). The resulting solution was extracted with 3 x 30 mL of dichloromethane and the organic layers combined and concentrated. The residue was applied onto a silica gel column with ethyl acetate/petroleum ether (1:2). This resulted in 240 mg(31.7%) of methyl 3 -formyl-1-methylindazole-6-carboxylate as a brown solid. **LCMS:** $[M+H]^+$=219.

Step 5. Into a 100-mL 3-necked round-bottom flask purged and maintained with an inert atmosphere of nitrogen, was placed methyl 3-formyl-1-methylindazole-6-carboxylate (240.00 mg, 1.100 mmol, 1.00 equiv), DCM (20.00 mL). This was followed by the addition of DAST (709.13 mg, 4.399 mmol, 4.00 equiv) dropwise with stirring at 0 °C. The resulting solution was stirred for 10 h at room temperature. The reaction was then quenched by the addition of 20 mL of water/ice. The resulting solution was extracted with 2 x 20 mL of dichloromethane and the organic layers combined and concentrated. The residue was applied onto a silica gel column with ethyl acetate/petroleum ether (1: 1). This resulted in 180 mg (68.1%) of methyl 3-(difluoromethyl)-1-methylindazole-6-carboxylate as a white solid. **LCMS:** $[M+H]^+$=241.

Step 6. Into a 50-mL round-bottom flask, was placed methyl 3-(difluoromethyl)-1-methylindazole-6-carboxylate (180.00 mg, 0.749 mmol, 1.00 equiv), MeOH (10.00 mL), $H_2O$ (10.00 mL), NaOH (59.94 mg, 1.498 mmol, 2.00 equiv). The

resulting solution was stirred for 6 h at room temperature. The resulting mixture was concentrated. The reaction was then quenched by the addition of 10 mL of water. The pH value of the solution was adjusted to with citric acid (aq). The solids were collected by filtration. This resulted in 150 mg (88.50%) of 3-(difluoromethyl)-1-methylindazole-6-carboxylic acid as a white solid. **LCMS:** $[M+H]^+$=227.

Preparation of Acid AS

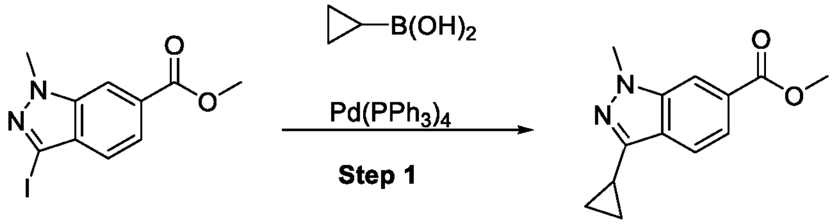


Into a 50-mL round-bottom flask purged and maintained with an inert atmosphere of nitrogen, was placed methyl 3-iodo-1-methylindazole-6-carboxylate (1.00 g, 3.164 mmol, 1.00 equiv), cyclopropylboronic acid (815.25 mg, 9.492 mmol, 3.00 equiv), $Pd(PPh_3)_4$ (365.57 mg, 0.316 mmol, 0.10 equiv), Toluene (20.00 mL), $H_2O$ (2.00 mL), $K_3PO_4$ (2.69 g, 12.656 mmol, 4.00 equiv). The resulting solution was stirred for 16 h at 110 °C in an oil bath. The solids were filtered out. The resulting mixture was concentrated. The residue was applied onto a silica gel column with ethyl acetate/petroleum ether (1:3). This resulted in 590 mg (81%) of methyl 3-cyclopropyl-1-methylindazole-6-carboxylate as a yellow solid. **LCMS:** $[M+H]^+$=231.

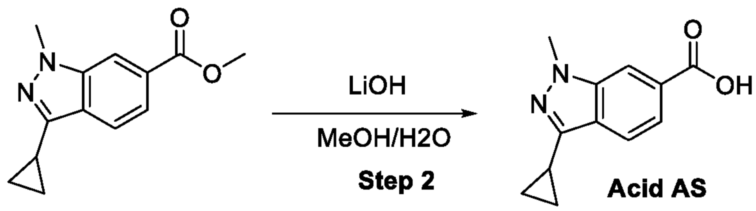

**Acid AS**

Into a 50-mL round-bottom flask, was placed methyl 3-cyclopropyl-1-methylindazole-6-carboxylate (590.00 mg, 2.56mmol, 1.00 equiv), LiOH (184.08 mg, 3.00 equiv), MeOH (10.00 mL), $H_2O$ (2.00 mL). The resulting solution was stirred for 6 h at room temperature. The resulting mixture was concentrated. The pH value of the solution was adjusted to 2~3 with citric acid(aq). The solids were collected by filtration. This resulted in 450 mg of 3-cyclopropyl-1-methylindazole-6-carboxylic acid as a white solid. **LCMS:** $[M+H]^+$=217.

Preparation of Acid AT

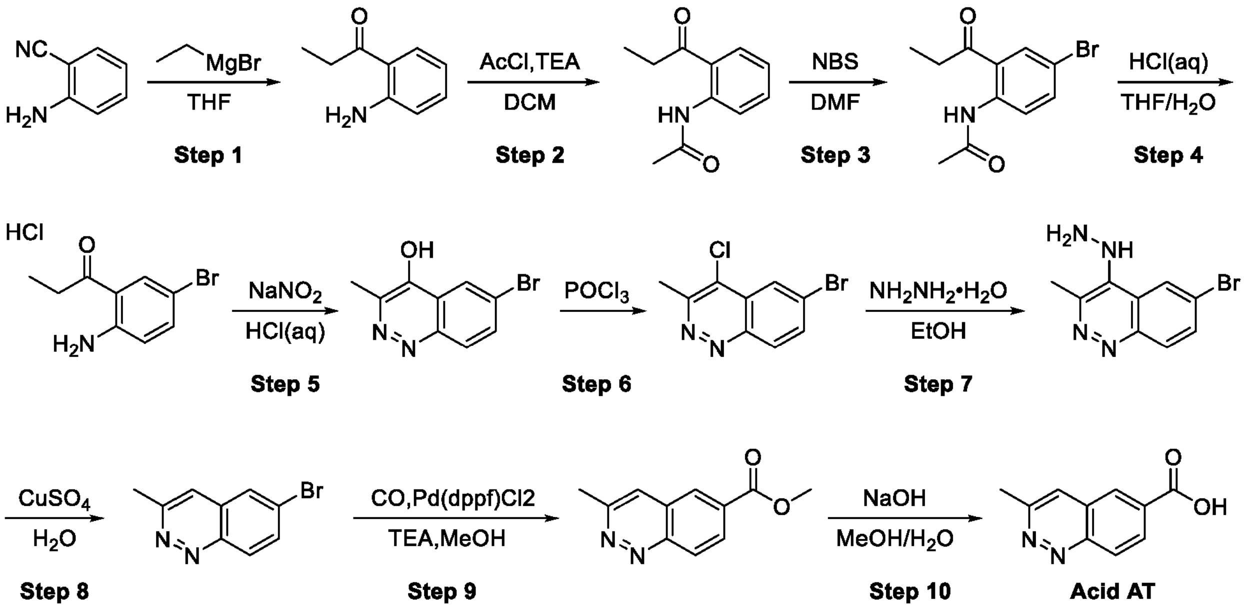


Step 1. Into a 500-mL 3-necked round-bottom flask purged and maintained with an inert atmosphere of nitrogen, was placed 2-amino-benzonitrile (11.00 g, 93.111 mmol, 1.00 equiv), tetrahydrofuran (220 mL). Ethylmagnesium bromide(2 mol/L in $Et_2O$) (139.67 mL, 279.33 mmol, 3.00 equiv) was added and the resulting solution was stirred for 30 min at -10 °C. The resulting solution was allowed to react, with stirring, for an additional 12 h at 25 °C. The reaction was then quenched by the addition of 300 mL of HCl(10%). The resulting solution was extracted with 3 x 100 mL of ethyl acetate and the aqueous layers combined. The resulting mixture was washed with 3 x 100mL of brine. The resulting mixture was concentrated. The residue was applied onto a silica gel column with ethyl acetate/hexane (1:10). This resulted in 11 g (79.19%) of 1-(2-aminophenyl)propan-1-one as a light yellow solid. **LCMS:** $[M+1]^+$=150.

Step 2. Into a 500-mL 3-necked round-bottom flask purged and maintained with an inert atmosphere of nitrogen, was placed 1-(2-aminophenyl)propan-1-one (11.0 g, 73.730 mmol, 1.00 equiv), TEA (22.38 g, 221.190 mmol, 3.00 equiv), DCM (220.00 mL), acetyl chloride (8.68 g, 110.595 mmol, 1.50 equiv). The resulting solution was stirred for 3 h at 25 °C. The reaction was then quenched by the addition of 600 mL of water/ice. The resulting solution was extracted with 3x500 mL of ethyl acetate and the aqueous layers combined. The resulting mixture was washed with 3 x400mL of brine. The resulting mixture was concentrated. The residue was applied onto a silica gel column with ethyl acetate/petroleum ether (1:10). This resulted in 10 g (70.9%) of N-(2-propanoylphenyl)acetamide as a brown solid. **LCMS:** $[M+1]^+$=192.

Step 3. Into a 250-mL 3-necked round-bottom flask purged and maintained with an inert atmosphere of nitrogen, was placed N-(2-propanoylphenyl)acetamide (10.0 g, 52.293 mmol, 1.00 equiv), DMF (150.00 mL), NBS (11168.79 mg, 62.752 mmol, 1.20 equiv). The resulting solution was stirred for 1 overnight at 40 °C. The resulting solution was extracted with 3 x 150 mL of ethyl acetate and the organic layers combined. The resulting mixture was washed with 2 x150 mL of brine. The resulting mixture was concentrated. This resulted in 13 g (92.03%) of N-(4-bromo-2-propanoylphenyl) acetamide as a solid. **LCMS:** $[M+1]^+$=270.

Step 4. Into a 250-mL round-bottom flask purged and maintained with an inert atmosphere of nitrogen, was placed N-(4-bromo-2-propanoylphenyl)acetamide (5.50 g, 0.020 mmol, 1.00 equiv), THF (60.00 mL), $H_2O$ (12.00 mL), HCl (12.00 mL). The resulting solution was stirred for 1 h at 70 °C.The resulting mixture was concentrated. This resulted in 5.8 g (crude) of 1-(2-amino-5-bromophenyl)propan-1-one hydrochloride as a brown solid. **LCMS:** $[M+1]^+$=228.

Step 5. Into a 100-mL round-bottom flask purged and maintained with an inert atmosphere of nitrogen, was placed 1-(2-amino-5-bromophenyl)propan-1-one (5.00 g, 21.921 mmol, 1.00 equiv), $H_2O$ (24.00 mL, 222.033 mmol), HCl(37%) (12.00 mL, 0.058 mmol), sodium nitrite (1.81 g, 26.305 mmol, 1.20 equiv). The resulting solution was stirred for 30 min at 25 °C. The resulting solution was allowed to react, with stirring, for an additional 10 h at 100 °C. The reaction mixture was cooled to 25 °C with a water/ice bath. The solids were collected by filtration. This resulted in 2.8 g (53.43%) of 6-bromo-3-methylcinnolin-4-ol as a solid. **LCMS:** $[M+1]^+$=239.

Step 6. Into a 50-mL round-bottom flask purged and maintained with an inert atmosphere of nitrogen, was placed 6-bromo-3-methylcinnolin-4-ol (2.8 g,11.76mmol, 1.00 equiv),$POCl_3$(20 mL). The resulting solution was stirred for 2 h at 100 °C. The resulting mixture was concentrated. This resulted in 2.8 g (92.84%) of 6-bromo-4-chloro-3-methylcinnoline as a brown solid. **LCMS:** $[M+1]^+$=257.

Step 7. Into a 100-mL round-bottom flask purged and maintained with an inert atmosphere of nitrogen, was placed 6-bromo-4-chloro-3-methylcinnoline (2.80 g, 10.873 mmol, 1.00 equiv), ethanol (56 mL), $NH_2NH_2.H_2O$ (10.89 g, 217.460 mmol, 20.00 equiv). The resulting solution was stirred for 3 days at 80 °C. The reaction was then quenched by the

addition of 200 mL of water/ice. The solids were collected by filtration. This resulted in 2.3 g (83.58%) of 6-bromo-4-hydrazinyl-3-methylcinnoline as a brown solid. **LCMS:** $[M+1]^+$=253.

Step 8. Into a 100-mL round-bottom flask purged and maintained with an inert atmosphere of nitrogen, was placed 6-bromo-4-hydrazinyl-3-methylcinnoline (2.30 g, 9.087 mmol, 1.00 equiv), water (40.00 mL), dioxo(sulfonylidene) copper (2.90 mg, 18.174 mmol, 2.00 equiv). The resulting solution was stirred for 2 h at 100 °C. The reaction was then quenched by the addition of 100 mL of water/ice. The pH value of the solution was adjusted to 8 with NaOH (1 mol/L). The resulting solution was extracted with 3x50 mL of ethyl acetate concentrated. The residue was applied onto a silica gel column with ethyl acetate/hexane (1:3).This resulted in 1 g (49.33%) of 6-bromo-3-methylcinnoline as a off-white solid. **LCMS:** $[M+1]^+$=223.

Step 9. Into a 50-mL pressure tank reactor, was placed 6-bromo-3-methylcinnoline (500.00 mg, 2.241 mmol, 1.00 equiv), methanol (10.00 mL), Pd(dppf)$Cl_2$ (164.00 mg, 0.224 mmol, 0.10 equiv), TEA (680.43 mg, 6.723 mmol, 3.00 equiv), carbon monoxide(20 atm) . The resulting solution was stirred for 16 h at 80 °C. The resulting mixture was concentrated. The residue was applied onto a silica gel column with ethyl acetate/hexane (1:3).This resulted in 400 mg (88.3%) of methyl 3-methylcinnoline-6-carboxylate as a off-white solid. **LCMS:** $[M+1]^+$=203.

Step 10. Into a 50-mL round-bottom flask purged and maintained with an inert atmosphere of nitrogen, was placed methyl 3-methylcinnoline-6-carboxylate (400.00 mg, 1.978 mmol, 1.00 equiv), methanol (10.00 mL), water (2.00 mL), sodium hydroxide (158.24 mg, 3.96 mmol, 2.00 equiv). The resulting solution was stirred for 16 h at 25 °C. The pH value of the solution was adjusted to 3 with HCl (37 %). The resulting mixture was concentrated. This resulted in 400 mg (crude) of 3-methylcinnoline-6-carboxylic acid as a off-white solid. **LCMS:** $[M+1]^+$=189.

Preparation of Acid AU

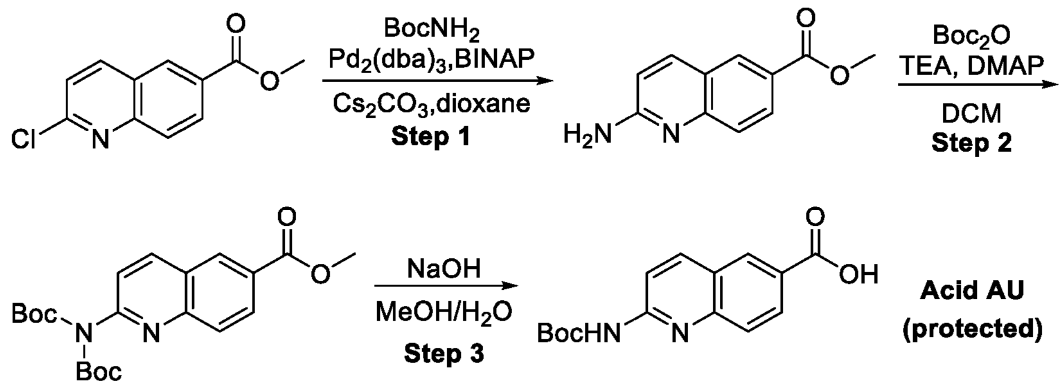


Step 1. Into a 40-mL vial purged and maintained with an inert atmosphere of nitrogen, was placed methyl 2-chloroquinoline-6-carboxylate (1.20 g, 5.414 mmol, 1.00 equiv), dioxane (24.00 mL), $BocNH_2$ (0.95 g, 8.110 mmol, 1.50 equiv), BINAP (0.34 g, 0.541 mmol, 0.10 equiv), $Cs_2CO_3$ (5.29 g, 16.242 mmol, 3.00 equiv), $Pd_2(dba)_3$ (0.25 g, 0.271 mmol, 0.05 equiv). The resulting solution was stirred for 20 hr at 100 °C in an oil bath. The resulting mixture was concentrated under vacuum. The resulting solution was diluted with 30 mL of $H_2O$. The resulting solution was extracted with 3 x 20 mL of ethyl acetate and the organic layers combined. The resulting mixture was washed with 2 x 20 mL of brine. The mixture was dried over anhydrous sodium sulfate and concentrated under vacuum. This resulted in 700 mg (63.9%) of methyl 2-aminoquinoline-6-carboxylate as a brown solid. **LCMS:** $[M+H]^+$=303.

Step 2. Into a 100-mL round-bottom flask, was placed methyl 2-aminoquinoline-6-carboxylate (700.00 mg, 3.462 mmol, 1.00 equiv), DCM (21.00 mL), $Boc_2O$ (1511.01 mg, 6.92 mmol, 2.00 equiv), $Et_3N$ (1050.9 mg, 10.385 mmol, 3.00 equiv), DMAP (84.58 mg, 0.692 mmol, 0.20 equiv). The resulting solution was stirred for 16 hr at room temperature. The resulting mixture was concentrated under vacuum. The resulting solution was diluted with 30 mL of $H_2O$. The resulting solution was extracted with 3 x 20 mL of ethyl acetate and the organic layers combined and dried over anhydrous sodium sulfate and concentrated under vacuum. This resulted in 1.5 g (69.76%) of methyl 2-[bis(tert-butoxycarbonyl)amino] quinoline-6-carboxylate as a brown solid. **LCMS:** $[M+H]^+$=403.

Step 3. Into a 100-mL round-bottom flask, was placed methyl 2-[bis(tert-butoxycarbonyl)amino]quinoline-6-carboxylate (1.50 g, 3.727 mmol, 1.00 equiv), $CH_3OH$ (30.00 mL), $H_2O$ (10.00 mL), sodium hydroxide (0.45 g, 11.251 mmol, 3.02 equiv). The resulting solution was stirred for 6 hr at room temperature. The resulting mixture was concentrated under vacuum. The resulting solution was diluted with 30 mL of $H_2O$. The resulting solution was extracted with 2 x 20 mL of ethyl acetate and the aqueous layers combined. The pH value of the solution was adjusted to 3 with HCl (3M). The solids were collected by filtration. This resulted in 800 mg (62.18%) of 2-[(tert-butoxycarbonyl)amino]quinoline-6-carboxylic acid

as a brown solid. **LCMS:** $[M+H]^+$=289.

Preparation of Acid AV

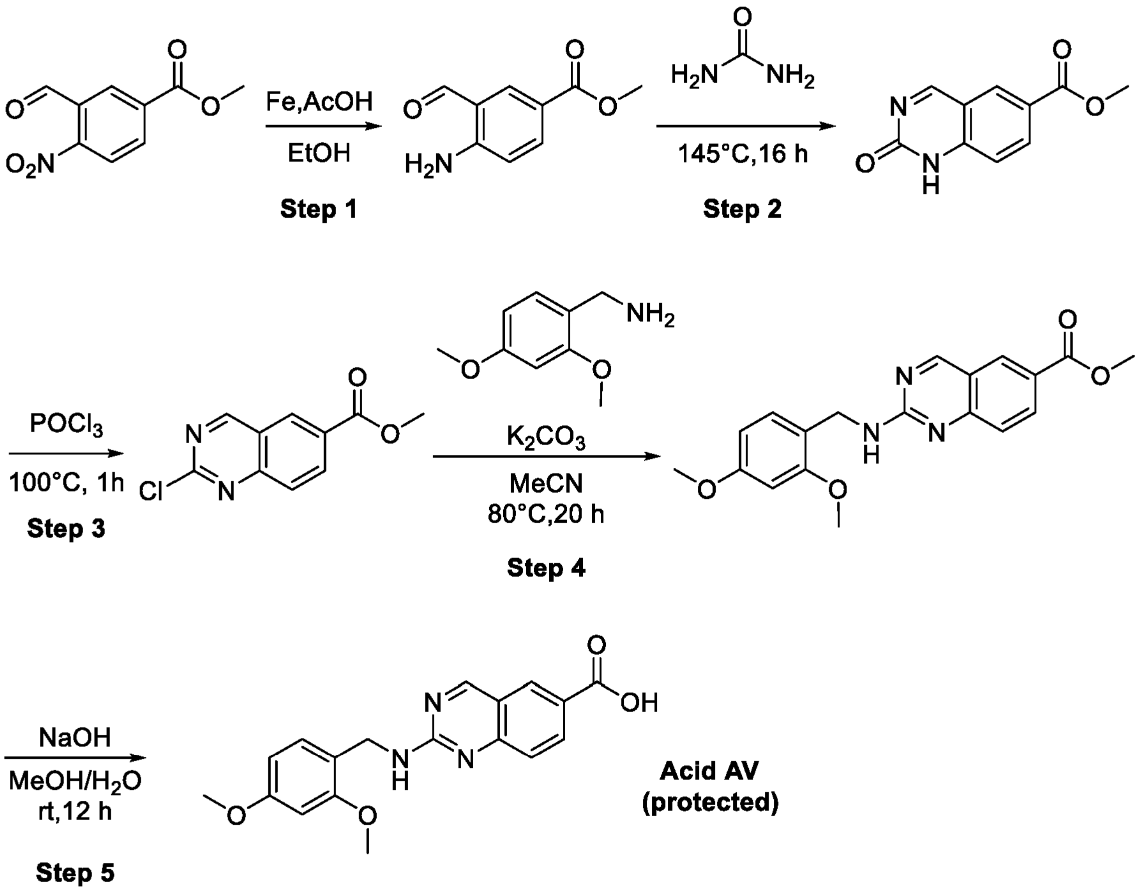


**Acid AV (protected)**

Step 1. Into a 50-mL 3-necked round-bottom flask purged and maintained with an inert atmosphere of nitrogen, was placed methyl 3-formyl-4-nitrobenzoate (1.10 g, 5.26 mmol, 1.00 equiv), AcOH (5.50 mL), EtOH (5.50 mL), Fe (881.10 mg, 15.78 mmol, 3.00 equiv). The resulting solution was stirred for 3 hr at room temperature. The solids were filtered out. The resulting mixture was concentrated. The residue was applied onto a silica gel column with ethyl acetate/petroleum ether (1:2). This resulted in 700 mg (74.28%) of methyl 4-amino-3-formylbenzoate as a yellow solid. **LCMS:** $[M+1]^+$=180.

Step 2. Into a 8-mL round-bottom flask, was placed methyl 4-amino-3-formylbenzoate (700.00 mg, 3.907 mmol, 1.00 equiv), urea (1173.13 mg, 19.534 mmol, 5.00 equiv). The resulting solution was stirred for 16 hr at 145 °C. The resulting solution was diluted with 10 mL of $H_2O$. The solids were collected by filtration. This resulted in 400 mg (50.14%) of methyl 2-oxo-1H-quinazoline-6-carboxylate as a yellow solid. **LCMS:** $[M+1]^+$=205.

Step 3. Into a 8-mL round-bottom flask, was placed methyl 2-oxo-1H-quinazoline-6-carboxylate (400.00 mg, 1 equiv), phosphorus oxychloride (1.00 mL). The resulting solution was stirred for 1 hr at 100 °C. The resulting mixture was concentrated. This resulted in 250 mg (57.32%) of methyl 2-chloroquinazoline-6-carboxylate as a yellow solid. **LCMS:** $[M+1]^+$=223.

Step 4. Into a 8-mL round-bottom flask, was placed methyl 2-chloroquinazoline-6-carboxylate (140.00 mg, 0.629 mmol, 1.00 equiv), ACN (2.00 mL), potassium carbonate (262.63 mg, 1.887 mmol, 3.0 equiv), 1-(2,4-dimethoxyphenyl) benzylamine (157.72 mg, 0.943 mmol, 1.50 equiv). The resulting solution was stirred for 20 hr at 80 °C. The resulting solution was diluted with 10 mL of $H_2O$. The resulting solution was extracted with 3 x 10 mL of ethyl acetate and the organic layers combined and concentrated. The residue was applied onto a silica gel column with ethyl acetate/petroleum ether (1:2). This resulted in 120 mg (54.00%) of methyl 2-[[(2,4-dimethoxyphenyl)methyl]amino]quinazoline-6-carboxylate as a light yellow solid. **LCMS:** $[M+1]^+$=354.

Step 5. Into a 8-mL round-bottom flask, was placed methyl 2-[[(2,4-dimethoxyphenyl)methyl]amino]quinazoline-6-carboxylate (120.00 mg, 0.340 mmol, 1.00 equiv), MeOH (2.00 mL), $H_2O$ (2.00 mL), sodium hydroxide (54.33 mg, 1.358 mmol, 4.00 equiv). The resulting solution was stirred for 12 hr at room temperature. The resulting mixture was

concentrated. The pH value of the solution was adjusted to 1-2 with HCL (1 mol/L). The solids were collected by filtration. This resulted in 90 mg (78.10%) of 2-[[(2,4-dimethoxyphenyl)methyl]amino]quinazoline-6-carboxylic acid as a white solid. **LCMS:** $[M+1]^+$=340.

Preparation of Acid AW

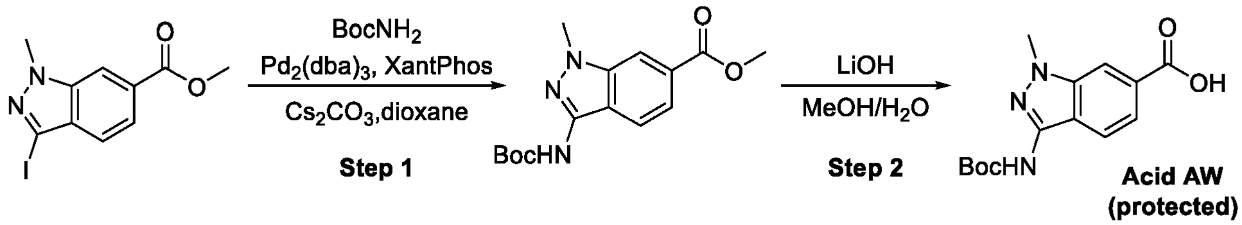


Step 1. Into a 50-mL round-bottom flask purged and maintained with an inert atmosphere of nitrogen, was placed methyl 3-iodo-1-methylindazole-6-carboxylate (1.00 g, 3.164 mmol, 1.00 equiv), tert-butyl carbamate (1111.82 mg, 0.000 mmol, 3.00 equiv), $Pd_2(dba)_3$ (289.69 mg, 0.316 mmol, 0.10 equiv), Xantphos (366.10 mg, 0.633 mmol, 0.20 equiv), $Cs_2CO_3$ (4123.02 mg, 12.656 mmol, 4.00 equiv), Dioxane (20.00 mL). The resulting solution was stirred for 20 h at 100 °C in an oil bath. The solids were filtered out. The resulting mixture was concentrated. The residue was applied onto a silica gel column with ethyl acetate/petroleum ether (1:2). This resulted in 850 mg of methyl 3-[(tert-butoxycarbonyl)amino]-1-methylindazole-6-carboxylate as a brown solid. **LCMS:** $[M+H]^+$=306.

Step 2. Into a 50-mL round-bottom flask, was placed methyl 3-[(tert-butoxycarbonyl)amino]-1-methylindazole-6-carboxylate (400.00 mg, 1.310 mmol, 1.00 equiv), LiOH (94.12 mg, 3.930 mmol, 3.00 equiv), MeOH (20.00 mL), $H_2O$ (5.00 mg). The resulting solution was stirred for 12 h at room temperature. The resulting mixture was concentrated. The pH value of the solution was adjusted to 2~3 with citric acid(aq). The solids were collected by filtration. This resulted in 280 mg (73.37%) of 3-[(tert-butoxycarbonyl)amino]-1-methylindazole-6-carboxylic acid as a off-white solid. **LCMS:** $[M+H]^+$=292.

Preparation of Acid AX

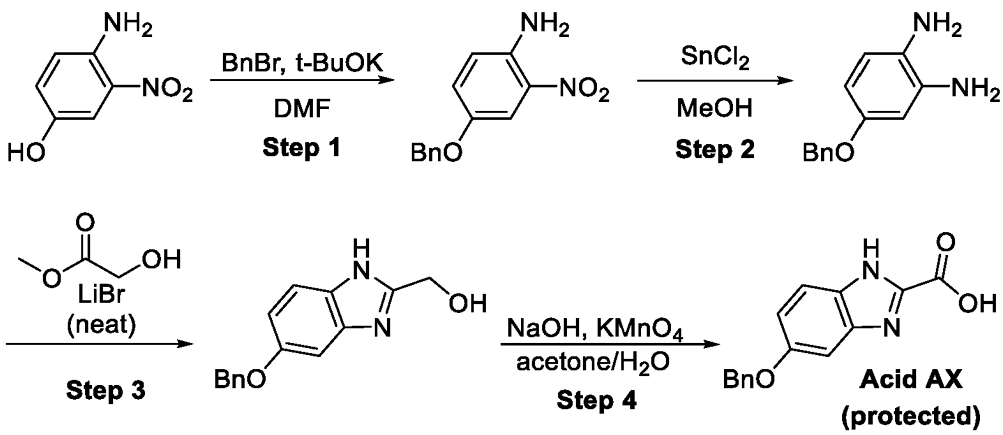


Step 1. Into a 250-mL 3-necked round-bottom flask purged and maintained with an inert atmosphere of nitrogen, was placed 4-amino-3-nitrophenol (10.00 g, 64.882 mmol, 1.00 equiv), dimethylformamide (100.00 mL), t-BuOK (7.28 g, 64.882 mmol, 1 equiv). The resulting solution was stirred for 0.5 hr at 0 °C in a water/ice bath. The BnBr (11.10 g, 64.882 mmol, 1 equiv) wsa placed into the flask. The resulting solution was stirred for 4 hr at room temperature. The resulting solution was diluted with 200 mL of $NH_4Cl$. The solids were collected by filtration. This resulted in 8 g (50.48%) of 4-(benzyloxy)-2-nitroaniline as a brown solid. **LCMS:** $[M+H]^+$=245.

Step 2. Into a 100-mL 3-necked round-bottom flask purged and maintained with an inert atmosphere of nitrogen, was placed 4-(benzyloxy)-2-nitroaniline (2.00 g, 8.188 mmol, 1.00 equiv), $CH_3OH$ (40.00 mL), stannous chloride (4.71 g, 24.579 mmol, 3.00 equiv). The resulting solution was stirred for 16 hr at 80 °C in an oil bath. The resulting mixture was concentrated under vacuum. The residue was applied onto a silica gel column with ethyl acetate/petroleum ether (1:3).

This resulted in 1.5 g (75.40%) of 4-(benzyloxy)benzene-1,2-diamine as a brown solid. **LCMS:** $[M+H]^+$=215.

Step 3. Into a 40-mL vial purged and maintained with an inert atmosphere of nitrogen, was placed 4-(benzyloxy) benzene-1,2-diamine (800.00 mg, 3.734 mmol, 1.00 equiv), methyl 2-hydroxyacetate (8.00 mL), LiBr (64.85 mg, 0.747 mmol, 0.2 equiv). The resulting solution was stirred for 12 hr at 80 °C in an oil bath. The residue was applied onto a silica gel column with ethyl acetate/petroleum ether (1:1). This resulted in 500 mg (44.89%) of [5-(benzyloxy)-1H-1,3-benzodiazol-2-yl]methanol as a brown solid. **LCMS:** $[M+H]^+$=255.

Step 4. Into a 40-mL vial purged and maintained with an inert atmosphere of nitrogen, was placed [5-(benzyloxy)-1H-1,3-benzodiazol-2-yl]methanol (500.00 mg, 1.966 mmol, 1.00 equiv), acetone (20.00 mL), $H_2O$ (5.00 mL), NaOH (165.15 mg, 4.129 mmol, 2.10 equiv), $KMnO_4$ (466.11 mg, 2.949 mmol, 1.50 equiv). The resulting solution was stirred for 2 hr at 100 °C in an oil bath. The reaction mixture was cooled. The resulting mixture was concentrated under vacuum. The resulting solution was diluted with 30 mL of $H_2O$. The solids were filtered out. The resulting solution was extracted with 2x20 mL of ethyl acetate and the aqueous layers combined. The pH value of the solution was adjusted to 3 with HCl (3M). The solids were collected by filtration. This resulted in 260 mg (49.29%) of 5-(benzyloxy)-1H-1,3-benzodiazole-2-carboxylic acid as a brown solid. **LCMS:** $[M+H]^+$=269.

Preparation of Acid AY

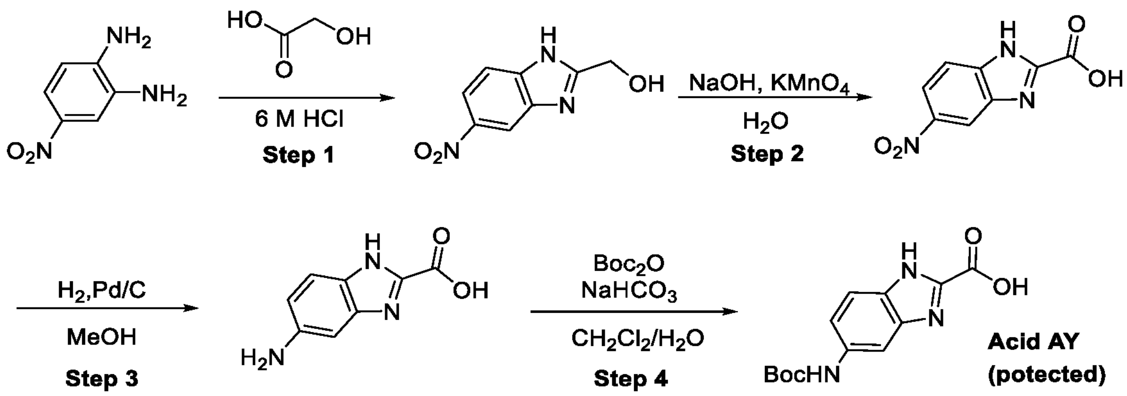


Step 1. Into a 100-mL 3-necked round-bottom flask purged and maintained with an inert atmosphere of nitrogen, was placed 2-amino-4-nitroaniline (5.00 g, 32.650 mmol, 1.00 equiv), HCl(6M) (50.00 mL), glycolic acid (2.48 g, 32.650 mmol, 1.00 equiv). The resulting solution was stirred for 3 hr at 100 °C in an oil bath. The resulting solution was extracted with 2x50 mL of ethyl acetate and the aqueous layers combined. The pH value of the solution was adjusted to pH 8 with $NaHCO_3$. The solids were collected by filtration. This resulted in 4 g (63.42%) of (5-nitro-1H-1,3-benzodiazol-2-yl) methanol as a brown solid. **LCMS:** $[M+H]^+$=194.

Step 2. Into a 100-mL 3-necked round-bottom flask purged and maintained with an inert atmosphere of nitrogen, was placed (5-nitro-1H-1,3-benzodiazol-2-yl)methanol (4.00 g, 20.708 mmol, 1.00 equiv), $H_2O$ (60.00 mL), NaOH (1.74 g, 0.044 mmol, 2.10 equiv), $KMnO_4$ (4.91 g, 31.069 mmol, 1.50 equiv). The resulting solution was stirred for 2 hr at room temperature. The resulting solution was diluted with 50 mL of $H_2O$. The resulting solution was extracted with 2 x 40 mL of ethyl acetate and the aqueous layers combined. The solids were filtered out. The pH value of the solution was adjusted to 3 with HCl (3M). The solids were collected by filtration. This resulted in 3.5 g (81.59%) of 5-nitro-1H-1,3-benzodiazole-2-carboxylic acid as a brown solid. **LCMS:** $[M+H]^+$=208.

Step 3. Into a 50-mL pressure tank reactor purged and maintained with an inert atmosphere of nitrogen, was placed 5-nitro-1H-1,3-benzodiazole-2-carboxylic acid (3.25 g, 15.689 mmol, 1.00 equiv), $CH_3OH$ (32 mL), Pd/C (2.00 g, 18.827 mmol, 1.20 equiv), $H_2$ (10 atm). The resulting solution was stirred for 4 hr at room temperature. The solids were filtered out. The resulting mixture was concentrated under vacuum. This resulted in 2.3 g (82.75%) of 5-amino-1H-1,3-benzodiazole-2-carboxylic acid as a light brown solid. **LCMS:** $[M+H]^+$=178.

Step 4. Into a 100-mL round-bottom flask, was placed 5-amino-1H-1,3-benzodiazole-2-carboxylic acid (2.30 g, 12.982 mmol, 1.00 equiv), DCM (23.00 mL), $H_2O$ (23.00 mL), $Boc_2O$ (5.67 g, 25.980 mmol, 2.00 equiv), $NaHCO_3$ (3.27 g, 38.947 mmol, 3 equiv). The resulting solution was stirred for 16 hr at room temperature. The resulting solution was diluted with 30 mL of $H_2O$. The resulting solution was extracted with 3 x 30 mL of dichloromethane and the organic layers combined and dried over anhydrous sodium sulfate and concentrated under vacuum. This resulted in 2.3 g (63.9%) of 5-[(tert-butoxycarbonyl)amino]-1H-1,3-benzodiazole-2-carboxylic acid as a brown solid. **LCMS:** $[M+H]^+$=278.

Preparation of Acid BA

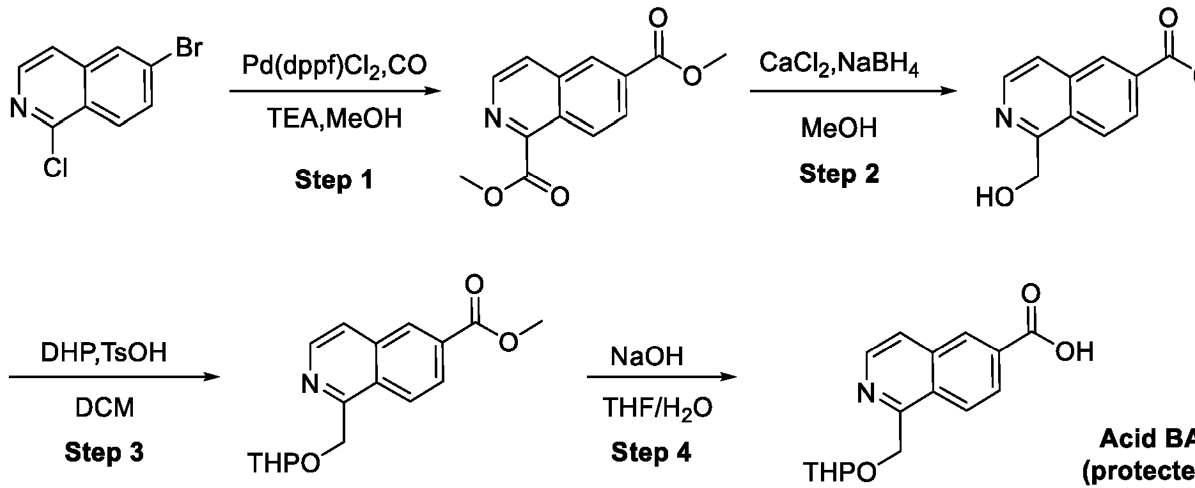


**Acid BA (protected)**

Into a 50-mL pressure tank reactor, was placed 6-bromo-1-chloroisoquinoline (2.00 g, 8.247 mmol, 1.00 equiv), TEA (4.17 g, 0.040 mmol, 5.00 equiv), MeOH (20.00 mL). The resulting solution was filled with CO(gas) so that the inner pressure was 0.3MPa ande then stirred for 16 h at 60 °C. The solids were filtered out. The resulting mixture was concentrated. This resulted in 1.52 g (75.15%) of 1,6-dimethyl isoquinoline-1,6-dicarboxylate as an off-white solid. **LCMS:** $[M+H]^+$=246.

Step 2. Into a 100-mL 3-necked round-bottom flask purged and maintained with an inert atmosphere of nitrogen, was placed 1,6-dimethyl isoquinoline-1,6-dicarboxylate (1.52 g, 6.198 mmol, 1.00 equiv), MeOH (30.00 mL), $CaCl_2$ (687.90 mg, 6.198 mmol, 1.00 equiv), $NaBH_4$ (281.39 mg, 7.438 mmol, 1.20 equiv). The resulting solution was stirred for 3 h at 0 °C. The reaction was then quenched by the addition of 10 mL of water/ice. The solids were filtered out. The resulting solution was extracted with 2x30 mL of ethyl acetate and the organic layers combined and concentrated. This resulted in 1.2 g (89.13%) of methyl 1-(hydroxymethyl)isoquinoline-6-carboxylate as an off-white solid. **LCMS:** $[M+H]^+$=218.

Step 3. Into a 100-mL 3-necked round-bottom flask, was placed methyl 1-(hydroxymethyl)isoquinoline-6-carboxylate (300.00 mg, 1.381 mmol, 1.00 equiv), DCM (20.00 mL), TsOH (23.78 mg, 0.138 mmol, 0.10 equiv), DHP (232.34 mg, 2.762 mmol, 2.00 equiv). The resulting solution was stirred for 40 h at room temperature. The resulting mixture was concentrated. The residue was applied onto a silica gel column with ethyl acetate/petroleum ether (1:3). This resulted in 400 mg (96.11%) of methyl 1-[(oxan-2-yloxy)methyl]isoquinoline-6-carboxylate as a brown solid. **LCMS:** $[M+H]^+$=302.

Step 4. Into a 50-mL round-bottom flask, was placed methyl 1-[(oxan-2-yloxy)methyl]isoquinoline-6-carboxylate (400.00 mg, 1.327 mmol, 1.00 equiv), NaOH (106.18 mg, 2.654 mmol, 2.00 equiv), THF (10.00 mL), $H_2O$ (10.00 mL). The resulting solution was stirred for 10 h at room temperature. The pH value of the solution was adjusted to 2~3 with citric acid(aq). The solids were collected by filtration. This resulted in 253 mg (66.34%) of 1-[(oxan-2-yloxy)methyl]isoquinoline-6-carboxylic acid as a white solid. **LCMS:** $[M+H]^+$=288.

Preparation of Acid BB

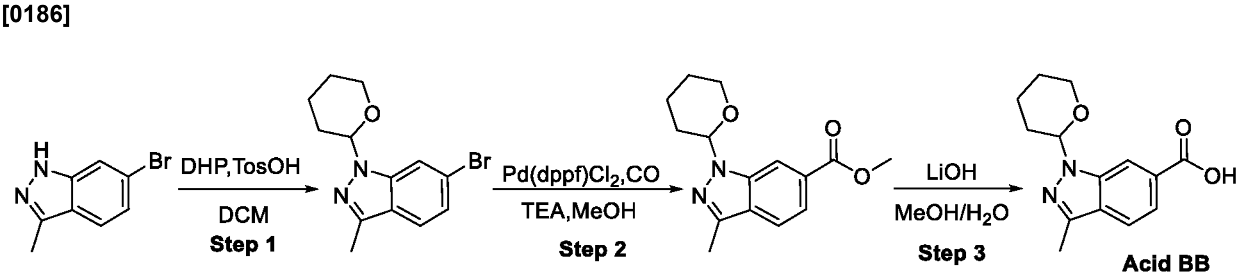


**Acid BB (protected)**

Step 1. Into a 100-mL 3-necked round-bottom flask purged and maintained with an inert atmosphere of nitrogen, was placed 6-bromo-3-methyl-1H-indazole (1.00 g, 4.738 mmol, 1.00 equiv), DHP (478.25 mg, 5.686 mmol, 1.20 equiv), DCM (10.00 mL), TsOH (81.59 mg, 0.474 mmol, 0.10 equiv). The resulting solution was stirred for 5 h at room temperature.

The solids were filtered out. The resulting mixture was concentrated. The residue was applied onto a silica gel column with ethyl acetate/petroleum ether (1:2). This resulted in 1.2 g (85.80%) of 6-bromo-3-methyl-1-(oxan-2-yl)indazole as a white solid. **LCMS:** [M+H] $^+$=295.

Step 2. Into a 50-mL pressure tank reactor, was placed methyl 6-bromo-3-methyl-1-(oxan-2-yl)indazole 3-methyl-1-(oxan-2-yl)indazole-6-carboxylate (1.20 g, 4.374 mmol, 1.00 equiv), TEA (2.21 g, 0.022 mmol, 5 equiv), MeOH (20.00 mL), Pd(dppf)$Cl_2$ $CH_2Cl_2$ (0.36 g, 0.000 mmol, 0.1 equiv). The resulting solution was stirred for 12 h at 80 °C under 0.3 MPa CO(gas) atmosphere. The solids were filtered out. The resulting mixture was concentrated. The residue was applied onto a silica gel column with ethyl acetate/petroleum ether (1:2). This resulted 3-methyl-1-(oxan-2-yl)indazole-6-carboxylate in 900 mg (69.70%) of 6-bromo-3-methyl-1-(oxan-2-yl)indazole as a white solid. **LCMS:** [M+H]$^+$=275.

Step 3. Into a 50-mL round-bottom flask, was placed methyl 3-methyl-1-(oxan-2-yl)indazole-6-carboxylate (400.00 mg, 1.458 mmol, 1.00 equiv), MeOH (10.00 mL), $H_2O$ (2.00 mL, 0.111 mmol), LiOH (69.84 mg, 2.916 mmol, 2.00 equiv). The resulting solution was stirred for 12 h at 40 °C in an oil bath. The resulting mixture was concentrated. The reaction was then quenched by the addition of 10 mL of water. The pH value of the solution was adjusted to 2~3 with citric acid(aq). The solids were collected by filtration. This resulted in 260 mg (68.50%) of 3-methyl-1-(oxan-2-yl)indazole-6-carboxylic acid as an off-white solid. **LCMS:** [M+H]$^+$=261.

Preparation of Acid BC

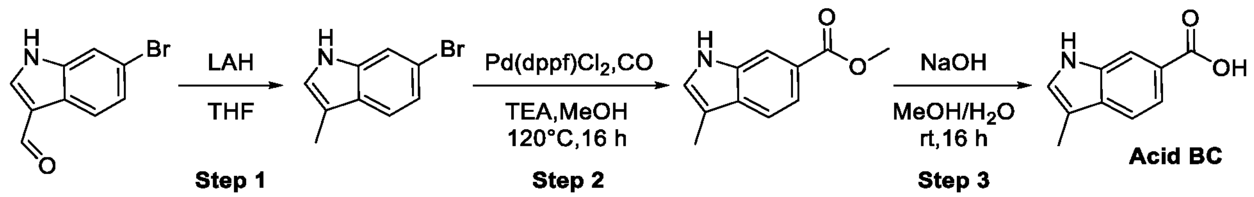


Step 1. Into a 50-mL 3-necked round-bottom flask purged and maintained with an inert atmosphere of nitrogen, was placed 6-bromo-1H-indole-3-carbaldehyde (2.00 g, 8.926 mmol, 1.00 equiv), THF (20.00 mL), LAH (914.73 mg, 24.101 mmol, 2.70 equiv). The resulting solution was stirred for 6 hr at room temperature. The reaction mixture was cooled to 0-5 °C with a water/ice bath. The resulting solution was diluted with 20 mL of $H_2O$. The resulting solution was extracted with 3 x 20 mL of ethyl acetate and the organic layers combined and concentrated. The residue was applied onto a silica gel column with ethyl acetate/petroleum ether (1:2). This resulted in 1 g (53.3%) of 6-bromo-3-methyl-1H-indole as a brown solid. **LCMS:** [M+1]$^+$=210.

Step 2. Into a 50-mL round-bottom flask, was placed 6-bromo-3-methyl-1H-indole (1.00 g, 4.760 mmol, 1.00 equiv), MeOH (10.00 mL), TEA (1445.06 mg, 14.281 mmol, 3.00 equiv), Pd(dppf)$Cl_2$ (174.15 mg, 0.238 mmol, 0.05 equiv), CO . The resulting solution was stirred for 16 hr at 120 °C. The solids were filtered out. The resulting mixture was concentrated. This resulted in 700 mg (77.72%) of methyl 3-methyl-1H-indole-6-carboxylate as a brown solid. **LCMS:** [M+1]$^+$=190.

Step 3. Into a 8-mL round-bottom flask, was placed methyl 3-methyl-1H-indole-6-carboxylate (400.00 mg, 2.114 mmol, 1.00 equiv), $H_2O$ (1.00 mL), MeOH (1.00 mL), sodium hydroxide (169.11 mg, 4.228 mmol, 2.00 equiv). The resulting solution was stirred for 16 hr at room temperature. The pH value of the solution was adjusted to 2-3 with HCL (1M). The solids were collected by filtration. This resulted in 300 mg (81.01%) of 3-methyl-1H-indole-6-carboxylic acid as a white solid. **LCMS:** [M+1]$^+$=176.

Preparation of Acid BE

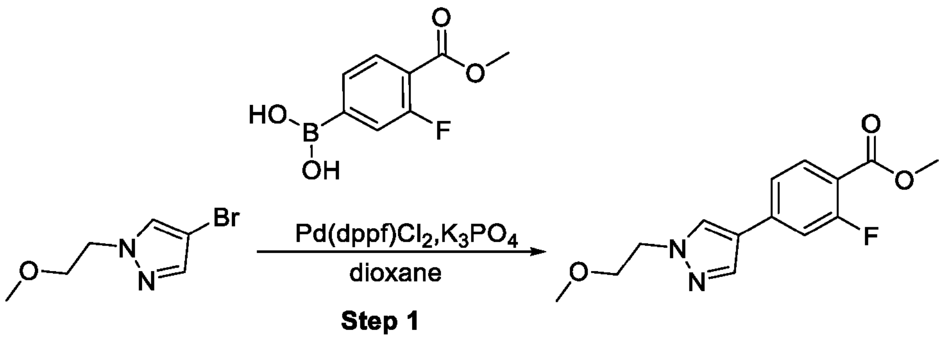


Step 1. Into a 50-mL round-bottom flask purged and maintained with an inert atmosphere of nitrogen, was placed 4-bromo-1-(2-methoxyethyl)pyrazole (1.00 g, 4.88 mmol, 1.00 equiv), 3-fluoro-4-(methoxycarbonyl)phenylboronic acid (1158.48 mg, 5.85 mmol, 1.20 equiv), Pd(dppf)$Cl_2$ (356.83 mg, 0.488 mmol, 0.10 equiv), $K_3PO_4$ (3105.50 mg, 14.63 mmol, 3.00 equiv), Dioxane (20.00 mL). The resulting solution was stirred for 5 h at 100 degrees C. The solids were filtered out. The resulting mixture was concentrated. The residue was applied onto a silica gel column with ethyl acetate/hexane (1:3). This resulted in 1.2 g (88.42%) of methyl 2-fluoro-4-[1-(2-methoxyethyl)pyrazol-4-yl]benzoate as a brown solid. **LCMS:** $[M+1]^+$=279.

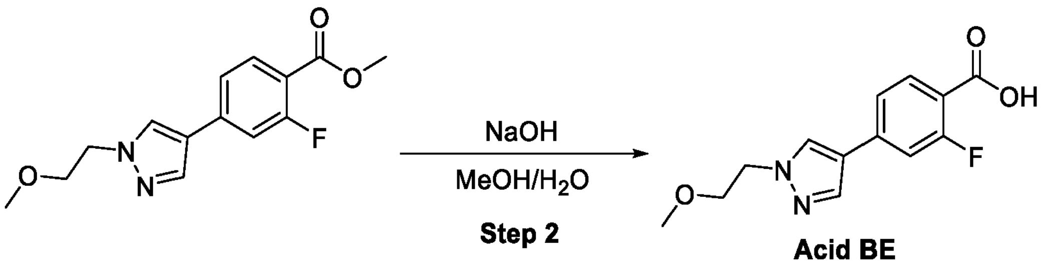


Acid BE

Step 2. Into a 50-mL round-bottom flask purged and maintained with an inert atmosphere of nitrogen, was placed methyl 2-fluoro-4-[1-(2-methoxyethyl)pyrazol-4-yl]benzoate (1.20 g, 4.31 mmol, 1.00 equiv), sodium hydroxide(344.95 mg, 0.008 mol, 2.00 equiv), MeOH (12.00 mL), $H_2O$ (3.00 mL). The resulting solution was stirred for 16 h at 25 °C. The pH value of the solution was adjusted to 3 with HCl (37 %). The resulting mixture was concentrated. This resulted in 1.7 g (crude) of 2-fluoro-4-[1-(2-methoxyethyl)pyrazol-4-yl]benzoic acid as a off-white solid. **LCMS:** $[M+1]^+$=265.

Preparation of Acid BG

Into a 50-mL round-bottom flask purged and maintained with an inert atmosphere of nitrogen, was placed methyl 5-bromopyridine-2-carboxylate (1.00 g, 4.629 mmol, 1.00 equiv), imidazole (630.26 mg, 9.258 mmol, 2.00 equiv), CuI (88.16 mg, 0.463 mmol, 0.1 equiv), 1,10-phenanthroline (166.83 mg, 0.926 mmol, 0.20 equiv), $K_2CO_3$ (1919.22 mg, 13.887 mmol, 3.00 equiv), DMF (20.00 mL). The resulting solution was stirred for 3 days at 75 °C. The solids were collected by filtration. The resulting solid was diluted with 20 mL of water. The pH value of the solution was adjusted to 5 with HCl (1M). The resulting mixture was concentrated. The crude product was purified by re-crystallization from MeOH. This resulted in 400 mg (crude) of 5-(imidazol-1-yl)pyridine-2-carboxylic acid as a off-white solid. LCMS: $[M+1]^+$=204.

Preparation of Acid BH

Into a 100-mL 3-necked round-bottom flask purged and maintained with an inert atmosphere of nitrogen, was placed 4-iodo-1-(triphenylmethyl)imidazole (1.00 g, 2.292 mmol, 1.00 equiv), methyl 5-(4,4,5,5-tetramethyl-1,3,2-dioxaborolan-2-yl)pyridine-2-carboxylate (0.90 g, 0.003 mmol, 1.50 equiv), Pd(dppf)$Cl_2$ (167.71 mg, 0.229 mmol, 0.10 equiv), $K_2CO_3$ (1.58 g, 11.432 mmol, 4.99 equiv), dioxane (20.00 mL), $H_2O$ (5.00 mL). The resulting solution was stirred for 20 h at room temperature. The solids were filtered out. The reaction was then quenched by the addition of 30 mL of water. The resulting solution was extracted with 3x20 mL of ethoxyethane and the aqueous layers combined. The pH value of the solution was adjusted to 3 with citric acid(aq). The solids were collected by filtration. This resulted in 352 mg (35.59%) of 5-[1-(triphenylmethyl)imidazol-4-yl]pyridine-2-carboxylic acid as a brown solid. **LCMS:** [M+H]=432.

Preparation of Acid BN

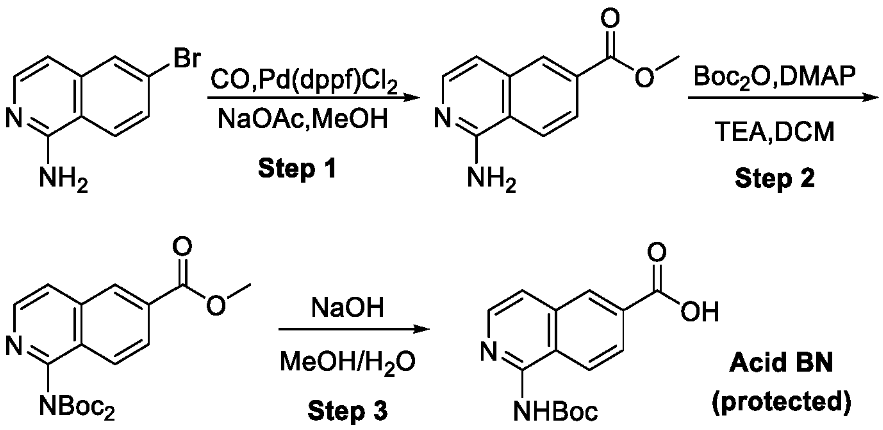


Step 1. Into a 50-mL pressure tank reactor purged and maintained with an inert atmosphere of nitrogen, was placed 6-bromoisoquinolin-1-amine (1.20 g, 5.379 mmol, 1.00 equiv), $CH_3OH$ (24.00 mL), Pd(dppf)$Cl_2$ (0.39 g, 0.000 mmol, 0.10 equiv), NaOAc (1.77 g, 21.576 mmol, 4.01 equiv), CO (10 atm). The resulting solution was stirred for 16 hr at 80 °C in an oil bath. The resulting mixture was concentrated under vacuum. The resulting solution was diluted with 30 mL of $H_2O$. The resulting solution was extracted with 3x30 mL of ethyl acetate and the organic layers combined. The resulting mixture was washed with 2x30 mL of brine. The mixture was dried over anhydrous sodium sulfate and concentrated under vacuum. The residue was applied onto a silica gel column with ethyl acetate/petroleum ether (1: 1). This resulted in 0.9 g (82.7%) of methyl 1-aminoisoquinoline-6-carboxylate as a light yellow solid. **LCMS:** $[M+H]^+$=203.

Step 2. Step 2. Into a 100-mL 3-necked round-bottom flask, was placed methyl 1-aminoisoquinoline-6-carboxylate (0.90 g, 4.45 mmol, 1.00 equiv), DCM (18.00 mL), $Boc_2O$ (2.43 g, 11.13 mmol, 2.50 equiv), TEA (1.80 g, 17.80 mmol, 4.00 equiv), DMAP (0.05 g, 0.45 mmol, 0.10 equiv). The resulting solution was stirred for 10 hr at room temperature. The resulting solution was diluted with 30 mL of $H_2O$. The resulting solution was extracted with 3x30 mL of dichloromethane and the organic layers combined and dried over anhydrous sodium sulfate and concentrated under vacuum. The residue was applied onto a silica gel column with ethyl acetate/petroleum ether (1:3). This resulted in 1 g (55.8%) of methyl 1-[bis(tert-butoxycarbonyl)amino]isoquinoline-6-carboxylate as brown oil. **LCMS:** $[M+H]^+$=403

Step 3. Into a 100-mL round-bottom flask, was placed methyl 1-[bis(tert-butoxycarbonyl)amino]isoquinoline-6-carboxylate (1.00 g, 2.49 mmol, 1.00 equiv), $CH_3OH$ (20.00 mL), $H_2O$ (7.00 mL), NaOH (0.30 g, 7.501 mmol, 3.02 equiv). The resulting solution was stirred for 16 hr at room temperature. The resulting solution was diluted with 20 mL of $H_2O$. The resulting mixture was concentrated under vacuum. The pH value of the solution was adjusted to 3 with HCl (3 mol/L). The resulting mixture was concentrated under vacuum. This resulted in 1 g (139.59%) of 1-[(tert-butoxycarbonyl)amino] isoquinoline-6-carboxylic acid as a light yellow solid. **LCMS:** $[M+H]^+$=289.

Preparation of Acid BO

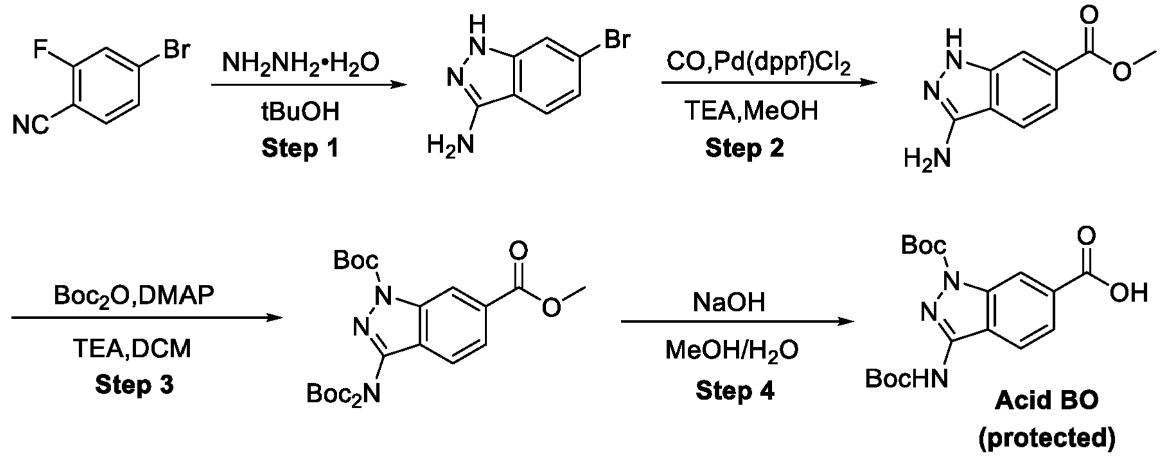


Step 1. Into a 250-mL 3-necked round-bottom flask purged and maintained with an inert atmosphere of nitrogen, was placed 4-bromo-2-fluorobenzonitrile (10.0 g, 50 mmol, 1.00 equiv), t-BuOH (100.00 mL), $NH_2NH_2.H_2O$ (7.51 g, 150.02 mmol, 3.00 equiv). The resulting solution was stirred for 12 hr at 110 °C in an oil bath. The reaction mixture was cooled. The solids were collected by filtration. This resulted in 6.5 g (61.3%) of 6-bromo-1H-indazol-3-amine as a white solid. **LCMS:** $[M+H]^+$=212.

Step 2. Into a 100-mL pressure tank reactor, was placed 6-bromo-1H-indazol-3-amine (6.50 g, 30.65 mmol, 1.00 equiv), $CH_3OH$ (65 mL), Pd(dppf)$Cl_2$ (2.24 g, 3.07 mmol, 0.10 equiv), triethylamine (9.31 g, 91.959 mmol, 3.00 equiv), CO(10 atm) . The resulting solution was stirred for 16 hr at 80 °C in an oil bath. The resulting mixture was concentrated. The resulting solution was diluted with 100 mL of $H_2O$. The resulting solution was extracted with 3x60 mL of ethyl acetate and the organic layers combined. The resulting mixture was washed with 2x60 mL of brine. The mixture was dried over anhydrous sodium sulfate and concentrated. The residue was applied onto a silica gel column with ethyl acetate/petroleum ether (1:1). This resulted in 3.5 g (59.7%) of methyl 3-amino-1H-indazole-6-carboxylate as a brown solid. **LCMS:** $[M+H]^+$=192.

Step 3. Into a 250-mL 3-necked round-bottom flask, was placed methyl 3-amino-1H-indazole-6-carboxylate (3.50 g, 18.31 mmol, 1.00 equiv), DCM (70.0 mL), $Boc_2O$ (11.99 g, 54.94 mmol, 3.00 equiv), triethylamine (7.41 g, 73.226 mmol, 4 equiv), DMAP (0.22 g, 1.831 mmol, 0.1 equiv). The resulting solution was stirred for 16 hr at room temperature. The resulting mixture was concentrated under vacuum. The resulting solution was diluted with 60 mL of $H_2O$. The resulting solution was extracted with 3x60 mL of ethyl acetate and the organic layers combined. The resulting mixture was washed with 2x60 mL of brine. The mixture was dried over anhydrous sodium sulfate and concentrated under vacuum. The residue was applied onto a silica gel column with ethyl acetate/petroleum ether (1:3). This resulted in 3 g (41.9%) of 1-tert-butyl 6-methyl 3-[(tert-butoxycarbonyl)amino]indazole-1,6-dicarboxylate as a brown solid. **LCMS:** $[M+H]^+$=492.

Step 4. Into a 100-mL round-bottom flask, was placed 1-tert-butyl 6-methyl 3-[(tert-butoxycarbonyl)amino] indazole-1,6-dicarboxylate (3.00 g, 7.67 mmol, 1.00 equiv), $CH_3OH$ (60.00 mL), $H_2O$ (20.00 mL), NaOH (0.92 g, 23.002 mmol, 3.00 equiv). The resulting solution was stirred for 12 hr at room temperature. The resulting mixture was concentrated under vacuum. The resulting solution was diluted with 20 mL of $H_2O$. The pH value of the solution was adjusted to 3 with HCl (3M). The resulting mixture was concentrated under vacuum. This resulted in 2 g of 1-(tert-butoxycarbonyl)-3-[(tert-butoxycarbonyl)amino]indazole-6-carboxylic acid as a brown solid. **LCMS:** $[M-H]^+$=376.

Preparation of Acid BP

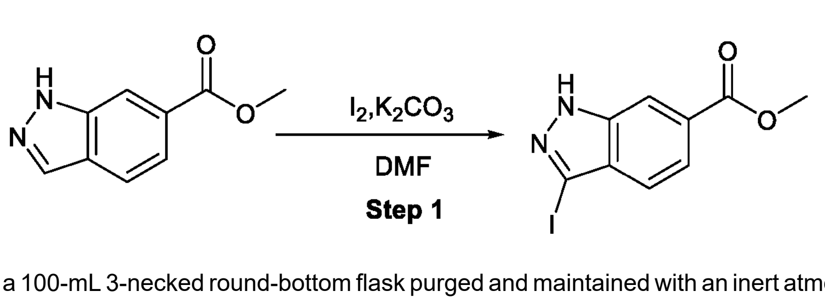


Step 1. Into a 100-mL 3-necked round-bottom flask purged and maintained with an inert atmosphere of nitrogen, was placed methyl 1H-indazole-6-carboxylate (2.00 g, 11.352 mmol, 1.00 equiv), DMF (40.00 mL), $K_2CO_3$ (3.14 g, 22.704 mmol, 2.00 equiv). The resulting solution was stirred at 0 °C in an ice/salt bath. Then the $I_2$ (24.32 g, 17.021 mmol, 1.50

equiv) was added into the flask. The resulting solution was allowed to react, with stirring, for 16 hr at room temperature. The resulting solution was diluted with 80 mL of $Na_2S_2O_3$. The solids were collected by filtration. This resulted in 3 g (87.48%) of methyl 3-iodo-1H-indazole-6-carboxylate as a yellow solid. **LCMS:** $[M+H]^+$=303.

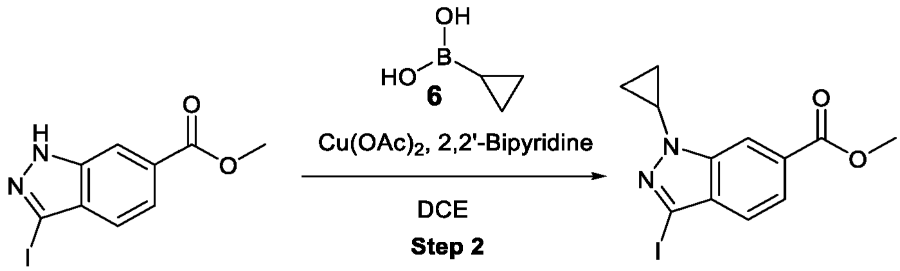


Step 2. Into a 100-mL 3-necked round-bottom flask purged and maintained with an inert atmosphere of nitrogen, was placed methyl 3-iodo-1H-indazole-6-carboxylate (3.00 g, 9.93 mmol, 1.00 equiv), DCE (60.00 mL), cyclopropylboronic acid (1.71 g, 19.86mmol, 2.00 equiv), $Cu(OAc)_2$ (1.80 g, 9.93 mmol, 1.00 equiv), 2-(pyridin-2-yl)pyridine (1.55 g, 9.92 mmol, 1.00 equiv). The resulting solution was stirred for 10 hr at 70 °C in an oil bath. The resulting solution was diluted with 90 mL of $H_2O$. The resulting solution was extracted with 3x60 mL of dichloromethane and the organic layers combined. The resulting mixture was washed with 2x60 mL of brine. The mixture was dried over anhydrous sodium sulfate and concentrated under vacuum. The residue was applied onto a silica gel column with ethyl acetate/petroleum ether (1:5). This resulted in 1 g (29.43%) of methyl 1-cyclopropyl-3-iodoindazole-6-carboxylate as a brown solid. **LCMS:** $[M+H]^+$=343.

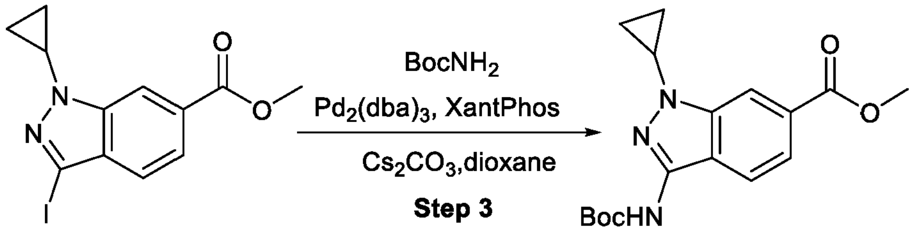


Step 3. Into a 40-mL vial purged and maintained with an inert atmosphere of nitrogen, was placed methyl 1-cyclopropyl-3-iodoindazole-6-carboxylate (900.0 mg, 2.63 mmol, 1.00 equiv), dioxane (18.0mL), tert-butyl carbamate (616.32 mg, 5.26 mmol, 2.00 equiv), $Pd_2(dba)_3$ (240.88 mg, 0.263 mmol, 0.10 equiv), XantPhos (304.41 mg, 0.53 mmol, 0.20 equiv), $Cs_2CO_3$ (2571.24 mg, 7.89 mmol, 3.00 equiv). The resulting solution was stirred for 20 hr at 100 °C in an oil bath. The resulting mixture was concentrated under vacuum. The resulting solution was diluted with 30 mL of $H_2O$. The resulting solution was extracted with 3x20 mL of ethyl acetate and the organic layers combined. The resulting mixture was washed with 2x30 mL of Brine. The mixture was dried over anhydrous sodium sulfate and concentrated under vacuum. The residue was applied onto a silica gel column with ethyl acetate/petroleum ether (1:3). This resulted in 190 mg (21.80%) of methyl 3-[(tert-butoxycarbonyl)amino]-1-cyclopropylindazole-6-carboxylate as a yellow solid. **LCMS:** $[M+H]^+$=332.

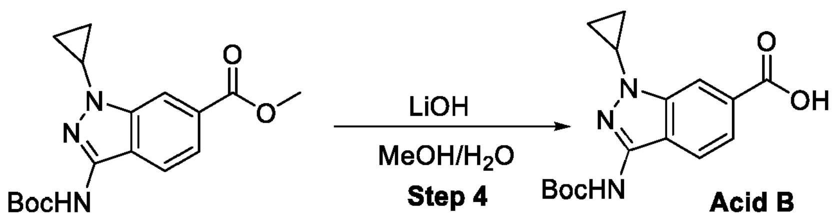


**Acid B (protected)**

Step 4. Into a 100-mL round-bottom flask, was placed methyl 3-[(tert-butoxycarbonyl)amino]-1-cyclopropylindazole-6-carboxylate (190.00 mg, 0.573 mmol, 1.00 equiv), $CH_3OH$ (3.80 mL), $H_2O$ (1.30 mL), lithium hydroxide (41.20 mg, 1.720 mmol, 3.00 equiv). The resulting solution was stirred for 12 hr at room temperature. The resulting solution was diluted with 10 mL of $H_2O$. The resulting mixture was concentrated under vacuum. The pH value of the solution was adjusted to 3 with HCl (3M). The resulting solution was extracted with 3x10 mL of ethyl acetate and the organic layers combined and dried over anhydrous sodium sulfate and concentrated under vacuum. This resulted in 150 mg (82.4%) of 3-[(tert-butoxycarbonyl)amino]-1-cyclopropylindazole-6-carboxylic acid as a light yellow solid. LCMS: $[M+H]^+$=318.

Preparation of Acid BQ

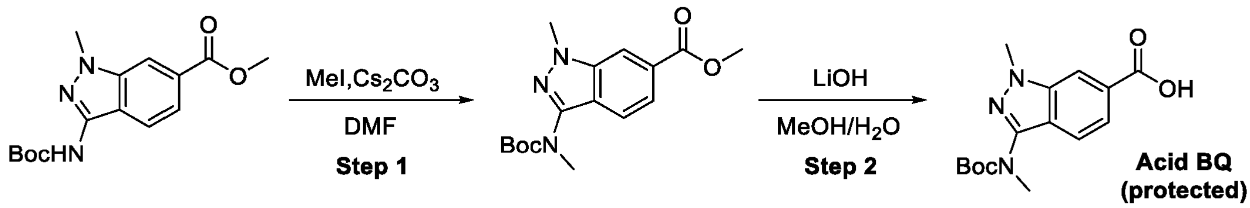


Step 1. Into a 8-mL round-bottom flask purged and maintained with an inert atmosphere of nitrogen, was placed methyl 3-[(tert-butoxycarbonyl)amino]-1-methylindazole-6-carboxylate (200.00 mg, 0.66 mmol, 1.00 equiv), $CH_3I$ (139.46 mg, 0.983 mmol, 1.50 equiv), $Cs_2CO_3$ (533.55 mg, 1.64 mmol, 2.50 equiv), DMF (3.00 mL). The resulting solution was stirred for 1 overnight at 25 °C. The reaction was then quenched by the addition of 10 mL of ice/salt. The resulting solution was extracted with 3x5 mL of ethyl acetate and the organic layers combined and concentrated. The residue was applied onto a silica gel column with ethyl acetate/hexane (1:3). This resulted in 120 mg (57.36%) of methyl 3-[(tert-butoxycarbonyl)(methyl)amino]-1-methylindazole-6-carboxylate as a brown solid. **LCMS:** $[M+1]^+$=320.

Step 2. Into a 8-mL vial purged and maintained with an inert atmosphere of nitrogen, was placed methyl 3-[(tert-butoxycarbonyl)(methyl)amino]-1-methylindazole-6-carboxylate (120.00 mg, 0.376 mmol, 1.00 equiv), lithium hydroxide (18.00 mg, 0.752 mmol, 2.00 equiv), MeOH (1.20 mL), $H_2O$ (0.30 mL). The resulting solution was stirred for 12 h at 25 °C.The resulting mixture was concentrated. This resulted in 110 mg (95.88%) of 3-[(tert-butoxycarbonyl)(methyl)amino]-1-methylindazole-6-carboxylic acid as a off-white solid. **LCMS:** $[M+1]^+$=306.

Preparation of Acid BR

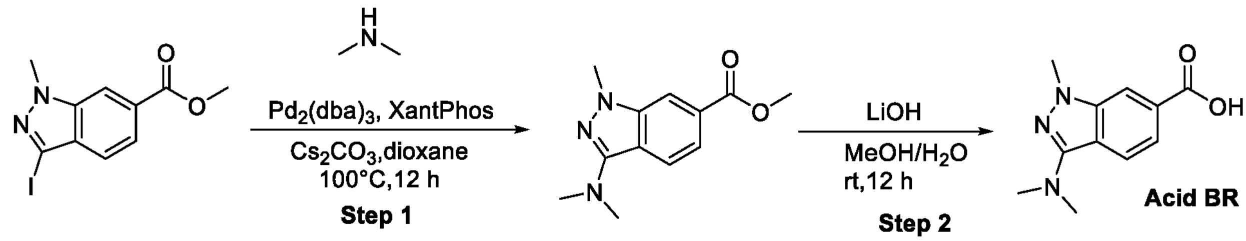


Step 1. Into a 40-mL vial purged and maintained with an inert atmosphere of nitrogen, was placed a solution of methyl 3-iodo-1-methylindazole-6-carboxylate (800 mg, 2.53 mmol, 1.00 equiv) in dioxane (20 mL), dimethylamine (2.53 mL, 5.06 mmol, 2.00 equiv), $Pd_2(dba)_3$ (23.18 mg, 0.025 mmol, 0.01 equiv), XantPhos (14.64 mg, 0.025 mmol, 0.01 equiv), $Cs_2CO_3$ (2473.81 mg, 7.59 mmol, 3.00 equiv). The resulting solution was stirred for 12 hr at 100 °C. The resulting mixture was concentrated under vacuum. The residue was applied onto a silica gel column with ethyl acetate/petroleum ether (1: 1). This resulted in 400 mg (67.8%) of methyl 3-(dimethylamino)-1-methylindazole-6-carboxylate as an yellow solid. **LCMS:** $[M+H]^+$=234.

Step 2. Into a 40-mL vial, was placed a solution of methyl 3-(dimethylamino)-1-methylindazole-6-carboxylate (400.00 mg, 1.715 mmol, 1.00 equiv) in MeOH (10 mL), a solution of lithium hydroxide (82.14 mg, 3.430 mmol, 2.00 equiv) in $H_2O$ (5 mL). The resulting solution was stirred for 16 hr at room temperature. The resulting mixture was concentrated under vacuum. HCl (1M) was employed to adjust the PH to 2. The crude product was purified by Flash-Prep-HPLC with C18 silica gel. This resulted in 200 mg (53.20%) of 3-(dimethylamino)-1-methylindazole-6-carboxylic acid as a yellow solid. **LCMS:** 220$[M+H]^+$.

Preparation of Acid BT

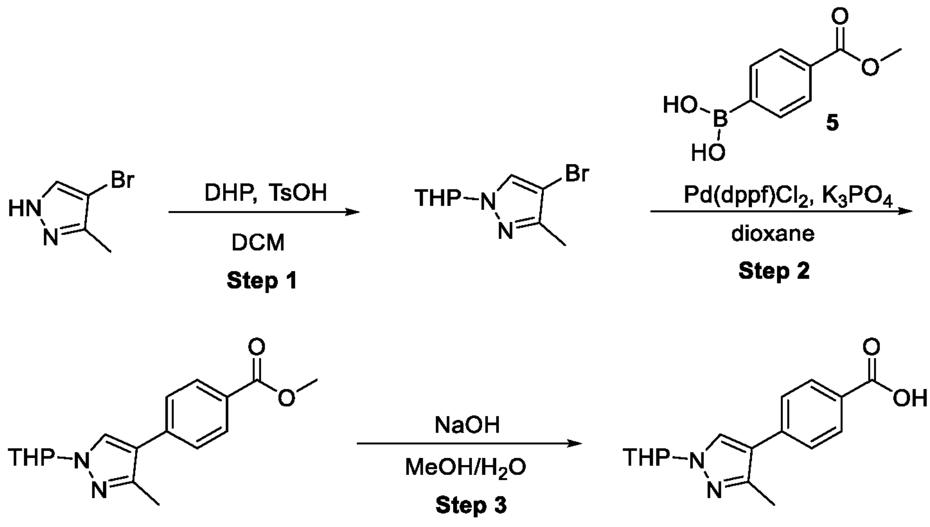


**Acid BT (protected)**

Step 1. Into a 250-mL 3-necked round-bottom flask purged and maintained with an inert atmosphere of nitrogen, was placed 4-bromo-3-methyl-1H-pyrazole (5.00 g, 31.06 mmol, 1.00 equiv), DCM (100.00 mL), DHP (3.92 g, 46.584 mmol, 1.50 equiv), TsOH (0.27 g, 1.568 mmol, 0.05 equiv). The resulting solution was stirred for 16 hr at room temperature. The resulting solution was diluted with 100 mL of $H_2O$. The resulting solution was extracted with 3x100 mL of dichloromethane and the organic layers combined. The resulting mixture was washed with 2x100 mL of brine. The mixture was dried over anhydrous sodium sulfate and concentrated under vacuum. This resulted in 6.5 g of 4-bromo-5-methyl-1-(oxan-2-yl)pyrazole as brown oil. **LCMS:** $[M+H]^+$=245.

Step 2. Into a 40-mL vial purged and maintained with an inert atmosphere of nitrogen, was placed 4-(methoxycarbonyl)phenylboronic acid (1.50 g, 8.34 mmol, 1.00 equiv), dioxane (24.00 mL), 4-bromo-3-methyl-1-(oxan-2-yl) pyrazole (2.04 g, 8.32mmol, 1.00 equiv), $Pd(dppf)Cl_2$ (0.61 g, 0.834 mmol, 0.10 equiv), $K_3PO_4$ (5.31 g, 25.02 mmol, 3.00 equiv). The resulting solution was stirred for 5 hr at 100 °C in an oil bath. The resulting mixture was concentrated under vacuum. The resulting solution was extracted with 3x30 mL of ethyl acetate and the organic layers combined. The resulting mixture was washed with 2 x30 mL of brine. The mixture was dried over anhydrous sodium sulfate and concentrated under vacuum. The residue was applied onto a silica gel column with ethyl acetate/petroleum ether (1: 1). This resulted in 1.3 g of methyl 4-[3-methyl-1-(oxan-2-yl)pyrazol-4-yl]benzoate as a brown solid. **LCMS:** $[M+H]^+$=301.

Step 3. Into a 100-mL round-bottom flask, was placed methyl 4-[3-methyl-1-(oxan-2-yl)pyrazol-4-yl]benzoate (1.30 g, 4.328 mmol, 1.00 equiv), $CH_3OH$ (26.00 mL), $H_2O$ (9.00 mL), sodium hydroxide (0.52 g, 12.984 mmol, 3.00 equiv). The resulting solution was stirred for 16 hr at room temperature. The resulting solution was extracted with 2x20 mL of ethyl acetate and the aqueous layers combined. The pH value of the solution was adjusted to 3 with HCl (3M). The solids were collected by filtration. This resulted in 0.9 g (72.6%) of 4-[3-methyl-1-(oxan-2-yl)pyrazol-4-yl]benzoic acid as a white solid. **LCMS:** $[M+H]^+$=287.

Preparation of Acid BU

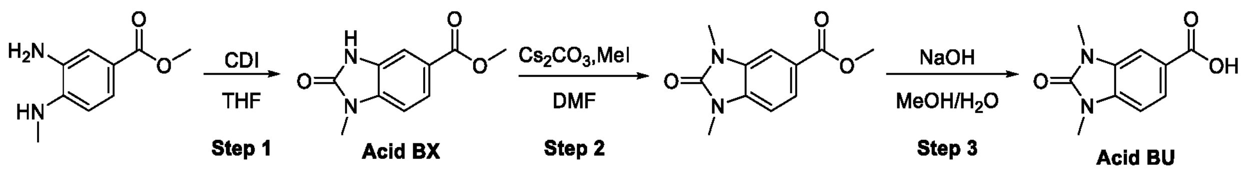


Step 1. Into a 50-mL round-bottom flask purged and maintained with an inert atmosphere of nitrogen, was placed THF (12.00 mL), methyl 3-amino-4-(methylamino)benzoate (600.00 mg, 3.33 mmol, 1.00 equiv), CDI (1619.62 mg, 9.99 mmol, 3.00 equiv). The resulting solution was stirred for 4 h at 70 °C. The resulting mixture was concentrated. The residue was applied onto a silica gel column with ethyl acetate/petroleum ether (1: 1). This resulted in 400 mg (58.26%) of methyl 1-methyl-2-oxo-3H-1,3-benzodiazole-5-carboxylate as a white solid. LCMS: $[M+1]^+$=207.

Step 2. Into a 8-mL vial purged and maintained with an inert atmosphere of nitrogen, was placed methyl 1-methyl-2-oxo-3H-1,3-benzodiazole-5-carboxylate (200.00 mg, 0.97 mmol, 1.00 equiv), dimethylformamide (4.00 mL), $Cs_2CO_3$ (632.04 mg, 1.940 mmol, 2.00 equiv), MeI (206.51 mg, 1.46mmol, 1.50 equiv). The resulting solution was stirred for 18 h at 25 °C. The reaction was then quenched by the addition of 15 mL of water/ice. The resulting solution was extracted with 3x4 mL of ethyl acetate and the organic layers combined. The resulting mixture was washed with 3 x4 mL of brine. This resulted in 180 mg (84.27%) of methyl 1,3-dimethyl-2-oxo-1,3-benzodiazole-5-carboxylate as a light brown solid. **LCMS:** $[M+1]^+$=221.

Step 3. Into a 8-mL vial purged and maintained with an inert atmosphere of nitrogen, was placed methyl 1,3-dimethyl-2-oxo-1,3-benzodiazole-5-carboxylate (180.00 mg, 0.817 mmol, 1.00 equiv), sodium hydroxide(65.38 mg, 1.634 mmol, 2.00 equiv), MeOH (1.60 mL), $H_2O$ (0.40 mL). The resulting solution was stirred for 16 h at 25 °C. The pH value of the solution was adjusted to 3 with HCl (37 %). The resulting mixture was concentrated. This resulted in 250 mg (crude) of 1,3-dimethyl-2-oxo-1,3-benzodiazole-5-carboxylic acid as a brown solid. **LCMS:** $[M+1]^+$=207.

Preparation of Acid BW

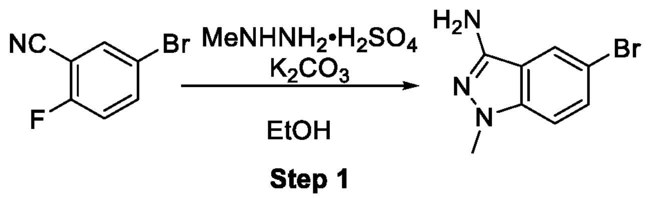


Step 1. To a stirred solution of methyl hydrazine; sulfuric acid (7.21 g, 49.998 mmol, 5 equiv) and $K_2CO_3$ (11.06 g, 79.996 mmol, 8 equiv) in EtOH (60.00 mL) was added methyl hydrazine; sulfuric acid (7.21 g, 49.998 mmol, 5 equiv) at 5 °C under nitrogen atmosphere. The resulting mixture was stirred for overnight at 80 °C under nitrogen atmosphere. The mixture was allowed to cool down to room temperature. The resulting mixture was concentrated under reduced pressure. The residue was dissolved in EtOAc (100 mL). The resulting mixture was washed with 3x20 mL of water. The resulting organic layer was dried over anhydrous $Na_2SO_4$. After filtration, the filtrate was concentrated under reduced pressure. The residue was purified by silica gel column chromatography, eluted with PE/EtOAc (2:3) to afford 5-bromo-1-methylindazol-3-amine (1.1 g, 48.66%) as a yellow solid. **LCMS:** $[M+1]^+$=226.

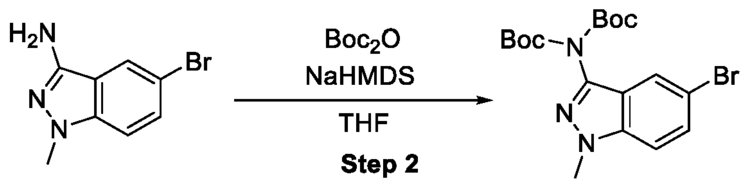


Step 2. To a stirred solution of 5-bromo-1-methylindazol-3-amine (0.95 g, 4.202 mmol, 1.00 equiv) and di-tert-butyl dicarbonate (2.29 g, 10.505 mmol, 2.50 equiv) in THF (30.00 mL)was added NaHMDS (2 mol/L, 5.67 mL, 11.345 mmol, 2.70 equiv) dropwise at 0 °C under nitrogen atmosphere. The resulting mixture was stirred for 4 h at 0 °C under nitrogen atmosphere. The reaction was quenched with Water at 0 °C. The mixture was allowed to warm to room temperature. The resulting mixture was extracted with EtOAc (3 x 30 mL). The combined organic layers were washed with brine (1x50 mL), dried over anhydrous $Na_2SO_4$. After filtration, the filtrate was concentrated under reduced pressure. The residue was purified by trituration with hexane/ethyl acetate=50/1 (50 mL). This resulted in tert-butyl N-(5-bromo-1-methylindazol-3-yl)-N-(tert-butoxycarbonyl)carbamate (1.5 g, 66.99%) as a light yellow solid. **LCMS:** $[M+1]^+$=426.

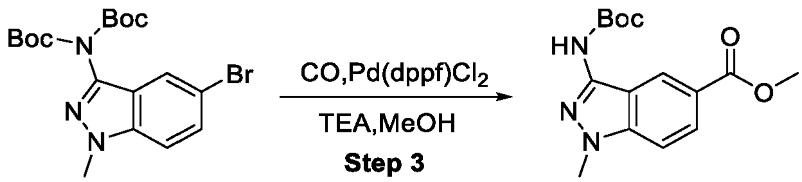


Step 3. Into a 30 mL pressure tank reactor were added tert-butyl N-(5-bromo-1-methylindazol-3-yl)-N-(tert-butoxycarbonyl)carbamate (500.0 mg, 1.173 mmol, 1.00 equiv), $Pd(dppf)Cl_2$ (85.82 mg, 0.117 mmol, 0.10 equiv), MeOH (10.00 mL) and triethylamine (593.40 mg, 5.865 mmol, 5.00 equiv) at room temperature. The resulting mixture was stirred

for overnight at 110 °C under CO(10 atm) atmosphere. The resulting mixture was concentrated under reduced pressure. The residue was purified by silica gel column chromatography, eluted with PE/EtOAc (1:1) to afford methyl 3-[(tert-butoxycarbonyl)amino]-1-methylindazole-5-carboxylate (290 mg, 80.98%) as a yellow solid. **LCMS:** $[M+1]^+$=306.

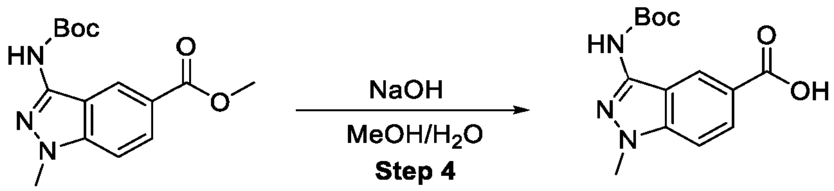


**Acid BW (protected)**

Step 4. Into a 25 mL round-bottom flask were added methyl 3-[(tert-butoxycarbonyl)amino]-1-methylindazole-5-carboxylate (290.00 mg, 0.950 mmol, 1.00 equiv), MeOH (5.00 mL), water (2.00 mL) and NaOH (151.95 mg, 3.800 mmol, 4.00 equiv) at room temperature. The resulting mixture was stirred for overnight at room temperature. The resulting mixture was concentrated under reduced pressure. The residue was dissolved in water (15 mL). The mixture was acidified to pH 5 with HCl (aq. 1M). The precipitated solids were collected by filtration and washed with water (2x10 mL). The resulting solid was dried under infrared light. This resulted in 3-[(tert-butoxycarbonyl)amino]-1-methylindazole-5-carboxylic acid (138 mg, 49.88%) as a light yellow solid. **LCMS:** $[M+1]^+$=292.

Preparation of Acid BY

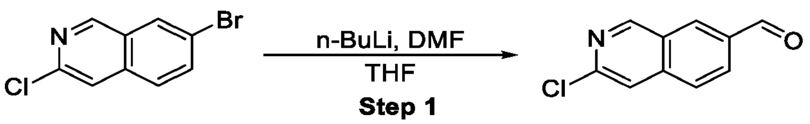


Step 1. Into a 100-mL 3-necked round-bottom flask purged and maintained with an inert atmosphere of nitrogen, was placed a solution of 7-bromo-3-chloroisoquinoline (1.00 g, 4.124 mmol, 1.00 equiv) in THF (20 mL). This was followed by the addition of n-BuLi (0.58 mL, 6.157 mmol, 1.49 equiv) dropwise with stirring at -78 °C. To this was added DMF (0.60 g, 8.209 mmol, 1.99 equiv) dropwise with stirring at -78 °C. The resulting solution was stirred for 30 min at -78 °C. The resulting solution was allowed to react, with stirring, for an additional 1hr at room temperature. The reaction was then quenched by the addition of 10 mL of water. The resulting solution was extracted with 3x30 mL of ethyl acetate dried over anhydrous sodium sulfate and concentrated under vacuum. The residue was applied onto a silica gel column with ethyl acetate/petroleum ether (1:5). This resulted in 230 mg (29.11%) of 3-chloroisoquinoline-7-carbaldehyde as a yellow solid. **LCMS:** $[M+H]^+$=192.

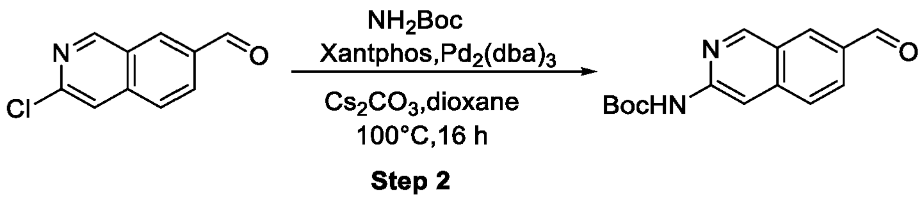


Step 2. Into a 100-mL 3-necked round-bottom flask purged and maintained with an inert atmosphere of nitrogen, was placed a solution of 3-chloroisoquinoline-7-carbaldehyde (230.00 mg, 1.200 mmol, 1.00 equiv) in dioxane (5 mL), tert-butyl carbamate (421.86 mg, 3.600 mmol, 3.00 equiv), Xantphos (69.45 mg, 0.120 mmol, 0.10 equiv), $Pd_2(dba)_3$ (109.92 mg, 0.120 mmol, 0.10 equiv), $Cs_2CO_3$ (782.20 mg, 2.400 mmol, 2.00 equiv). The resulting solution was stirred for 16 hr at 100 °C. The resulting solution was diluted with 100 mL of $H_2O$. The resulting solution was extracted with 3x20 mL of ethyl acetate The resulting mixture was washed with 3x20 ml of brine. The mixture was dried over anhydrous sodium sulfate and concentrated under vacuum. The residue was applied onto a silica gel column with ethyl acetate/petroleum ether (1: 1). This resulted in 100 mg (30.59%) of tert-butyl N-(7-formylisoquinolin-3-yl)carbamate as a light yellow solid. **LCMS:** $[M+H]^+$=273.

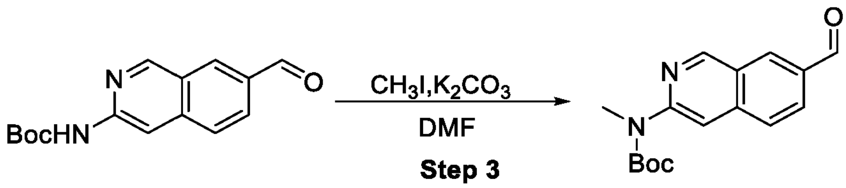


Step 3. Into a 8-mL vial purged and maintained with an inert atmosphere of nitrogen, was placed a solution of tert-butyl N-(7-formylisoquinolin-3-yl)carbamate (100.00 mg, 0.367 mmol, 1.00 equiv) in DMF (2 mL), $CH_3I$ (104.25 mg, 0.734 mmol, 2 equiv), $K_2CO_3$ (101.51 mg, 0.734 mmol, 2 equiv). The resulting solution was stirred for 12 hr at room temperature. The resulting solution was diluted with 30 mL of ethyl acetate. The resulting mixture was washed with 3x10 mL of brine. The mixture was dried over anhydrous sodium sulfate and concentrated under vacuum. This resulted in 52 mg (49.45%) of tert-butyl N-(7-formylisoquinolin-3-yl)-N-methylcarbamate as ayellow solid. **LCMS:** $[M+H]^+$=287.

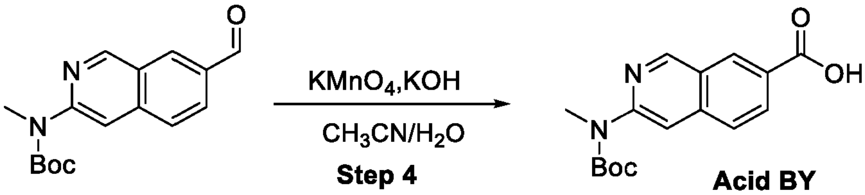


**Acid BY (protected)**

Step 4. Into a 8-mL vial, was placed a solution of tert-butyl N-(7-formylisoquinolin-3-yl)-N-methylcarbamate (52.00 mg, 0.182 mmol, 1.00 equiv) in $CH_3CN$ (5 mL), $KMnO_4$ (71.75 mg, 0.455 mmol, 2.50 equiv), a solution of KOH (30.57 mg, 0.546 mmol, 3.00 equiv) in $H_2O$ (5 mL). The resulting solution was stirred for 12 hr at room temperature. Ctric acide (3 mol/L) was employed to adjust the pH to 3-4. The resulting solution was extracted with 2x20 mL of dichloromethane dried over anhydrous sodium sulfate and concentrated under vacuum. This resulted in 21 mg (38.25%) of 3-[(tert-butoxycarbonyl)(methyl)amino]isoquinoline-7-carboxylic acid as a yellow solid. **LCMS:** $[M+H]^+$=303.

Preparation of Acid CA

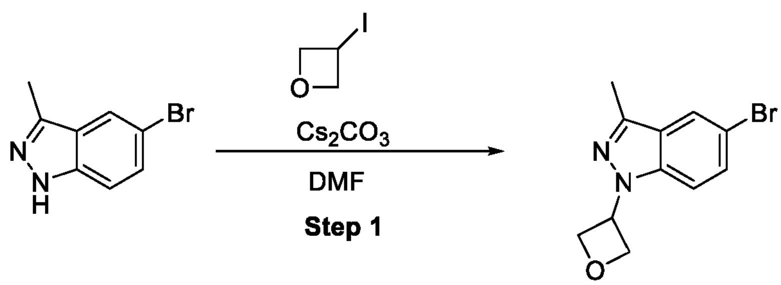


Step 1. Into a 50-mL round-bottom flask, was placed 5-bromo-3-methyl-1H-indazole (1.00 g, 4.738 mmol, 1.00 equiv), 3-iodooxetane (1.05 g, 0.000 mmol, 1.20 equiv), $Cs_2CO_3$ (3087.43 mg, 9.476 mmol, 2.00 equiv), DMF (20.00 mL). The resulting solution was stirred for 2 h at 100 °C in an oil bath. The solids were filtered out. The resulting mixture was concentrated. The residue was applied onto a silica gel column with ethyl acetate/petroleum ether (1:2). This resulted in 907 mg (71.66%) of 5-bromo-3-methyl-1-(oxetan-3-yl)indazole as a white solid. **LCMS:** [M+H]=267.

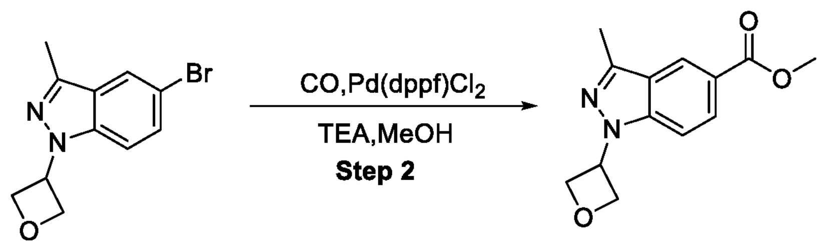

Step 2. Into a 50-mL pressure tank reactor was placed 5-bromo-3-methyl-1-(oxetan-3-yl)indazole (700.00 mg, 2.620 mmol, 1.00 equiv), Pd(dppf)$Cl_2$ $CH_2Cl_2$ (213.47 mg, 0.262 mmol, 0.10 equiv), triethylamine(1325.83 mg, 0.000 mmol, 5.00 equiv), MeOH (20.00 mL). The resulting solution was stirred for 16 h at room temperature under CO(3.0Mpa) atmosphere. The solids were filtered out. The resulting mixture was concentrated. The residue was applied onto a silica gel column with ethyl acetate/petroleum ether (1:1). This resulted in 380 mg (58.88%) of methyl 3-methyl-1-(oxetan-3-yl) indazole-5-carboxylate as a brown solid. **LCMS:** [M+H]=247.

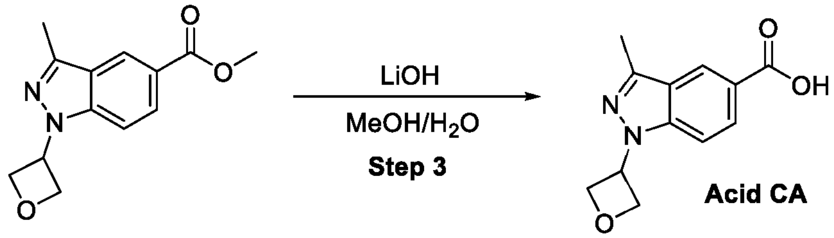


Step 3. Into a 50-mL round-bottom flask, was placed methyl 3-methyl-1-(oxetan-3-yl)indazole-5-carboxylate (380.00 mg, 1.543 mmol, 1.00 equiv), LiOH (147.81 mg, 6.172 mmol, 4.00 equiv), MeOH (10.00 mL), $H_2O$ (2.00 mL). The resulting solution was stirred for 16 h at room temperature. The resulting mixture was concentrated. The reaction was then quenched by the addition of 10 mL of water/ice. The pH value of the solution was adjusted to 3 with citric acid(aq). The solids were collected by filtration. This resulted in 323 mg (90.13%) of 3-methyl-1-(oxetan-3-yl)indazole-5-carboxylic acid as a white solid. **LCMS:** [M+H]=233.

Preparation of Acid CB

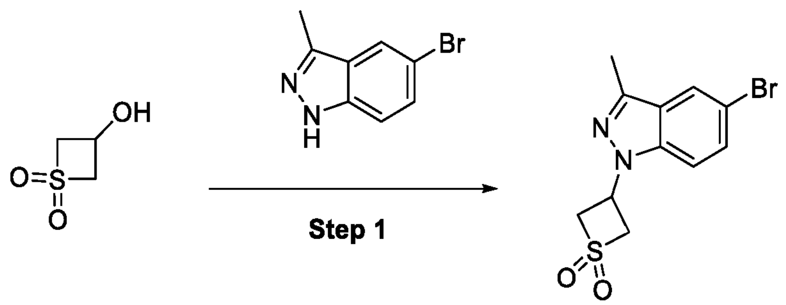


Step 1. Into a 50-mL round-bottom flask, was placed 3-hydroxythietane 1,1-dioxide (600.00 mg, 4.912 mmol, 1.00 equiv), MsCl (675.26 mg, 5.894 mmol, 1.20 equiv), DCM (5.00 mL), TEA (1491.26 mg, 14.736 mmol, 3.00 equiv). The resulting solution was stirred for 1 h at room temperature. Into a 50-mL round-bottom flask, was placed the crude thietane 1,1-dioxide 3-methanesulfonate in DCM, 5-bromo-3-methyl-1H-indazole (933.14 mg, 4.421 mmol, 0.90 equiv), $Cs_2CO_3$ (4801.67 mg, 14.736 mmol, 3.00 equiv), DMF (5.00 mL). The resulting solution was stirred for 16 h at room temperature. The reaction was then quenched by the addition of 50 mL of water. The resulting solution was extracted with 2x20 mL of ethyl acetate and the organic layers combined and concentrated. This resulted in 820 mg (52.96%) of 3-(5-bromo-3-methyl-1H-indazol-1-yl)thietane 1,1-dioxide as a light brown solid. **LCMS:** [M+H]=315.

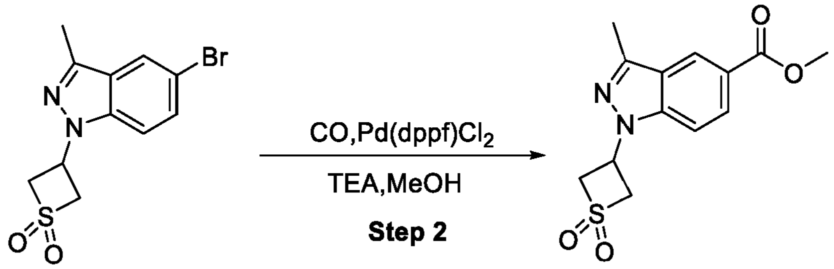


Step 2. Into a 50-mL pressure tank reactor, was placed 3-(5-bromo-3-methyl-1H-indazol-1-yl)thietane 1,1-dioxide (500.00 mg, 1.586 mmol, 1.00 equiv), TEA (802.61 mg, 7.930 mmol, 5.00 equiv), MeOH (10.00 mL), Pd(dppf)$Cl_2$

$CH_2Cl_2$ (129.23 mg, 0.159 mmol, 0.10 equiv). The resulting solution was stirred for 3 h at 100 °C under CO(3MPa) atmosphere. The solids were filtered out. The resulting mixture was concentrated. The residue was applied onto a silica gel column with ethyl acetate/hexane (1:3). This resulted in 360 mg (77.10%) of methyl 1-(1,1-dioxidothietan-3-yl)-3-methyl-1H-indazole-5-carboxylate as a brown solid. **LCMS:** [M+H]=295.

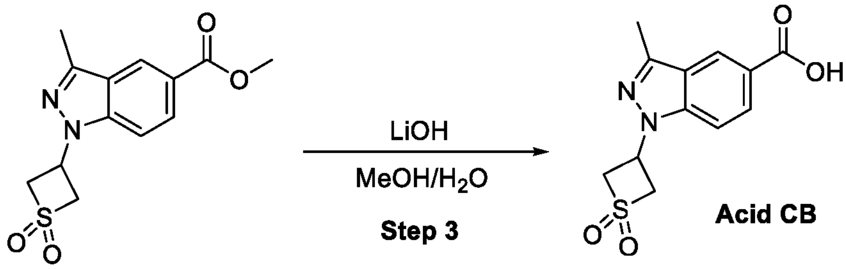


Step 3. Into a 50-mL round-bottom flask, was placed methyl 1-(1,1-dioxidothietan-3-yl)-3-methylindazole-5-carboxylate (360.00 mg, 1.223 mmol, 1.00 equiv), LiOH (58.58 mg, 2.446 mmol, 2.00 equiv), MeOH (10.00 mL), $H_2O$ (2.00 mL). The resulting solution was stirred for 16 h at room temperature. The resulting mixture was concentrated. The pH value of the solution was adjusted to 3 with citric acid(aq). The solids were collected by filtration. This resulted in 247 mg (72.05%) of 1-(1,1-dioxidothietan-3-yl)-3-methylindazole-5-carboxylic acid as a white solid. **LCMS:** [M+H]=281.

Preparation of Acid CC

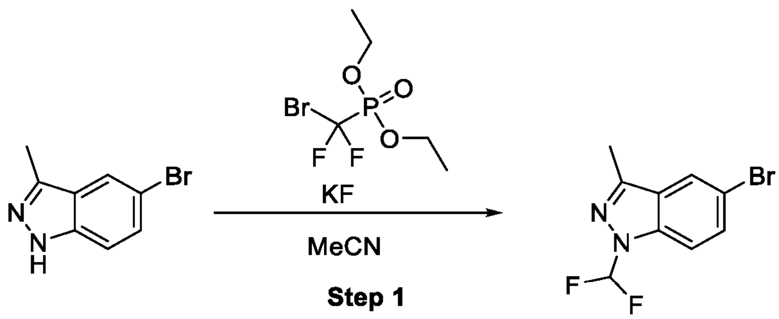


Step 1. Into a 100-mL 3-necked round-bottom flask, was placed 5-bromo-3-methyl-1H-indazole (1.00 g, 4.74 mmol, 1.00 equiv), diethyl bromodifluoromethylphosphonate (1265.06 mg, 1.00 equiv), MeCN (35.00 mL), KF (550.52 mg, 9.476 mmol, 2.00 equiv). The resulting solution was stirred for 3 h at room temperature. The solids were filtered out. The resulting mixture was concentrated. The residue was applied onto a silica gel column with ethyl acetate/petroleum ether (1:1). This resulted in 1.0 g (80.84%) of 5-bromo-1-(difluoromethyl)-3-methylindazole as a white solid. LCMS: [M+H]=261.

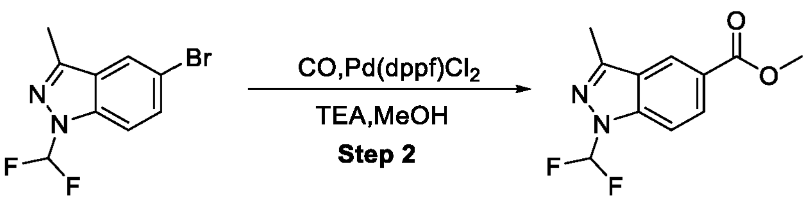


Step 2. Into a 50-mL pressure tank reactor, was placed 5-bromo-1-(difluoromethyl)-3-methylindazole (550.00 mg, 2.107 mmol, 1.00 equiv), triethylamine (1065.89 mg, 0.000 mmol, 5.00 equiv), MeOH (20.00 mL), $Pd(dppf)Cl_2.CH_2Cl_2$ (171.62 mg, 0.211 mmol, 0.10 equiv). The resulting solution was stirred for 16 h at 105 °C under CO(3.0Mpa) atmosphere. The solids were filtered out. The resulting mixture was concentrated. The residue was applied onto a silica gel column with ethyl acetate/petroleum ether (1:1). This resulted in 420 mg (83.00%) of methyl 1-(difluoromethyl)-3-methylindazole-5-carboxylate as a brown solid. **LCMS:** [M+H]=241.

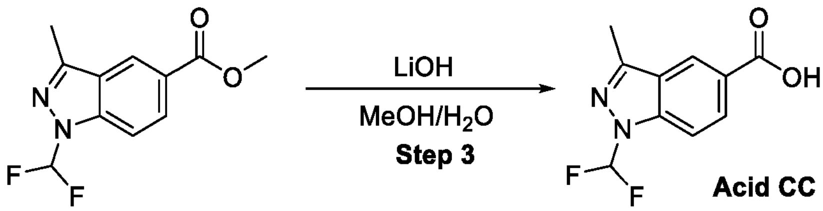


Step 3. Into a 50-mL round-bottom flask, was placed methyl 1-(difluoromethyl)-3-methylindazole-5-carboxylate (420.00 mg, 1.748 mmol, 1.00 equiv), MeOH (10.00 mL), $H_2O$ (1.00 mL), LiOH (125.62 mg, 5.24 mmol, 3.00 equiv). The resulting solution was stirred for 16 h at room temperature. The resulting mixture was concentrated. The pH value of the solution was adjusted to 3 with citric acid(aq). The solids were collected by filtration. This resulted in 343 mg (86.73%) of 1-(difluoromethyl)-3-methylindazole-5-carboxylic acid as a light brown solid. **LCMS:** [M+H]=227.

Preparation of Acid CE

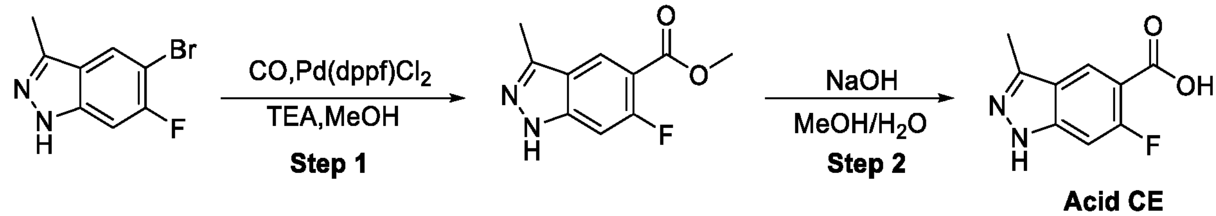


Step 1. Into a 50-mL pressure tank reactor, was placed 5-bromo-6-fluoro-3-methyl-1H-indazole (550.0 mg, 2.4 mmol, 1.00 equiv), MeOH (20.0 mL), $Pd(dppf)Cl_2$ (175.7 mg, 0.24 mmol, 0.10 equiv), TEA (971.9 mg, 9.61 mmol, 4.00 equiv), CO (10 atm). The resulting solution was stirred for 16 hr at 80 °C. The resulting mixture was concentrated under vacuum. The resulting solution was diluted with 50 mL of $H_2O$. The resulting solution was extracted with 3x20 mL of ethyl acetate and the organic layers combined. The resulting mixture was washed with 3x20 mL of brine. The mixture was dried over anhydrous sodium sulfate and concentrated under vacuum. The residue was applied onto a silica gel column with ethyl acetate/petroleum ether (1:1). This resulted in 280 mg (56%) of methyl 6-fluoro-3-methyl-1H-indazole-5-carboxylate as an orange solid. **LCMS:** $[M+H]^+$=209.

Step 2. Into a 50-mL round-bottom flask, was placed methyl 6-fluoro-3-methyl-1H-indazole-5-carboxylate (280.0 mg, 1.35 mmol, 1.00 equiv), $CH_3OH$ (9.00 mL), $H_2O$ (3.00 mL), sodium hydroxide (161.38 mg, 4.0 mmol, 3.0 equiv). The resulting solution was stirred for 16 hr at room temperature. The resulting solution was diluted with 20 mL of $H_2O$. The resulting solution was extracted with 2x20 mL of ethyl acetate and the aqueous layers combined. The pH value of the solution was adjusted to 3 with HCl (3 mol/L). The solids were collected by filtration. This resulted in 210 mg of 6-fluoro-3-methyl-1H-indazole-5-carboxylic acid as a off-white solid. **LCMS:** $[M+H]^+$=195.

Preparation of Acid CF

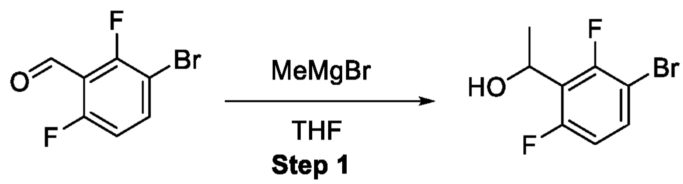


Step 1. Into a 250-mL 3-necked round-bottom flask purged and maintained with an inert atmosphere of nitrogen, was placed 3-bromo-2,6-difluorobenzaldehyde (5.5 g, 24.89 mmol, 1.00 equiv), tetrahydrofuran (110.0 mL).MeMgBr(in $Et_2O$) (20.70 mL, 62.2 mmol, 2.50 equiv) was added and the resulting solution was stirred for 30 min at -75 °C. The resulting solution was allowed to react, with stirring, for an additional 2.5 h at room temperature. The reaction was then quenched by the addition of 300 mL of water/ice. The resulting solution was extracted with 3x100 mL of ethyl acetate and the organic layers combined. The resulting mixture was washed with 3 x100 mL of brine. The resulting solution was concentrated. This

resulted in 5.5 g (93.23%) of 1-(3-bromo-2,6-difluorophenyl)ethanol as a light yellow oil. **LCMS:** $[M+1]^+$= 237.

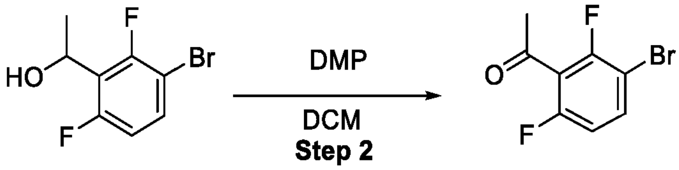


Step 2. Into a 250-mL 3-necked round-bottom flask purged and maintained with an inert atmosphere of nitrogen, was placed 1-(3-bromo-2,6-difluorophenyl)ethanol (5.50 g, 23.202 mmol, 1.00 equiv), DCM (110.0 mL), DMP (14.76 g, 34.80 mmol, 1.50 equiv). The resulting solution was stirred for 18 h at room temperature. The reaction was then quenched by the addition of 250 mL of saturated $Na_2S_2O_3$. The resulting solution was extracted with 3x100 mL of ethyl acetate and the organic layers combined. The resulting mixture was washed with 2 x100 ml of saturated $NaHCO_3$. The resulting mixture was washed with 2x100 mL of brine. The resulting mixture was concentrated. The residue was applied onto a silica gel column with PE/THF (1:3). This resulted in 5.5 g (100%) of 1-(3-bromo-2,6-difluorophenyl)ethanone as light yellow oil. **LCMS:** $[M+1]^+$= 235.

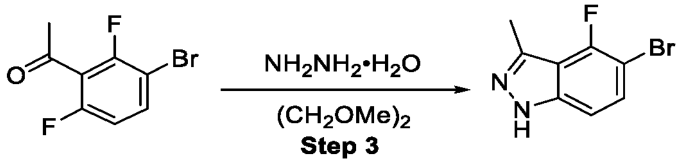


Step 3. Into a 250-mL 3-necked round-bottom flask purged and maintained with an inert atmosphere of nitrogen, was placed 1-(3-bromo-2,6-difluorophenyl)ethanone (5.50 g, 23.40 mmol, 1.00 equiv), DME (110.0 mL), $NH_2NH_2.H_2O$ (11.7 g, 0.23 mmol, 10.00 equiv). The resulting solution was stirred for 12 h at 90 °C. The reaction was then quenched by the addition of 500 mL of water/ice. The solids were collected by filtration. The residue was applied onto a silica gel column with ethyl acetate/petroleum ether (1:3).This resulted in 670 mg (12.50%) of 5-bromo-4-fluoro-3-methyl-1H-indazole as an off-white solid. **LCMS:** $[M+1]^+$= 229.

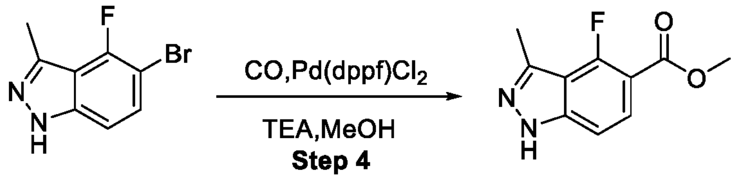


Step 4. Into a 50-mL sealed tube, was placed 5-bromo-4-fluoro-3-methyl-1H-indazole (600.0 mg, 2.62mmol, 1.00 equiv), Pd(dppf)$Cl_2$ (383 mg, 0.52 mmol, 0.20 equiv), TEA (795.20 mg, 7.86 mmol, 3.00 equiv), MeOH (20.00 mL), CO(5atm) .The resulting solution was stirred for 16 h at 80 °C. The resulting mixture was concentrated. The residue was applied onto a silica gel column with ethyl acetate/petroleum ether (1:3). This resulted in 450 mg (83%) of methyl 4-fluoro-3-methyl-1H-indazole-5-carboxylate as a brown solid. **LCMS:** $[M+1]^+$= 209.

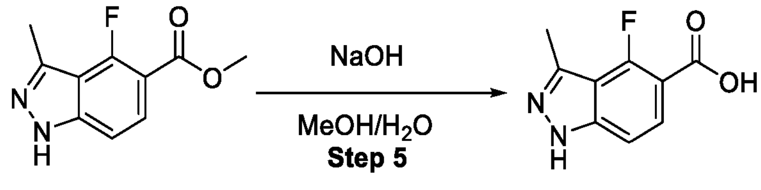


**Acid CF**

Step 5. Into a 50-mL round-bottom flask purged and maintained with an inert atmosphere of nitrogen, was placed methyl 4-fluoro-3-methyl-1H-indazole-5-carboxylate (450.00 mg, 2.161 mmol, 1.00 equiv), caustic soda (172.90 mg, 4.323 mmol, 2.00 equiv), MeOH (8.00 mL), $H_2O$ (2.00 mL). The resulting solution was stirred for 16 h at room temperature. The pH value of the solution was adjusted to 3 with HCl (37 %). The resulting mixture was concentrated. This resulted in 600 mg (crude) of 4-fluoro-3-methyl-1H-indazole-5-carboxylic acid as a brown solid. **LCMS:** $[M+1]^+$= 195.

Preparation of Acid CH

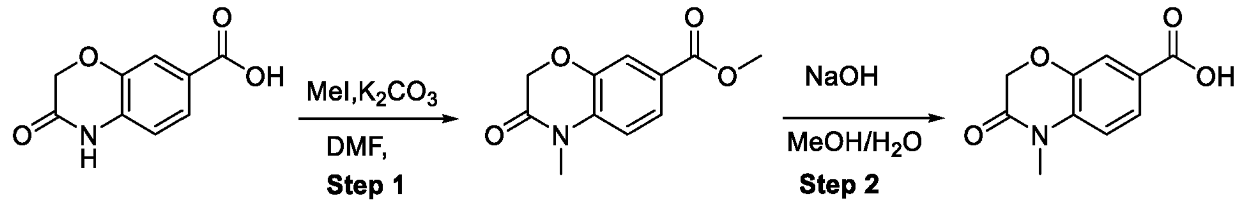


**Acid CH**

Step 1. Into a 50-mL round-bottom flask, was placed 3-oxo-2,4-dihydro-1,4-benzoxazine-7-carboxylic acid (CAS: 214848-62-1, 500.0 mg, 2.59 mmol, 1.00 equiv), MeI (808.32 mg, 5.696 mmol, 2.20 equiv), K2CO3 (1073.26 mg, 7.767 mmol, 3.00 equiv), DMF (10.00 mL). The resulting solution was stirred for 10h at room temperature. The solids were filtered out. The resulting mixture was concentrated. The residue was applied onto a silica gel column with ethyl acetate/petroleum ether (1:2). This resulted in 373 mg (65%) of methyl 4-methyl-3-oxo-2H-1,4-benzoxazine-7-carboxylate as a yellow solid. **LCMS:** $[M+H]^+$=222.

Step 2. Into a 100-mL round-bottom flask, was placed methyl 4-methyl-3-oxo-2H-1,4-benzoxazine-7-carboxylate (373.00 mg, 1.686 mmol, 1.00 equiv), NaOH (134.88 mg, 3.372 mmol, 2.00 equiv), MeOH (5.00 mL) and $H_2O$ (1.00 mL, 0.056 mmol, 0.03 equiv). The resulting solution was stirred for 5 h at room temperature. The resulting mixture was concentrated. The pH value of the solution was adjusted to 5 with citric. acid. The solids were collected by filtration. This resulted in 277 mg (79.3%) of 4-methyl-3-oxo-2H-1,4-benzoxazine-7-carboxylic acid as an off-white solid. **LCMS:** $[M+H]^+$=208.

Preparation of Acid CI

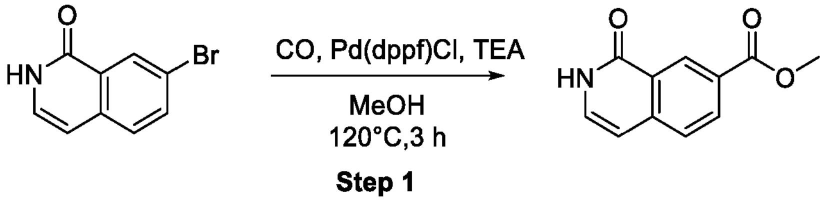


Step 1. Into a 50-mL pressure tank reactor, was placed 7-bromo-2H-isoquinolin-1-one (200.00 mg, 0.893 mmol, 1.00 equiv), MeOH (5.0 mL), TEA (270.98 mg, 2.678 mmol, 3 equiv), Pd(dppf)$Cl_2$ (32.66 mg, 0.045 mmol, 0.05 equiv). This was followed by the addition of CO (5 atm). The resulting solution was stirred for 3h at 120 °C. The solids were filtered out. The resulting mixture was concentrated. This resulted in 120 mg (66.16%) of methyl 1-oxo-2H-isoquinoline-7-carboxylate as a brown solid. **LCMS:** $[M+1]^+$=204.

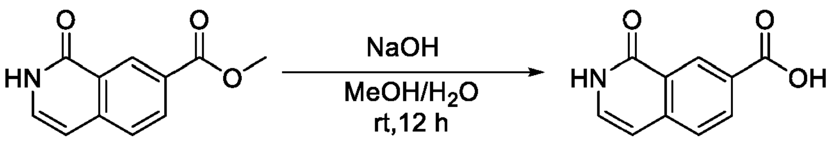


**Step 2** **Acid CI**

Step 2. Into a 50-mL round-bottom flask purged and maintained with an inert atmosphere of nitrogen, was placed methyl 1-oxo-2H-isoquinoline-7-carboxylate (120.0 mg, 0.591 mmol, 1.00 equiv), $H_2O$ (3.0 mL), MeOH (3.0 mL) and sodium hydroxide (94.5 mg, 2.362 mmol, 4.00 equiv). The resulting solution was stirred for 12h at room temperature. The resulting mixture was concentrated. The pH value of the solution was adjusted to 2-3 with 1M HCl. The solids were collected by filtration. This resulted in 80 mg (72%) of 1-oxo-2H-isoquinoline-7-carboxylic acid as a light yellow solid. **LCMS:** $[M+1]^+$=190.

Preparation of Acid CJ

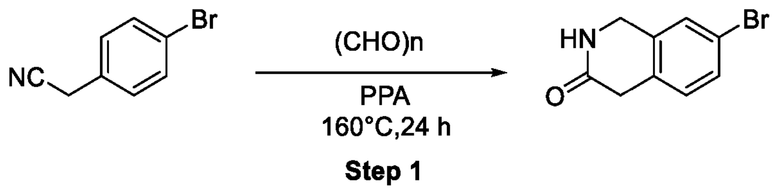


Step 1. Into a 40-mL round-bottom flask, was placed 4-bromophenylacetonitrile (1.10 g, 5.611 mmol, 1.00 equiv), Paraformaldehyde (556 mg, 6.172 mmol, 1.10 equiv), PPA (6.5g, 56.1 mmol, 10.00 equiv). The resulting solution was stirred for 24h at 160 °C. The resulting solution was diluted with 20 mL of $H_2O$. The pH value of the solution was adjusted to 7-8 with 1M $NaHCO_3$. The resulting solution was extracted with 3x20 mL of ethyl acetate and the organic layers combined and concentrated. The residue was applied onto a silica gel column with ethyl acetate/petroleum ether (1:1). This resulted in 600 mg (47%) of 7-bromo-2,4-dihydro-1H-isoquinolin-3-one as a yellow solid. LCMS: $[M+1]^+$=226.

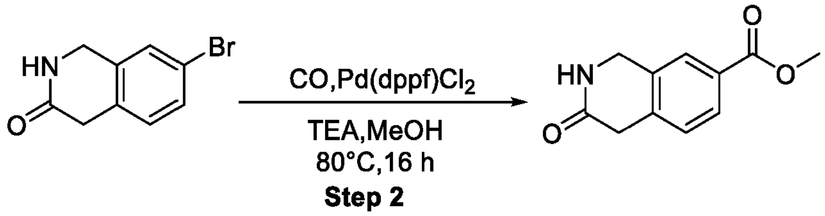


Step 2. Into a 50-mL round-bottom flask, was placed 7-bromo-2,4-dihydro-1H-isoquinolin-3-one (600.0 mg, 2.65 mmol, 1.00 equiv), MeOH (12.0 mL), TEA (805.7 mg, 7.962 mmol, 3.00 equiv), $Pd(dppf)Cl_2$ (97 mg, 0.133 mmol, 0.05 equiv), CO (5-atm). The resulting solution was stirred for 16h at 120 °C. The solids were filtered. The resulting mixture was concentrated. This resulted in 450 mg (82.62%) of methyl 3-oxo-2,4-dihydro-1H-isoquinoline-7-carboxylate as a dark brown solid. **LCMS:** $[M+1]^+$=206.

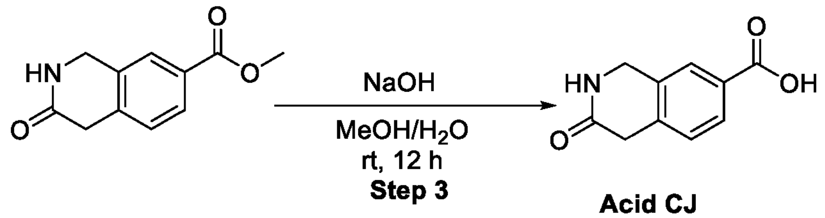


**Acid CJ**

Step 3. Into a 50-mL round-bottom flask purged and maintained with an inert atmosphere of nitrogen, was placed methyl 3-oxo-2,4-dihydro-1H-isoquinoline-7-carboxylate (450.0 mg, 2.19 mmol, 1.00 equiv), MeOH (9 mL), $H_2O$ (9 mL), sodium hydroxide (350.83 mg, 8.77 mmol, 4.00 equiv). The resulting solution was stirred for 12 hr at room temperature. The resulting mixture was washed with 1 x20 ml of DCM. The solids were filtered out. This resulted in 200 mg (47.71%) of 3-oxo-2,4-dihydro-1H-isoquinoline-7-carboxylic acid as a white solid. **LCMS:** $[M+1]^+$=192.

Preparation of Acid CK

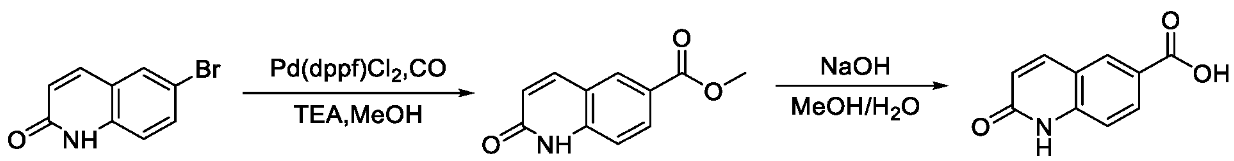


**CK-1** **CK-2** **Acid CK**

Methyl 2-oxo-1H-quinoline-6-carboxylate (CK-2)

Into a 50-mL pressure tank reactor, was placed 6-bromo-1H-quinolin-2-one (**CK-1**, 2.00 g, 8.926 mmol, 1.00 equiv), $CH_3OH$ (20.00 mL), Pd(dppf)$Cl_2$ (0.65 g, 0.888 mmol, 0.10 equiv), TEA (3.61 g, 35.675 mmol, 4.00 equiv), CO(10 atm) . The resulting solution was stirred for 16 hr at 80 °C in an oil bath. The resulting mixture was concentrated under vacuum. The resulting solution was diluted with 40 mL of $H_2O$. The resulting solution was extracted with 3 x 30 mL of ethyl acetate and the organic layers combined. The resulting mixture was washed with 2 x 30 mL of brine. The mixture was dried over anhydrous sodium sulfate and concentrated under vacuum. This resulted in 1.9 g (73.45%) of methyl 2-oxo-1H-quinoline-6-carboxylate (**CK-2**) as a red solid. **LCMS:** $[M+H]^+$=204.

2-oxo-1H-quinoline-6-carboxylic acid (Acid CK)

Into a 100-mL round-bottom flask, was placed methyl 2-oxo-1H-quinoline-6-carboxylate (1.90 g, 9.351 mmol, 1.00 equiv), $CH_3OH$ (38.00 mL), $H_2O$ (12.00 mL), sodium hydroxide (1.12 g, 28.002 mmol, 2.99 equiv). The resulting solution was stirred for 12 hr at room temperature. The resulting mixture was concentrated under vacuum. The resulting solution was diluted with 30 mL of $H_2O$. The resulting solution was extracted with 2 x 20 mL of ethyl acetate and the aqueous layers combined. The pH value of the solution was adjusted to 3 with HCl (3M). The solids were collected by filtration. This resulted in 1.2 g (67.84%) of 2-oxo-1H-quinoline-6-carboxylic acid (**Acid CK**) as a red solid. **LCMS:** $[M+H]^+$=190.

Preparation of Acid CL

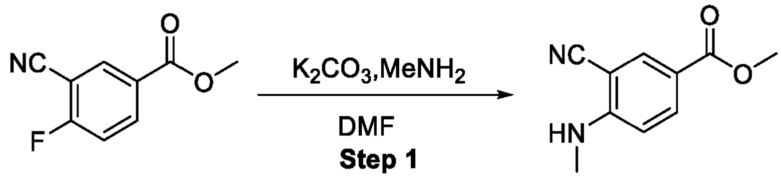


Step 1. Into a 100-mL 4-necked round-bottom flask, was placed a solution of methyl 3-cyano-4-fluorobenzoate (2.00 g, 11.164 mmol, 1.00 equiv) in DMF (30 mL), potassium carbonate (4662.45 mg, 33.491 mmol, 3.00 equiv), $CH_3NH_2HCl$ (2261.28 mg, 33.492 mmol, 3.00 equiv). The resulting solution was stirred for 12 hr at room temperature. The reaction was then quenched by the addition of 100 mL of water/ice. The solids were collected by filtration. This resulted in 1800 mg (84.77%) of methyl 3-cyano-4-(methylamino)benzoate as a white solid. **LCMS:** $[M+H]^+$=191.

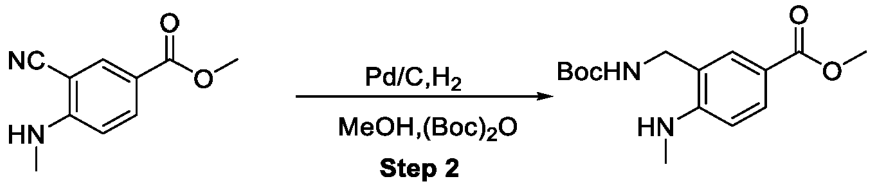


Step 2. Into a 250-mL pressure tank reactor purged and maintained with an inert atmosphere of nitrogen, was placed a solution of methyl 3-cyano-4-(methylamino)benzoate (1800.00 mg, 9.464 mmol, 1.00 equiv) in MeOH (50 mL), Pd/C (500.00 mg, 4.698 mmol, 0.50 equiv).$Boc_2O$(6196.22mg, 28.391mmol, 3 equiv). $H_2$(g) was introduced and the resulting solution was stirred for 5 hr at room temperature. The solids were filtered. The resulting mixture was concentrated under vacuum. This resulted in 1.67 g (59.95%) of methyl 3-[[(tert-butoxycarbonyl)amino]methyl]-4-(methylamino) benzoate as a yellow solid. **LCMS:** $[M+H]^+$=295.

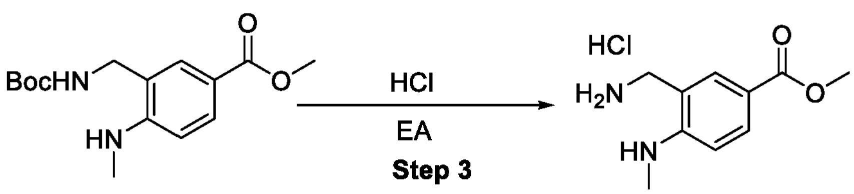

Step 3. Into a 100-mL round-bottom flask purged and maintained with an inert atmosphere of nitrogen, was placed methyl 3-[[(tert-butoxycarbonyl)amino]methyl]-4-(methylamino)benzoate (1.67 g, 5.67mmol, 1.00 equiv), hydrogen chloride in ethyl acetate(2M) (20 mL). The resulting solution was stirred for 5 hr at room temperature. The solids were collected by filtration. This resulted in 1.1 g (84.05%) of methyl 3-(aminomethyl)-4-(methylamino)benzoate hydrochloride as a yellow solid. **LCMS:** [M+H]$^+$=19.

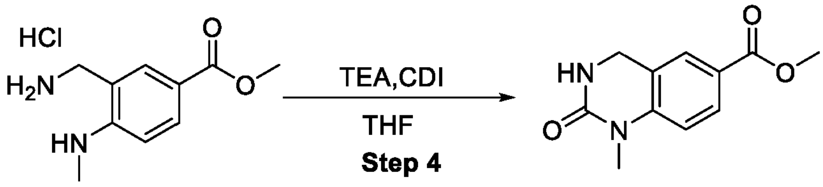


Step 4. Into a 50-mL round-bottom flask purged and maintained with an inert atmosphere of nitrogen, was placed a solution of methyl 3-(aminomethyl)-4-(methylamino)benzoate hydrochloride (1100.00 mg, 4.768 mmol, 1.00 equiv) in THF (10 mL), triethylamine(1447.51 mg, 14.304 mmol, 3.00 equiv), CDI (1159.76 mg, 7.152 mmol, 1.50 equiv). The resulting solution was stirred for 5 hr at room temperature. The reaction was then quenched by the addition of 30 mL of water. The resulting solution was extracted with 3x30 mL of ethyl acetate dried over anhydrous sodium sulfate and concentrated under vacuum. This resulted in 680 mg (64.8%) of methyl 1-methyl-2-oxo-3,4-dihydroquinazoline-6-carboxylate as a white solid. **LCMS:** [M+H]$^+$=221.

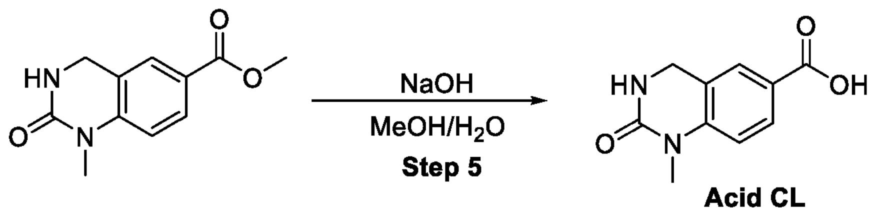


**Acid CL**

Step 5. Into a 20-mL vial purged and maintained with an inert atmosphere of nitrogen, was placed a solution of methyl 1-methyl-2-oxo-3,4-dihydroquinazoline-6-carboxylate (680.00 mg, 3.09 mmol, 1.00 equiv) in MeOH (5 mL), a solution of sodium hydroxide(247.00 mg, 6.18 mmol, 2.00 equiv) in $H_2O$ (5 mL). The resulting solution was stirred for 12 hr at room temperature. The resulting mixture was concentrated under vacuum. HCl (1M) was employed to adjust the pH to 2-3. The solids were collected by filtration. This resulted in 510 mg (80.10%) of 1-methyl-2-oxo-3,4-dihydroquinazoline-6-carboxylic acid as a white solid. **LCMS:** [M+H]$^+$=207.

Preparation of Acid CM

Into a 8-mL round-bottom flask, was placed methyl 3-oxo-2H-isoquinoline-7-carboxylate (CAS: 1416713-96-6, 100.0 mg, 0.492 mmol, 1.00 equiv), $H_2O$ (1 mL), MeOH (1 mL), sodium hydroxide (78.7 mg, 1.97 mmol, 4.00 equiv). The resulting solution was stirred for 12h at room temperature. The resulting solution was diluted with 10 mL of $H_2O$. The pH value of the solution was adjusted to 2-3 with 1M HCL. The solids were collected by filtration. This resulted in 70 mg (75%) of 3-oxo-2H-isoquinoline-7-carboxylic acid as a white solid. **LCMS:** [M+1]$^+$=190.

Preparation of Acid CN

Prepared in a similar fashion as Acid CK, except that **CK-1** is replaced with 6-bromoquinolin-2(1H)-one. **LCMS:** [M+1]$^+$=190.

The following amines used are depicted in **Table** C. Synthetic schemes are depicted in the specification.

Table C

| Amine | Structure |
|---|---|
| D | 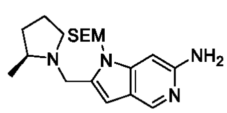 |

(continued)

| Amine | Structure |
|---|---|
| E | 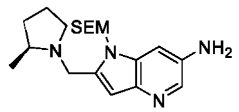 |
| F | 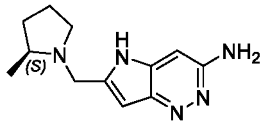 |

Synthesis of Amine D

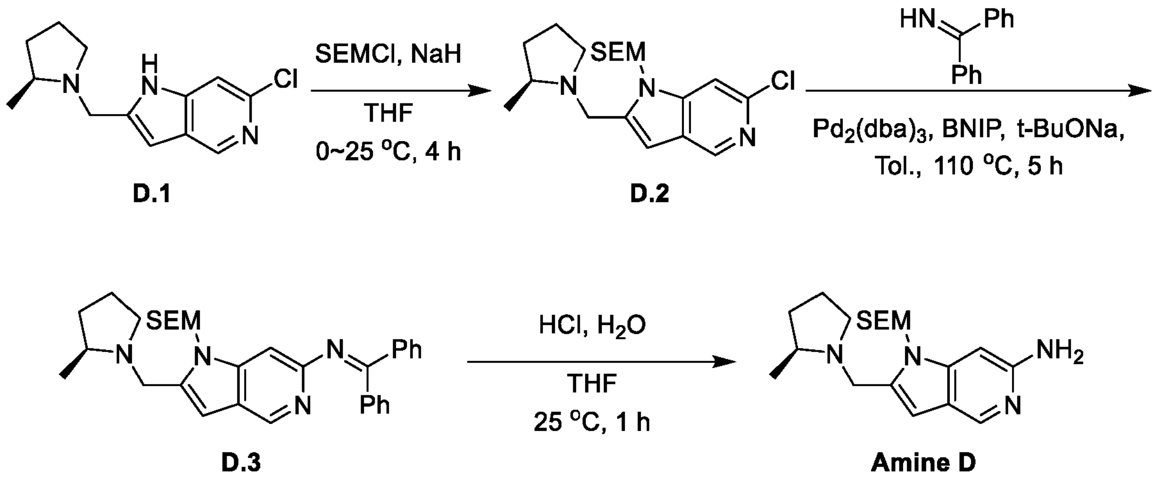


Scheme D

2-[[6-chloro-2-[[(2S)-2-methylpyrrolidin-1-yl]methyl]pyrrolo[3,2-c]pyridin-1-yl]methoxy]ethyl-trimethyl-silane (D.2)

To a solution of 6-chloro-2-[[(2S)-2-methylpyrrolidin-1-yl]methyl]-1H-pyrrolo[3,2-c] pyridine (1.30 g, 5.21 mmol, 1 *eq)* in THF (30 mL) was added NaH **(D.1,** 625 mg, 15.6 mmol, 60 wt% oil dispersion, 3 *eq)* at 0 °C. After stirring at 0 °C for 1 hr, SEM-Cl (868 mg, 5.21 mmol, 0.921 ml, 1 *eq*) was added at 0 °C. The resulting mixture was stirred at 25 °C for 3 hr. The reaction mixture was quenched by addition of $H_2O$ (10 ml). The mixture was extracted with EtOAc (10 ml x 3). The combined organic layers were washed with brine (10 ml x 2), dried over $Na_2SO_4$, and filtered. The solution was concentrated under reduced pressure. The residue was purified by column chromatography ($SiO_2$, petroleum ether/ethyl acetate gradient of 1:0 to 2:1) to furnish 2-[[6-chloro-2-[[(2S)-2-methylpyrrolidin-1-yl]methyl]pyrrolo[3,2-c]pyridin-1-yl] methoxy]ethyl-trimethylsilane.

N-[2-[[(2S)-2-methylpyrrolidin-1-yl]methyl]-1-(2-trimethylsilylethoxymethyl)pyrrolo[3,2-c]pyridin-6-yl]-1,1-diphenyl-methanimine (D.3)

A mixture of 2-[[6-chloro-2-[[(2S)-2-methylpyrrolidin-1-yl]methyl]pyrrolo[3,2-c]pyridin-1- yl]methoxy]ethyl-trimethylsilane (**D.1,** 0.600 g, 1.58 mmol, 1 eq), diphenylmethanimine (429 mg, 2.37 mmol, 0.397 mL, 1.5 eq), $Pd_2(dba)_3$ (145 mg, 0.158 mmol, 0.1 eq), BINAP (98 mg, 0.16 mmol, 0.1 eq), and t-BuONa (303 mg, 3.16 mmol, 2 eq) in toluene (15 mL) was degassed and purged with $N_2$ (3X). The mixture was stirred at 110 °C for 4 hr under a $N_2$ atmosphere. The reaction mixture was filtered, and the filtrate was concentrated under reduced pressure to furnish N-[2-[[(2S)-2-methylpyrrolidin-1-yl]methyl]-1-(2-trimethylsilylethoxymethyl)pyrrolo[3,2-c]pyridin-6-yl]-1,1-diphenyl-methanimine.

2-[[(2S)-2-methylpyrrolidin-1-yl]methyl]-1-(2-trimethylsilylethoxymethyl)pyrrolo[3,2-c]-pyridine-6-amine (Amine D)

To a solution of N-[2-[[(2S)-2-methylpyrrolidin-1-yl]methyl]-1-(2-trimethylsilylethoxy methyl)pyrrolo[3,2-c]pyridin-6-yl]-1,1-diphenyl-methanimine **(D.2,** 0.900 g, 1.72 mmol, 1 *eq)* in THF (10 mL) was added HCl (10 mL). The mixture was stirred at 25 °C for 1 hr. Water (15 mL) and EtOAc (15 mL) were added to the reaction mixture. The layers were separated, and the aqueous phase was adjusted to pH = 9-10 by addition of aqueous 1N NaOH. The aqueous layer was extracted with EtOAc (15 ml x 3). The combined organic layers were dried over $Na_2SO_4$, filtered, and concentrated under reduced pressure to furnish 2-[[(2S)-2-methylpyrrolidin-1-yl]methyl]-1-(2-trimethylsilylethoxymethyl)pyrrolo[3,2-c]pyridin-6-amine.

Synthesis of Amine E

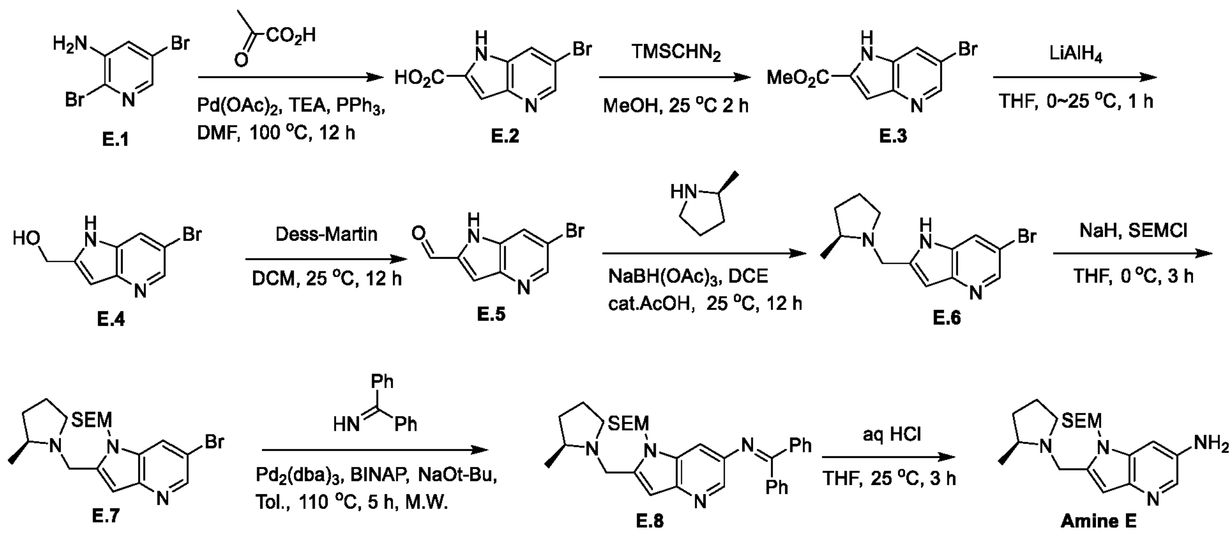


Scheme E

6-bromo-1H-pyrrolo[3,2-b]pyridine-2-carboxylic acid (E.2)

To a solution of 2,5-dibromopyridin-3-amine (5.00 g, 19.9 mmol, 1 *eq)* in DMF (120 mL) was added TEA (8.84 g, 87.3 mmol, 12.2 mL, 4.4 *eq*), $PPh_3$ (4.58 g, 17.5 mmol, 0.88 *eq),* $Pd(OAc)_2$ (980 mg, 4.37 mmol, 0.22 *eq),* and 2-oxopropanoic acid (4.63 g, 52.6 mmol, 3.71 mL, 2.65 *eq).* The mixture was stirred at 100 °C for 12 hr. The reaction mixture was concentrated under reduced pressure. The residue was diluted with $H_2O$ (50 mL) and EtOAc (50 mL). The solid was filtered and dried to furnish 6-bromo-1H-pyrrolo[3,2-b]pyridine-2-carboxylic acid. **$^1$H-NMR:** 400MHz, DMSO-d6: $\delta$ 12.17 (br s, 1H), 8.50 (d, *J*= 2.0 Hz, 1H), 8.00 (d, *J*= 1.3 Hz, 1H), 7.17 (d, *J* = 1.0 Hz, 1H).

Methyl 6-bromo-1H-pyrrolo[3,2-b]pyridine-2-carboxylate (E.3)

To a solution of 6-bromo-1H-pyrrolo[3,2-b]pyridine-2-carboxylic acid (4.00 g, 16.6 mmol, 1 *eq)* in MeOH (30 mL) and DCM (30 mL) was added $TMSCHN_2$ (2 M, 32.61 mL) dropwise. The mixture was stirred at 25 °C for 2 hr. Acetic acid (3 mL) was added to the mixture. The reaction mixture was filtered, and the solution was concentrated under reduced pressure to furnish methyl 6-bromo-1H-pyrrolo[3,2-b]pyridine-2-carboxylate. **$^1$H-NMR:** 400MHz, DMSO-d6: $\delta$ 12.36 (br s, 1H), 8.53 (d, *J* = 2.0 Hz, 1H), 8.03 (d, *J*= 1.4 Hz, 1H), 7.25 (s, 1H), 3.92 (s, 3H)

(6-bromo-1H-pyrrolo[3,2-b]pyridin-2-yl)methanol (E.4)

To a mixture of $LiAlH_4$ (565 mg, 14.9 mmol, 2 *eq)* in THF (25 mL) was added methyl 6-bromo-1H-pyrrolo[3,2-b]pyridine-2-carboxylate (1.90 g, 7.45 mmol, 1 *eq)* at 0°C. The mixture was warmed to 25°C and stirred at 25°C for 1hr. The reaction mixture was cooled to 0 °C. Aqueous NaOH (20 mL, 10%) and $H_2O$ (30 mL) were added. The mixture was stirred at 0 °C for 0.5 hr. The mixture was filtered and extracted with EtOAc (20 mL x 3). The organic layers were dried over $Na_2SO_4$

and filtered. The solution was concentrated under reduced pressure to furnish (6-bromo-1H-pyrrolo[3,2-b]pyridin-2-yl) methanol. **LCMS:** M+H$^+$:149.0

6-bromo-1H-pyrrolo[3,2-b]pyridine-2-carbaldehyde (E.5)

To a solution of (6-bromo-1H-pyrrolo[3,2-b]pyridin-2-yl)methanol (1.55 g, 6.83 mmol, 1 *eq)* in DCM (100 mL) was added Dess-Martin (8.69 g, 20.5 mmol, 3 *eq).* The mixture was stirred at 25 °C for 12 hr. The reaction was diluted with saturated aq. $NaHCO_3$ (50 mL) and extracted with EtOAc (20 mL x 3). The combined organic layers were washed with brine (50 mL), dried over $Na_2SO_4$, and filtered. The solution was concentrated under reduced pressure to give 6-bromo-1H-pyrrolo[3,2-b]pyridine-2-carbaldehyde. **$^1$H-NMR:** 400MHz, DMSO-d6: δ 10.00 (s, 1H), 8.59 (d, *J* = 2.0 Hz, 1H), 8.09 (d, *J* = 1.3 Hz, 1H), 7.55 (s, 1H)

6-bromo-2-[[(2S)-2-methylpyrrolidin-1-yl]methyl]-1H-pyrrolo[3,2-b]pyridine (E.6)

To a solution of 6-bromo-1H-pyrrolo[3,2-b]pyridine-2-carbaldehyde (1.59 g, 7.07 mmol, 1 *eq)* in DCE (20 mL) was added (2S)-2-methylpyrrolidine (602 mg, 7.07 mmol, 1 *eq)* and AcOH (424 mg, 7.07 mmol, 0.404 mL, 1 *eq).* After stirring at 25 °C for 0.5 hr, $NaBH(OAc)_3$ (2.99 g, 14.1 mmol, 2 *eq)* was added. The mixture was stirred at 25 °C for 11.5 hr. The reaction mixture was diluted with saturated aq. $NaHCO_3$ and extracted with EtOAc (20 mL x 3). The combined organic layers were washed with brine (50 mL), dried over $Na_2SO_4$, and filtered. The solution was concentrated under reduced pressure. The residue was purified by column chromatography ($SiO_2$, petroleum ether/ethyl acetate gradient of 1/0 to 0/1) to furnish 6-bromo-2-[[(2S)-2-methylpyrrolidin-1-yl]methyl]-1H-pyrrolo[3,2-b]pyridine.

2-[[6-bromo-2-[[(2S)-2-methylpyrrolidin-1-yl]methyl]pyrrolo[3,2-b]pyridin-1-yl]methoxy]-ethyl-trimethyl-silane (E.7)

To a solution of 6-bromo-2-[[(2S)-2-methylpyrrolidin-1-yl]methyl]-1H-pyrrolo[3,2-b]pyridine (0.400 g, 1.36 mmol, 1 *eq)* in THF (10 mL) was added NaH (163 mg, 4.08 mmol, 60 wt % oil dispersion, 3 *eq)* at 0°C. After stirring at 0 °C for 1hr, SEM-Cl (227 mg, 1.36 mmol, 0.241 mL, 1 *eq)* was added. The mixture was stirred at 0 °C for 2hr. The reaction mixture was diluted with $H_2O$ (50 mL) and extracted with EtOAc (20 mL x 3). The combined organic layers were washed with brine (50 mL), dried over $Na_2SO_4$, and filtered. The solution was concentrated under reduced pressure. The residue was purified by column chromatography ($SiO_2$, petroleum ether/ethyl acetate gradient of 1/0 to 1/2) to furnish 2-[[6-bromo-2- [[(2S)-2-methylpyrrolidin-1-yl]methyl]pyrrolo[3,2-b]pyridin-1-yl]methoxy]ethyl-trimethyl-silane. **LCMS:** M+H$^+$:426.2

N-[2-[[(2S)-2-methylpyrrolidin-1-yl]methyl]-1-(2-trimethylsilylethoxymethyl)pyrrolo[3,2-b]pyridin-6-yl]-1,1-diphenyl-methanimine (E.8)

A mixture of 2-[[6-bromo-2-[[(2S)-2-methylpyrrolidin-1-yl]methyl]pyrrolo[3,2-b]pyridin-1-y l]methoxy]ethyl-trimethylsilane (0.200 g, 0.471 mmol, 1 *eq),* diphenylmethanimine (128 mg, 0.707 mmol, 0.119 mL, 1.5 *eq*), $Pd_2(dba)_3$ (43 mg, 0.047 mmol, *0.1 eq),* BINAP (29 mg, 0.047 mmol, 0.1 *eq)* and t-BuONa (91 mg, 0.94 mmol, 2 *eq)* in toluene (4 mL) was stirred at 110 °C for 5 hr under microwave irradiation. The reaction mixture was filtered and concentrated under reduced pressure. The residue was purified by column chromatography ($SiO_2$, petroleum ether/ethyl acetate gradient of 1/0 to 0/1) to furnish N-[2-[[(2S)-2-methy lpyrrolidin-1-yl]methyl]-1-(2-trimethylsilylethoxymethyl)pyrrolo[3,2-b]pyridin-6-yl]-1,1-diphenyl-methanimine.

2-[[(2S)-2-methylpyrrolidin-1-yl]methyl]-1-(2-trimethylsilylethoxymethyl)pyrrolo[3,2-b]pyridin-6-amine (Amine E)

To a solution of N-[2-[[(2S)-2-methylpyrrolidin-1-yl]methyl]-1-(2-trimethylsilylethoxymethy l)pyrrolo[3,2-b]pyridin-6-yl]-1,1-diphenyl-methanimine (0.140 g, 0.267 mmol, 1 *eq)* in THF (5 mL) was added aqueous HCl (1 M, 140 mL). The mixture was stirred at 25 °C for 3 hr. The reaction mixture was concentrated under reduced pressure. The residue was diluted with $H_2O$ (5 mL) and extracted with EtOAc (15 mL). The aqueous was adjusted to pH = 8 with saturated aq. $NaHCO_3$, and extracted with EtOAc(15 mL x 2 ). The combined organic layers were washed with brine (20 mL), dried over $Na_2SO_4$, and filtered. The solution was concentrated under reduced pressure to furnish 2-[[(2S)-2-methylpyrrolidin-1-yl]methyl]-1-(2-trimethylsilylethoxymethyl)pyrrolo[3,2-b]pyridin-6-amine. **LCMS:** M+H$^+$:361.2

Synthesis of Amine F

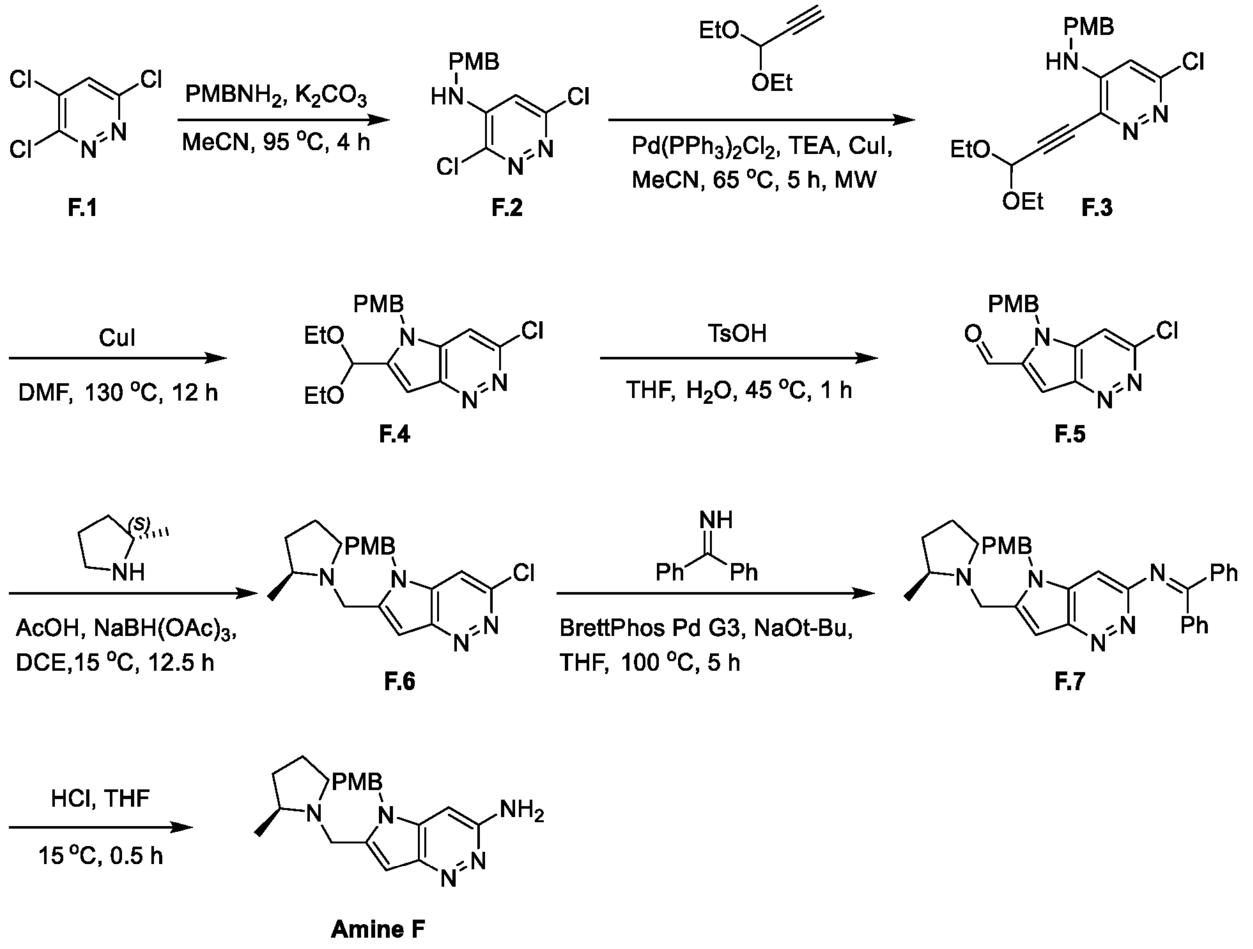


Scheme F

3,6-dichloro-N-[(4-methoxyphenyl)methyl]pyridazin-4-amine (F.2)

A mixture of 3,4,6-trichloropyridazine (3 g, 16.36 mmol, 1 *eq*), $PMBNH_2$ (2.24 g, 16.36 mmol, 2.12 mL, 1 *eq*), $K_2CO_3$ (2.26 g, 16.36 mmol, 1 *eq)* in MeCN (30 mL) was degassed and purged with $N_2$ for 3 times, and then the mixture was stirred at 95 °C for 4 h under $N_2$ atmosphere. The reaction mixture was concentrated under reduced pressure to remove solvent. The residue was purified by column chromatography (SiO2, Petroleum ether/Ethyl acetate=100/1 to 4/1). Compound 3,6-dichloro-N-[(4-methoxyphenyl)methyl]pyridazin-4-amine (4 g, 85% purity) was obtained as a white solid. **$^1$H-NMR:** (400 MHz, $CDCl_3$, ppm): δ 7.30 (d, *J* = 3.89 Hz, 2H), 6.97 (d, *J*= 8.53 Hz, 2H), 6.58 (s, 1H), 5.42 (br s, 1H), 4.38 (d, *J*= 5.27 Hz, 2H), 3.87 (s, 3H).

6-chloro-3-(3,3-diethoxyprop-1-ynyl)-N-[(4-methoxyphenyl)methyl]pyridazin-4-amine (F.3)

A mixture of 3,6-dichloro-N-[(4-methoxyphenyl)methyl]pyridazin-4-amine (1 g x 4 , 3.52 mmol, 1 *eq),* 3,3-diethoxyprop-1-yne (473.63 mg x 4, 3.70 mmol, 529.78 uL, 1.05 *eq),* $Pd(PPh_3)_2Cl_2$ (74.11 mg x 4, 105.58 umol, 0.03 *eq),* TEA (1.78 g x 4, 17.60 mmol, 2.45 mL, 5 *eq)* and CuI (40.22 mg x 4, 211.16 umol, 0.06 *eq)* in MeCN (7 mL) was degassed and purged with $N_2$, and then the mixture was stirred at 65 °C for 5 h under microwave. The reaction mixture was concentrated under reduced pressure to remove solvent. The residue was purified by column chromatography ($SiO_2$, Petroleum ether/Ethyl acetate = 50/1 to 3/1). 6-chloro-3-(3,3-diethoxyprop-1-ynyl)-N-[(4-methoxyphenyl)methyl]pyridazin-4-amine (3.6 g, crude) was obtained as a brown oil, which was used into next step without further purification.

3-chloro-6-(diethoxymethyl)-5- [(4-methoxyphenyl)methyl]pyrrolo[3,2-c]pyridazine (F.4)

To a solution of 6-chloro-3-(3,3-diethoxyprop-1-ynyl)-N-[(4-methoxyphenyl)methyl] pyridazin-4-amine (2.8 g, 4.47 mmol, 1 *eq)* in dry DMF (20 mL) was added CuI (170.26 mg, 893.98 umol, 0.2 *eq).* The reaction mixture was heated to

130 °C for 12 h. The mixture was poured into water (50 mL) and extracted with ethyl acetate (30 mL x 3). The combined organic phase was washed with brine (50 mL), dried with anhydrous $Na_2SO_4$, filtered and concentrated in vacuum. The residue was purified by column chromatography ($SiO_2$, Petroleum ether/Ethyl acetate = 50/1 to 3/1). Compound 3-chloro-6-(diethoxymethyl)-5- [(4-methoxyphenyl)methyl]pyrrolo[3,2-c]pyridazine (0.9 g, crude) was obtained as brown oil.

3-chloro-5-[(4-methoxyphenyl)methyl]pyrrolo[3,2-c]pyridazine-6- carbaldehyde (F.5)

A mixture of 3-chloro-6-(diethoxymethyl)-5-[(4-methoxyphenyl)methyl]pyrrolo[3,2-c] pyridazine (0.9 g, 2.39 mmol, 1 *eq*), TsOH.$H_2O$ (683.25 mg, 3.59 mmol, 1.5 *eq)* in THF (10 mL) and $H_2O$ (2 mL) was stirred at 45 °C for 1 h. The mixture was poured into $NaHCO_3$ (50 mL) and extracted with ethyl acetate (30 mL x 3). The combined organic phase was washed with brine (30 mL), dried with anhydrous $Na_2SO_4$, filtered and concentrated in vacuum. Compound 3-chloro-5-[(4-methoxyphenyl)methyl]pyrrolo[3,2-c]pyridazine-6- carbaldehyde (0.8 g, crude) was obtained as a brown solid,

3-chloro-5-[(4-methoxyphenyl)methyl]-6-[[(2S)-2-methylpyrrolidin-1-yl]methyl]pyrrolo[3,2-c]pyridazine (F.6)

To the solution of 3-chloro-5-[(4-methoxyphenyl)methyl]pyrrolo[3,2-c]pyridazine-6-carbaldehyde (950 mg, 1.57 mmol, 1 *eq)* and (2S)-2-methylpyrrolidine (160.85 mg, 1.89 mmol, *1.2 eq)* in DCE (20 mL) was added AcOH (94.53 mg, 1.57 mmol, 90.03 uL, 1 *eq*). The mixture was stirred for 0.5 h at 15°C. $NaBH(OAc)_3$ (667.30 mg, 3.15 mmol, 2 *eq)* was added to the solution. The mixture was stirred for 12 h at 15°C. The reaction mixture was concentrated under reduced pressure to remove solvent. The residue was purified by column chromatography ($SiO_2$, Petroleum ether/Ethyl acetate = 20/1 to 0/1). Compound 3-chloro-5-[(4-methoxyphenyl)methyl]-6-[[(2S)-2-methylpyrrolidin-1-yl]methyl]pyrrolo[3,2-c]pyridazine (800 mg, crude) was obtained as a brown oil. **$^1$H-NMR:** (400 MHz ,DMSO-$d_6$,ppm): $\delta$ 7.94 (s, 1H), 7.01 (d, *J* = 8.63Hz, 2H), 6.94-6.83 (m, 3H), 5.52 (s, 2H), 4.04-3.99 (m, 1H), 3.70 (s, 3H), 3.42 (d, *J*= 14.13Hz, 1H), 2.84-2.76 (m, 1H), 2.46-2.39 (m, 1H), 2.17 (q, *J* = 8.67Hz, 1H), 1.94-1.90 (m, 1H), 1.67-1.53 (m, 2H), 1.33 (dq, *J* = 12.07, 8.15Hz, 1H), 1.03 (d, *J* = 6.00Hz, 3H).

N-[5-[(4-methoxyphenyl)methyl]-6-[[(2S)-2-methylpyrrolidin-1-yl]methyl]pyrrolo[3,2-c]pyridazin-3-yl]-1,1-diphenyl-methanimine (F.7)

A mixture of 3-chloro-5-[(4-methoxyphenyl)methyl]-6-[[(2S)-2-methylpyrrolidin-1-yl] methyl]pyrrolo[3,2-c]pyridazine (850 mg, 2.29 mmol, 1 *eq),* diphenylmethanimine (498.44 mg, 2.75 mmol, 461.51 uL, 1.2 *eq),* tBuONa (440.50 mg, 4.58 mmol, 2 *eq)* and [2-(2-aminophenyl)phenyl]-methylsulfonyloxy-palladium;dicyclohexyl-[3,6-dimethoxy-2-(2,4,6-triisopropylphenyl)phenyl]phosphane (207.76 mg, 229.19 umol, 0.1 *eq)* in THF (20 mL) was degassed and purged with $N_2$ for 3 times, and then the mixture was stirred at 100 °C for 5 h under $N_2$ atmosphere. The reaction mixture was concentrated under reduced pressure to remove solvent. The residue was purified by column chromatography ($SiO_2$, Petroleum ether/Ethyl acetate = 3/1 to 0/1). Compound N-[5-[(4-methoxyphenyl)methyl]-6-[[(2S)-2-methylpyrrolidin-1-yl]methyl] pyrrolo[3,2-c]pyridazin-3-yl]-1,1-diphenyl-methanimine ( crude) (0.5 g ) was obtained as brown oil. **$^1$H-NMR:** (400 MHz ,DMSO-$d_6$,ppm) $\delta$ 7.76-7.68 (m, 2H), 7.61-7.55 (m, 1H), 7.54-7.48 (m, 2H), 7.31-7.18 (m, 4H), 7.10-7.05 (m, 2H), 6.89-6.77 (m, 4H), 6.65 (s, 1H), 5.32 (s, 2H), 3.95 (d, *J* = 13.88Hz, 1H), 3.71-3.68 (m, 1H), 2.80-2.72 (m, 1H), 2.42-2.37 (m, 1H), 2.18-2.07 (m, 1H), 1.94-1.88 (m, 1H), 1.61-1.52 (m, 2H), 1.38-1.26 (m, 1H), 1.03 (d, *J* = 5.88Hz, 3H)

5-[(4-methoxyphenyl)methyl]-6-[[(2S)-2-methylpyrrolidin-1-yl] methyl]pyrrolo[3,2-c]pyridazin-3-amine (Amine F)

N-[5-[(4-methoxyphenyl)methyl]-6-[[(2S)-2-methylpyrrolidin-1-yl]methyl]pyrrolo[3,2-c]pyridazin-3-yl]-1,1-diphenyl-methanimine (400 mg, 775.72 umol, 1 *eq)* in HCl (4 mL) and THF (4 mL) was degassed and purged with $N_2$ 3 times, and then the mixture was stirred at 15 °C for 0.5 h under $N_2$ atmosphere. $H_2O$ (20 mL) and EtOAc (10 mL) were added to the mixture, and then the organic phase was separated. The aqueous phase was basified using $NaHCO_3$ adjust pH = 8. The aqueous layer was extracted with EtOAc (15 mL x 3), the combined organic phases were dried over $Na_2SO_4$, filtered and concentrated under reduced pressure. Compound 5-[(4-methoxyphenyl)methyl]-6-[[(2S)-2-methylpyrrolidin-1-yl] methyl] pyrrolo[3,2-c]pyridazin-3-amine (0.27 g, 553.15 umol, 71.31% yield, 72% purity) was obtained as a brown solid. The crude product was used into the next step without further purification. **LCMS:** $[M+H]^+$=352.

SYNTHESIS OF EXAMPLES ACCORDING TO THE INVENTION (examples 18 and 19 are reference examples for comparative purposes)

Preparation of Example 1

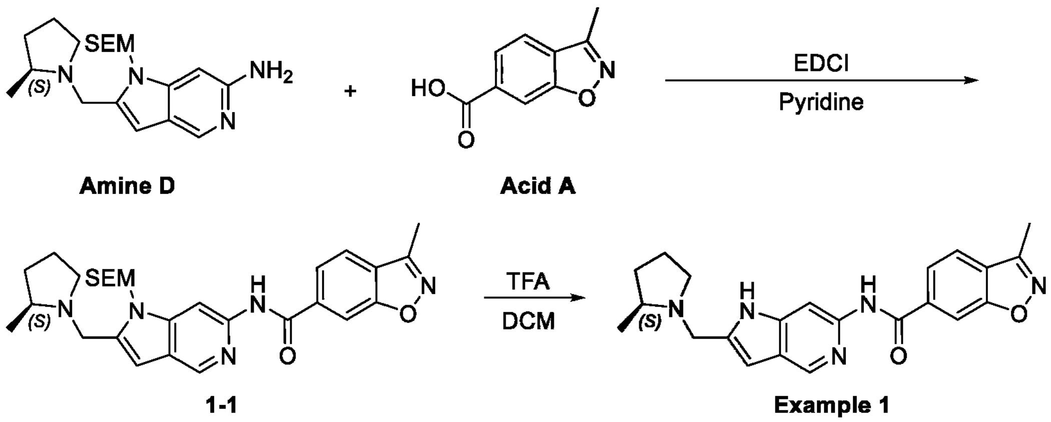


**Amine D** **Acid A**

**1-1** **Example 1**

3-methyl-N-(2-[[(2S)-2-methylpyrrolidin-1-yl]methyl]-1-[[2-(trimethylsilyl)ethoxy]methyl]pyrrolo[3,2-c]pyridin-6-yl)-1,2-benzoxazole-6-carboxamide (1-1)

Into a 8-mL vial purged and maintained under an inert atmosphere of nitrogen, was placed 2-[[(2S)-2-methylpyrrolidin-1-yl]methyl]-1-[[2-(trimethylsilyl)ethoxy]methyl]pyrrolo[3,2-c]pyridin-6-amine (**Amine D,** 170.0 mg, 0.471 mmol, 1.0 equiv), 3-methyl-1,2-benzoxazole-6-carboxylic acid **(Acid A,** CAS: 478169-72-1, 91.9 mg, 0.52 mmol, 1.10 equiv), EDCI (135.6 mg, 0.71 mmol, 1.5 equiv), and pyridine (2.0 mL). The resulting solution was stirred for 12 h at 50 °C. The reaction was then quenched by the addition of 8 mL of water/ice. The resulting solution was extracted with 3x3mL of ethyl acetate and the organic layers combined. The resulting mixture was washed with 3x3mL of brine. The resulting mixture was concentrated. This resulted in 280 mg (crude) of 3-methyl-N-(2-[[(2S)-2-methylpyrrolidin-1-yl] methyl]-1-[[2-(trimethylsilyl)ethoxy]methyl]pyrrolo[3,2-c]pyridin-6-yl)-1,2-benzoxazole-6-carboxamide **(1-1)** as a brown liquid. **LCMS:** $[M+1]^+$=520.

(S)-3-methyl-N-(2-((2-methylpyrrolidin-1-yl)methyl)-1H-pyrrolo[3,2-c]pyridin-6-yl)benzo[d]isoxazole-6-carboxamide (Example 1)

Into a 8-mL vial purged and maintained under an inert atmosphere of nitrogen, was placed 3-methyl-N-(2-[[(2S)-2-methylpyrrolidin-1-yl]methyl]-1-[[2-(trimethylsilyl)ethoxy]methyl]pyrrolo[3,2-c]pyridin-6-yl)-1,2-benzoxazole-6-carboxamide **(1-1,** 280.0 mg, 0.54 mmol, 1.0 equiv), TFA (2.0 mL, 50 equiv), DCM (2.0 mL). The resulting solution was stirred for 12 h at 25 °C. The resulting mixture was concentrated. The crude product was purified by Prep-HPLC with the following conditions: Column, XBridge Prep C18 OBD Column,5um, 19x 150mm ; mobile phase, Water(0.05% $NH_3.H_2O$) and ACN (12% PhaseB up to 34% in 7 min); Detector,UV 254nm. This resulted in 40.9 mg (19.5%) of (S)-3-methyl-N-(2-((2-methylpyrrolidin-1-yl)methyl)-1H-pyrrolo[3,2-c]pyridin-6-yl)benzo[d]isoxazole-6-carboxamide **(Example 1)** as an off-white solid. **LCMS:** $[M+1]^+$= 390. **$^1$H-NMR** (300 MHz, Methanol-$d_4$, ppm): δ 8.57 (d, *J* = 1.0 Hz, 1H), 8.26-8.19 (m, 2H), 8.04-7.90 (m, 2H), 6.55 (s, 1H), 4.19 (d, *J* = 14.1 Hz, 1H), 3.61 (d, *J* = 14.1 Hz, 1H), 3.08 (s, 1H), 2.65 (s, 4H), 2.45 (d, *J* = 10.5 Hz, 1H), 2.07(m,1H),1.77 (d, *J* =8.7 Hz, 2H), 1.52 (s, 1H), 1.25 (d, *J* = 6.3 Hz, 3H).

The following examples in **Table D** were prepared in a similar fashion to that shown above for **Example 1** using **Amine D** and the appropriate reagents and conditions.

Table D

| Ex. | Acid | Structure | LCMS | $^{1}$H-NMR |
|---|---|---|---|---|
| 15 | L | 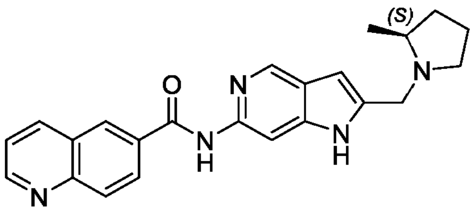 | 386 | (300 MHz, Methanol-$d_4$, ppm): δ 8.99 (s, 1H), 8.66 (d, $J$ = 2.1 Hz, 1H), 8.56 (m, 2H), 8.36 (d, $J$ = 2.1 Hz, 1H), 8.25 (s, 1H), 8.17 (d, $J$ = 9.0 Hz, 1H), 7.67 (m, 1H), 6.54 (s, 1H), 4.16 (d, $J$ = 13.8 Hz, 1H), 3.56 (d, $J$ = 13.8 Hz, 1H), 3.09-3.03 (m, 1H), 2.58-2.56 (m, 1H), 2.40-2.38 (m, 1H), 2.06-2.04 (m, 1H), 1.81-1.73 (m, 2H), 1.54-1.51 (m, 1H), 1.24 (d, $J$ = 6.3 Hz, 3H) |
| 31 | W | 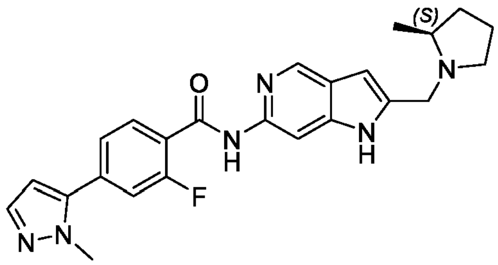 | 433 | (300 MHz, Methanol-$d_4$, ppm) δ 8.54 (d, $J$ = 1.0 Hz, 1H), 8.31 (s, 1H), 8.07 (t, $J$ = 8.0 Hz, 1H), 7.60-7.47 (m, 3H), 6.55 (d, $J$ = 2.0 Hz, 2H), 4.18 (d, $J$ = 13.9 Hz, 1H), 3.98 (s, 3H), 3.59 (d, $J$ = 14.6 Hz, 1H), 2.62 (s, 1H), 2.43 (d, $J$ = 9.1 Hz, 1H), 1.78 (m, 2H), 1.58 (m, 1H)1.25 (d, $J$ = 6.1 Hz, 3H) |
| 32 | X | 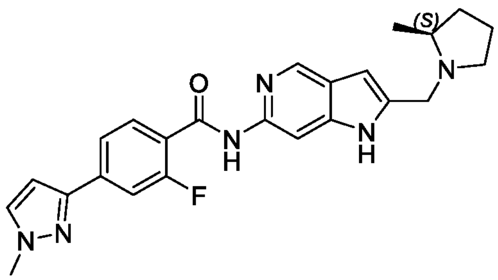 | 433 | (300 MHz, Methanol-$d_4$, ppm) δ 8.52 (d, $J$ = 1.0 Hz, 1H), 8.30 (s, 1H), 8.00 (t, $J$ = 8.0 Hz, 1H), 7.78-7.64 (m, 3H), 6.78 (d, $J$ = 2.4 Hz, 1H), 6.52 (s, 1H), 4.14 (d, $J$ = 13.9 Hz, 1H), 3.99 (s, 3H), 3.54 (d, $J$ = 13.9 Hz, 1H), 3.04 (m, 1H), 2.55 (m, 1H), 2.38 (m, 1H), 2.05 (m, 1H), 1.79 (m, 1H), 1.75 (m, 1H), 1.60-1.44 (m, 1H), 1.23 (d, $J$ = 6.1 Hz, 3H) |
| 33 | Y | 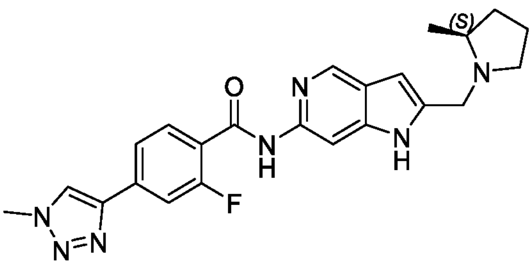 | 434 | (300 MHz, Methanol-$d_4$, ppm) δ 8.57-8.48 (m, 1H), 8.46 (s, 1H), 8.31 (s, 1H), 8.06 (t, $J$ = 7.9 Hz, 1H), 7.86-7.73 (m, 2H), 6.55 (s, 1H), 4.75 (s, 1H), 4.20 (s, 3H), 4.17 (s, 1H), 3.58 (d, $J$ = 13.9 Hz, 1H), 2.62 (m, 1H), 2.43 (d, $J$ = 9.2 Hz, 1H), 2.08 (m, 1H), 1.78 (d, $J$ = 9.3 Hz, 2H), 1.53 (s, 1H), 1.24 (d, $J$ = 6.1 Hz, 3H) |

(continued)

| Ex. | Acid | Structure | LCMS | $^1$H-NMR |
| --- | --- | --- | --- | --- |
| **34** | **Z** | 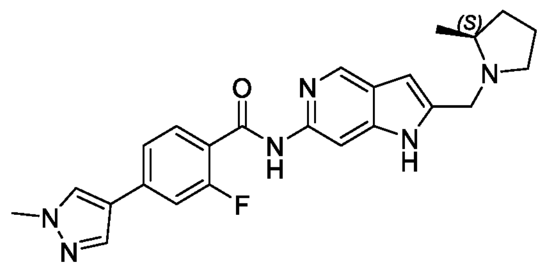 | **433** (PH-PUK) | **$^1$H-NMR**14 (PH-PUK): (300 MHz, Methanol-$d_4$, ppm) δ 8.52 (s, 1H), 8.30 (s, 1H), 8.15 (s, 1H), 8.04-7.92 (m, 2H), 7.62-7.47 (m, 2H), 6.51 (s, 1H), 4.14 (d, *J* = 13.8 Hz, 1H), 3.97 (s, 3H), 3.53 (d, *J* = 13.9 Hz, 1H), 3.04 (m, 1H), 2.54 (m, 1H), 2.37 (m, 1H), 2.03 (m, 1H), 1.83-1.69(m, 2H), 1.50 (m, 1H), 1.23 (d, *J* = 6.1 Hz, 3H) |
| **35** | **AA** | 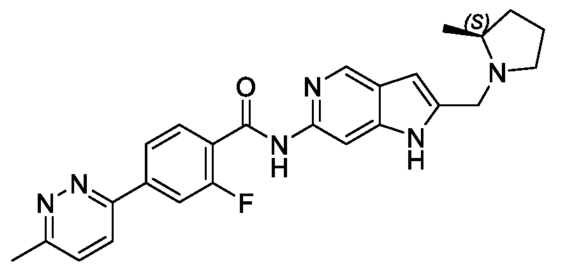 | **445** | (300 MHz, Methanol-$d_4$, ppm) δ 8.57 (d, *J* = 1.0 Hz, 1H), 8.34 (s, 1H), 8.20 (d, *J* = 8.8 Hz, 1H), 8.14-8.05 (m, 3H), 7.75 (d, *J* = 8.8 Hz, 1H), 6.63 (s, 1H), 4.30 (d, *J* = 14.0 Hz, 1H), 3.79 (d, *J* = 14.1 Hz, 1H), 3.20 (s, 1H), 2.86 (s, 1H), 2.78 (s, 3H), 2.67 (s, 1H), 2.15 (dd, *J* = 13.0, 7.3 Hz, 1H), 1.85 (m, 2H), 1.68-1.52 (m, 1H), 1.31 (d, *J* = 6.2 Hz, 3H)<br>$^{19}$F-NMR: (300 MHz, Methanol-$d_4$, ppm) δ - 114.372 |
| **36** | **AB** | 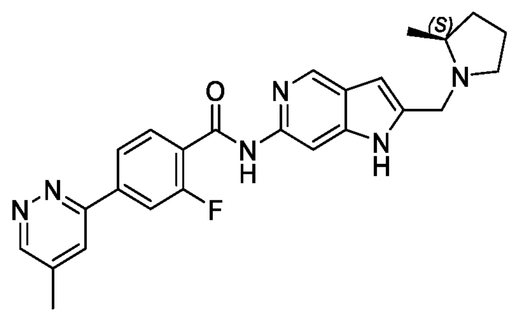 | **445** | (300 MHz, Methanol-$d_4$, ppm) δ 9.12 (d, *J* = 1.8 Hz, 1H), 8.54 (d, *J* = 1.0 Hz, 1H), 8.31 (s, 1H), 8.17 (s, 1H), 8.15-8.05 (m, 3H), 6.53 (s, 1H), 4.15 (d, *J* = 13.8 Hz, 1H), 3.55 (d, *J* = 13.8 Hz, 1H), 3.05 (m, 1H), 2.58 (d, *J* = 7.2 Hz, 1H), 2.54 (s, 3H), 2.38 (m, 1H), 1.79 (m, 1H), 1.52 (dd, *J* = 17.8, 10.2 Hz, 1H), 1.24 (d, *J* = 6.1 Hz, 3H)<br>$^{19}$F-NMR: (300 MHz, Methanol-$d_4$, ppm) δ - 114.378 |

Preparation of Example 2

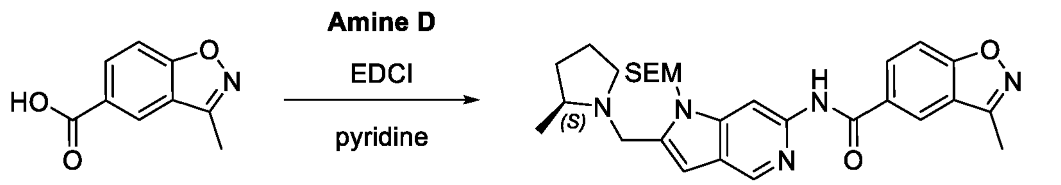


**Acid B**

Into a 8-mL vial purged and maintained under an inert atmosphere of nitrogen, was placed 2-[[(2S)-2-methylpyrrolidin -1-yl]methyl]-1-[[2-(trimethylsilyl)ethoxy]methyl]pyrrolo[3,2-c]pyridin-6-amine **(Amine D,** 150.0 mg, 0.42 mmol, 1.0 equiv), pyridine (3.00 mL), 3-methyl-1,2-benzoxazole-5-carboxylic acid (81.1 mg, 0.46 mmol, 1.10 equiv), EDCI (119.6 mg, 0.62 mmol, 1.5 equiv). The resulting solution was stirred at room temperature for 24hr. The resulting mixture was concentrated and diluted with of $H_2O$. The resulting solution was extracted with 3x20 mL of ethyl acetate and the organic layers combined. The resulting mixture was washed with 2 x20 mL of brine. The resulting mixture was concentrated. This resulted in 300 mg (crude) of 3-methyl-N-(2-[[(2S)-2-methylpyrrolidin-1-yl]methyl] -1-[[2-(trimethylsilyl)ethoxy]methyl] pyrrolo[3,2-c]pyridin-6-yl)-1,2-benzoxazole-5-carboxamide as a brown solid. **LCMS:** $[M+H]^+$=520.

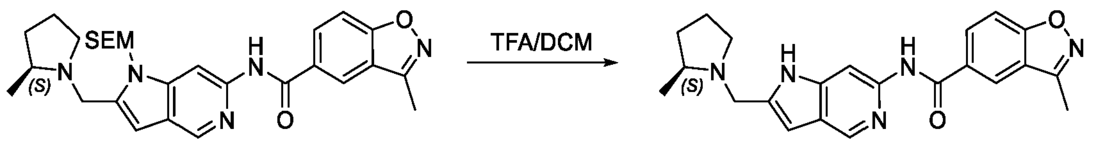


**Example 2**

Into a 8-mL vial purged and maintained under an inert atmosphere of nitrogen, was placed 3-methyl-N-(2-[[(2S)-2-methylpyrrolidin-1-yl]methyl]-1-[[2-(trimethylsilyl)ethoxy]methyl]pyrrolo[3,2-c]pyridin-6-yl)-1,2-benzoxazole-5-carboxamide (300.00 mg, 0.577 mmol, 1.00 equiv), DCM (3.00 mL), TFA (3 mL). The resulting solution was stirred for 16h at room temperature. The resulting mixture was concentrated. The pH value of the solution was adjusted to 8 with $Et_2NH$. The crude product (110 mg) purified by Prep-HPLC with the following conditions: Column, XBridge Prep C18 OBD Column, 5um,19x150mm; mobile phase, Water(0.05%$NH_3H_2O$) and ACN (25% PhaseB up to 50% in 7 min); Detector, UV.254 nm. This resulted in 47.3 mg (21.04%) of 3-methyl-N-(2-[[(2S)-2-methylpyrrolidin-1-yl]methyl]-1H-pyrrolo[3,2-c] pyridin-6-yl)-1,2-benzoxazole -5-carboxamide as a white solid. **LCMS:** $[M+H]^+$=390. **$^1$H-NMR:** (300 MHz, Methanol-$d_4$, ppm) δ 8.57 (d, *J* = 1.0 Hz, 1H), 8.52-8.46 (m, 1H), 8.27 (dd, *J* = 8.8, 1.8 Hz, 1H), 8.21 (t, *J* = 1.0 Hz, 1H), 7.74 (dd, *J* = 8.8, 0.8 Hz, 1H), 6.55 (s, 1H), 4.19 (d, *J* = 13.9 Hz, 1H), 3.62 (d, *J* = 14.0 Hz, 1H), 3.09 (m, 1H), 2.67 (s, 3H), 2.63 (s, 1H), 2.46 (m, 1H), 2.08 (m, 1H), 1.80 (s, 1H), 1.88-1.71 (m, 2H), 1.62-1.43 (m, 1H), 1.25 (d, *J* = 6.1 Hz, 3H).

The following examples in **Table E** were prepared in a similar fashion to that shown above for **Example 2** using **Amine D** and the appropriate reagents and conditions.

Table E

| Ex. | Acid | Structure | LCMS | $^1$H-NMR |
|---|---|---|---|---|
| **5** | **E** | 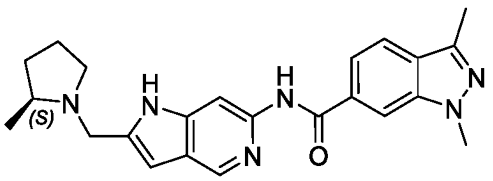 | **403** | (300 MHz, Methanol-$d_4$, ppm) δ 8.57 (s, 1H), 8.27-8.16 (m, 2H), 7.87 (dd, *J* = 8.4, 0.9 Hz, 1H), 7.75 (dd, *J* = 8.5, 1.4 Hz, 1H), 6.54 (s, 1H), 4.17 (d, *J* = 13.9 Hz, 1H), 4.11 (s, 3H), 3.58 (d, *J* = 13.5 Hz, 1H), 3.07 (m, 1H), 2.61 (s, 3H), 2.41 (m, 1H), 2.13-1.98 (m, 1H), 1.83-1.73 (m, 2H), 1.50 (dd, *J* = 17.8, 9.2 Hz, 1H), 1.25 (d, *J* = 6.1 Hz, 3H). |
| **6** | **F** | 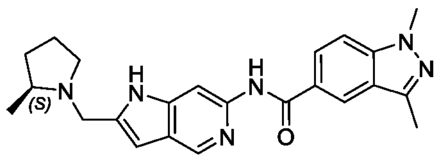 | **403** | (300 MHz, Methanol-$d_4$, ppm) δ 8.56 (d, *J* = 1.0 Hz, 1H), 8.5-8.44 (m, 1H), 8.22 (m, 1H), 8.08 (dd, *J* = 8.8, 1.7 Hz, 1H), 7.62 (dd, *J* = 8.9, 0.8 Hz, 1H), 6.53 (s, 1H), 4.16 (d, *J* = 13.9 Hz, 1H), 4.06 (s, 3H), 3.57 (d, *J* = 13.9 Hz, 1H), 3.07 (m, 1H), 2.64 (s, 3H), 2.59 (d, *J* = 7.0 Hz, 1H), 2.41 (m, 1H), 2.05 (m, 1H), 1.78 (m, 2H), 1.87-1.70 (m, 1H), 1.61-1.45 (m, 1H), 1.24 (d, *J* = 6.1 Hz, 3H). |

(continued)

| Ex. | Acid | Structure | LCMS | $^1$H-NMR |
|---|---|---|---|---|
| **8** | **H** | 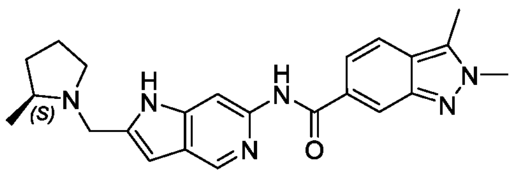 | **403** | (300 MHz, Methanol-$d_4$, ppm) δ 8.55 (d, *J* = 1.0 Hz, 1H), 8.24 (m, 2H), 7.80 (dd, *J* = 8.8, 0.9 Hz, 1H), 7.62 (dd, *J* = 8.8, 1.5 Hz, 1H), 6.52 (s, 1H), 4.17 (s, 3H), 4.12 (s, 1H), 3.55 (d, *J* = 13.9 Hz, 1H), 3.05 (m, 1H), 2.71 (s, 3H), 2.55 (m, 1H), 2.39 (m, 1H), 1.77 (m, 2H), 1.50 (m, 1H), 1.23 (d, *J* = 6.1 Hz, 3H). |
| **9** | **I** | 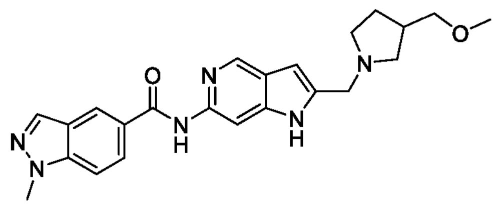 | **419** | (300 MHz, Methanol-$d_4$, ppm) δ8.55 (d, *J* = 0.9 Hz, 1H), 8.51 (s, 1H), 8.21 - 8.20 (m, 2H), 8.08 (dd, *J* = 9, 1.7 Hz, 1H), 7.71 (d, *J* = 9 Hz, 1H), 6.51 (s, 1H), 4.15 (s, 3H), 3.82 (s, 2H), 3.36 (d, *J* = 1.5 Hz, 1H), 3.34(s, 3H), 2.85-2.80 (m, 1H), 2.74-2.61 (m, 2H), 2.55 - 2.41 (m, 2H), 2.06 - 1.95 (m, 1H), 1.60-1.49 (m, 1H). |
| **92** | **BW** | 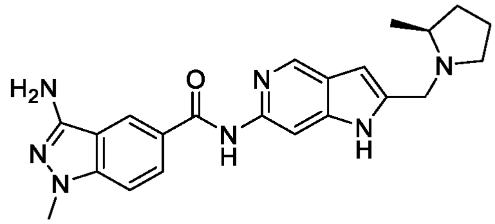 | **404** | (300 MHz, DMSO-$d_6$, ppm)δ 11.33 (s, 1H), 10.07 (s, 1H), 8.52 (d, *J* = 8.1 Hz, 2H), 8.23 (s, 1H), 8.00-7.97 (m, 1H), 7.39 (d, *J* = 8.7 Hz, 1H), 6.36 (s, 1H), 5.64 (s, 2H), 4.01 (d, *J* = 13.5 Hz, 1H), 3.78 (s, 3H), 3.40 (d, *J* = 13.5 Hz, 1H), 2.88-2.73 (m, 1H), 2.50-2.40(m, 1H), 2.28-2.15 (m, 1H), 1.97-1.90 (m, 1H), 1.68-1.61 (m, 2H), 1.39-1.36(m, 1H), 1.11 (d, *J* = 6.0 Hz, 3H). |

Preparation of Example 3

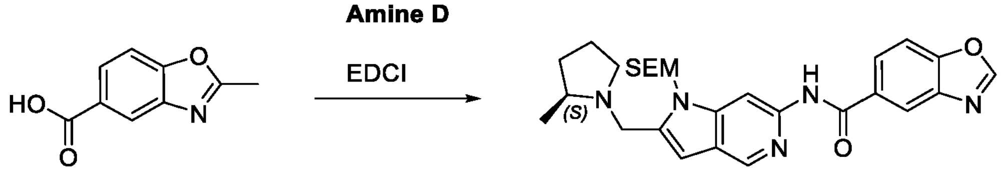


**Acid C**

Into a 50-mL round-bottom flask, was placed 2-[[(2S)-2-methylpyrrolidin-1-yl]methyl]-1-[[2-(trimethylsilyl) ethoxy]methyl]pyrrolo[3,2-c]pyridin-6-amine **(Amine D**, 150.0 mg, 0.42 mmol, 1.0 equiv), 2-methyl-1,3-benzoxazole-5-carboxylic acid (CAS: 90322-32-0, 81.07 mg, 0.458 mmol, 1.10 equiv), EDCI (119.6 mg, 0.62 mmol, 1.5 equiv), pyridine (1.5 mL). The resulting solution was stirred for 1 hr at 50 °C. The reaction was then quenched by the addition of 5 mL of water and the resulting solution was extracted with 3x10 mL of ethyl acetate and the organic layers combined and concentrated. This resulted in 300 mg (crude) of 2-methyl-N-(2-[[(2S)-2-methylpyrrolidin-1-yl]methyl]-1-[[2-(trimethylsilyl) ethoxy]methyl]pyrrolo[3,2-c]pyridin-6-yl)-1,3-benzoxazole-5-carboxamide as a brown solid. **LCMS:** $[M+H]^+$ =506.

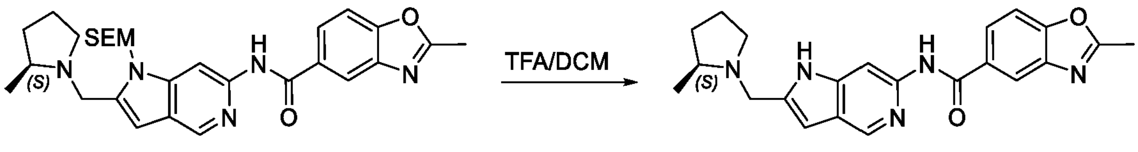


**Example 3**

Into a 50-mL round-bottom flask, was placed 2-methyl-N-(2-[[(2S)-2-methylpyrrolidin-1-yl]methyl]-1-[[2-(trimethylsilyl)ethoxy]methyl]pyrrolo[3,2-c]pyridin-6-yl)-1,3-benzoxazole-5-carboxamide (300.0 mg, crude), TFA (0.50 mL), DCM (0.50 mL). The resulting solution was stirred for 2 hr at room temperature. The resulting mixture was concentrated. The crude product (100 mg) was purified by Prep-HPLC with the following conditions: Column, XBridge Prep C18 OBD Column, 5um, 19x150mm; mobile phase, Water(0.05%$NH_3H_2O$) and ACN (32% PhaseB up to 63% in 7 min); Detector, UV 254nm. This resulted in 33.6 mg (21% for two steps)of 2-methyl-N-(2-[[(2S)-2-methylpyrrolidin-1-yl]methyl]-1H-pyrrolo[3,2-c]pyridin-6-yl)-1,3-benzoxazole-5-carboxamide as a light brown solid. **LCMS:** $[M+H]^+$ =390. **$^1$H-NMR:** (300 MHz,$CD_3OD$-*$d_4$*, *ppm):* δ 8.54 (d, *J* = 1.0 Hz, 1H), 8.30 (d, *J* = 1.8 Hz, 1H), 8.20 (d, *J* = 1.1 Hz, 1H), 8.05 (dd, *J* = 8.5, 1.8 Hz, 1H), 7.72 (d, *J* = 8.6 Hz, 1H), 6.51 (s, 1H), 4.13 (d, *J* = 13.9 Hz, 1H), 3.53 (d, *J*= 13.9 Hz, 1H), 3.04-3.00 (m, 1H), 2.70 (s, 3H), 2.63-2.47 (m, 1H), 2.36 (q, *J* = 9.0 Hz, 1H), 2.13 - 1.95 (m, 1H), 1.76-1.70 (m, 2H), 1.59-1.40 (m, 1H), 1.22 (d, *J* = 6.1 Hz, 3H).

The following examples in **Table F** were prepared in a similar fashion to that shown above for **Example 3** using **Amine D** and the appropriate reagents and conditions.

Table F

| Ex. | Acid | Structure | LCMS | $^1$H-NMR |
|---|---|---|---|---|
| **4** | **D** | 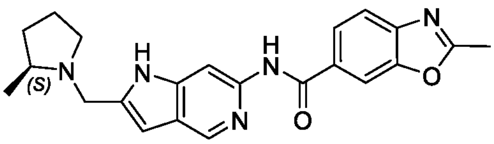 | **390** | (300 MHz,$CD_3OD$-*$d_4$*, *ppm*): δ8.56 (d, *J* = 1.0 Hz, 1H), 8.28 - 8.17 (m, 2H), 8.05 (dd, *J* = 8.3, 1.7 Hz, 1H), 7.77 (d, *J* = 8.5 Hz, 1H), 6.52 (s, 1H), 4.15 (d, *J* = 13.9 Hz, 1H), 3.55 (d, *J* = 13.8 Hz, 1H), 3.05-3.0 (m, 1H), 2.72 (s, 3H), 2.56-2.50 (m, 1H), 2.38 (q, *J* = 9.0 Hz, 1H), 2.14-1.96 (m, 1H), 1.77-1.70 (m, 2H), 1.60-1.41 (m, 1H), 1.23 (d, *J* = 6.1 Hz, 3H). |
| **7** | **G** | 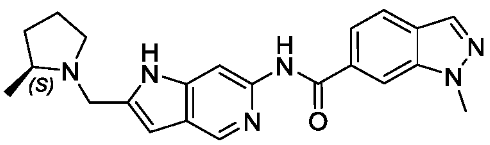 | **389** | (300 MHz,$CD_3OD$-*$d_4$*, *ppm*): δ 8.57 (d, *J* = 1.0 Hz, 1H), 8.26-8.20 (m, 2H), 8.12 (d, *J* = 1.0 Hz, 1H), 7.92 (dd, *J* = 8.5, 0.9 Hz, 1H), 7.78 (dd, *J* = 8.5, 1.4 Hz, 1H), 6.53 (s, 1H), 4.19 (s, 4H), 3.55 (d, *J* = 13.8 Hz, 1H), 3.12 - 2.99 (m, 1H), 2.56 (q, *J* = 7.2 Hz, 1H), 2.37-2.30 (m, 1H), 2.14-1.96 (m, 1H), 1.76-1.70(m, 2H), 1.60-1.41 (m, 1H), 1.34-1.20 (m, 3H). |

Preparation of Example 10

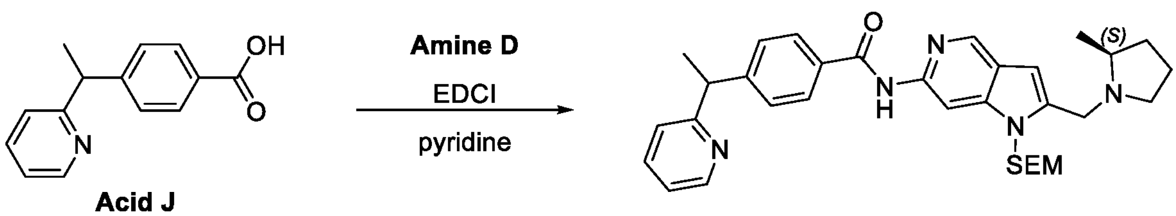


**Acid J**

Into a 8 mL vial was added 2-[[(2S)-2-methylpyrrolidin-1-yl]methyl]-1-[[2-(trimethylsilyl)ethoxy]methyl]pyrrolo[3,2-c]pyridin-6-amine **(Amine D,** 111 mg, 0.308 mmol, 1.00 equiv), 4-[1-(pyridin-2-yl)ethyl]benzoic acid (100 mg, 0.308 mmol, 1.00 equiv), pyridine (3.00 mL) and EDCI (89mg, 0.462 mmol, 1.50 equiv) at room temperature. The resulting mixture was stirred for 6 h at 50 °C under nitrogen atmosphere. The resulting mixture was concentrated under reduced pressure. The residue was dissolved in EtOAc (50 mL) and the resulting mixture was washed with 3x20 mL of brine. The organic layer was dried over anhydrous $Na_2SO_4$.The resulting mixture was concentrated under reduced pressure. This resulted in N-(2-[[(2S)-2-methylpyrrolidin-1-yl]methyl]-1-[[2-(trimethylsilyl)ethoxy]methyl]pyrrolo[3,2-c]pyridin-6-yl)-4-[1-(pyridin-2-yl)ethyl]benzamide (100 mg, 48.43%) as a brown oil. **LCMS:** $[M+1]^+$=570.

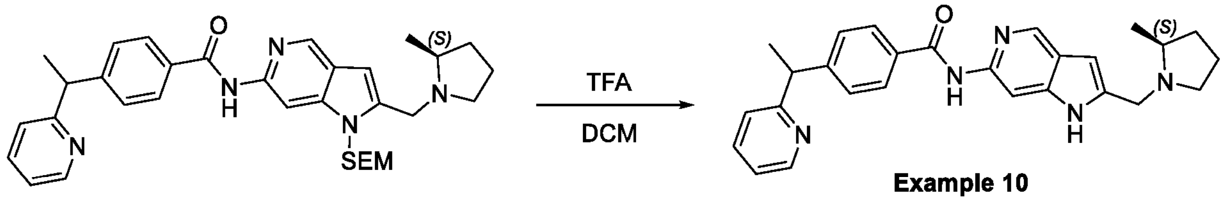


**Example 10**

Into a 8 mL vial were added N-(2-[[(2S)-2-methylpyrrolidin-1-yl]methyl]-1-[[2-(trimethylsilyl)ethoxy]methyl]pyrrolo[3,2-c]pyridin-6-yl)-4-[1-(pyridin-2-yl)ethyl]benzamide (100. mg, 0.175 mmol, 1.00 equiv), DCM (2.00 mL) and TFA (2.00 mL) at room temperature. The resulting mixture was stirred for overnight at room temperature under nitrogen atmosphere. The resulting mixture was concentrated under reduced pressure. The residue was dissolved in DMF (3 mL). The mixture was basified to pH 9 with Ammonium hydroxide, then it was purified by Prep-HPLC with the following conditions (Column: XBridge Prep C18 OBD Column, 5um, 19x150mm; Mobile Phase A:Water(0.05%$NH_3H_2O$), Mobile Phase B:ACN; Flow rate:20 mL/min; Gradient:44 B to 66 B in 7 min, 220 nm;) to afford N-(2-[[(2S)-2-methylpyrrolidin-1-yl]methyl]-1H-pyrrolo[3,2-c]pyridin-6-yl)-4-[1-(pyridin-2-yl)ethyl]benzamide (24.7 mg, 35.8%) as a white solid. **LCMS:** $[M+1]^+$=440. **$^1$H-NMR:** (300 MHz, Methanol-$d_4$, ppm)δ8.55 (s, 1H), 8.51 - 8.49 (m, 1H), 8.20 (s, 1H), 7.95 (d, *J* = 8.4 Hz, 2H), 7.82-7.76 (m, 1H), 7.48 (d, *J* = 8.4 Hz, 2H), 7.39 (d, *J* = 8.1 Hz, 1H), 7.30-7.26 (m, 1H), 6.56 (s, 1H), 4.43 (q, *J* = 7.2 Hz, 1H), 4.22 (d, *J* = 14.1 Hz, 1H), 3.66 (d, *J* = 14.1 Hz, 1H), 3.12 - 3.09 (m, 1H), 2.75-2.65 (m, 1H), 2.56-2.46 (m, 1H), 2.13-2.06 (m, 1H), 1.88 - 1.71 (m, 5H), 1.63 - 1.44 (m, 1H), 1.26 (d, *J* = 6.3 Hz, 3H).

Preparation of Example 14

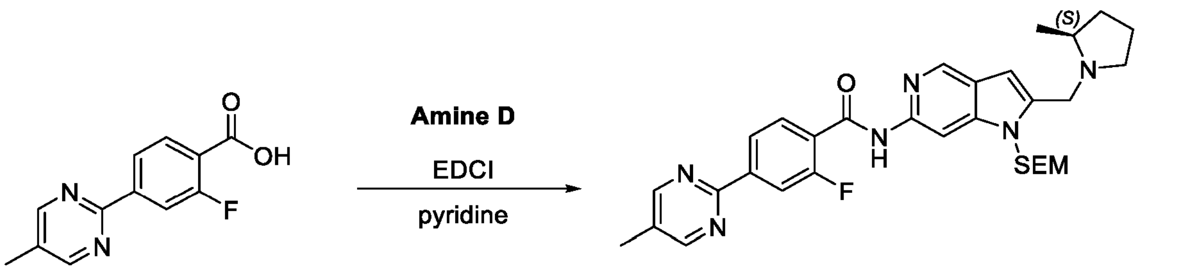


**Acid K**

Into a 8-mL vial purged and maintained with an inert atmosphere of nitrogen, was placed 2-[[(2S)-2-methylpyrrolidin-1-yl]methyl]-1-[[2-(trimethylsilyl)ethoxy]methyl]pyrrolo[3,2-c]pyridin-6-amine **(Amine D,** 124.22 mg, 0.345 mmol, 1.00 equiv), pyridine (2.5 mL), 2-fluoro-4-(5-methylpyrimidin-2-yl)benzoic acid (80.0 mg, 0.345 mmol, 1.00 equiv), EDCI (99.06 mg, 0.517 mmol, 1.50 equiv). The resulting solution was stirred for 16 h at 50 °C in an oil bath. The resulting mixture was concentrated under vacuum. The resulting solution was diluted with 30 mL of $H_2O$. The resulting solution was extracted with 2x20 mL of ethyl acetate and the organic layers combined. The resulting mixture was washed with 2 x20 mL of brine. The mixture was dried over anhydrous sodium sulfate and concentrated under vacuum. This resulted in 200 mg (70.5%) of 2-fluoro-4-(5-methylpyrimidin-2-yl) -N-(2-[[(2S)-2-methylpyrrolidin-1-yl]methyl]-1-[[2-(trimethylsilyl)ethoxy]methyl]pyrrolo[3,2-c]pyridin-6-yl)benzamide as brown oil. **LCMS:** $[M+H]^+$=575.

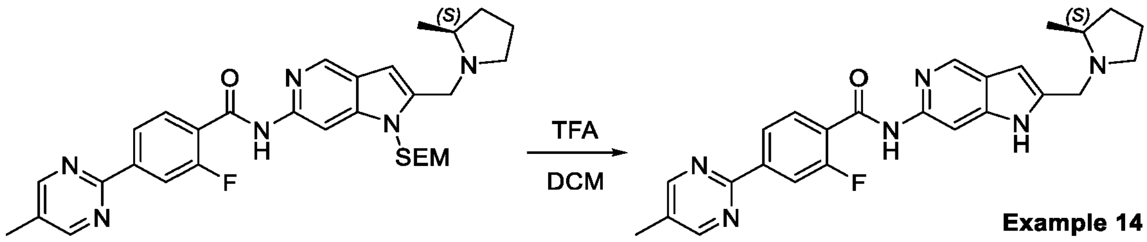


**Example 14**

Into a 8-mL vial, was placed 2-fluoro-4-(5-methylpyrimidin-2-yl)-N-(2-[[(2S)-2-methylpyrrolidin-1-yl]methyl]-1 -[[2-(trimethylsilyl)ethoxy]methyl]pyrrolo[3,2-c]pyridin-6-yl)benzamide (200 mg, 0.348 mmol, 1.00 equiv), DCM (2.0 mL), TFA (2.0 mL). The resulting solution was stirred for 6 h at room temperature. The resulting mixture was concentrated under vacuum. The pH value of the solution was adjusted to 8 with $Et_2NH$. The crude product (100 mg) was purified by Prep-HPLC with the following conditions Column, XBridge Prep C18 OBD Column,, 5um,19x150mm; mobile phase, Water(0.05%$NH_3H_2O$) and ACN (25% PhaseB up to 50% in 7 min); Detector, UV 254 nm. This resulted in 38.3 mg (24.76%) of 2-fluoro-4-(5-methylpyrimidin-2-yl)-N-(2-[[(2S)-2-methylpyrrolidin-1-yl]methyl]-1H-pyrrolo[3,2-c]pyridin-6-yl) benzamide as a yellow solid. **LCMS:** $[M+H]^+$=445. **$^1$H-NMR:** (300 MHz, Methanol-*d*$_4$, ppm) δ 8.77 (d, *J* = 0.8 Hz, 2H), 8.54 (d, *J* = 1.0 Hz, 1H), 8.39 (dd, *J* = 8.2, 1.5 Hz, 1H), 8.34-8.23 (m, 2H), 8.05 (t, *J* = 7.9 Hz, 1H), 6.56 (s, 1H), 4.21 (d, *J* = 13.9 Hz, 1H), 3.65 (d, *J* = 14.0 Hz, 1H), 3.11 (m, 1H), 2.68 (s, 1H), 2.48 (d, *J* = 9.7 Hz, 1H), 2.41 (s, 3H), 2.15-2.02 (m, 1H), 1.80 (m, 2H), 1.54 (m, 1H), 1.26 (d, *J*= 6.1 Hz, 3H). **$^{19}$F-NMR:** (300 MHz, Methanol-*d*$_4$, ppm): δ-115.236.

Preparation of Example 16

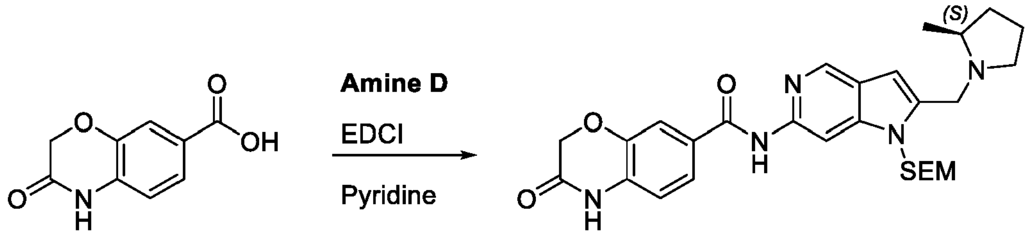


**Acid CG**

Into a 8-mL sealed tube, was placed 3-oxo-2,4-dihydro-1,4-benzoxazine-7-carboxylic acid (CAS: 214848-62-1, 35.36 mg, 0.183 mmol, 1.10 equiv), 2-[[(2S)-2-methylpyrrolidin-1-yl]methyl]-1-[[2-(trimethylsilyl)ethoxy]methyl]pyrrolo [3,2-c]pyridin-6-amine **(Amine D,** 60.00 mg, 0.166 mmol, 1.00 equiv), EDCI (47.85 mg, 0.249 mmol, 1.50 equiv), and pyridine (1.00 mL). The resulting solution was stirred for 1h at room temperature. The resulting mixture was concentrated. The resulting solution was extracted with 2x20 mL of ethyl acetate and the organic layers combined. This resulted in 200 mg (crude) of N-(2-[[(2S)-2-methylpyrrolidin-1-yl]methyl]-1-[[2-(trimethylsilyl)ethoxy]methyl]pyrrolo[3,2-c]pyridin-6-yl)-3-oxo-2,4-dihydro-1,4-benzoxazine-7-carboxamide as Light brown a solid. **LCMS:** $[M+H]^+$=536.

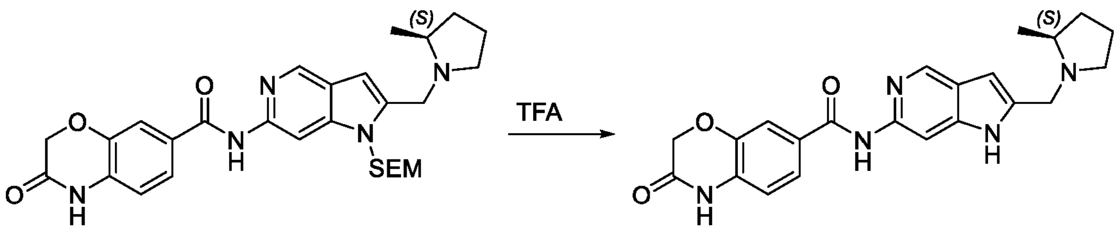


**Example 16**

Into a 8-mL sealed tube, was placed N-(2-[[(2S)-2-methylpyrrolidin-1-yl]methyl]-1-[[2-(trimethylsilyl)ethoxy] methyl]pyrrolo[3,2-c]pyridin-6-yl)-3-oxo-2,4-dihydro-1,4-benzoxazine-7-carboxamide (200.0 mg, 0.373 mmol, 1.00 equiv), TFA (0.5 mL), DCM (0.5 mL). The resulting solution was stirred for 2h at room temperature. The resulting mixture was concentrated. The pH value of the solution was adjusted to 7 with $NaHCO_3$(aq). The solids were collected by filtration. The crude product (100 mg) was purified by Prep-HPLC with the following conditions: Column, XBridge Prep C18 OBD Column,, 5um, 19x150m ; mobile phase, Water(0.05%$NH_3H_2O$) and ACN (15% PhaseB up to 45% in 7 min); Detector, UV

254nm. This resulted in 21.7 mg (17.3% for 2 steps) of N-(2-[[(2S)-2-methylpyrrolidin-1-yl]methyl]-1H-pyrrolo[3,2-c]pyridin-6-yl)-3-oxo-2,4-dihydro-1,4-benzoxazine-7-carboxamide as a brown solid. **LCMS:** (ES, m/z): $[M+H]^+$=389. **$^1$H-NMR:** (300 MHz,DMSO-$d_6$, *ppm):* δ11.33 (s, 1H), 10.96 (s, 1H), 10.33 (s, 1H), 8.50 (s, 1H), 8.18 (d, *J* = 1.2 Hz, 1H), 7.77 - 7.64 (m, 2H), 6.97 (d, *J* = 8.2 Hz, 1H), 6.36 (s, 1H), 4.65 (s, 2H), 4.01 (d, *J* = 13.9 Hz, 1H), 3.40 (d, *J* = 13.8 Hz, 1H), 2.89 (d, *J* = 6.4 Hz, 1H), 2.43 (q, *J* = 6.9 Hz, 1H), 2.19 (q, *J* = 8.8 Hz, 1H), 1.93-1.90 (m,1H), 1.63 (d, *J* = 8.1 Hz, 1H), 1.10 (d, *J* = 6.0 Hz, 3H).

Preparation of Example 17

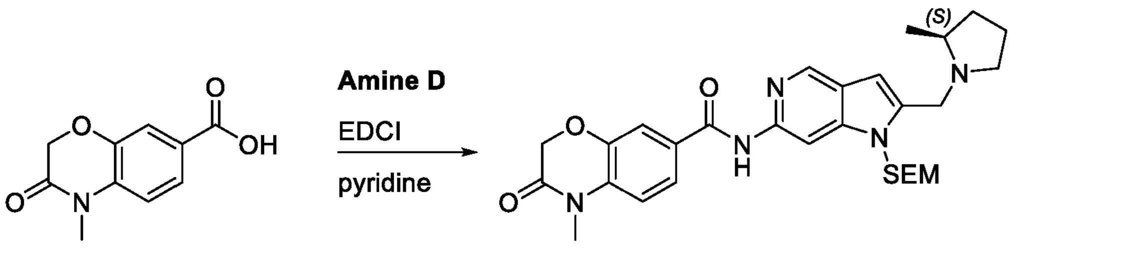


**Acid CH**

Into a 8-mL sealed tube, was placed 2-[[(2S)-2-methylpyrrolidin-1-yl]methyl]-1-[[2-(trimethylsilyl)ethoxy]methyl]pyrrolo[3,2-c]pyridin-6-amine **(Amine D,** 145.00 mg, 0.402 mmol, 1.00 equiv), 4-methyl-3-oxo-2H-1,4-benzoxazine-7-carboxylic acid (99.98 mg, 0.483 mmol, 1.20 equiv), EDCI (115.63 mg, 0.603 mmol, 1.50 equiv), and pyridine (5.00 mL). The resulting solution was stirred for 20 h at 50 °C in an oil bath. The resulting mixture was concentrated. The reaction was then quenched by the addition of 20 mL of water. The resulting solution was extracted with 2x20 mL of ethyl acetate and the organic layers combined and concentrated. This resulted in 200 mg (crude) of 4-methyl-N-(2-[[(2S)-2-methylpyrrolidin-1-yl]methyl]-1-[[2-(trimethylsilyl)ethoxy]methyl]pyrrolo[3,2-c]pyridin-6-yl)-3-oxo-2H-1,4-benzoxazine-7-carboxamide as brown oil. **LCMS:** $[M+H]^+$=550.

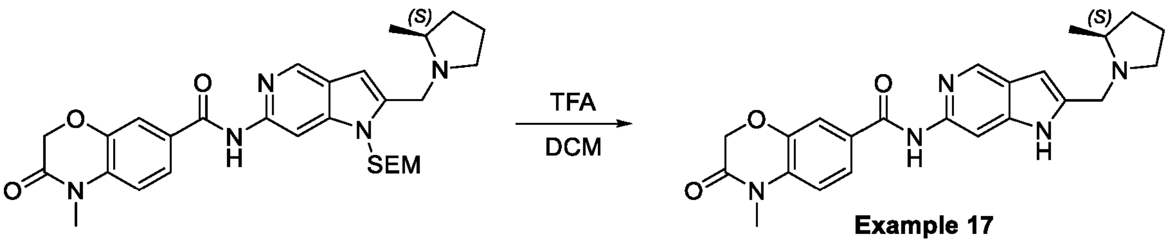


**Example 17**

Into a 50-mL round-bottom flask, was placed 4-methyl-N-(2-[[(2S)-2-methylpyrrolidin-1-yl]methyl]-1-[[2-(trimethylsilyl)ethoxy]methyl]pyrrolo[3,2-c]pyridin-6-yl)-3-oxo-2H-1,4-benzoxazine-7-carboxamide (200.00 mg, 0.364 mmol, 1.00 equiv), TFA (2 mL), DCM (1mL). The resulting solution was stirred for 10 h at room temperature. The resulting mixture was concentrated. The reaction was then quenched by the addition of 20 mL of water. The pH value of the solution was adjusted to 7 with $NaHCO_3$(aq). The resulting solution was extracted with 2x20 mL of ethyl acetate and the organic layers combined and concentrated. The crude product (130 mg) was purified by Prep-HPLC with the following conditions: Column, XBridge Prep C18 OBD Column, 5um, 19x150mm; mobile phase, Water(0.05%$NH_3H_2O$) and ACN (27% PhaseB up to 60% in 7 min); Detector, UV. 254nm. This resulted in 25.1 mg (12.41% for two steps) of 4-methyl-N-(2-[[(2S)-2-methylpyrrolidin-1-ylmethyl]-1H-pyrrolo[3,2-c]pyridin-6-yl)-3-oxo-2H-1,4-benzoxazine-7-carboxamide as a light brown solid. LCMS: $[M+H]^+$=420. **$^1$H-NMR:** (300 MHz,DMSO-$d_6$, *ppm):* δ8.54 (d, *J* = 1.0 Hz, 1H), 8.18 (t, *J* = 1.0 Hz, 1H), 7.77 (dd, *J* = 8.4, 2.1 Hz, 1H), 7.66 (d, *J* = 2.0 Hz, 1H), 7.31 (d, *J* = 8.5 Hz, 1H), 6.52 (s, 1H), 4.72 (s, 2H), 4.14 (d, *J* = 13.9 Hz, 1H), 3.54 (d, *J* = 13.9 Hz, 1H), 3.44 (s, 3H), 3.37 (s, 3H), 3.04 (t, *J* = 6.7 Hz, 1H), 2.55-2.50 (m, 1H), 2.37-2.30 (m, 1H), 2.05 (t, *J* = 6.8 Hz, 1H), 1.76 (dd, *J* = 10.4, 4.7 Hz, 2H), 1.52 (dd, *J* = 18.7, 9.4 Hz, 1H), 1.23 (d, *J* = 6.1 Hz, 3H).

Preparation of Example 20

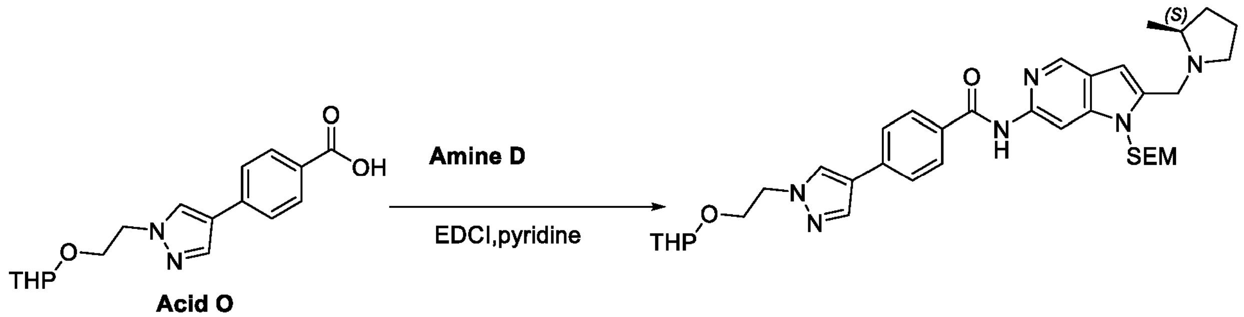


Acid O

Into a 40-mL vial, was placed a solution of 4-[1-[2-(oxan-2-yloxy)ethyl]pyrazol-4-yl]benzoic acid (120.00 mg, 0.379 mmol, 1.00 equiv) in pyridine(10 mL), 2-[[(2S)-2-methylpyrrolidin-1-yl]methyl]-1-[[2-(trimethylsilyl)ethoxy]methyl] pyrrolo[3,2-c]pyridin-6-amine **(Amine D,** 136.77 mg, 0.379 mmol, 1.00 equiv) and EDCI (109.07 mg, 0.569 mmol, 1.50 equiv). The resulting solution was stirred for 5 h at room temperature. The resulting mixture was concentrated under vacuum. The resulting solution was diluted with 20 mL of EA. The resulting mixture was washed with 2x10 mL of brine. The mixture was dried over anhydrous sodium sulfate and concentrated under vacuum. This resulted in 100 mg (40.01%) of N-(2-[[(2S)-2-methylpyrrolidin-1-yl]methyl]-1-[[2-(trimethylsilyl)ethoxy]methyl]pyrrolo[3,2-c]pyridin-6-yl)-4-[1-[2-(oxan-2-yloxy)ethyl]pyrazol-4-yl]benzamide as yellow oil. **LCMS:** 659.

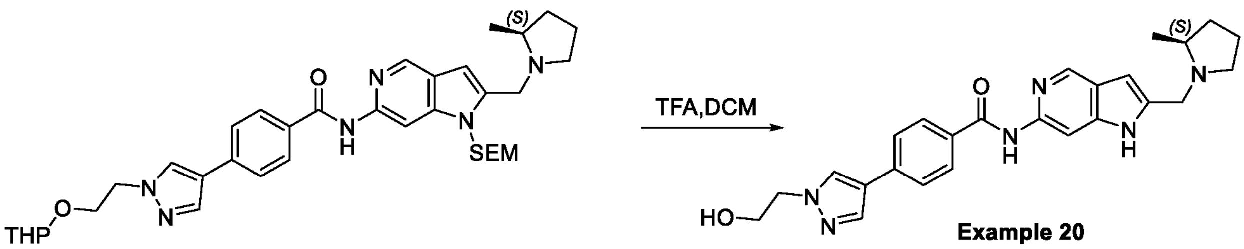


Example 20

Into a 20-mL vial, was placed a solution of N-(2-[[(2S)-2-methylpyrrolidin-1-yl]methyl]-1-[[2-(trimethylsilyl) ethoxy]methyl]pyrrolo[3,2-c]pyridin-6-yl)-4-[1-[2-(oxan-2-yloxy)ethyl]pyrazol-4-yl]benzamide (100.0 mg, 0.152 mmol, 1.00 equiv) in DCM (5 mL) and TFA (2.00 mL, 26.926 mmol, 177.42 equiv). The resulting solution was stirred for 5h at room temperature and concentrated under vacuum. The crude product was purified by Prep-HPLC with the following conditions: Column, XBridge Prep C18 OBD Column, 5um,19x150mm; mobile phase, Water(0.05%$NH_3$.$H_2O$) and ACN (29% PhaseB up to 46% in 7 min); Detector, UV 254nm. This resulted in 23 mg of 4-[1-(2-hydroxyethyl)pyrazol-4-yl]-N-(2-[[(2S)-2-methylpyrrolidin-1-yl]methyl]-1H-pyrrolo[3,2-c]pyridin-6-yl)benzamide as a white solid. **LCMS:** $[M+H]^+$=445. **$^1$H-NMR:** (300 MHz, Methanol-$d_4$, ppm): δ 8.55 (d, *J* = 1.0 Hz, 1H), 8.25 - 8.14 (m, 2H), 8.07 - 7.96 (m, 3H), 7.81 - 7.71 (m, 2H), 6.52 (s, 1H), 4.31 (m, 2H), 4.15 (d, *J* = 13.9 Hz, 1H), 3.95 (m, 2H), 3.55 (d, *J* = 13.9 Hz, 1H), 3.05 (s, 1H), 2.57 (m, 1H), 2.39 (m, 1H), 2.05 (m, 1H), 1.77 (s, 1H), 1.86 - 1.69 (m, 2H), 1.51 (s, 1H), 1.24 (d, *J* = 6.1 Hz, 3H).

Preparation of Example 21

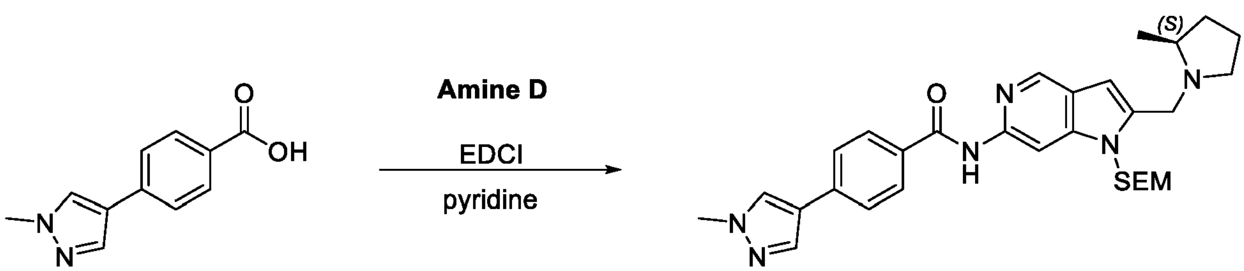


Acid P

Into a 20-mL vial, was placed a solution of 4-(1-methylpyrazol-4-yl)benzoic acid (100.00 mg, 0.495 mmol, 1.00 equiv) in pyridine(10mL), 2-[[(2S)-2-methylpyrrolidin-1-yl]methyl]-1-[[2-(trimethylsilyl)ethoxy]methyl]pyrrolo[3,2-c]pyridin-6-amine **(Amine D,** 178.32 mg, 0.495 mmol, 1.00 equiv), EDCI (142.20 mg, 0.742 mmol, 1.50 equiv). The resulting

solution was stirred for 12h at room temperature. The resulting solution was diluted with 20 mL of EA. The resulting mixture was washed with 3x10 mL of brine. The mixture was dried over anhydrous sodium sulfate and concentrated under vacuum. This resulted in 150 mg (crude) of 4-(1-methylpyrazol-4-yl)-N-(2-[[(2S)-2-methylpyrrolidin-1-yl]methyl]-1-[[2-(trimethylsilyl)ethoxy]methyl]pyrrolo[3,2-c]pyridin-6-yl)benzamide as yellow oil. **LCMS:** $[M+H]^+$=545.

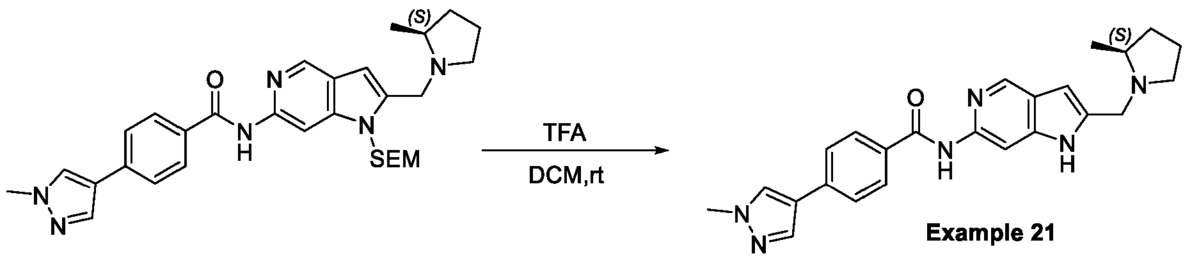


Example 21

Into a 20-mL vial, was placed 4-(1-methylpyrazol-4-yl)-N-(2-[[(2S)-2-methylpyrrolidin-1-yl]methyl]-1-[[2-(trimethylsilyl)ethoxy]methyl]pyrrolo[3,2-c]pyridin-6-yl)benzamide (150.00 mg, 0.275 mmol, 1.00 equiv), DCM (5.00 mL, 78.650 mmol, 285.64 equiv), TFA (5.00 mL, 0.044 mmol, 0.16 equiv). The resulting solution was stirred for 16 hr at room temperature. The resulting mixture was concentrated under vacuum. The crude product was purified by Prep-HPLC with the following conditions: Column, XBridge Prep C18 OBD Column, 5um, 19 x 150mm; mobile phase, Water(0.05% $NH_3.H_2O$) and ACN (33% PhaseB up to 55% in 7 min); Detector, UV 254nm.This resulted in 26 mg of 4-(1-methylpyrazol-4-yl)-N-(2-[[(2S)-2-methylpyrrolidin-1-yl]methyl]-1H-pyrrolo[3,2-c]pyridin-6-yl)benzamide as an white solid. **LCMS:** $[M+H]^+$=415. **$^1$H-NMR:** (300 MHz, Methanol-$d_4$, ppm) δ 8.59 (s, 1H), 8.25 (s, 1H), 8.11 (s, 1H), 8.07 - 7.97 (m, 2H), 7.95 (d, *J* = 0.8 Hz, 1H), 7.79 - 7.70 (m, 2H), 6.63 (s, 1H), 4.30 (d, *J* = 13.9 Hz, 1H), 3.97 (s, 3H), 3.79 (d, *J* = 14.0 Hz, 1H), 3.19 (m, 1H), 2.85 (s, 1H), 2.68 (s, 1H), 2.64 (s, 1H), 2.15 (m, 7.3 Hz, 1H), 1.86 (m, 2H), 1.68 - 1.49 (m, 1H), 1.30 (d, *J* = 6.2 Hz, 3H).

The following examples in **Table G** were prepared in a similar fashion to that shown above for **Example 21** using **Amine D** and the appropriate reagents and conditions.

Table G

| Ex. | Acid | Structure | LCMS | $^1$H-NMR |
|---|---|---|---|---|
| 22 | Q | 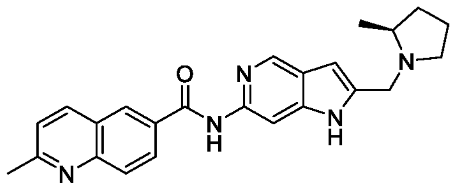 | 400 | (300 MHz, Methanol-$d_4$,ppm): δ 8.58 (dd, *J* = 7.6, 1.5 Hz, 2H), 8.42 (d, *J* = 8.5 Hz, 1H), 8.31 (m, 1H), 8.24 (m, 1H), 8.10 (d, *J* = 8.8 Hz, 1H), 7.56 (d, *J* = 8.5 Hz, 1H), 6.53 (s, 1H), 4.15 (d, *J* = 13.9 Hz, 1H), 3.54 (d, *J* = 13.8 Hz, 1H), 3.11 - 2.99 (m, 1H), 2.79 (s, 3H), 2.61 - 2.48 (m, 1H), 2.37 (m, 1H), 2.11 - 1.96 (m, 1H), 1.77 (s, 2H), 1.60 - 1.41 (m, 1H), 1.23 (d, *J* = 6.1 Hz, 3H). |
| 25 | CI | 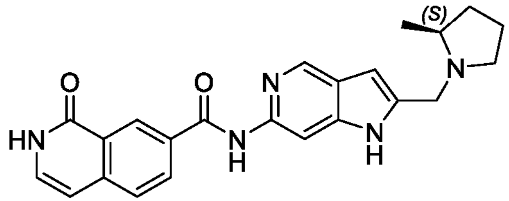 | 402 | (300 MHz, Methanol-d4): δ 11.44 - 11.33 (m, 2H), 10.73 (s, 1H), 8.83 (d, *J* = 1.9 Hz, 1H), 8.53 (s, 1H), 8.31 (dd, *J* = 8.3 Hz, 1H), 8.25 (d, *J* = 1.1 Hz, 1H), 7.76 (d, *J* = 8.4 Hz, 1H), 7.35 - 7.25 (m, 1H), 6.63 (d, *J* = 7.1 Hz, 1H), 6.38 (s, 1H), 4.02 (d, *J* = 13.8 Hz, 1H), 3.42 (d, *J* = 13.9 Hz, 1H), 2.89 (s, 1H), 2.44 (d, *J* = 6.9 Hz, 1H), 2.21 (m, 1H), 1.94 (dd, *J* = 13.1 Hz, 1H), 1.64 (t, *J* = 7.5 Hz, 2H), 1.38 (m, 1H), 1.11 (d, *J* = 6.0 Hz, 3H). |
| 26 | CJ | 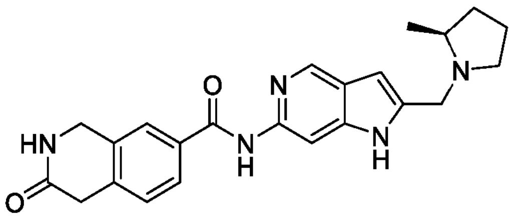 | 404 | (300 MHz, Methanol-$d_4$, ppm): δ 8.55 (d, *J* = 1.1 Hz, 1H), 8.19 (t, *J* = 1.0 Hz, 1H), 7.92 (d, *J* = 7.2 Hz, 2H), 7.42 (d, *J* = 8.1 Hz, 1H), 6.52 (s, 1H), 4.60 (s, 2H), 4.11 (t, *J* = 14.1 Hz, 1H), 3.69 (s, 2H), 3.53 (d, *J* = 13.9 Hz, 1H), 3.04 (m, 1H), 2.54 (m, 1H), 2.37 (m, 1H), 2.11 - 1.96 (m, 1H), 1.82 - 1.71 (m, 1H), 1.56 - 1.44 (m, 1H), 1.23 (d, *J* = 6.1 Hz, 3H). |
| 47 | AJ | 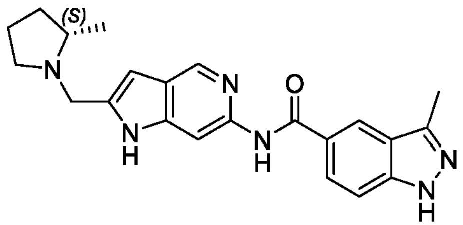 | 389 | (300 MHz, Methanol-$d_4$,*ppm*): δ 8.59-8.47 (m, 2H), 8.22 (d, *J* = 1.0 Hz, 1H), 8.05 (dd, *J* = 8.8, 1.7 Hz, 1H), 7.61 (dd, *J* = 8.8, 0.8 Hz, 1H), 6.53 (s, 1H), 4.15 (d, *J* = 13.9 Hz, 1H), 3.54 (d, *J* = 13.9 Hz, 1H), 3.10-3.00 (m, 1H), 2.67 (s, 3H), 2.60-2.48 (m, 1H), 2.40-2.30(m, 1H), 2.10-2.00 (m, 1H), 1.86-1.69 (m, 2H), 1.60-1.41 (m, 1H), 1.24 (d, *J* = 6.1 Hz, 3H). |
| 48 | AK | 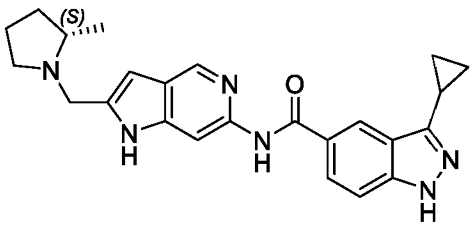 | 415 | (300 MHz, Methanol-$d_4$,*ppm*): δ 8.56 (dd, *J* = 1.7, 0.8 Hz, 2H), 8.23 (d, *J* = 1.0 Hz, 1H), 8.03 (dd, *J* = 8.8, 1.7 Hz, 1H), 7.58 (dd, *J* = 8.8, 0.8 Hz, 1H), 6.52 (d, *J* = 0.9 Hz, 1H), 4.14 (d, *J* = 13.9 Hz, 1H), 3.53 (d, *J* = 13.9 Hz, 1H), 3.10-3.00 (m, 1H), 2.61-2.50 (m, 1H), 2.50-2.29 (m, 2H), 2.13-1.93 (m, 1H), 1.86-1.68 (m, 2H), 1.59-1.41 (m, 1H), 1.23 (d, *J* = 6.1 Hz, 3H), 1.20-1.13 (m, 2H), 1.13-1.10 (m, 2H). |

(continued)

| Ex. | Acid | Structure | LCMS | $^1$H-NMR |
|---|---|---|---|---|
| **54** | **AQ** | 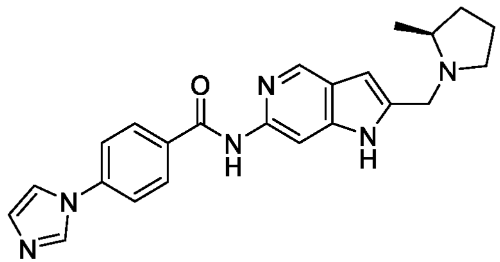 | **401** | (300 MHz, Methanol-$d_4$, ppm): δ 8.56 (d, $J$ = 1.0 Hz, 1H), 8.31 (m, 1H), 8.24 - 8.13 (m, 3H), 7.85 - 7.75 (m, 2H), 7.73 (m, 1H), 7.25 - 7.19 (m, 1H), 6.52 (s, 1H), 4.14 (d, $J$ = 13.8 Hz, 1H), 3.53 (d, $J$ = 13.9 Hz, 1H), 3.10 - 2.97 (m, 1H), 2.54 (m, 1H), 2.36 (m, 1H), 2.11 - 1.96 (m, 1H), 1.77 (s, 2H), 1.82 - 1.68 (m, 1H), 1.59 - 1.44 (m, 1H), 1.23 (d, $J$ = 6.1 Hz, 3H). |
| **55** | **AR** | 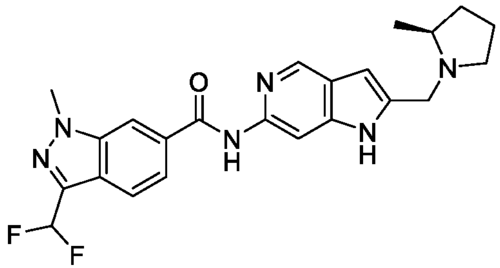 | **439** | (PH-PUK) (300 MHz, Methanol-$d_4$,*ppm*): δ 8.57 (d, $J$ = 1.0 Hz, 1H), 8.34 (t, $J$ = 1.2 Hz, 1H), 8.24 (d, $J$ = 1.1 Hz, 1H), 8.02 (d, $J$ = 8.6 Hz, 1H), 7.89 (dd, $J$ = 8.5, 1.4 Hz, 1H), 7.11 (t, $J$ = 54.3 Hz, 1H), 6.53 (s, 1H), 4.23 (t, $J$ = 1.3 Hz, 3H), 4.15 (d, $J$ = 13.9 Hz, 1H), 3.54 (d, $J$ = 13.9 Hz, 1H), 3.11-2.99 (m, 1H), 2.61-2.46 (m, 1H), 2.45-2.30 (m, 1H), 2.2-1.98 (m, 1H), 1.87-1.70 (m, 2H), 1.60-1.44 (m, 1H), 1.24 (d, $J$ = 6.1 Hz, 3H).<br>$^{19}$F-NMR (300 MHz, Methanol-$d_4$,*ppm*):-112.9. |
| **56** | **AS** | 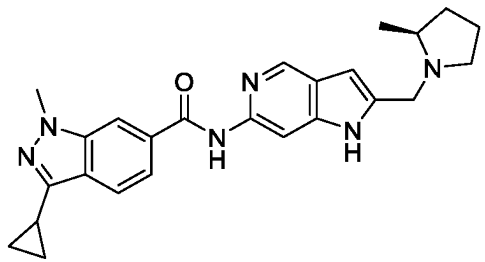 | **429** | (300 MHz, Methanol-$d_4$,*ppm*): δ 8.57 (d, $J$ = 1.0 Hz, 1H), 8.25 (d, $J$ = 1.0 Hz, 1H), 8.18 (t, $J$ = 1.1 Hz, 1H), 7.93 (dd, $J$ = 8.5, 0.8 Hz, 1H), 7.74 (dd, $J$ = 8.5, 1.5 Hz, 1H), 6.53 (s, 1H), 4.15 (d, $J$ = 13.9 Hz, 1H), 4.08 (s, 3H), 3.54 (d, $J$ = 13.9 Hz, 1H), 3.12-3.00 (m, 1H), 2.61-2.46 (m, 1H), 2.45-2.25 (m, 2H), 2.14-1.97 (m, 1H), 1.87-1.69 (m, 2H), 1.60-1.42 (m, 1H), 1.24 (d, $J$ = 6.1 Hz, 3H), 1.17-1.01 (m, 4H). |
| **58** | **AU** | 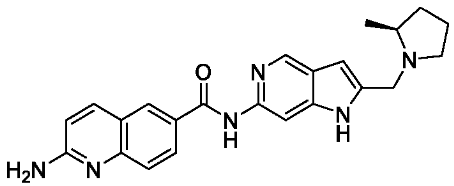 | **401** | (300 MHz, Methanol-$d_4$, ppm) δ 8.55 (d, $J$ = 1.3 Hz, 1H), 8.35 (t, $J$ = 1.8 Hz, 1H), 8.22 (d, $J$ = 1.3 Hz, 1H), 8.18-8.03 (m, 2H), 7.63 (d, $J$ = 8.8 Hz, 1H), 6.93 (dd, $J$ = 8.9, 1.3 Hz, 1H), 6.51 (s, 1H), 4.14 (d, $J$ = 13.9 Hz, 1H), 3.53 (d, $J$ = 13.9 Hz, 1H), 3.04 (s, 1H), 2.55 (m, 1H), 2.36 (m, 1H), 2.05 (m, 1H), 1.75 (d, $J$ = 9.1 Hz, 2H), 1.59-1.46 (m, 1H), 1.23 (dd, $J$ = 6.1, 1.3 Hz, 3H). |

(continued)

| Ex. | Acid | Structure | LCMS | $^1$H-NMR |
|---|---|---|---|---|
| **59** | **AV** | 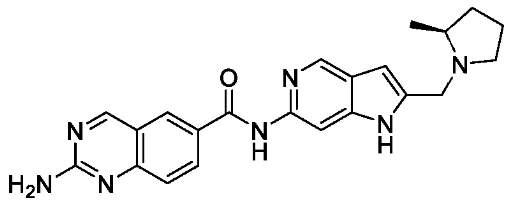 | **402** | (300 MHz, Methanol-$d_4$, ppm): δ 9.24 (d, *J* = 0.8 Hz, 1H), 8.60 - 8.47 (m, 2H), 8.32 (dd, *J* = 8.9, 2.2 Hz, 1H), 8.21 (s, 1H), 7.61 (d, *J* = 8.9 Hz, 1H), 6.52 (s, 1H), 4.14 (d, *J* = 13.9 Hz, 1H), 3.54 (d, *J* = 14.0 Hz, 1H), 3.04 (s, 1H), 2.58 - 2.48 (m, 1H), 2.35 (m, 1H), 1.76 (d, *J* = 8.7 Hz, 3H), 1.50 (s, 1H), 1.23 (d, *J* = 6.1 Hz, 3H). |
| **60** | **AW** | 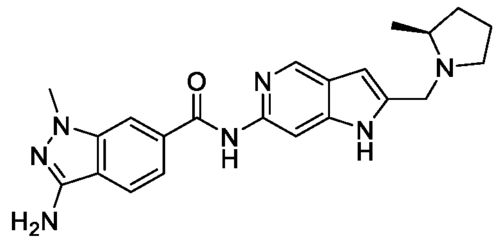 | **404** | (300 MHz, Methanol-$d_4$,*ppm*): δ 8.57 (d, *J* = 1.0 Hz, 1H), 8.25 (d, *J = 1.0* Hz, 1H), 8.05 - 7.99 (m, 1H), 7.84 (dd, *J* = 8.5, 0.8 Hz, 1H), 7.61 (dd, *J* = 8.4, 1.5 Hz, 1H), 6.54 (s, 1H), 4.16 (d, *J* = 13.9 Hz, 1H), 3.92 (s, 3H), 3.56 (d, *J* = 13.9 Hz, 1H), 3.14-3.01 (m, 1H), 2.63-2.51 (m, 1H), 2.47-2.33 (m, 1H), 2.14-1.98 (m, 1H), 1.88-1.70 (m, 2H), 1.61-1.42 (m, 1H), 1.25 (d, *J* = 6.1 Hz, 3H). |
| **62** | **CK** | 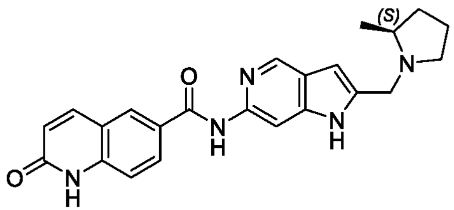 | **402** | (300 MHz, Methanol-$d_4$, ppm) δ 8.56 (d, *J* = 1.0 Hz, 1H), 8.37 (d, *J* = 2.0 Hz, 1H), 8.23-8.14 (m, 2H), 8.10 (d, *J* = 9.5 Hz, 1H), 7.49 (d, *J* = 8.7 Hz, 1H), 6.72 (d, *J* = 9.5 Hz, 1H), 6.52 (s, 1H), 4.14 (d, *J* = 13.9 Hz, 1H), 3.53 (d, *J* = 13.9 Hz, 1H), 3.10-2.98 (m, 1H), 2.54 (m, 1H), 2.36 (m, 1H), 2.13-1.93 (m, 1H), 1.74 (m, 2H), 1.59-1.44 (m, 1H), 1.23 (d, *J* = 6.1 Hz, 3H). |
| **67** | **BB** | 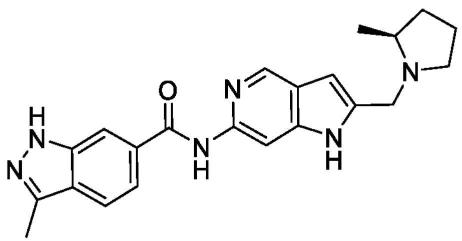 | **389** | (300 MHz, DMSO-$d_6$,*ppm*): δ 12.97 (s, 1H), 11.41-11.24 (m, 1H), 10.55 (s, 1H), 8.54 (s, 1H), 8.29-8.09 (m, 2H), 7.87-7.63 (m, 2H), 6.38 (d, *J* = 1.7 Hz, 1H), 4.03 (d, *J* = 13.9 Hz, 1H), 3.42 (d, *J* = 13.9 Hz, 1H), 2.96-2.76 (m, 1H), 2.55 (s, 3H), 2.49-2.37 (m, 1H), 2.28-2.15 (m, 1H), 2.02-1.89 (m, 1H), 1.64 (d, *J* = 8.3 Hz, 2H), 1.47-1.30 (m, 1H), 1.12 (d, *J* = 6.1 Hz, 3H). |

(continued)

| Ex. | Acid | Structure | LCMS | $^1$H-NMR |
|---|---|---|---|---|
| 71 | BD | 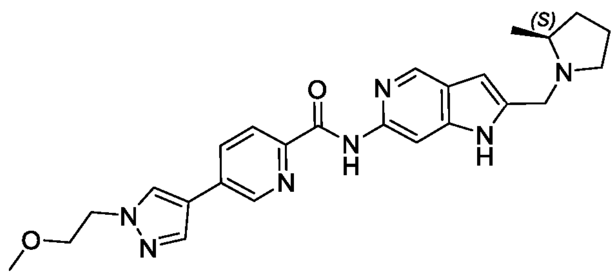 | 459 | (300 MHz, Methanol-$d_4$, ppm): δ 8.96 (s, 1H), 8.52 (d, *J* = 1.1 Hz, 1H), 8.39 (d, *J* = 1.0 Hz, 1H), 8.30 - 8.15 (m, 3H), 8.07 (s, 1H), 6.52 (s, 1H), 4.39 (m,, 2H), 4.13 (d, *J* = 13.9 Hz, 1H), 3.80 (m, 2H), 3.53 (d, *J* = 13.9 Hz, 1H), 3.36 (s, 3H), 3.03 (d, *J* = 7.0 Hz, 1H), 2.54 (d, *J* = 6.0 Hz, 1H), 2.36 (m, 1H), 2.10 - 1.95 (m, 1H), 1.76 (s, 2H), 1.49 (s, 1H), 1.23 (d, *J* = 6.1 Hz, 3H). |
| 74 | BF | 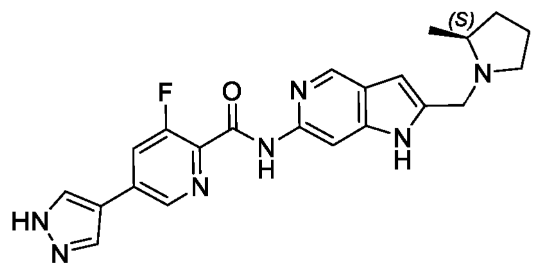 | 420 | (300 MHz, Methanol-$d_4$, ppm): δ 8.85 (d, *J* = 1.8 Hz, 1H), 8.53 (d, *J* = 1.0 Hz, 1H), 8.37 (s, 1H), 8.27 (s, 2H), 8.04 (dd, *J* = 12.4, 1.8 Hz, 1H), 6.52 (s, 1H), 4.59 (s, 4H), 4.14 (d, *J* = 13.9 Hz, 1H), 3.53 (d, *J* = 13.9 Hz, 1H), 3.04 (s, 1H), 2.54 (d, *J* = 7.3 Hz, 1H), 2.37 (m, 1H), 2.05 (s, 1H), 1.76 (d, *J* = 8.9 Hz, 2H), 1.50 (s, 1H), 1.23 (d, *J* = 6.1 Hz, 3H). |
| 76 | BH | 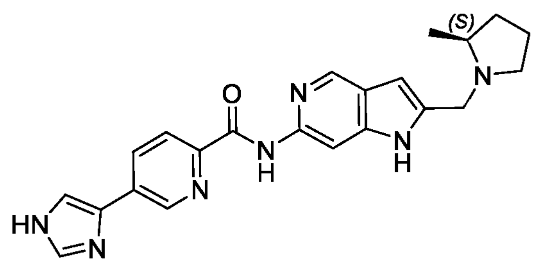 | 402 | (300 MHz, Methanol-$d_4$,*ppm*): δ 9.14 (d, *J* = 2.1 Hz, 1H), 8.54 (d, *J* = *1.0* Hz, 1H), 8.41 (t, *J* = 1.0 Hz, 1H), 8.38 - 8.32 (m, 1H), 8.28 (dd, *J* = 8.2, 0.9 Hz, 1H), 7.89 (d, *J* = 1.1 Hz, 1H), 7.81 (s, 1H), 6.53 (s, 1H), 4.15 (d, *J* = 13.9 Hz, 1H), 3.54 (d, *J* = 13.9 Hz, 1H), 3.12-2.99 (m, 1H), 2.62-2.49 (m, 1H), 2.46-2.31 (m, 1H), 2.12-1.97 (m, 1H), 1.78 (s, 2H), 1.87-1.69 (m, 1H), 1.61-1.42 (m, 1H), 1.24 (d, *J* = 6.1 Hz, 3H). |
| 77 | BI | 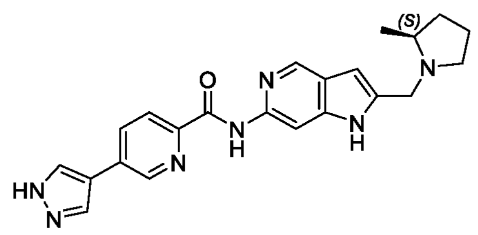 | 402 | (300 MHz, Methanol-$d_4$, ppm) δ 9.04-8.97 (m, 1H), 8.53 (d, *J* = 1.0 Hz, 1H), 8.40 (t, *J* = 1.0 Hz, 1H), 8.25 (m, 4H), 6.52 (s, 1H), 4.13 (d, *J* = 14.0 Hz, 1H), 3.53 (d, *J* = 13.9 Hz, 1H), 3.11-2.98 (m, 1H), 2.54 (m, 1H), 2.36 (m, 1H), 2.04 (m, 1H), 1.77 (m, 2H), 1.59-1.44 (m, 1H), 1.23 (d, *J* = 6.1 Hz, 3H). |

(continued)

| Ex. | Acid | Structure | LCMS | $^1$H-NMR |
|---|---|---|---|---|
| 78 | BJ | 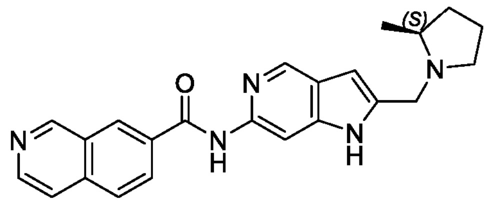 | 386 | (300 MHz, Methanol-d4,ppm): 9.43 (s, 1H),8.80 (s, 1H) ,δ 8.58 (dd, J = 7.6, 1.5 Hz, 2H), 8.42 (d, J = 8.5 Hz, 1H), 8.31 (dd, J = 8.8, 2.1 Hz, 1H), 8.24 (m, 1H), 8.10 (d, J = 8.8 Hz, 1H), 7.56 (d, J = 8.5 Hz, 1H), 6.53 (s, 1H), 4.15 (d, J = 13.9 Hz, 1H), 3.54 (d, *J* = 13.8 Hz, 1H), 3.11 - 2.99 (m, 1H), 2.79 (s, 3H), 2.61 - 2.48 (m, 1H), 2.37 (m, 1H), 2.11 - 1.96 (m, 1H), 1.77 (s, 2H), 1.60 - 1.41 (m, 1H), 1.23 (d, J = 6.1 Hz, 3H) |
| 79 | BK | 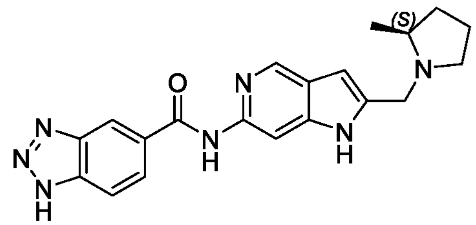 | 376 | (300 MHz, Methanol-$d_4$, ppm): δ 8.65 - 8.55 (m, 2H), 8.23 (m, 1H), 8.09 (dd, J = 8.7, 1.6 Hz, 1H), 7.99 (dd, J = 8.7, 0.8 Hz, 1H), 6.56 (s, 1H), 4.20 (d, J = 13.9 Hz, 1H), 3.63 (d, J = 13.9 Hz, 1H), 3.10 (m, 1H), 2.73 - 2.59 (m, 1H), 2.48 (m, 1H), 2.17 - 2.00 (m, 1H), 1.80 (m, 2H), 1.63 - 1.44 (m, 1H), 1.26 (d, J = 6.1 Hz, 3H). |
| 80 | BL | HCl 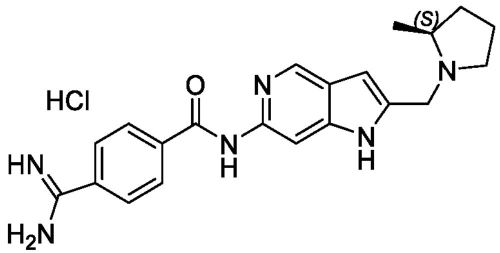 | 377 | (300 MHz, Methanol-$d_4$,ppm): δ 9.01 (s, 1H), 8.35 (d, *J* = 8.1 Hz, 2H), 8.04 (d, *J* = 7.8 Hz, 3H), 7.29 (s, 1H), 4.57 (d, *J* = 13.6 Hz, 1H), 3.72 (d, *J* = 7.8 Hz, 1H), 3.62 (s, 1H), 2.43 (d, *J* = 9.4 Hz, 1H), 2.13(m, 2H),1.85 (s, 1H), 1.55 (d, *J* = 6.5 Hz, 3H). |
| 81 | BM | 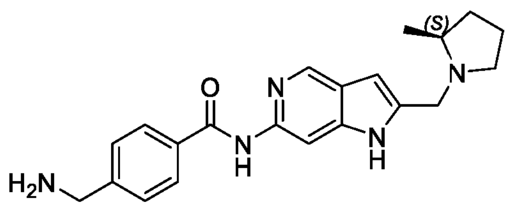 | 364 | (300 MHz, Methanol-$d_4$,ppm): δ 8.54 (d, *J* = 1.0 Hz, 1H), 8.20 (t, *J* = 1.0 Hz, 1H), 8.05 - 7.95 (m, 2H), 7.58 - 7.49 (m, 2H), 6.51 (d, *J* = 0.9 Hz, 1H), 4.13 (d, *J* = 13.9 Hz, 1H), 3.91 (s, 2H), 3.52 (d, *J* = 13.9 Hz, 1H), 3.03 (m, 1H), 2.60 - 2.46 (m, 1H), 2.35 (q, *J* = 9.0 Hz, 1H), 2.13 - 1.93 (m, 1H), 1.76 (m, 2H), 1.59 - 1.40 (m, 1H), 1.22 (d, *J* = 6.1 Hz, 3H). |

(continued)

| Ex. | Acid | Structure | LCMS | $^1$H-NMR |
|---|---|---|---|---|
| **82** | **BN** | 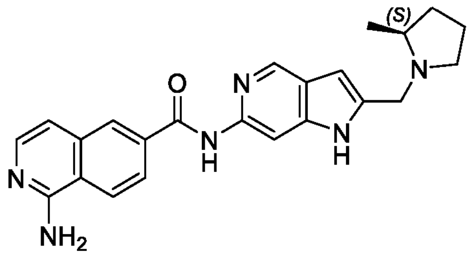 | **401** | (300 MHz, Methanol-$d_4$, ppm) δ 8.56 (d, $J$ = 1.0 Hz, 1H), 8.36 (s, 1H), 8.32-8.20 (m, 2H), 8.05 (dd, $J$ = 8.7, 1.8 Hz, 1H), 7.85 (d, $J$ = 6.0 Hz, 1H), 7.15 (d, $J$ = 6.0 Hz, 1H), 6.52 (s, 1H), 4.14 (d, $J$ = 13.9 Hz, 1H), 3.53 (d, $J$ = 13.8 Hz, 1H), 3.04 (m, 1H), 2.54 (m, 1H), 2.36 (m, 1H), 2.01 (m, 1H), 1.75 (d, $J$ = 8.4 Hz, 2H), 1.49 (m, 1H), 1.23 (d, $J$ = 6.1 Hz, 3H). |
| **83** | **BO** | 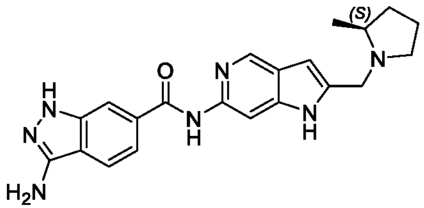 | **390** | (300 MHz, Methanol-$d_4$, ppm) δ 8.55 (d, $J$ = 1.0 Hz, 1H), 8.23 (d, $J$ = 1.0 Hz, 1H), 8.01-7.94 (m, 1H), 7.85 (dd, $J$ = 8.4, 0.8 Hz, 1H), 7.60 (dd, $J$ = 8.4, 1.5 Hz, 1H), 6.51 (s, 1H), 4.13 (d, $J$ = 13.8 Hz, 1H), 3.53 (d, $J$ = 13.9 Hz, 1H), 3.04 (m, 1H), 2.60-2.47 (m, 1H), 2.36 (m, 1H), 2.03 (d, $J$ = 7.6 Hz, 1H), 1.73 (m, 2H), 1.49 (m, 1H), 1.22 (d, $J$ = 6.1 Hz, 3H). |
| **84** | **BP** | 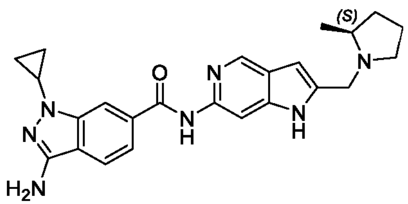 | **430** (PH-PUK) | **$^1$H-NMR**14 (PH-PUK): (300 MHz, Methanol-$d_4$, ppm) δ 8.55 (d, $J$ = 1.0 Hz, 1H), 8.24 (d, $J$ = *1.0* Hz, 1H), 8.16-8.09 (m, 1H), 7.82 (dd, $J$ = 8.4, 0.8 Hz, 1H), 7.63 (dd, $J$ = 8.4, 1.5 Hz, 1H), 6.55-6.48 (m, 1H), 4.13 (d, $J$ = 13.9 Hz, 1H), 3.52 (d, $J$ = 13.9 Hz, 1H), 3.47-3.35 (m, 1H), 3.04 (m, 1H), 2.60-2.47 (m, 1H), 2.36 (m, 1H), 2.03 (m, 1H), 1.85-1.67 (m, 2H), 1.59-1.40 (m, 1H), 1.23 (d, $J$ = 6.1 Hz, 3H), 1.20-1.11 (m, 4H). |
| **98** | **CL** | 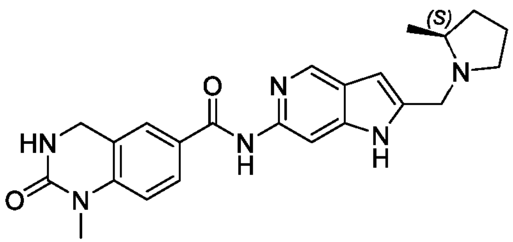 | **419** | (300 MHz, Methanol-$d_4$,ppm): δ 8.53 (d, $J$ = 1.0 Hz, 1H), 8.18 (m, 1H), 7.97 (dd, $J$ = 8.6, 2.2 Hz, 1H), 7.83 - 7.76 (m, 1H), 7.14 (d, $J$ = 8.6 Hz, 1H), 6.51 (d, $J$ = 0.9 Hz, 1H), 4.50 (s, 2H), 4.13 (d, $J$ = 13.9 Hz, 1H), 3.53 (d, $J$ = 13.9 Hz, 1H), 3.10 - 2.97 (m, 1H), 2.54 (m, 1H), 2.36 (m, 1H), 2.04 (m, 1H), 1.75 (m 2H), 1.59 - 1.40 (m, 1H), 1.22 (d, $J$ = 6.1 Hz, 3H). |

(continued)

| Ex. | Acid | Structure | LCMS | $^1$H-NMR |
|---|---|---|---|---|
| 100 | CA | 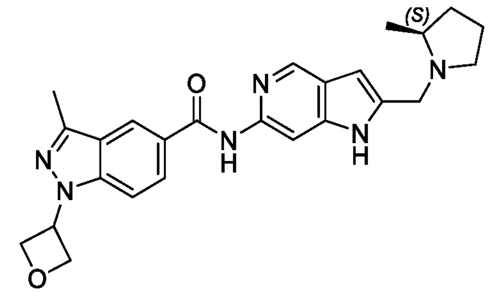 | 445 | (300 MHz, Methanol-$d_4$,*ppm*): δ8.56 (s, 1H), 8.49 (s, 1H), 8.21 (s, 1H), 8.11 - 8.02 (m, 1H), 7.69 (d, *J* = 8.9 Hz, 1H), 6.53 (s, 1H),6.06-5.94 (m, 1H), 5.24 (t, *J* = 6.4 Hz, 2H), 5.16 (t, *J* = 7.1 Hz, 2H), 4.15 (d, *J* = 13.9 Hz, 1H), 3.56 (d, *J* = 13.9 Hz, 1H), 3.11-2.99 (m, 1H), 2.70 (s, 3H), 2.57 (q, *J* = 7.1 Hz, 1H), 2.45-2.34 (m, 1H), 2.16-1.96 (m, 1H), 1.84-1.71 (m, 2H), 1.57-1.44 (m, 1H), 1.24 (d, *J* = 6.0 Hz, 3H). |
| 101 | CB | 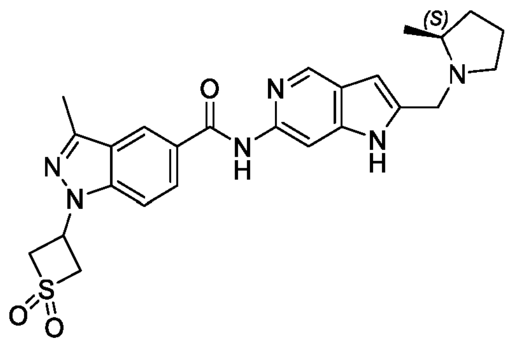 | 493 | (300 MHz, DMSO-$d_6$, *ppm*): δ 11.36 (s, 1H), 10.51 (s, 1H), 8.68 (s, 1H), 8.53 (s, 1H), 8.25 (s, 1H), 8.12 (dd, *J* = 8.9, 1.8 Hz, 1H), 7.77 (d, *J* = 8.9 Hz, 1H), 6.37 (s, 1H), 5.91-5.74 (m, 1H), 4.91-4.70 (m, 4H), 4.02 (d, *J* = 13.8 Hz, 1H), 3.40 (s, 1H), 2.93-2.82 (m, 1H), 2.63 (s, 3H), 2.48-2.38 (m, 1H), 2.27-2.14 (m, 1H), 2.02 - 1.85 (m, 1H), 1.64 (t, *J* = 7.9 Hz, 2H), 1.42-1.30 (m, 1H), 1.11 (d, *J* = 5.9 Hz, 3H). |
| 102 | CC | 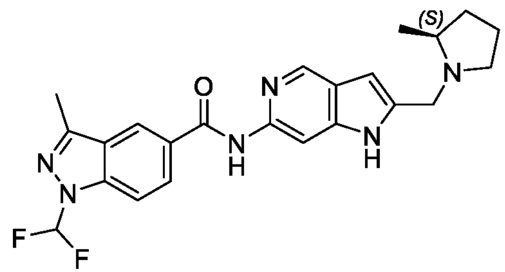 | 439 | (300 MHz, Methanol-$d_4$,ppm): δ8.59-8.49 (m, 2H), 8.21 (t, *J* = 1.0 Hz, 1H), 8.10-7.86 (m, 2H), 7.77-7.66 (m, 1H), 6.53 (s, 1H), 4.15 (d, *J* = 13.9 Hz, 1H), 3.56 (d, *J* = 13.9 Hz, 1H), 3.14-3.00 (m, 1H), 2.93 (d, *J* = 1.0 Hz, 3H), 2.65-2.51(m, 1H), 2.48-2.32 (m, 1H), 2.14-1.90 (m, 1H), 1.86-1.69 (m, 2H), 1.60-1.42 (m, 1H), 1.24 (d, *J* = 6.1 Hz, 3H).<br>$^{19}$F-NMR (300 MHz, Methanol-$d_4$,*ppm*):96.84(s,2F). |

Preparation of Example 23

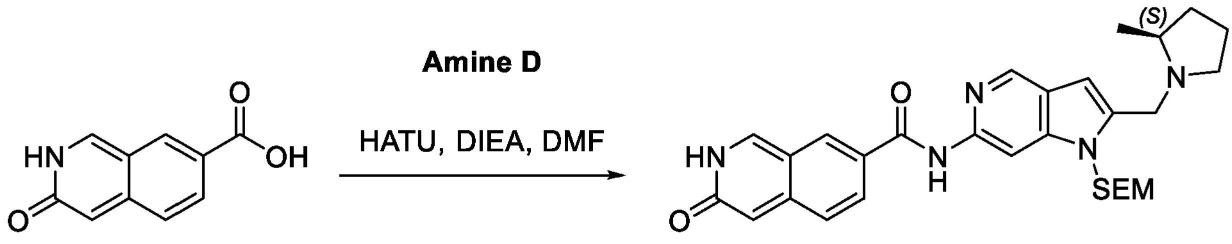


Acid CM

Into a 8-mL round-bottom flask, was placed 3-oxo-2H-isoquinoline-7-carboxylic acid (60.00 mg, 0.317 mmol, 1.00 equiv), 2-[[(2S)-2-methylpyrrolidin-1-yl]methyl]-1-[[2-(trimethylsilyl)ethoxy]methyl]pyrrolo[3,2-c]pyridin-6-amine **(Amine D,** 114.37 mg, 0.317 mmol, 1.00 equiv), DMF (2.00 mL), HATU (180.90 mg, 0.476 mmol, 1.5 equiv) and DIEA (163.97 mg, 1.269 mmol, 4 equiv). The resulting solution was stirred for 15h at room temperature. The resulting solution was diluted with 10 mL of $H_2O$. The resulting solution was extracted with 3x10 mL of ethyl acetate and the organic layers combined. The resulting mixture was washed with 3 x10 ml of brine. The resulting mixture was concentrated. This resulted in 80 mg (47.43%) of N-(2-[[(2S)-2-methylpyrrolidin-1-yl]methyl]-1-[[2-(trimethylsilyl)ethoxy]methyl]pyrrolo[3,2-c]pyridin-6-yl)-3-oxo-2H-isoquinoline-7-carboxamide as yellow oil. **LCMS:** $[M+1]^+$=532.

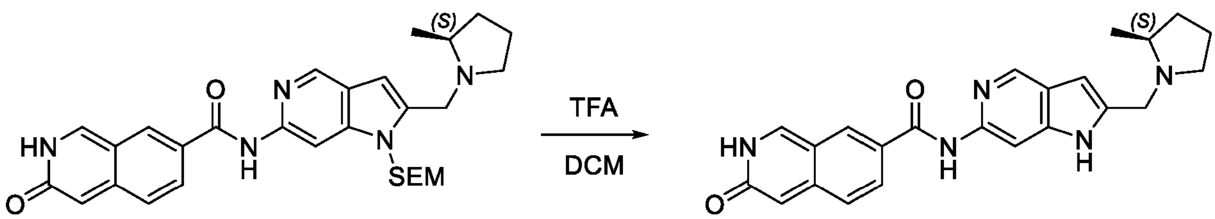


Example 23

Into a 8-mL round-bottom flask, was placed N-(2-[[(2S)-2-methylpyrrolidin-1-yl]methyl]-1-[[2-(trimethylsilyl) ethoxy]methyl]pyrrolo[3,2-c]pyridin-6-yl)-3-oxo-2H-isoquinoline-7-carboxamide (60.00 mg, 0.113 mmol, 1.00 equiv), DCM (2.00 mL), TFA (55.30 mg, 0.564 mmol, 5.00 equiv). The resulting solution was stirred for 16h at room temperature. The resulting solution was diluted with 10 mL of $H_2O$. The resulting solution was extracted with 3x10 mL of dichloromethane and the organic layers combined and concentrated. The crude product was purified by Prep-HPLC with the following conditions: Column: HPH C18, 50x3.0 mm, 2.6 um; Mobile Phase A: Water/0.05% $NH_3.H_2O$, Mobile Phase B: ACN; Flow rate: 1.2 mL/min; Gradient: 5%B to 100%B in 1.1 min, hold 0.7 min), Detector, UV 254 nm. This resulted in 19 mg (42%) of N-(2-[[(2S)-2-methylpyrrolidin-1-yl]methyl]-1H-pyrrolo[3,2-c]pyridin-6-yl)-3-oxo-2H-isoquinoline-7-carboxamide as a white solid. **LCMS:** $M+1]^+$=402. **$^1$H-NMR:** (300 MHz, Methanol-$d_4$, ppm): δ 11.40 - 11.33 (m, 1H), 10.56 (s, 1H), 9.02 (s, 1H), 8.74 (d, *J* = 1.7 Hz, 1H), 8.53 (s, 1H), 8.26 (s, 1H), 8.14 (dd, *J* = 8.9 Hz, 1H), 7.77 (d, *J* = 8.9 Hz, 1H), 6.95 (s, 1H), 6.37 (s, 1H), 4.02 (d, *J* = 13.9 Hz, 1H), 3.41 (d, *J* = 13.9 Hz, 1H), 2.88 (s, 1H), 2.47 - 2.37 (m, 1H), 2.20 (m, 1H), 1.92 (d, *J* = 11.6 Hz, 1H), 1.63 (d, *J* = 8.2 Hz, 3H), 1.35 (d, *J* = 9.0 Hz, 1H), 1.11 (d, *J* = 6.0 Hz, 3H).

Preparation of Example 24

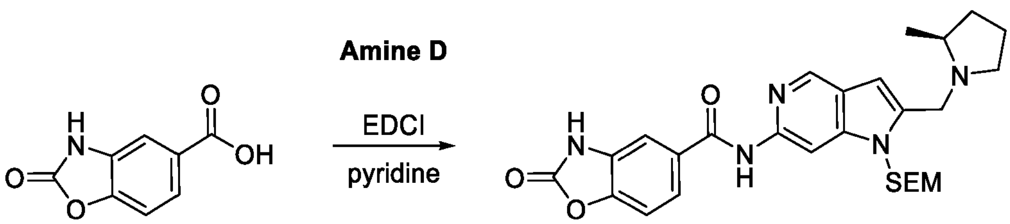


Acid R

Into a 12-mL vial purged and maintained with an inert atmosphere of nitrogen, was placed a solution of 2-oxo-3H-1,3-benzoxazole-5-carboxylic acid (100.0 mg, 0.558 mmol, 1.00 equiv) in pyridine (5 mL), 2-[[(2S)-2-methylpyrrolidin-1-yl]methyl]-1-[[2-(trimethylsilyl)ethoxy]methyl]pyrrolo[3,2-c]pyridin-6-amine **(Amine D,** 201.29 mg, 0.558 mmol, 1.00 equiv), EDCI (160.53 mg, 0.837 mmol, 1.50 equiv). The resulting solution was stirred for 12h at room temperature. The resulting mixture was concentrated under vacuum. The resulting solution was diluted with 20 mL of DCM. The resulting mixture was washed with 2x10 mL of $H_2O$. The mixture was dried over anhydrous sodium sulfate and concentrated under vacuum. This resulted in 150 mg (51.50%) of N-(2-[[(2S)-2-methylpyrrolidin-1-yl]methyl]-1-[[2-(trimethylsilyl)ethoxy]methyl]pyrrolo[3,2-c]pyridin-6-yl)-2-oxo-3H-1,3-benzoxazole-5-carboxamide as brown crude oil. **LCMS:** $[M+H]^+$=522.

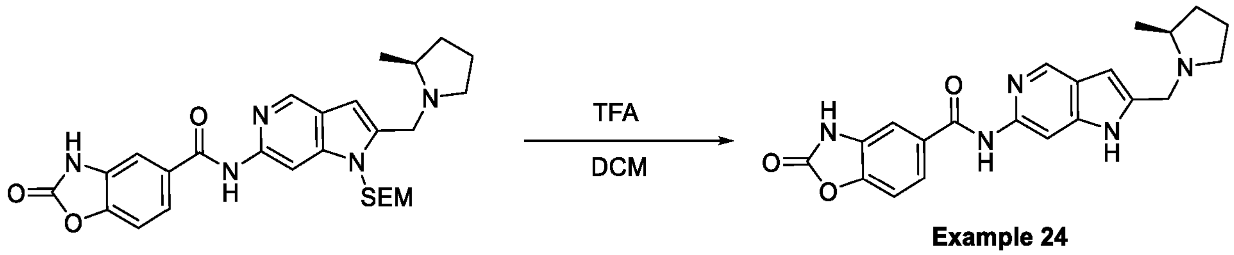


**Example 24**

Into a 10-mL vial purged and maintained with an inert atmosphere of nitrogen, was placed a solution of N-(2-[[(2S)-2-methylpyrrolidin-1-yl]methyl]-1-[[2-(trimethylsilyl)ethoxy]methyl]pyrrolo[3,2-c]pyridin-6-yl)-2-oxo-3H-1,3-benzoxazole-5-carboxamide (150.00 mg, 0.288 mmol, 1.00 equiv, crude) in DCM (2 mL) and TFA (2 mL). The resulting solution was stirred for 16h at room temperature. The resulting mixture was concentrated under vacuum. The crude product was purified by Prep-HPLC with the following conditions: Column, XBridge Prep C18 OBD Column, 5um,19x150mm; mobile phase, Water(0.05%$NH_3$.$H_2O$) and ACN (15% PhaseB up to 55% in 7 min); Detector, UV 254nm. This resulted in 2 mg of N-(2-[[(2S)-2-methylpyrrolidin-1-yl]methyl]-1H-pyrrolo[3,2-c]pyridin-6-yl)-2-oxo-3H-1,3-benzoxazole-5-carboxamide as an white solid. **LCMS:** $[M+H]^+$=392. **$^1$H-NMR:** (300 MHz, DMSO-$d_6$,ppm): δ 11.34 (s, 1H), 10.50 (s, 1H), 8.51 (s, 1H), 8.18 (s, 1H), 7.84 (d, *J* = 9.9 Hz, 1H), 7.75 (s, 1H), 7.38 (d, *J* = 8.3 Hz, 1H), 6.37 (s, 1H), 4.01 (d, *J* = 14.0 Hz, 1H), 2.87 (s, 1H), 2.43 (d, *J* = 6.4 Hz, 1H), 2.20 (d, *J= 8.9* Hz, 1H), 1.64 (s, 2H), 1.10 (d, *J* = 6.0 Hz, 3H).

The following examples in **Table H** were prepared in a similar fashion to that shown above for **Example 24** using **Amine D** and the appropriate reagents and conditions.

Table H

| Ex. | Acid | Structure | LCMS | $^1$H-NMR |
|---|---|---|---|---|
| **61** | **CN** | 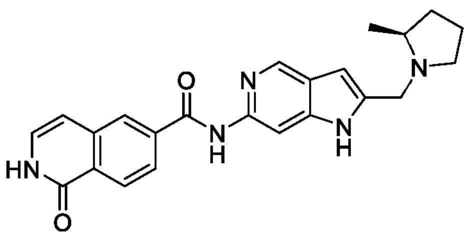 | **402** | (300 MHz, DMSO-$d_6$, ppm) δ11.37 (s, 1H), 10.77 (s, 1H), 8.53 (d, *J* = 1.0 Hz, 1H), 8.34 (d, *J* = 1.7 Hz, 1H), 8.26 (d, *J* = 7.9 Hz, 2H), 8.03 (dd, *J* = 8.3, 1.7 Hz, 1H), 7.26 (d, *J* = 7.1 Hz, 1H), 6.65 (d, *J* = 7.2 Hz, 1H), 6.38 (s, 1H), 4.02 (d, *J* = 13.9 Hz, 1H), 3.41 (d, *J* = 13.9 Hz, 1H), 2.88 (m, 1H), 2.47-2.36 (m, 1H), 2.20 (m, 1H), 2.00-1.89 (m, 1H), 1.73-1.56 (m, 2H), 1.36 (s, 1H), 1.11 (d, *J* = 6.0 Hz, 3H). |

(continued)

| Ex. | Acid | Structure | LCMS | $^1$H-NMR |
|---|---|---|---|---|
| 63 | AX | 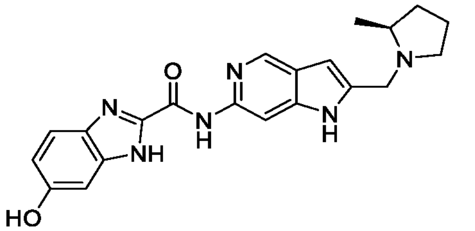 | 391 | (300 MHz, Methanol-$d_4$, ppm) δ 8.54 (s, 1H), 8.30 (s, 1H), 7.57 (s, 1H), 7.01 (s, 1H), 6.92 (d, *J* = 9.5 Hz, 1H), 6.53 (s, 1H), 4.14 (d, *J* = 13.9 Hz, 1H), 3.54 (d, *J* = 13.9 Hz, 1H), 3.05 (s, 1H), 2.62-2.49 (m, 1H), 2.45-2.25 (m, 1H), 2.11-1.96 (m, 1H), 1.86-1.69 (m, 2H), 1.52 (dd, *J* = 18.9, 9.5 Hz, 1H), 1.30 (s, 1H), 1.23 (d, *J* = 6.1 Hz, 3H). |
| 64 | AY | 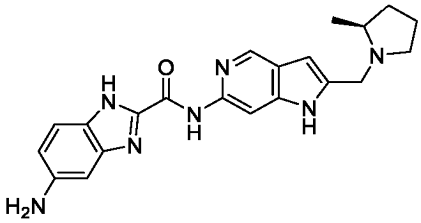 | 390 | (300 MHz, Methanol-$d_4$, ppm) δ 8.54 (d, *J* = 1.1 Hz, 1H), 8.29 (t, *J* = 1.0 Hz, 1H), 7.50 (d, *J* = 8.6 Hz, 1H), 6.87 (d, *J* = 8.9 Hz, 1H), 6.52 (s, 1H), 4.14 (d, *J* = 13.9 Hz, 1H), 3.51 (d, *J* = 13.5 Hz, 1H), 3.04 (m, 1H), 2.58-2.47 (m, 1H), 2.35 (m, 1H), 2.03 (d, *J* = 12.5 Hz, 1H), 1.77 (m, 2H), 1.50 (m, 1H), 1.23 (d, *J* = 6.1 Hz, 3H). |
| 86 | BR | 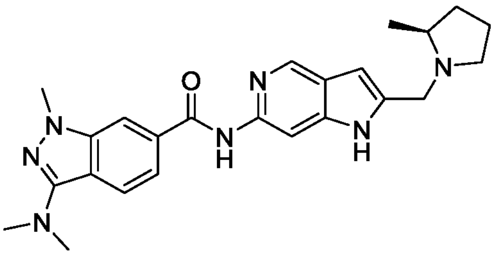 | 432 | (300 MHz, Methanol-$d_4$,ppm): δ 8.55 (d, *J* = 1.1 Hz, 1H), 8.23 (m, 1H), 8.04 (dd, *J* = 1.5, 0.7 Hz, 1H), 7.97 (dd, *J* = 8.6, 0.8 Hz, 1H), 7.60 (dd, *J* = 8.6, 1.5 Hz, 1H), 6.52 (s, 1H), 4.14 (d, *J* = 13.8 Hz, 1H), 3.96 (s, 3H), 3.53 (d, *J* = 13.9 Hz, 1H), 3.13 (s, 6H), 3.10 - 2.98 (m, 1H), 2.60 - 2.47 (m, 1H), 2.36 (m, 1H), 2.13 - 1.96 (m, 1H), 1.77 (s, 2H), 1.85 - 1.68 (m, 1H), 1.59 - 1.41 (m, 1H), 1.23 (d, *J* = 6.1 Hz, 3H). |
| 88 | BS | 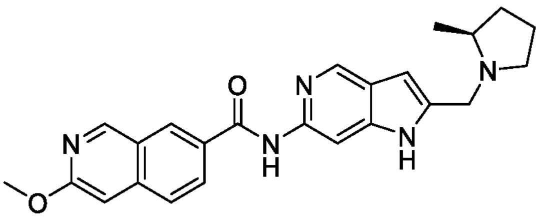 | 416 | (300 MHz, Methanol-$d_4$ ,ppm): δ 9.15 (s, 1H), 8.70 (d, *J* = 1.6 Hz, 1H), 8.57 (d, *J* = 1.0 Hz, 1H), 8.26 - 8.16 (m, 2H), 7.94 (d, *J* = 8.8 Hz, 1H), 7.24 (s, 1H), 6.53 (s, 1H), 4.15 (d, J = 13.9 Hz, 1H), 4.07 (s, 3H), 3.54 (d, *J* = 13.8 Hz, 1H), 3.05 (s, 1H), 2.61 - 2.48 (m, 1H), 2.37 (m, 1H), 2.05 (m, 1H), 1.86 - 1.68 (m, 2H), 1.60 - 1.44 (m, 1H), 1.22-1.24 (d, *J* = 6.1 Hz, 3H). |

(continued)

| Ex. | Acid | Structure | LCMS | $^1$H-NMR |
|---|---|---|---|---|
| **89** | **BT** | 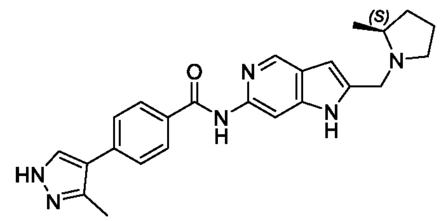 | **415** | (300 MHz, Methanol-$d_4$, ppm) δ 8.55 (d, *J* = 1.5 Hz, 1H), 8.22 (s, 1H), 8.05 (dd, *J* = 8.4, 1.7 Hz, 2H), 7.85 (s, 1H), 7.66 (dd, *J* = 8.4, 1.6 Hz, 2H), 6.51 (s, 1H), 4.13 (d, *J* = 13.9 Hz, 1H), 3.55 (d, *J* = 13.8 Hz, 1H), 3.04 (m, 1H), 2.56 (d, *J* = 7.6 Hz, 1H), 2.50 (s, 3H), 2.36 (m, 1H), 2.05 (m, 1H), 1.75 (d, *J* = 8.2 Hz, 2H), 1.56-1.44 (m, 1H), 1.23 (dd, *J* = 6.0, 1.5 Hz, 3H). |
| **91** | **BV** | 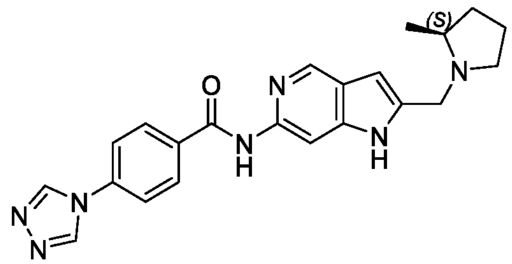 | **402** (PH-PUK) | (PH-PUK): (300 MHz, DMSO-$d_6$, ppm)δ 11.38 (s, 1H), 10.67 (s, 1H), 9.27 (s, 2H), 8.53 (s, 1H), 8.27-8.23 (m, 3H), 7.89 (d, *J* = 8.4 Hz, 2H), 6.38 (s, 1H), 4.02 (d, *J* = 13.8 Hz, 1H), 3.40 (d, *J* = 13.8 Hz, 1H), 2.90-2.85 (m, 1H), 2.46-2.40 (m, 1H), 2.20 (q, *J* = 8.7 Hz, 1H), 1.97-1.90 (m, 1H), 1.67-1.62 (m, 2H), 1.46 - 1.31 (m, 1H), 1.11 (d, *J* = 6 Hz, 3H). |
| **97** | **BY** | 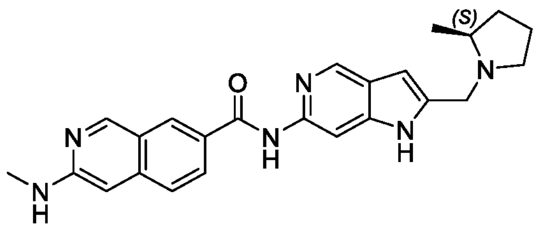 | **415** | (300 MHz, Methanol-$d_4$,ppm):δ 8.95 (s, 1H), 8.64 (s, 1H), 8.52 (d, *J* = 3.9 Hz, 1H), 8.28 (s, 1H), 8.06 (dd, *J* = 8.8, 1.9 Hz, 1H), 7.71 (d, *J* = 8.8 Hz, 1H), 6.69 (d, *J* = 13.4 Hz, 2H), 4.42 (d, *J* = 14.0 Hz, 1H), 3.99 (d, *J* = 14.1 Hz, 1H), 2.99 (s, 3H), 2.92 - 2.79 (m, 1H), 2.22 (m , 1H), 2.02 - 1.85 (m, 2H), 1.75 - 1.56 (m, 1H), 1.40 - 1.28 (m, 4H). |

Preparation of Example 27

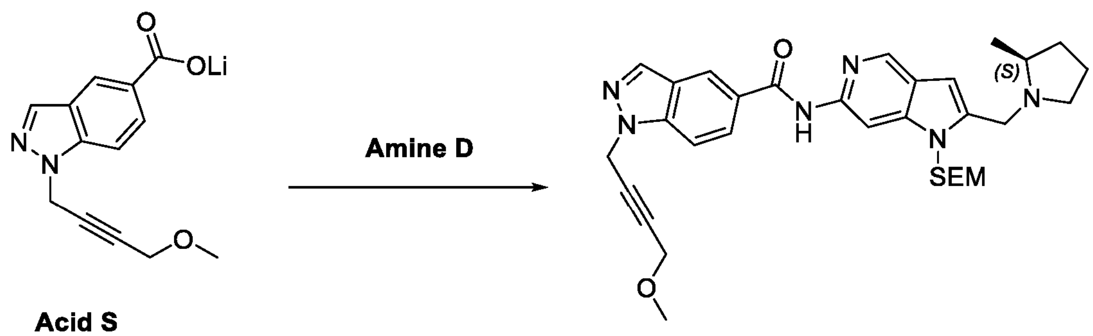


Acid S

Into a 8-mL sealed tube, was placed lithio 1-(4-methoxybut-2-yn-1-yl)indazole-5-carboxylate (87.42 mg, 0.350 mmol, 1.40 equiv), 2-[[(2S)-2-methylpyrrolidin-1-yl]methyl]-1-[[2-(trimethylsilyl)ethoxy]methyl]pyrrolo[3,2-c]pyridin-6-amine **(Amine D,** 90.00 mg, 0.250 mmol, 1.00 equiv), HATU (170.83 mg, 0.450 mmol, 1.80 equiv), DIPEA (96.78 mg, 0.750 mmol, 3.00 equiv), DMF (1.00 mL, 12.922 mmol, 51.77 equiv). The resulting solution was stirred for 20h at room temperature. The reaction was then quenched by the addition of 10 mL of water. The resulting solution was extracted with 2x10 mL of ethyl acetate and the organic layers combined and concentrated. This resulted in 200 mg (crude) of 1-(4-methoxybut-2-yn-1-yl)-N-(2-[[(2S)-2-methylpyrrolidin-1-yl]methyl]-1-[[2-(trimethylsilyl)ethoxymethyl]pyrrolo[3,2-c]pyridin-6-yl)indazole-5-carboxamide as a brown solid. **LCMS:** [M+H]=587.

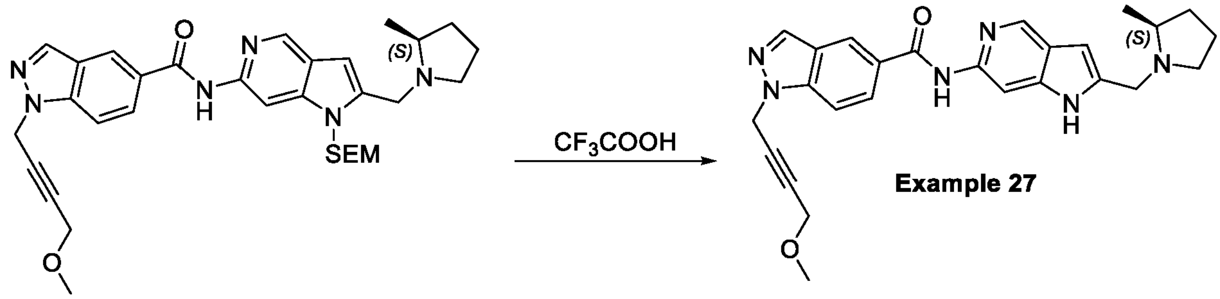


Into a 50-mL round-bottom flask, was placed 1-(4-methoxybut-2-yn-1-yl)-N-(2-[[(2S)-2-methylpyrrolidin-1-yl]methyl]-1-[[2-(trimethylsilyl)ethoxy]methyl]pyrrolo[3,2-c]pyridin-6-yl)indazole-5-carboxamide (200.00 mg, 0.341 mmol, 1.00 equiv), $CF_3COOH$ (1.00 mL), DCM (2.00 mL). The resulting solution was stirred for 2 h at room temperature. The resulting mixture was concentrated. The reaction was then quenched by the addition of 10 mL of water. The pH value of the solution was adjusted to 7 with $NaHCO_3$(aq). The resulting solution was extracted with 2x20 mL of ethyl acetate and the organic layers combined and concentrated. The crude product (120 mg) was purified by Prep-HPLC with the following conditions: Column, XBridge Prep C18 OBD Column,, 5um,19x150mm; mobile phase, Water(0.05%$NH_3H_2O$) and ACN (41% PhaseB up to 54% in 7 min); Detector, UV 254nm. This resulted in 12.2 mg (12% for two steps) of 1-(4-methoxybut-2-yn-1-yl)-N-(2-[[(2S)-2-methylpyrrolidin-1-yl]methyl]-1H-pyrrolo[3,2-c]pyridin-6-yl)indazole-5-carboxamide as a light brown solid. **LCMS:** $[M+H]^+$=457. **$^1$H-NMR:** (300 MHz, Methanol-$d_4$,*ppm*): δ 8.59 - 8.50 (m, 2H), 8.28 - 8.18 (m, 2H), 8.12 (dd, *J* = 8.9, 1.7 Hz, 1H), 7.82 (dd, *J* = 8.8, 1.1 Hz, 1H), 6.52 (s, 1H), 5.41 (t, *J* = 2.0 Hz, 2H), 4.20 - 4.09 (m, 3H), 3.53 (d, *J* = 13.9 Hz, 1H), 3.11 - 2.98 (m, 1H), 2.61 - 2.48 (m, 1H), 2.41-2.30 (m, 1H), 2.11 - 1.99 (m, 1H), 1.76-1.70 (m, 2H), 1.60 - 1.44 (m, 1H), 1.24 (d, *J* = 6.1 Hz, 3H).

The following examples in **Table I** were prepared in a similar fashion to that shown above for **Example 27** using **Amine D** and the appropriate reagents and conditions.

Table I

| Ex. | Acid | Structure | LCMS | $^1$H-NMR |
|---|---|---|---|---|
| 28 | T | 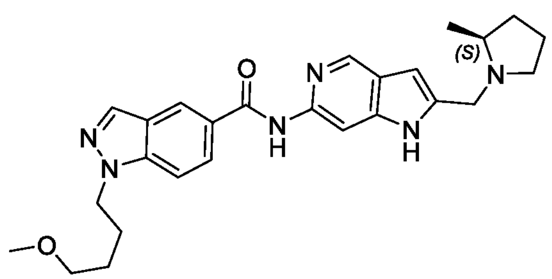 | 461 | (300 MHz, Methanol-$d_4$,*ppm*): δ 8.58 - 8.47 (m, 2H), 8.21 (d, *J* = 1.0 Hz, 2H), 8.07 (dd, *J* = 8.9, 1.7 Hz, 1H), 7.73 (d, *J* = 8.9 Hz, 1H), 6.51 (s, 1H), 4.55-4.49 (m, 2H), 4.13 (d, *J* = 13.9 Hz, 1H), 3.52 (d, *J* = 13.9 Hz, 1H), 3.41 (t, *J* = 6.3 Hz, 2H), 3.31 (s, 8H), 3.08-3.00 (m, 1H), 2.58-2.47 (m, 1H), 2.40-2.28 (m, 1H), 2.13 - 1.94 (m, 3H), 1.55-1.67 (m, 2H), 1.65 - 1.42 (m, 3H), 1.23 (d, *J* = 6.1 Hz, 3H). |

Preparation of Example 29

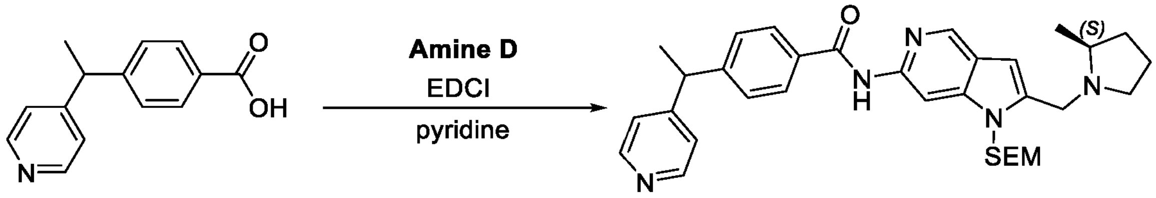


Acid U

Into a 8 mL vial were added 4-[1-(pyridin-4-yl)ethyl]benzoic acid (83.20 mg, 0.366 mmol, 1.20 equiv), pyridine (2.50 mL) and EDCI (87.72 mg, 0.458 mmol, 1.50 equiv) at room temperature. To the above mixture was added 2-[[(2S)-2-methylpyrrolidin-1-yl]methyl]-1-[[2-(trimethylsilyl)ethoxy]methyl]pyrrolo[3,2-c]pyridin-6-amine **(Amine D,** 110.00 mg, 0.305 mmol, 1.00 equiv). The resulting mixture was stirred for additional 6 h at 50 °C. The resulting mixture was concentrated under reduced pressure. This resulted in N-(2-[[(2S)-2-methylpyrrolidin-1-yl]methyl]-1-[[2-(trimethylsilyl) ethoxy]methyl]pyrrolo[3,2-c]pyridin-6-yl)-4-[1-(pyridin-4-yl)ethyl]benzamide (260 mg, crude) as a brown oil. The crude product was used in the next step directly without further purification. **LCMS:** $[M+1]^+$=570.

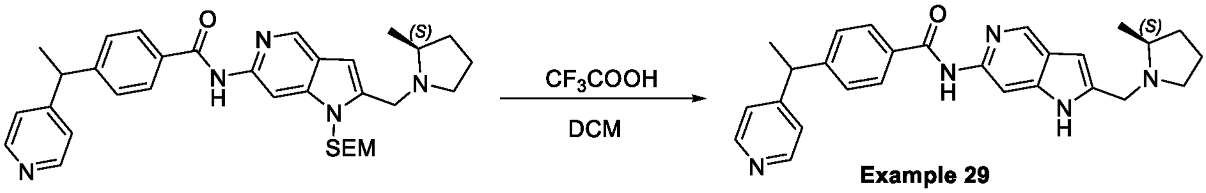


**Example 29**

Into a 20 mL vial were added N-(2-[[(2S)-2-methylpyrrolidin-1-yl]methyl]-1-[[2-(trimethylsilyl)ethoxy]methyl] pyrrolo[3,2-c]pyridin-6-yl)-4-[1-(pyridin-4-yl)ethyl]benzamide (240.00 mg, 0.421 mmol, 1.00 equiv, 50%), DCM (4.00 mL) and $CF_3COOH$ (4.00 mL) at room temperature. The resulting mixture was stirred for overnight at room temperature. The resulting mixture was concentrated under vacuum. The residue was dissolved in DMF (3 mL). The mixture was basified to pH 10 with ammonium hydroxide. The crude product was purified by Prep-HPLC with the following conditions (Column: XBridge Prep C18 OBD Column, 5um, 19x150mm; Mobile Phase A: Water(0.05% $NH_3H_2O$), Mobile Phase B: ACN; Flow rate: 20 mL/min; Gradient: 25 B to 50 B in 7 min, 220 nm;) to afford N-(2-[[(2S)-2-methylpyrrolidin-1-yl] methyl]-1H-pyrrolo[3,2-c]pyridin-6-yl)-4-[1-(pyridin-4-yl)ethyl]benzamide (60 mg, 64.82%) as a white solid. **LCMS:** $[M+1]^+$=440. **$^1$H-NMR:** (300 MHz, Methanol-$d_4$, ppm)δ 8.53 (d, *J* = 0.9 Hz, 1H), 8.47 - 8.45 (m, 2H), 8.19 (s, 1H), 7.98 (d, *J* = 8.1 Hz, 2H), 7.47 (d, *J* = 8.1 Hz, 2H), 7.39-7.37 (m, 2H), 6.51 (s, 1H), 4.35 (q, *J* = 7.2 Hz, 1H), 4.14 (d, *J* = 13.8 Hz, 1H), 3.53 (d, *J* = 13.8 Hz, 1H), 3.07 - 3.00 (m, 1H), 2.55-2.53 (m, 1H), 2.37 (q, *J* = 9.0 Hz, 1H), 2.06-2.00 (m, 1H), 1.79-1.72 (m, 5H), 1.53-1.49 (m, 1H), 1.23 (d, *J* = 6.0 Hz, 3H).

The following examples in **Table J** were prepared in a similar fashion to that shown above for **Example 29** using **Amine D** and the appropriate reagents and conditions.

Table J

| Ex. | Acid | Structure | LCMS | $^1$H-NMR |
|---|---|---|---|---|
| **30** | **V** | 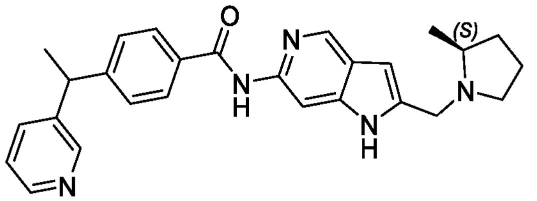 | **440** | (300 MHz, Methanol-$d_4$, ppm)δ 8.53 (d, *J* = 0.9 Hz, 1H), 8.50 (d, *J* = 2.1 Hz, 1H), 8.40 (dd, *J* = 4.8, 1.5 Hz, 1H), 8.19 (s, 1H), 7.99-7.96 (m, 2H), 7.79 (dt, *J* = 7.8, 1.8 Hz, 1H), 7.49 - 7.46 (m, 2H), 7.40 (ddd, *J* = 8.1, 4.8, 0.9 Hz, 1H), 6.51 (s, 1H), 4.38 (q, *J* = 7.2 Hz, 1H), 4.13 (d, *J* = 13.8 Hz, 1H), 3.53 (d, *J* = 13.8 Hz, 1H), 3.05-3.00 (m, 1H), 2.54 (q, *J* = 6.9 Hz, 1H), 2.36 (q, *J* = 9.3 Hz, 1H), 2.02 (m, 1H), 1.79-1.71 (m, 5H), 1.49 (m, 1H), 1.22 (d, *J* = 6.3 Hz, 3H). |

Preparation of Example 37

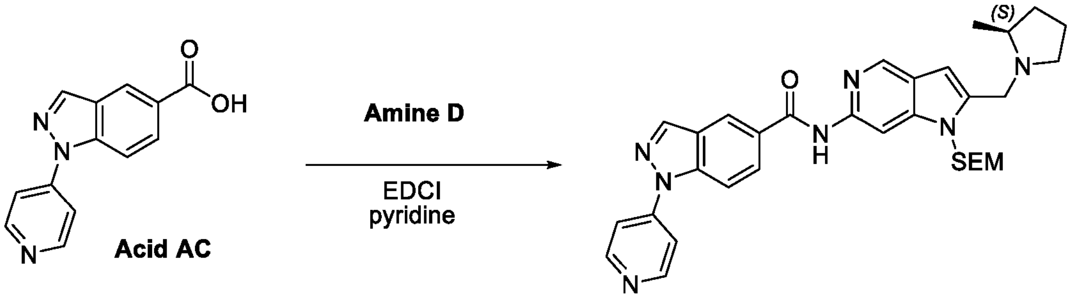


Into a 8-mL vial, was placed 1-(pyridin-4-yl)indazole-5-carboxylic acid (70.00 mg, 0.293 mmol, 1.00 equiv), Pyridine (2.00 mL), 2-[[(2S)-2-methylpyrrolidin-1-yl]methyl]-1-[[2-(trimethylsilyl)ethoxy]methyl]pyrrolo[3,2-c]pyridin-6-amine **(Amine D,** 105.51 mg, 0.293 mmol, 1.00 equiv), EDCI (84.14 mg, 0.439 mmol, 1.50 equiv). The resulting solution was stirred for 16 hr at room temperature. The resulting mixture was concentrated under vacuum. The resulting solution was diluted with 20 mL of $H_2O$. The resulting solution was extracted with 3x15 mL of ethyl acetate and the organic layers combined. The resulting mixture was washed with 2 x20 mL of brine. The mixture was dried over anhydrous sodium sulfate and concentrated under vacuum. This resulted in 150 mg (76.11%) of N-(2-[[(2S)-2-methylpyrrolidin-1-yl] methyl]-1-[[2-(trimethylsilyl)ethoxy]methyl]pyrrolo[3,2-c]pyridin-6-yl)-1-(pyridin-4-yl)indazole-5-carboxamide as brown oil. **LCMS:** $[M+H]^+$=582.

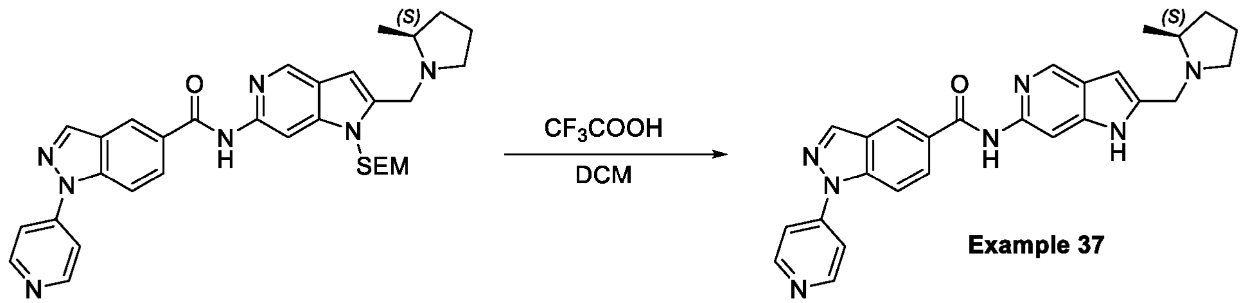


Into a 8-mL vial, was placed N-(2-[[(2S)-2-methylpyrrolidin-1-yl]methyl]-1-[[2-(trimethylsilyl)ethoxy]methyl]pyrrolo [3,2-c]pyridin-6-yl)-1-(pyridin-4-yl)indazole-5-carboxamide (150.00 mg, 0.258 mmol, 1 equiv), DCM (2.00 mL), $CF_3COOH$ (2.00 mL). The resulting solution was stirred for 16 hr at room temperature. The resulting mixture was concentrated under vacuum. The pH value of the solution was adjusted to 8 with $Et_2NH$. The crude product (110 mg) was purified by Prep-HPLC with the following conditions: Column, XBridge Prep C18 OBD Column, 5um,19x150mm; mobile phase, Water(0.05%$NH_3H_2O$) and ACN (31% PhaseB up to 48% in 7 min); Detector, UV 254 nm. This resulted in 38.1 mg (32.73%) of N-(2-[[(2S)-2-methylpyrrolidin-1-yl]methyl]-1H-pyrrolo[3,2-c]pyridin-6-yl)-1-(pyridin-4-yl) indazole-5-carboxamide as a white solid. **LCMS:** $[M+H]^+$=452. **$^1$H-NMR:** (300 MHz, Methanol-$d_4$, ppm) δ 8.74-8.72 (m, 2H), 8.62-8.55 (m, 3H), 8.23 (m, 3H), 8.04-8.02 (m, 2H), 6.53 (s, 1H), 4.15 (d, *J* = 13.9 Hz, 1H), 3.54 (d, *J* = 13.9 Hz, 1H), 3.05 (m, 1H), 2.56 (m, 1H), 2.38 (m, 1H), 2.05 (m, 1H), 1.76 (m, 2H), 1.60-1.45 (m, 1H), 1.24 (d, *J* = 6.1 Hz, 3H).

The following examples in **Table K** were prepared in a similar fashion to that shown above for **Example 37** using **Amine D** and the appropriate reagents and conditions.

Table K

| Ex. | Acid | Structure | LCMS | $^1$H-NMR |
|---|---|---|---|---|
| 38 | AD | 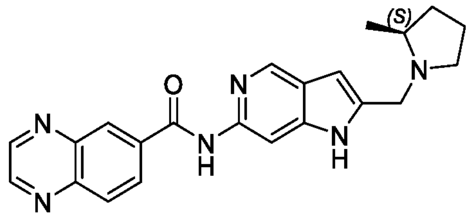 | 387 | (300 MHz, Methanol-$d_4$, *ppm*): δ 9.05 - 8.97 (m, 2H), 8.77 (d, *J* = 2.0 Hz, 1H), 8.58 (d, *J* = 1.1 Hz, 1H), 8.42 (dd, *J* = 8.8, 2.0 Hz, 1H), 8.27 (d, *J* = 8.9 Hz, 2H), 6.53 (s, 1H), 4.15 (d, *J* = 13.9 Hz, 1H), 3.54 (d, *J* = 13.8 Hz, 1H), 3.12 - 2.99 (m, 1H), 2.61-2.50 (m, 1H), 2.44-2.33 (m, 1H), 2.12-1.96 (m, 1H), 1.86 - 1.69 (m, 2H), 1.60 - 1.41 (m, 1H), 1.24 (d, *J* = 6.1 Hz, 3H). |
| 39 | AE | 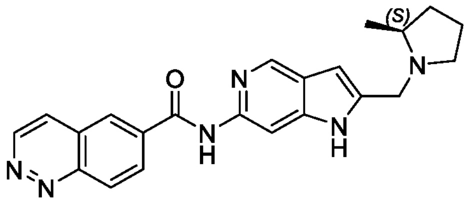 | 387 | (300 MHz, Methanol-$d_4$, ppm) δ 9.45 (d, *J* = 5.8 Hz, 1H), 8.68 (d, *J* = 1.9 Hz, 1H), 8.66-8.56 (m, 2H), 8.48 (dd, *J* = 9.0, 1.9 Hz, 1H), 8.38 (d, *J* = 5.8 Hz, 1H), 8.26 (s, 1H), 6.57 (s, 1H), 4.20 (d, *J* = 14.0 Hz, 1H), 3.62 (d, *J* = 14.5 Hz, 1H), 3.33-3.09(m, 1H), 2.64 (s, 1H), 2.45 (s, 1H), 2.08 (s, 1H), 1.78 (d, *J* = 8.8 Hz, 3H), 1.52 (s, 1H), 1.25 (d, *J* = 6.1 Hz, 3H). |
| 44 | AI | 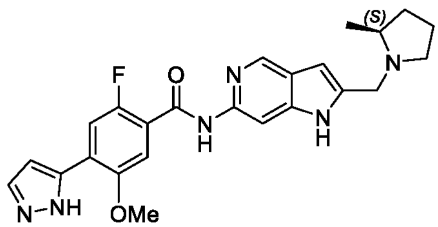 | 449 | (300 MHz, DMSO-$d_6$, ppm) δ 13.11 (s, 1H), 11.39 (s, 1H), 10.30 (s, 1H), 8.50 (d, *J* = 0.9 Hz, 1H), 8.25 (s, 1H), 7.78 (d, *J* = 11.8 Hz, 1H), 7.44 (d, *J* = 6.1 Hz, 1H), 6.88 (d, *J* = 2.1 Hz, 1H), 6.37 (s, 1H), 4.07-3.94 (m, 4H), 3.40 (d, *J* = 13.9 Hz, 1H), 2.94-2.82 (m, 1H), 2.42 (m, 1H), 2.20 (m, 1H), 2.00-1.85 (m, 1H), 1.73-1.59 (m, 2H), 1.37 (t, *J* = 9.9 Hz, 1H), 1.11 (d, *J* = 6.0 Hz, 3H).<br>$^{19}$F-NMR: (300 MHz, DMSO-$d_6$, ppm) δ - 124.195 |

(continued)

| Ex. | Acid | Structure | LCMS | 1H-NMR |
|---|---|---|---|---|
| 49 | AL | 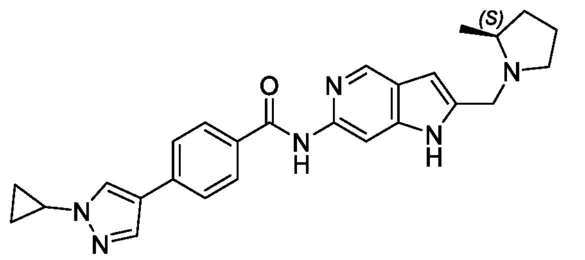 | 441 | (300 MHz, Methanol-$d_4$, ppm): δ 8.60 (s, 1H), 8.23 (d, $J$ = 15.2 Hz, 2H), 8.07 - 7.98 (m, 2H), 7.95 (d, $J$ = 0.8 Hz, 1H), 7.81 - 7.71 (m, 2H), 6.66 (s, 1H), 4.35 (d, $J$ = 13.9 Hz, 1H), 3.87 (d, $J$ = 14.0 Hz, 1H), 3.74 (m, 1H), 3.23 (s, 1H), 2.95 (s, 1H), 2.74 (s, 1H), 2.18 (dd, $J$ = 13.1, 6.7 Hz, 1H), 1.94 - 1.83 (m, 2H), 1.70 - 1.54 (m, 1H), 1.33 (d, $J$ = 6.2 Hz, 3H), 1.24 - 1.04 (m, 4H). |
| 50 | AM | 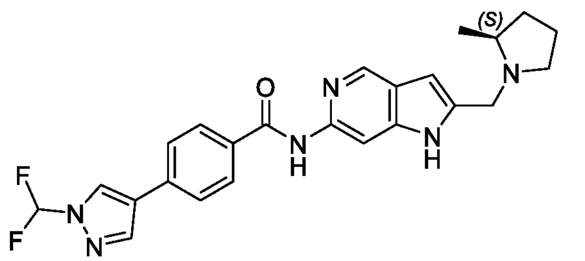 | 451 | (300 MHz, Methanol-$d_4$, ppm): δ 8.66 - 8.56 (m, 2H), 8.25 (d, $J$ = 16.5 Hz, 2H), 8.11 - 8.03 (m, 2H), 7.85 (d, $J$ = 8.3 Hz, 2H), 7.55 (s, 0H), 6.71 (s, 1H), 4.41 (d, $J$ = 14.0 Hz, 1H), 3.97 (d, $J$ = 14.0 Hz, 1H), 3.09 (s, 1H), 2.86 (s, 1H), 2.20 (m, 1H), 1.94 (m, 2H), 1.74 - 1.58 (m, 1H), 1.36 (d, $J$ = 6.3 Hz, 3H) |
| 51 | AN | 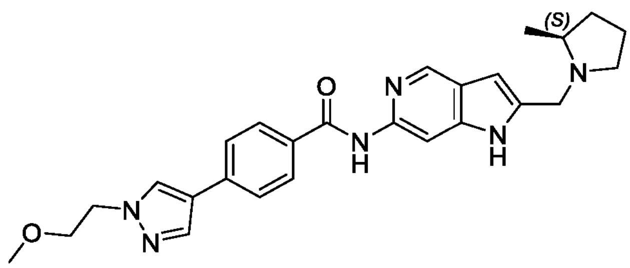 | 459 | (300 MHz, Methanol-$d_4$, ppm): δ 8.55 (d, $J$ = 1.0 Hz, 1H), 8.21 (m, 1H), 8.15 (d, $J$ = 0.8 Hz, 1H), 8.08 - 7.95 (m, 3H), 7.80 - 7.71 (m, 2H), 6.55 - 6.49 (m, 1H), 4.37 (t, $J$ = 5.2 Hz, 2H), 4.14 (d, $J$ = 13.8 Hz, 1H), 3.80 (m, 2H), 3.53 (d, $J$ = 13.9 Hz, 1H), 3.37 (s, 3H), 3.04 (m, 1H), 2.55 (m, 1H), 2.37 (m, 1H), 2.13 - 1.96 (m, 1H), 1.76 (m, 2H), 1.59 - 1.44 (m, 1H), 1.23 (d, $J$ = 6.1 Hz, 3H). |
| 52 | AO | 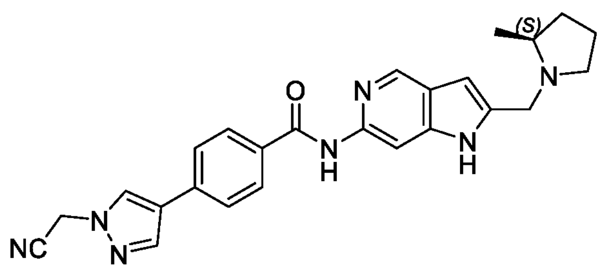 | 440 | (300 MHz, Methanol-$d_4$, ppm): δ 11.35 (s, 1H), 10.44 (s, 1H), 8.52 (d, $J$ = 1.0 Hz, 1H), 8.42 (s, 1H), 8.26 - 8.15 (m, 2H), 8.08 (d, $J$ = 8.4 Hz, 2H), 7.74 (d, $J$ = 8.4 Hz, 2H), 6.37 (s, 1H), 5.55 (s, 2H), 4.01 (d, $J$ = 13.9 Hz, 1H), 3.40 (d, $J$ = 13.9 Hz, 1H), 2.88 (s, 1H), 2.43 (m, 1H), 2.20 (m, 1H), 1.94 (m, 1H), 1.63 (d, $J$ = 8.2 Hz, 3H), 1.39 (s, 1H), 1.11 (d, $J$ = 6.0 Hz, 3H). |

(continued)

| Ex. | Acid | Structure | LCMS | $^1$H-NMR |
|---|---|---|---|---|
| 65 | AZ | 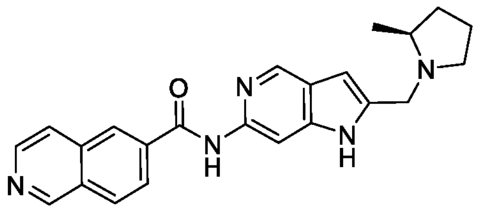 | 386 | (300 MHz, Methanol-$d_4$, *ppm*): δ 9.38 (d, *J* = 1.0 Hz, 1H), 8.65-8.54 (m, 3H), 8.34-8.20 (m, 3H), 8.02 (d, *J* = 5.9 Hz, 1H), 6.53 (s, 1H), 4.15 (d, *J* = 13.9 Hz, 1H), 3.53 (d, *J* = 13.9 Hz, 1H), 3.11-2.98 (m, 1H), 2.6 -2.48 (m, 1H), 2.44-2.30 (m, 1H), 2.15-1.98 (m, 1H), 1.77 (s, 2H), 1.86-1.68 (m, 1H), 1.60-1.44 (m, 1H), 1.24 (d, *J* = 6.1 Hz, 3H). |
| 66 | BA | 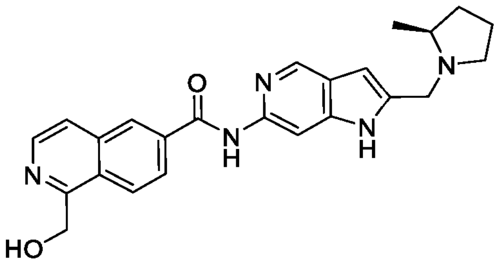 | 416 | (300 MHz, Methanol-$d_4$, *ppm*):δ8.61 (d, *J* = 1.8 Hz, 1H), 8.58 (d, *J* = 1.0 Hz, 1H), 8.56-8.47 (m, 2H), 8.29-8.19 (m, 2H), 7.93 (d, *J* = 5.8 Hz, 1H), 6.53 (s, 1H), 5.26 (s, 2H), 4.15 (d, *J* = *13.9* Hz, 1H), 3.54 (d, *J* = 13.9 Hz, 1H), 3.12-3.00 (m, 1H), 2.62-2.49 (m, 1H), 2.45-2.30 (m, 1H), 2.14-1.96 (m, 1H), 1.86-1.70 (m, 2H), 1.60-1.41 (m, 1H), 1.24 (d, *J* = 6.1 Hz, 3H). |
| 68 | BC | 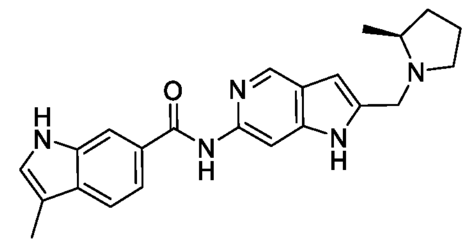 | 388 | (300 MHz, Methanol-d4): δ 8.54 (d, *J* = 1.0 Hz, 1H), 8.25 (t, *J* = 1.0 Hz, 1H), 8.06 (d, *J* = 1.1 Hz, 1H), 7.74 - 7.60 (m, 2H), 7.24 (d, *J* = 1.1 Hz, 1H), 6.52 (s, 1H), 4.15 (d, *J* = 13.9 Hz, 1H), 3.54 (d, *J* = 13.8 Hz, 1H), 3.05 (s, 1H), 2.56 (d, *J* = 7.8 Hz, 1H), 2.37 (d, *J* = 1.1 Hz, 4H), 2.04 (s, 1H), 1.77 (s, 2H), 1.50 (s, 1H), 1.24 (d, *J* = 6.1 Hz, 3H). |

Preparation of Example 40

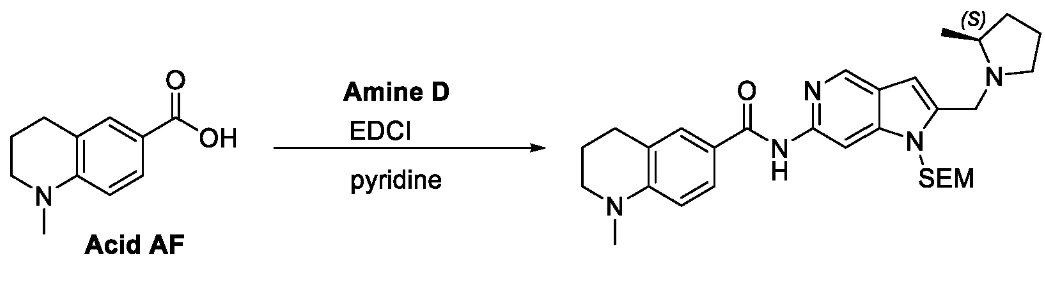


Into a 8-mL sealed tube, was placed 1-methyl-3,4-dihydro-2H-quinoline-6-carboxylic acid (50.00 mg, 0.261

mmol, 1.00 equiv), 2-[[(2S)-2-methylpyrrolidin-1-yl]methyl]-1-[[2-(trimethylsilyl)ethoxy]methyl]pyrrolo[3,2-c]pyridin-6-amine **(Amine D,** 94.28 mg, 0.261 mmol, 1.00 equiv), Pyridine (1.00 mL), EDCI (75.19 mg, 0.392 mmol, 1.50 equiv). The resulting solution was stirred for 12 h at 50 °C in an oil bath. The resulting mixture was concentrated. The reaction was then quenched by the addition of 20 mL of water. The resulting solution was extracted with 2x20 mL of ethyl acetate and the organic layers combined and concentrated. This resulted in 150 mg (crude) of 1-methyl-N-(2-[[(2S)-2-methylpyrrolidin-1-yl]methyl]-1-[[2-(trimethylsilyl)ethoxy]methyl]pyrrolo[3,2-c]pyridin-6-yl)-3,4-dihydro-2H-quinoline-6-carboxamide as a light brown solid. **LCMS:** $[M+H]^+$=534.

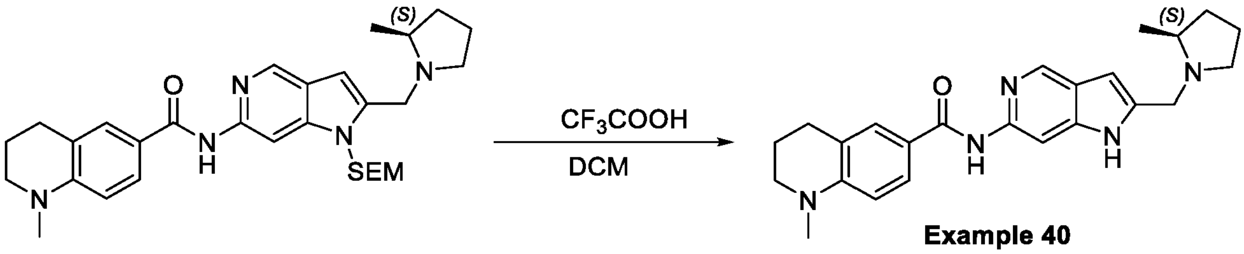


**Example 40**

Into a 50-mL round-bottom flask, was placed 1-methyl-N-(2-[[(2S)-2-methylpyrrolidin-1-yl]methyl]-1-[[2-(trimethylsilyl)ethoxy]methyl]pyrrolo[3,2-c]pyridin-6-yl)-3,4-dihydro-2H-quinoline-6-carboxamide (150.0 mg, 0.281 mmol, 1.00 equiv), $CF_3COOH$ (1.0 mL), DCM (5.0 mL). The resulting solution was stirred for 16 h at room temperature. The resulting mixture was concentrated. The reaction was then quenched by the addition of 20 mL of water. The pH value of the solution was adjusted to 8 with $NaHCO_3$(aq). The resulting solution was extracted with 2x20 mL of ethyl acetate and the organic layers combined and concentrated. The crude product (120 mg) was purified by Prep-HPLC with the following conditions: Column, XBridge Prep C18 OBD Column,, 5um,19x150mm; mobile phase, Water(0.05%$NH_3H_2O$) and ACN (50% PhaseB up to 75% in 7 min); Detector, UV 254nm. This resulted in 25.7 mg (22 % for two steps) of 1-methyl-N-(2-[[(2S)-2-methylpyrrolidin-1-yl]methyl]-1H-pyrrolo[3,2-c]pyridin-6-yl)-3,4-dihydro-2H-quinoline-6-carboxamide as a light brown solid. **LCMS:** [M+H]+=404. **$^1$H-NMR:** (300 MHz, Methanol-$d_4$,*ppm*): δ 8.50 (d, *J* = 1.0 Hz, 1H), 8.17 (t, *J* = 1.0 Hz, 1H), 7.73 (dd, *J* = 8.7, 2.4 Hz, 1H), 7.60 (d, *J* = 2.4 Hz, 1H), 6.66 (d, *J* = 8.7 Hz, 1H), 6.48 (s, 1H), 4.12 (d, *J* = 13.8 Hz, 1H), 3.50 (d, *J* = 13.9 Hz, 1H), 3.43 - 3.35 (m, 2H), 3.00 (s, 4H), 2.84 (t, *J* = 6.3 Hz, 2H), 2.58-2.44 (m, 1H), 2.42-2.29 (m, 1H), 2.11-1.94 (m, 3H), 1.84 - 1.66 (m, 2H), 1.58 - 1.39 (m, 1H), 1.22 (d, *J* = 6.1 Hz, 3H).

Preparation of Example 42

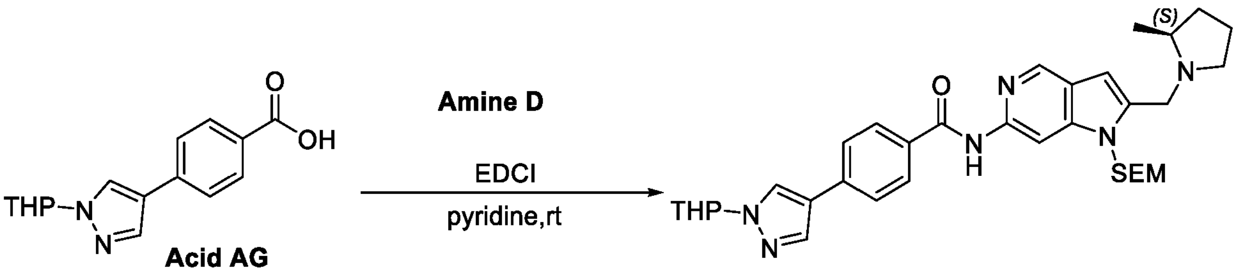


**Acid AG**

Into a 8-mL round-bottom flask, was placed 2-[[(2R)-2-methylpyrrolidin-1-yl]methyl]-1-[[2-(trimethylsilyl)ethoxy]methyl]pyrrolo[3,2-c]pyridin-6-amine **(Amine D,** 264.83 mg, 0.734 mmol, 1.00 equiv), Pyridine (2.00 mL), EDCI (563.20 mg, 2.938 mmol, 4.00 equiv), 4-[1-(oxan-2-yl)pyrazol-4-yl]benzoic acid (200.00 mg, 0.734 mmol, 1.00 equiv). The resulting solution was stirred for 4 hr at room temperature. The resulting solution was diluted with 10 mL of $H_2O$. The resulting solution was extracted with 3x10 mL of ethyl acetate and the organic layers combined and concentrated. This resulted in 100 mg (22.14%) of N-(2-[[(2R)-2-methylpyrrolidin-1-yl]methyl]-1-[[2-(trimethylsilyl)ethoxy]methyl]pyrrolo[3,2-c]pyridin-6-yl)-4-[1-(oxan-2-yl)pyrazol-4-yl]benzamide as yellow oil. **LCMS:** $[M+1]^+$=615

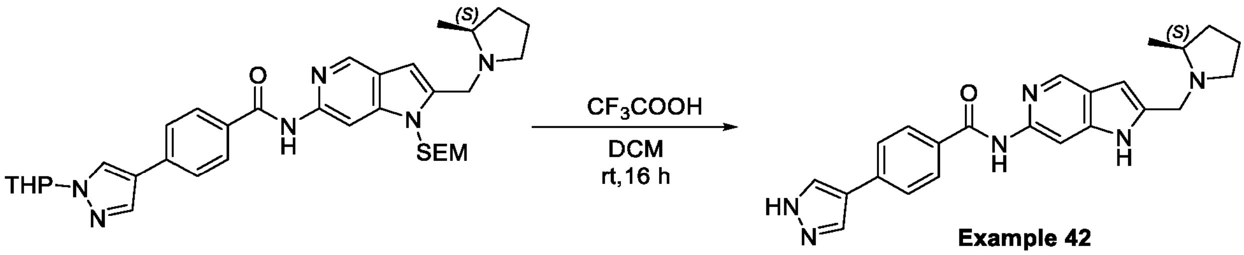


Example 42

Into a 8-mL round-bottom flask, was placed N-(2-[[(2S)-2-methylpyrrolidin-1-yl]methyl]-1-[[2-(trimethylsilyl) ethoxy]methyl]pyrrolo[3,2-c]pyridin-6-yl)-4-[1-(oxan-2-yl)pyrazol-4-yl]benzamide (100.00 mg, 0.163 mmol, 1.00 equiv), DCM (2.00 mL), $CF_3COOH$(79.71 mg, 0.813 mmol, 5.00 equiv). The resulting solution was stirred for 16 hr at room temperature. The resulting solution was diluted with 10 mL of $H_2O$. The pH value of the solution was adjusted to 7-8 with $NaHCO_3$ (1 mol/L). The resulting solution was extracted with 3x10 mL of ethyl acetate and the organic layers combined and concentrated. The crude product was purified by Prep-HPLC with the following conditions: Column: HPH C18, 50x3.0 mm, 2.6 um; Mobile Phase A: Water/0.05% $NH_3.H_2O$, Mobile Phase B: ACN; Flow rate: 1.2 mL/min; Gradient: 5%B to 100%B in 1.1 min, hold 0.7 min), Detector, UV 254 nm. This resulted in 8.6 mg (13.20%) of N-(2-[[(2S)-2-methylpyrrolidin-1-yl] methyl]-1H-pyrrolo[3,2-c]pyridin-6-yl)-4-(1H-pyrazol-4-yl)benzamide as a white solid. **LCMS:** $[M+1]^+$=401. **$^1$H-NMR:** (300 MHz, Methanol-$d_4$, ppm): δ 13.01 (s, 1H), 11.35 (s, 1H), 10.40 (s, 1H), 8.52 (d, *J* = 1.1 Hz, 1H), 8.21 (d, *J* = 7.3 Hz, 3H), 8.06 (d, *J* = 8.4 Hz, 2H), 7.79 - 7.70 (m, 2H), 6.37 (s, 1H), 4.01 (d, *J* = 13.9 Hz, 1H), 3.40 (d, *J* = 13.8 Hz, 1H), 2.88 (s, 1H), 2.43 (m, 1H), 2.20 (m, 1H), 1.92 (m, 1H), 1.64 (s, 2H), 1.37 (s, 1H), 1.11 (d, *J* = 6.0 Hz, 3H).

Preparation of Example 43

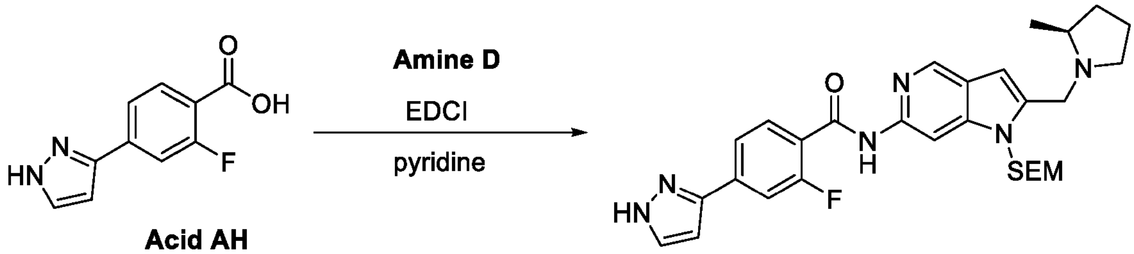


Acid AH

Into a 8-mL vial, was placed 2-[[(2S)-2-methylpyrrolidin-1-yl]methyl]-1-[[2-(trimethylsilyl)ethoxy]methyl]pyrrolo [3,2-c]pyridin-6-amine (Amine D, 104.93 mg, 0.291 mmol, 1.00 equiv), pyridine (2.10 mL), 2-fluoro-4-(1H-pyrazol-3-yl) benzoic acid (Prepared according Example 43, step 2 above, 60.00 mg, 0.291 mmol, 1.00 equiv), EDCI (83.68 mg, 0.437 mmol, 1.50 equiv). The resulting solution was stirred for 6 hr at 50 °C in an oil bath. The resulting mixture was concentrated under vacuum. The resulting solution was diluted with 15 mL of $H_2O$. The resulting solution was extracted with 3x10 mL of ethyl acetate and the organic layers combined. The resulting mixture was washed with 2 x20 mL of Brine. The mixture was dried over anhydrous sodium sulfate and concentrated under vacuum. This resulted in 160 mg (81.41%) of 2-fluoro-N-(2-[[(2S)-2-methylpyrrolidin-1-yl]methyl]-1-[[2-(trimethylsilyl)ethoxy]methyl]pyrrolo[3,2-c]pyridin-6-yl)-4-(1H-pyrazol-3-yl)benzamide as a brown oil. **LCMS:** $[M+H]^+$=549.

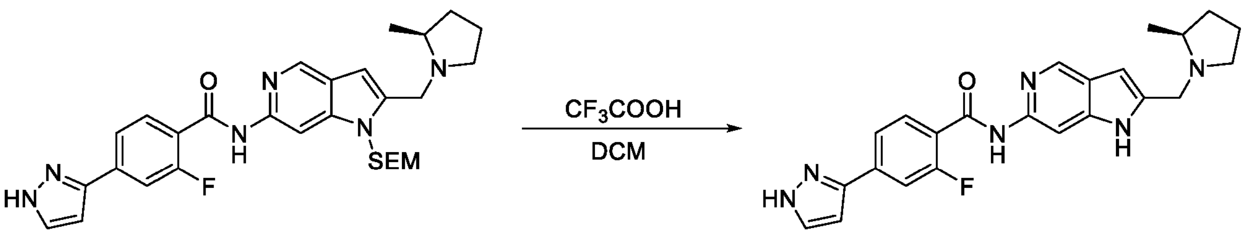


Example 43

Into a 8-mL vial, was placed 2-fluoro-N-(2-[[(2S)-2-methylpyrrolidin-1-yl]methyl]-1-[[2-(trimethylsilyl)ethoxy] methyl]pyrrolo[3,2-c]pyridin-6-yl)-4-(1H-pyrazol-3-yl)benzamide (160.00 mg, 0.292 mmol, 1 equiv), DCM (2.00 mL), $CF_3COOH$ (2.00 mL). The resulting solution was stirred for 16 hr at room temperature. The resulting mixture was

concentrated under vacuum. The pH value of the solution was adjusted to 8 with $Et_2NH$. The crude product (90 mg) was purified by Prep-HPLC with the following conditions: Column, XBridge Prep C18 OBD Column, 5um,19x150mm; mobile phase, Water(0.05%$NH_3H_2O$) and ACN (36% PhaseB up to 59% in 7 min); Detector, UV 254 nm. This resulted in 25.8 mg (21.14%) of 2-fluoro-N-(2-[[(2S)-2-methylpyrrolidin-1-yl]methyl]-1H-pyrrolo[3,2-c]pyridin-6-yl)-4-(1H-pyrazol-3-yl)benzamide as a light yellow solid. **LCMS:** $[M+H]^+$=419. **$^1$H-NMR:** (300 MHz, Methanol-$d_4$, ppm) δ 8.53 (d, *J* = 1.0 Hz, 1H), 8.31 (s, 1H), 8.02 (t, *J* = 8.0 Hz, 1H), 7.76 (d, *J* = 12.2 Hz, 3H), 6.85 (d, *J* = 2.4 Hz, 1H), 6.54 (s, 1H), 4.17 (d, *J* = 14.0 Hz, 1H), 3.60 (d, *J* = 30.3 Hz, 1H), 3.06 (s, 1H), 2.59 (s, 1H), 2.42 (d, *J* = 9.4 Hz, 1H), 1.77 (d, *J* = 8.1 Hz, 2H), 1.61-1.48 (m, 1H), 1.25 (d, *J* = 6.1 Hz, 3H). **$^{19}$F-NMR:** (300 MHz, Methanol-$d_4$, ppm) δ -115.077.

Preparation of Example 53

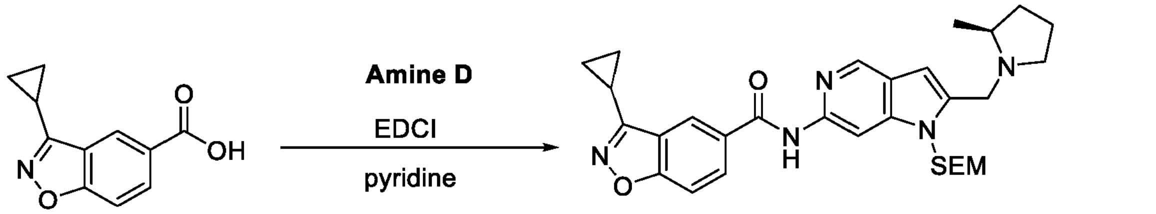


Acid AP

Into a 8-mL vial purged and maintained with an inert atmosphere of nitrogen, was placed 3-cyclopropyl-1,2-benzoxazole-5-carboxylic acid (54.10 mg, 0.266 mmol, 1.20 equiv), 2-[[(2S)-2-methylpyrrolidin-1-yl]methyl]-1-[[2-(trimethylsilyl)ethoxy]methyl]pyrrolo[3,2-c]pyridin-6-amine **(Amine D,** 80.00 mg, 0.222 mmol, 1.00 equiv), EDCI (63.80 mg, 0.333 mmol, 1.5 equiv), Pyridine (3.00 mL). The resulting solution was stirred for 12 h at 25 °C. The reaction was then quenched by the addition of 15 mL of water/ice. The resulting solution was extracted with 3x10 mL of ethyl acetate and the organic layers combined. The resulting mixture was washed with 3 x10 mL of brine. The resulting mixture was concentrated. This resulted in 100 mg (crude) of 3-cyclopropyl-N-(2-[[(2S)-2-methylpyrrolidin-1-yl]methyl]-1-[[2-(trimethylsilyl)ethoxy]methyl]pyrrolo[3,2-c]pyridin-6-yl)-1,2-benzoxazole-5-carboxamide as brown oil. **LCMS:** $[M+1]^+$=546.

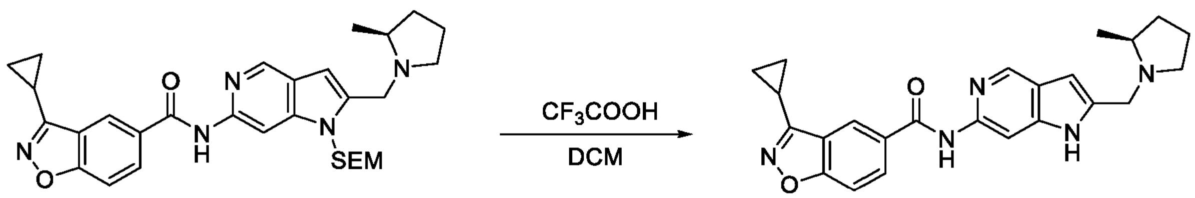


Example 53

Into a 50-mL round-bottom flask purged and maintained with an inert atmosphere of nitrogen, was placed 3-cyclopropyl-N-(2-[[(2S)-2-methylpyrrolidin-1-yl]methyl]-1-[[2-(trimethylsilyl)ethoxy]methyl]pyrrolo[3,2-c]pyridin-6-yl)-1,2-benzoxazole-5-carboxamide (100.00 mg, 0.183 mmol, 1.00 equiv), $CF_3COOH$ (2.00 mL), DCM (2.00 mL). The resulting solution was stirred for 16 h at 25 °C.The resulting mixture was concentrated. The crude product was purified by Prep-HPLC with the following conditions: Column,XBridge Prep C18 OBD Column,,5um,19x150mm ; mobile phase, Water(0.05%$NH_3.H_2O$) and ACN (41% PhaseB up to 66% in 7 min);Detector,UV 254 nm.This resulted in 24 mg (31.52%) of 3-cyclopropyl-N-(2-[[(2S)-2-methylpyrrolidin-1-yl]methyl]-1H-pyrrolo[3,2-c]pyridin-6-yl)-1,2-benzoxazole-5-carboxamide as a light yellow solid. **LCMS:** $[M+1]^+$= 416. **H-NMR:** (300 MHz, Methanol-$d_4$, ppm):δ 8.57(s,1H),8.51(s,1H),8.26(d,*J*=6.9Hz, 1H),8.20(s,1H) 7.73 (d, *J* = 8.7 Hz, 1H), 6.53 (s, 1H), 4.15 (d, *J* = 13.8 Hz, 1H), 3.54 (d, *J* = 13.8 Hz, 1H), 3.05 (s, 1H), 2.57(s, 1H), 2.46-2.33 (m, 2H), 2.04 (s, 1H), 1.77 (s, 2H), 1.52 (s, 2H), 1.28-1.22 (m,7H).

The following examples in **Table L** were prepared in a similar fashion to that shown above for **Example 53** using **Amine D** and the appropriate reagents and conditions.

Table L

| Ex. | Acid | Structure | LCMS | $^1$H-NMR |
|---|---|---|---|---|
| **57** | **AT** | 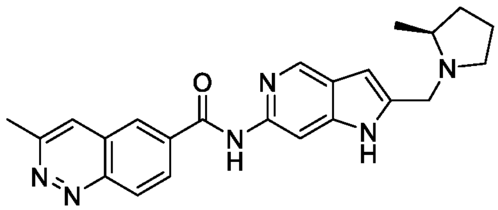 | **401** | (300 MHz, Methanol-$d_4$, ppm): δ 8.58-8.53 (m, 3H), 8.38 (d, $J$ = 9.0, 1H), 8.24 (s, 1H), 8.19 (s, 1H), 6.53 (s, 1H), 4.15 (d, $J$ = 13.8 Hz, 1H), 3.54 (d, $J$ = 13.8Hz, 1H), 3.08-2.99 (m, 4H), 2.58-2.51 (m,1H), 2.37 (m, 1H), 2.06-2.02 (m, 1H), 1.81-1.73(m, 2H), 1.55-1.50 (m, 1H), 1.23 (d, $J$ = 6.0 Hz, 3H). |
| **72** | **BE** | 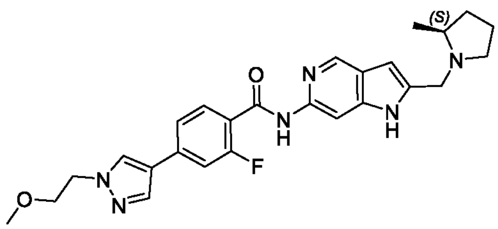 | **477** | (300 MHz, Methanol-$d_4$, ppm):δ 8.51 (s, 1H), 8.30 (s, 1H), 8.18 (s, 1H), 8.00-7.95 (m, 2H), 7.59-7.51 (m, 2H), 6.51 (s, 1H), 4.37 (t, $J$ = 5.2 Hz, 2H), 4.13 (d, $J$ = 13.9 Hz, 1H), 3.80 (t, $J$ = 5.2 Hz, 2H), 3.52 (d, $J$ = 13.9 Hz, 1H), 3.36-3.32 (m, 3H), 3.07-3.00 (m, 1H),2.57-2.52 (m, 1H), 2.40-2.31 (m, 1H), 2.10-2.00 (m, 1H), 1.76 (s, 2H), 1.56-1.46 (m, 1H), 1.23 (d, $J$ = 6.0 Hz, 3H).<br>F-NMR (300 MHz, Methanol-$d_4$, ppm):-114.764 |
| **75** | **BG** | 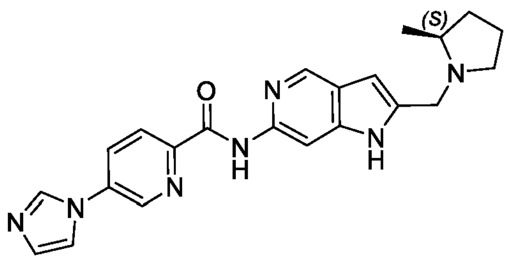 | **402** | (300 MHz, Methanol-$d_4$, ppm)δ 9.07 (d, $J$ = 2.1 Hz, 1H), 8.54 (s, 1H), 8.45-8.42 (m, 3H), 8.32 (dd, $J$ = 8.5, 2.6 Hz, 1H), 7.83 (s, 1H), 7.28 (s, 1H), 6.54 (s, 1H), 4.14 (d, $J$ = 15.0 Hz, 1H), 3.54 (d, $J$ = 14.1 Hz, 1H), 3.09-3.01 (s, 1H), 2.55 (s, 1H), 2.41-2.32 (m, 1H),2.06(s,1H),1.77 (s, 2H), 1.50 (s, 1H), 1.23 (d, $J$ = 6.0 Hz, 3H). |
| **85** | **BQ** | 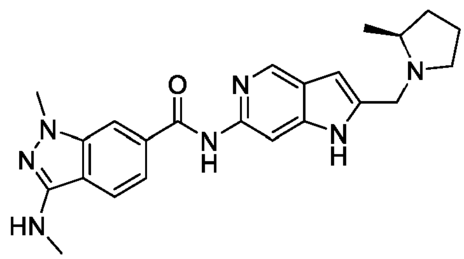 | **418** | (300 MHz, Methanol-$d_4$, ppm)δ 8.56 (s, 1H), 8.23 (s, 1H), 7.98 (s, 1H), 7.78 (d, $J$ = 8.4, 1H), 7.56 (dd, J = 8.4, 1.5 Hz, 1H), 6.52 (s, 1H), 4.14 (d, $J$ = 13.8 Hz, 1H), 3.92 (s, 3H), 3.53 (d, $J$ = 14.1 Hz, 1H), 3.02 (s, 4H), 2.58-2.50 (m, 1H), 2.36 (q, $J$ = 9.0 Hz, 1H), 2.08-2.01(m, 1H), 1.81-1.72 (m, 2H), 1.56-1.51 (m, 1H), 1.23 (d, $J$ = 6.0 Hz, 3H). |
| **90** | **BU** | 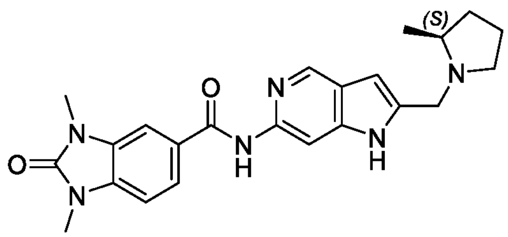 | **419** | (300 MHz, Methanol-$d_4$, ppm):δ 8.54 (d, $J$ = 0.6 Hz, 1H), 8.20 (s, 1H), 7.89-7.81(m, 2H), 7.28 (d, $J$ = 8.1 Hz, 1H), 6.52 (s, 1H), 4.15 (d, $J$ = 14.1 Hz, 1H), 3.57 (s, 1H), 3.41 (d, $J$ = 10.5 Hz, 6H), 3.07-3.02 (m, 1H), 2.56 (q, $J$ = 7.2 Hz, 1H), 2.38 (q, $J$ = 9.0 Hz, 1H), 2.08-2.02 (m, 1H), 1.80-1.73 (m, 2H), 1.54 - 1.40 (m, 1H), 1.23 (d, $J$ = 6.3 Hz, 3H). |

(continued)

| Ex. | Acid | Structure | LCMS | $^1$H-NMR |
|---|---|---|---|---|
| 93 | BX | 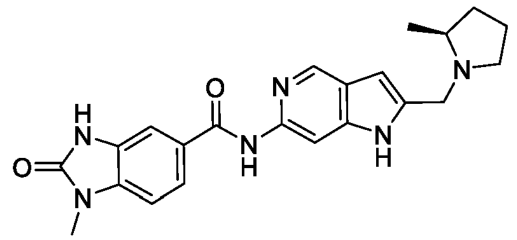 | 405 | (300 MHz, Methanol-$d_4$, ppm)δ 8.54 (d, $J$ = 0.9 Hz, 1H), 8.20 (s, 1H), 7.84 (dd, $J$ = 8.4, 1.5 Hz, 1H), 7.74 (d, $J$ = 1.5 Hz, 1H), 7.27 (d, $J$ = 8.4 Hz, 1H), 6.51 (s, 1H), 4.14 (d, $J$ = 13.8 Hz, 1H), 3.53 (d, $J$ = 14.1 Hz, 1H), 3.47 (s, 3H), 3.10-3.01 (m, 1H), 2.57-2.50 (m, 1H), 2.36 (q, $J$ = 9.1 Hz, 1H), 2.08-1.91 (m, 1H), 1.79-1.72 (m, 2H), 1.56-1.47 (m, 1H), 1.23 (d, $J$ = 6.1 Hz, 3H). |

Preparation of Example 69

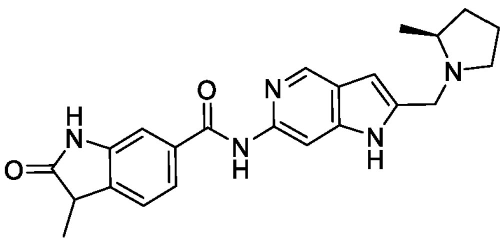

Into a 8-mL round-bottom flask, was placed 3-methyl-N-(2-[[(2S)-2-methylpyrrolidin-1-yl]methyl]-1H-pyrrolo [3,2-c]pyridin-6-yl)-1H-indole-6-carboxamide **(Example 61,** 40.00 mg, 0.103 mmol, 1.00 equiv), hydrogen chloride (1.00 mL), DMSO (2.00 mL). This was followed by the addition of $H_2O$ (1.00 mL) dropwise with stirring at °C in 5 min. The resulting solution was stirred for 12 hr at room temperature. The resulting mixture was concentrated. The resulting solution was diluted with 10 mL of $H_2O$. The resulting solution was extracted with 3 x 10 mL of dichloromethane and the organic layers combined and concentrated. The crude product was purified by Prep-HPLC with the following conditions: Column: HPH C18, 50x3.0 mm, 2.6 um; Mobile Phase A: Water/0.05% $NH_3.H_2O$, Mobile Phase B: ACN; Flow rate: 1.2 mL/min; Gradient: 5%B to 100%B in 1.1 min, hold 0.7 min), Detector, UV 254 nm. This resulted in 12.5 mg (30.01%) of 3-methyl-N-(2-[[(2S)-2-methylpyrrolidin-1-yl]methyl]-1H-pyrrolo[3,2-c]pyridin-6-yl)-2-oxo-1,3-dihydroindole-6-carboxamide as a white solid. **LCMS:** $[M+1]^+$=404. **$^1$H-NMR:** (300 MHz, Methanol-$d_4$, ppm): δ 8.54 (d, $J$ = 1.0 Hz, 1H), 8.19 (d, $J$ = 1.0 Hz, 1H), 7.70 (dd, $J$ = 7.7, 1.7 Hz, 1H), 7.54 - 7.41 (m, 2H), 6.52 (s, 1H), 4.14 (d, $J$ = 14.0 Hz, 1H), 3.53 (d, $J$ = 13.9 Hz, 1H), 3.04 (s, 1H), 2.59 - 2.48 (m, 1H), 2.37 (m, 1H), 2.11 - 1.96 (m, 1H), 1.75 (m, 2H), 1.50 (s, 3H), 1.23 (d, $J$ = 6.1 Hz, 3H).

Preparation of Example 87

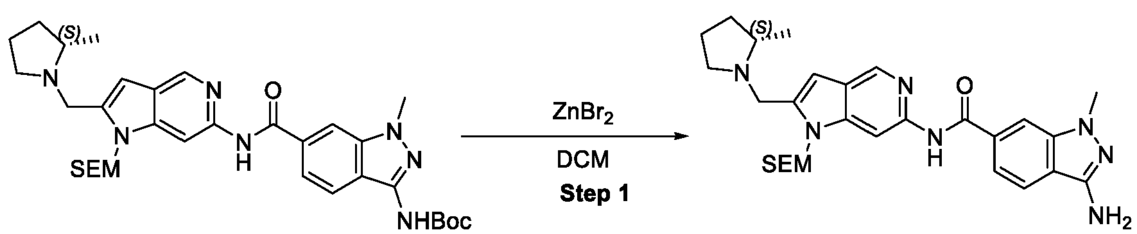


Into a 8-mL sealed tube, was placed tert-butyl N-[1-methyl-6-[(2-[[(2S)-2-methylpyrrolidin-1-yl]methyl]-1-[[2-(trimethylsilyl)ethoxy]methyl]pyrrolo[3,2-c]pyridin-6-yl)carbamoyl]indazol-3-yl]carbamate (200.00 mg, 0.316 mmol, 1.00 equiv), DCM (5.00 mL), $ZnBr_2$ (213.18 mg, 0.947 mmol, 3.00 equiv). The resulting solution was stirred for 16 h at room temperature. The reaction was then quenched by the addition of 20 mL of water. The pH value of the solution was adjusted

to 8 with $NaHCO_3$(aq). The solids were filtered and the resulting solution was extracted with 2x30 mL of ethyl acetate and the organic layers combined and concentrated. This resulted in 156 mg (crude) of 3-amino-1-methyl-N-(2-[[(2S)-2-methylpyrrolidin-1-yl]methyl]-1-[[2-(trimethylsilyl)ethoxy]methyl]pyrrolo[3,2-c]pyridin-6-yl)indazole-6-carboxamide as a brown solid. **LCMS:** [M+H]=534.

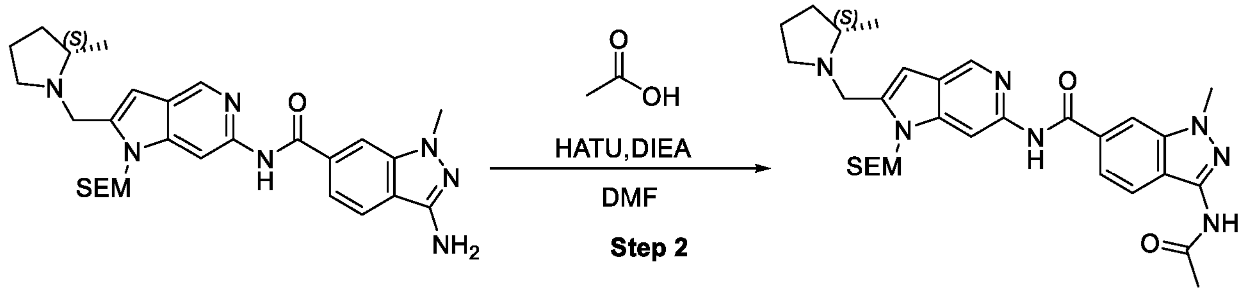


Into a 50-mL round-bottom flask, was placed 3-amino-1-methyl-N-(2-[[(2S)-2-methylpyrrolidin-1-yl]methyl]-1-[[2-(trimethylsilyl)ethoxy]methyl]pyrrolo[3,2-c]pyridin-6-yl)indazole-6-carboxamide (150.00 mg, 0.281 mmol, 1.00 equiv), acetic acid (20.25 mg, 0.337 mmol, 1.20 equiv), DMF (10.00 mL), HATU (160.28 mg, 0.422 mmol, 1.50 equiv). The resulting solution was stirred for 6 h at room temperature. The reaction was then quenched by the addition of 20 mL of water. The resulting solution was extracted with 2x20 mL of ethyl acetate and the organic layers combined and concentrated. This resulted in 200 mg (crude) of 3-acetamido-1-methyl-N-(2-[[(2S)-2-methylpyrrolidin-1-yl]methyl]-1-[[2-(trimethylsilyl)ethoxy]methyl]pyrrolo[3,2-c]pyridin-6-yl)indazole-6-carboxamide as a brown solid. **LCMS:** [M+H]=576.

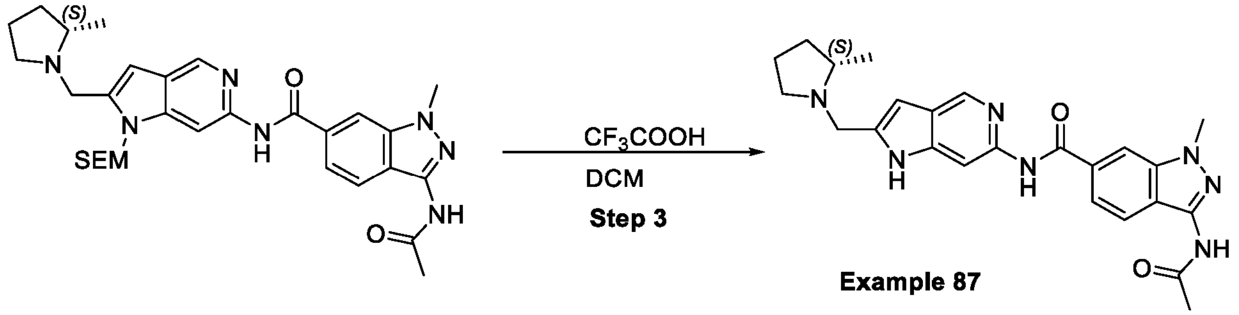


**Example 87**

Into a 50-mL round-bottom flask, was placed 3-acetamido-1-methyl-N-(2-[[(2S)-2-methylpyrrolidin-1-yl]methyl]-1-[[2-(trimethylsilyl)ethoxy]methyl]pyrrolo[3,2-c]pyridin-6-yl)indazole-6-carboxamide (200.00 mg, 1 equiv, crude), $CF_3COOH$ (10.00 mL), DCM (10.00 mL). The resulting solution was stirred for 16 h at room temperature. The resulting mixture was concentrated. The reaction was then quenched by the addition of 10 mL of water. The pH value of the solution was adjusted to 9 with $NaHCO_3$(aq). The resulting solution was extracted with 2x20 mL of ethyl acetate and the organic layers combined and concentrated. The crude product (110 mg) was purified by Prep-HPLC with the following conditions: Column, XBridge Shield RP18 OBD Column, 5um,19x150mm; mobile phase, Water(0.05%$NH_3H_2O$) and ACN (19% PhaseB up to 44% in 7 min); Detector, UV 254nm. This resulted in 22.4 mg (18% for two steps) of 3-acetamido-1-methyl-N-(2-[[(2S)-2-methylpyrrolidin-1-yl]methyl]-1H-pyrrolo[3,2-c]pyridin-6-yl)indazole-6-carboxamide as a light brown solid. **LCMS:** [M+H]=446. **$^1$H-NMR** (300 MHz, Methanol-$d_4$,*ppm*): δ8.56 (d, *J* = 1.0 Hz, 1H), 8.26-8.16 (m, 2H), 7.96 (d, *J* = 8.6 Hz, 1H), 7.73 (dd, *J* = 8.6, 1.4 Hz, 1H), 6.52 (s, 1H), 4.14 (d, *J* = 14.3 Hz, 1H), 4.11 (s, 3H), 3.53 (d, *J* = 14.0 Hz, 1H), 3.11-2.98 (m, 1H), 2.63-2.46 (m, 1H), 2.44-2.31 (m, 1H), 2.26 (s, 3H), 2.13-1.98 (m, 1H), 1.77 (s, 2H), 1.86-1.68 (m, 1H), 1.59-1.41 (m, 1H), 1.23 (d, *J* = 6.1 Hz, 3H).

Preparation of Example 94

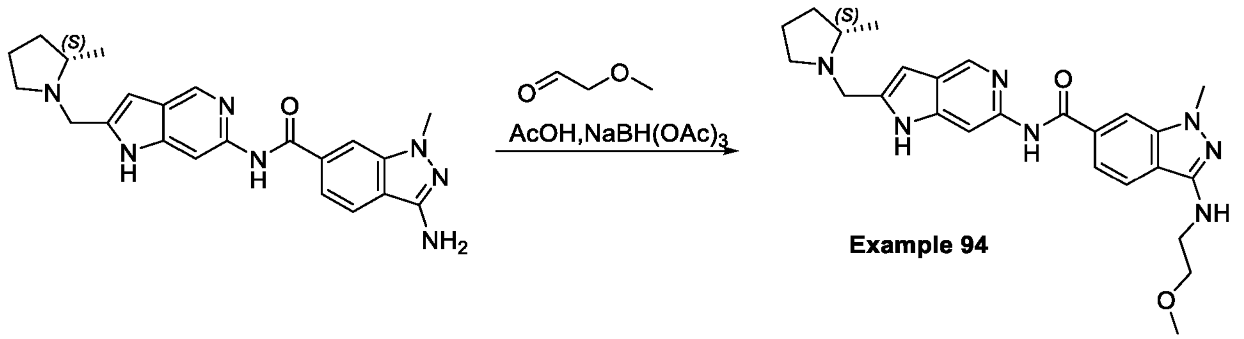


Into a 50-mL 3-necked round-bottom flask purged and maintained with an inert atmosphere of nitrogen, was placed methoxy acetaldehyde (51.41 mg, 0.692 mmol, 4.00 equiv), 3-amino-1-methyl-N-(2-[[(2S)-2-methylpyrrolidin-1-yl] methyl]-1H-pyrrolo[3,2-c]pyridin-6-yl)indazole-6-carboxamide **(Example 87, Step 2,** 70.00 mg, 0.173 mmol, 1.00 equiv), DCE (5.00 mL), $NaBH(AcO)_3$ (367.69 mg, 1.730 mmol, 10.00 equiv). The resulting solution was stirred for 12 h at room temperature. The reaction was then quenched by the addition of 10 mL of water. The resulting solution was extracted with 2x20 mL of dichloromethane and the organic layers combined and concentrated. The crude product (40 mg) was purified by Prep-HPLC with the following conditions: Column, XBridge Shield RP18 OBD Column, 5um, 19x150mm; mobile phase, Water (0.05%$NH_3H_2O$) and ACN (20% PhaseB up to 45% in 7 min); Detector, UV 254nm. This resulted in 9.4 mg (11.74%) of 3-[(2-methoxyethyl)amino]-1-methyl-N-(2-[[(2S)-2-methylpyrrolidin-1-yl]methyl]-1H-pyrrolo[3,2-c]pyridin-6-yl)indazole-6-carboxamide as a light brown solid. **LCMS:** [M+H]=462. **$^1$H-NMR** (300 MHz, Methanol-*$d_4$*,*ppm*): δ8.56 (d, *J* = 1.0 Hz, 1H), 8.23 (t, *J* = 1.0 Hz, 1H), 8.02 - 7.95 (m, 1H), 7.83 (dd, *J* = 8.4, 0.8 Hz, 1H), 7.57 (dd, *J* = 8.4, 1.5 Hz, 1H), 6.52 (s, 1H), 4.14 (d, *J* = 13.8 Hz, 1H), 3.92 (s, 3H), 3.70 (t, *J* = 5.5 Hz, 2H), 3.62-3.48 (m, 3H), 3.43 (s, 3H), 3.05 (t, *J* = 6.7 Hz, 1H), 2.60-2.48 (m, 1H), 2.37 (q, *J*= 9.0 Hz, 1H), 2.12-1.99 (m, 1H), 1.77 (dd, *J* = 11.1, 5.7 Hz, 2H), 1.52 (dd, *J* = 17.3, 9.9 Hz, 1H), 1.23 (d, *J* = 6.1 Hz, 3H).

Preparation of Example 95

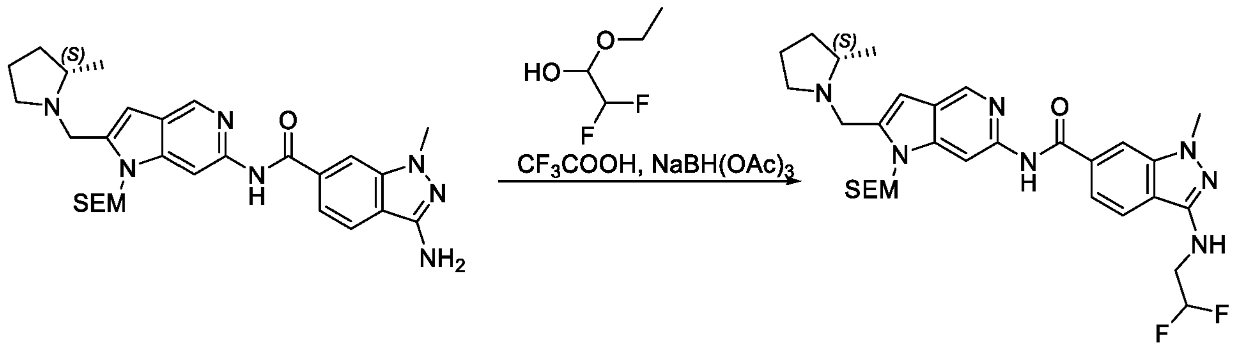


Into a 50-mL round-bottom flask, was placed 3-amino-1-methyl-N-(2-[[(2S)-2-methylpyrrolidin-1-yl] methyl]-1-[[2-(trimethylsilyl)ethoxy]methyl]pyrrolo[3,2-c]pyridin-6-yl)indazole-6-carboxamide **(Example 87, Step 1**, 95 mg, 0.178 mmol, 1.00 equiv), 1-ethoxy-2,2-difluoroethanol (44.89 mg, 0.000 mmol, 2.00 equiv), DCM (6.00 mL), $CF_3COOH$ (40.59 mg, 0.356 mmol, 2.00 equiv). This was followed by the addition of $NaBH(AcO)_3$ (377.22 mg, 1.780 mmol, 10.00 equiv), in portions at 0 °C. The resulting solution was stirred for 1 h at 0 °C. The reaction was then quenched by the addition of 10 mL of water. The resulting solution was extracted with 2x20 mL of ethyl acetate and the organic layers combined and concentrated. This resulted in 120 mg (crude) of 3-[(2,2-difluoroethyl)amino]-1-methyl-N-(2-[[(2S)-2-methylpyrrolidin-1-yl]methyl]-1-[[2-(trimethylsilyl)ethoxy]methyl]pyrrolo[3,2-c]pyridin-6-yl)indazole-6-carboxamide as a brown solid. **LCMS:** [M+H]=598.

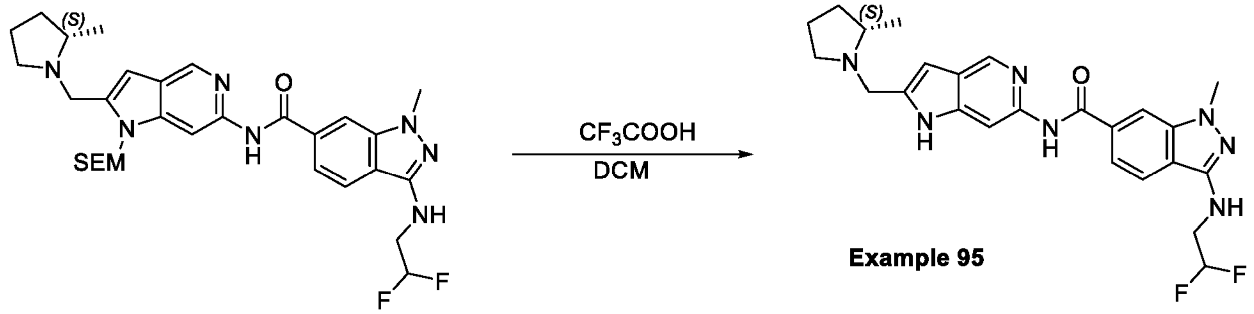

Example 95

Into a 8-mL sealed tube, was placed 3-[(2,2-difluoroethyl)amino]-1-methyl-N-(2-[[(2S)-2-methylpyrrolidin-1-yl]methyl]-1-[[2-(trimethylsilyl)ethoxy]methyl]pyrrolo[3,2-c]pyridin-6-yl)indazole-6-carboxamide (120.00 mg), $CF_3COOH$ (1.00 mL), DCM (1.00 mL). The resulting solution was stirred for 16 h at room temperature. The resulting mixture was concentrated. The pH value of the solution was adjusted to 8 with $NaHCO_3$(aq). The resulting solution was extracted with 2x20 mL of ethyl acetate and the organic layers combined and concentrated. The crude product (100 mg) was purified by Prep-HPLC with the following conditions: Column, XBridge Shield RP18 OBD Column,, 5um, 19x150mm; mobile phase, Water(0.05%$NH_3H_2O$) and ACN (26% PhaseB up to 50% in 7 min); Detector, UV 254nm. This resulted in 19.1(23% for two steps) mg of 3-[(2,2-difluoroethyl)amino]-1-methyl-N-(2-[[(2S)-2-methylpyrrolidin-1-yl]methyl]-1H-pyrrolo[3,2-c]pyridin-6-yl)indazole-6-carboxamide as a light brown solid. **LCMS:** [M+H]=468. **$^1$H-NMR** (300 MHz, Methanol-$d_4$,*ppm*): δ 8.55 (d, *J* = 1.0 Hz, 1H), 8.23 (t, *J* = 1.0 Hz, 1H), 7.99 (t, *J* = 1.1 Hz, 1H), 7.82 (dd, *J* = 8.4, 0.8 Hz, 1H), 7.58 (dd, *J* = 8.5, 1.4 Hz, 1H), 6.52 (s, 1H), 6.14 (t, *J* = 4.4 Hz, 1H), 4.14 (d, *J* = 13.9 Hz, 1H), 3.93 (s, 3H), 3.84-3.67 (m, 2H), 3.53 (d, *J* = 13.9 Hz, 1H), 3.10-2.98 (m, 1H), 2.61-2.47 (m, 1H), 2.43-2.29 (m, 1H), 2.13-1.95 (m, 1H), 1.85-1.68 (m, 2H), 1.58-1.41(m, 1H), 1.23 (d, *J* = 6.1 Hz, 3H). $^{19}$F-NMR (PH-PUK): (300 MHz, Methanol-$d_4$,*ppm*):124.1-124.2(s,2F)

Preparation of Example 99

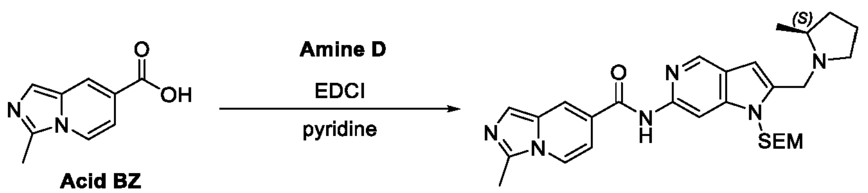

Acid BZ

Into a 8-mL vial purged and maintained with an inert atmosphere of nitrogen, was placed 3-methylimidazo[1,5-a]pyridine-7-carboxylic acid (80.00 mg, 0.454 mmol, 1.00 equiv), pyridine (4.00 mL), 2-[[(2S)-2-methylpyrrolidin-1-yl]methyl]-1-[[2-(trimethylsilyl)ethoxy]methyl]pyrrolo[3,2-c]pyridin-6-amine (163.74 mg, 0.454 mmol, 1.00 equiv), EDCI (130.58 mg, 0.681 mmol, 1.50 equiv). The resulting solution was stirred for 16 hr at room temperature. The resulting mixture was concentrated under vacuum. The resulting solution was diluted with 20 mL of $H_2O$. The resulting solution was extracted with 3x10 mL of ethyl acetate and the organic layers combined. The resulting mixture was washed with 2x10 mL of brine. The mixture was dried over anhydrous sodium sulfate and concentrated under vacuum. This resulted in 100 mg (42.5%) of 3-methyl-N-(2-[[(2S)-2-methylpyrrolidin-1-yl]methyl]-1-[[2-(trimethylsilyl)ethoxy]methyl]pyrrolo[3,2-c]pyridin-6-yl)imidazo[1,5-a]pyridine-7-carboxamide as brown oil. **LCMS:** $[M+H]^+$=519.

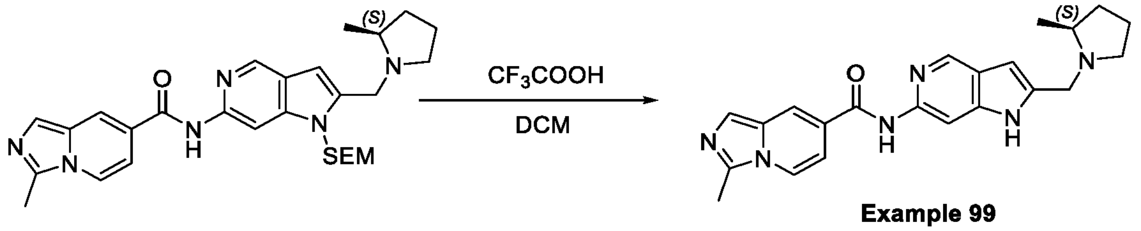

Example 99

Into a 50-mL round-bottom flask, was placed 3-methyl-N-(2-[[(2S)-2-methylpyrrolidin-1-yl]methyl]-1-[[2-(trimethylsilyl)ethoxy]methyl]pyrrolo[3,2-c]pyridin-6-yl)imidazo[1,5-a]pyridine-7-carboxamide (100.00 mg, 1 equiv), DCM (3.00 mL), $CF_3COOH$ (3.00 mL). The resulting solution was stirred for 16 hr at room temperature. The resulting mixture

was concentrated under vacuum. The resulting solution was diluted with 4 mL of DMF. The pH value of the solution was adjusted to 8 with $NH_3$/H2O. The crude product (80 mg) was purified by Prep-HPLC with the following conditions: Column, XBridge Shield RP18 OBD Column, 5um,19x150mm; mobile phase, Water(0.05%$NH_3H_2O$) and ACN (25% PhaseB up to 45% in 7 min); Detector, UV 254 nm. This resulted in 31.3 mg (41.80%) of 3-methyl-N-(2-[[(2S)-2-methylpyrrolidin-1-yl]methyl]-1H-pyrrolo[3,2-c]pyridin-6-yl)imidazo[1,5-a]pyridine-7-carboxamide as a white solid. **LCMS:** $[M+H]^+$=389. **$^1$H-NMR:** (300 MHz, Methanol-$d_4$, ppm) δ 8.56 (d, $J$ = 1.0 Hz, 1H), 8.30 (t, $J$ = 1.5 Hz, 1H), 8.18 (t, $J$ = 1.0 Hz, 1H), 8.15-8.07 (m, 1H), 7.62 (d, $J$ = 0.9 Hz, 1H), 7.25 (dd, $J$ = 7.5, 1.8 Hz, 1H), 6.53 (s, 1H), 4.16 (d, $J$ = 13.9 Hz, 1H), 3.57 (d, $J$ = 13.8 Hz, 1H), 3.11-3.00 (m, 1H), 2.72 (s, 3H), 2.59 (m, 1H), 2.41 (m, 1H), 2.15-1.97 (m, 1H), 1.83-1.70 (m, 2H), 1.60-1.42 (m, 1H), 1.24 (d, $J$ = 6.1 Hz, 3H).

Preparation of Example 41

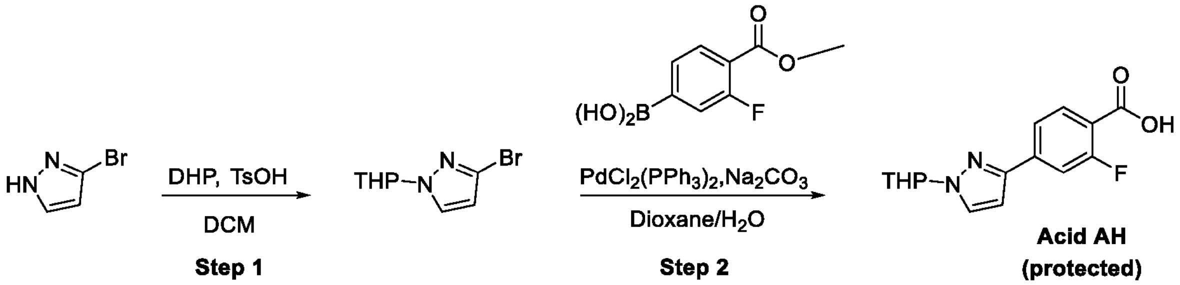


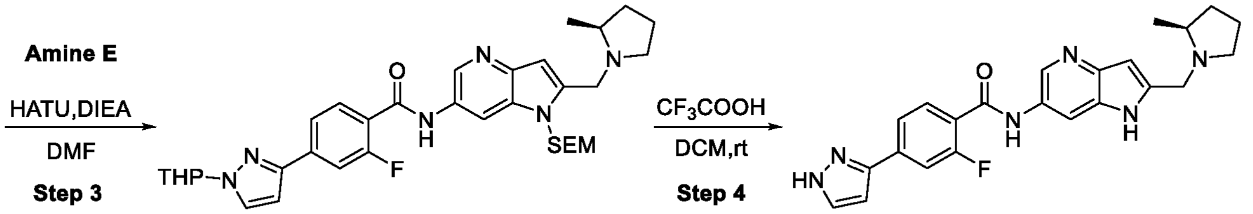


**Example 41**

Step 1. Into a 100-mL round-bottom flask, was placed 3-bromo-1H-pyrazole (1.50 g, 10.206 mmol, 1.00 equiv), DCM (30.00 mL), dihydropyran (1.29 g, 15.336 mmol, 1.50 equiv), TsOH (0.05 g, 0.510 mmol, 0.05 equiv). The resulting solution was stirred for overnight at 80 °C in an oil bath. The resulting solution was diluted with 50 mL of $NaHCO_3$ (5%). The resulting solution was extracted with 3x30 mL of dichloromethane and the organic layers combined and dried over anhydrous sodium sulfate and concentrated under vacuum. This resulted in 2 g (84.8%) of 3-bromo-1-(oxan-2-yl)pyrazole as brown oil. **LCMS:** $[M+H]^+$=231.

Step 2. Into a 40-mL vial purged and maintained with an inert atmosphere of nitrogen, was placed 3-fluoro-4-(methoxycarbonyl)phenylboronic acid (0.86 g, 4.327 mmol, 1.00 equiv), dioxane (12.00 mL), $H_2O$(12.00 mL), 3-bromo-1-(oxan-2-yl)pyrazole (1.00 g, 4.327 mmol, 1.00 equiv), $Na_2CO_3$ (1.38 g, 13.02 mmol, 3.01 equiv), $Pd(PPh_3)_2Cl_2$ (0.30 g, 0.427 mmol, 0.10 equiv), BINAP (0.54 g, 0.867 mmol, 0.20 equiv). The resulting solution was stirred for 16 hr at 80 °C in an oil bath. The resulting mixture was concentrated under vacuum. The resulting solution was diluted with 30 mL of $H_2O$. The resulting solution was extracted with 2x20 mL of ethyl acetate and the aqueous layers combined. The pH value of the solution was adjusted to 3 with HCl (3 mol/L). The solids were collected by filtration. This resulted in 1 g (62.38%) of 2-fluoro-4-[1-(oxan-2-yl)pyrazol-3-yl]benzoic acid as a white solid. **LCMS:** $[M+H]^+$=291.

Step 3. Into a 8-mL vial purged and maintained with an inert atmosphere of nitrogen, was placed 2-fluoro-4-[1-(oxan-2-yl)pyrazol-3-yl]benzoic acid **(Acid AH,** 100.00 mg, 0.344 mmol, 1.00 equiv), DMF (2.50 mL), HATU (157.18 mg, 0.413 mmol, 1.20 equiv), DIEA (89.04 mg, 0.689 mmol, 2.00 equiv), 2-[[(2S)-2-methylpyrrolidin-1-yl]methyl]-1-[[2-(trimethylsilyl)ethoxy]methyl]pyrrolo[3,2-b]pyridin-6-amine **(Amine E,** 124.21 mg, 0.344 mmol, 1.00 equiv). The resulting solution was stirred for 16 hr at room temperature. The resulting solution was diluted with 15 mL of $H_2O$. The resulting solution was extracted with 3x10 mL of ethyl acetate and the organic layers combined. The resulting mixture was washed with 4x10 mL of Brine. The mixture was dried over anhydrous sodium sulfate and concentrated under vacuum. his resulted in 200 mg (76.18%) of 2-fluoro-N-(2-[[(2S)-2-methylpyrrolidin-1-yl]methyl]-1-[[2-(trimethylsilyl)ethoxy]methyl]pyrrolo[3,2-b]pyridin-6-yl)-4-[1-(oxan-2-yl)pyrazol-3-yl]benzamide as a brown solid. **LCMS:** $[M+H]^+$=633.

Step 4. Into a 8-mL vial, was placed 2-fluoro-N-(2-[[(2S)-2-methylpyrrolidin-1-yl]methyl] -1-[[2-(trimethylsilyl) ethoxy]methyl]pyrrolo[3,2-b]pyridin-6-yl)-4-[1-(oxan-2-yl)pyrazol-3-yl]benzamide (200.00 mg, 0.316 mmol, 1.00 equiv), DCM (2.00 mL), $CF_3COOH$ (2 mL). The resulting solution was stirred for 16 hr at room temperature. The resulting mixture was concentrated under vacuum. The pH value of the solution was adjusted to 8 with $Et_2NH$. The crude product (100 mg) was purified by Prep-HPLC with the following conditions: Column, XBridge Prep C18 OBD Column, 5um, 19x150mm; mobile phase, Water(0.05%$NH_3H_2O$) and ACN (30% PhaseB up to 55% in 7 min); Detector, UV 254 nm. This resulted in 26 mg (19.66%) of 2-fluoro-N-(2-[[(2S)-2-methylpyrrolidin-1-yl] ethyl]-1H-pyrrolo[3,2-b]pyridin-6-yl)-4-(1H-pyrazol-3-yl)benzamide as a white solid. **LCMS:** $[M+H]^+$=419. **$^1$H-NMR:** (300 MHz, Methanol-$d_4$, ppm) δ 8.44 -8.14 (m, 2H), 7.87 (t, *J* = 7.6 Hz, 1H), 7.80 (s, 1H), 7.74 (d, *J* = 13.1 Hz, 2H), 6.83 (d, *J* = 2.4 Hz, 1H), 6.53 (s, 1H), 4.18 (d, *J* = 13.9 Hz, 1H), 3.58 (d, *J* = 14.0 Hz, 1H), 3.09-3.00 (m, 1H), 2.57 (m, 1H), 2.39 (m, 1H), 2.14-1.97 (m, 1H), 1.78 (m, 2H), 1.60-1.45 (m, 1H), 1.24 (d, *J* = 6.1 Hz, 3H). **$^{19}$F-NMR:** (300 MHz, Methanol-$d_4$, ppm) δ -115.644.

Preparation of Example 105

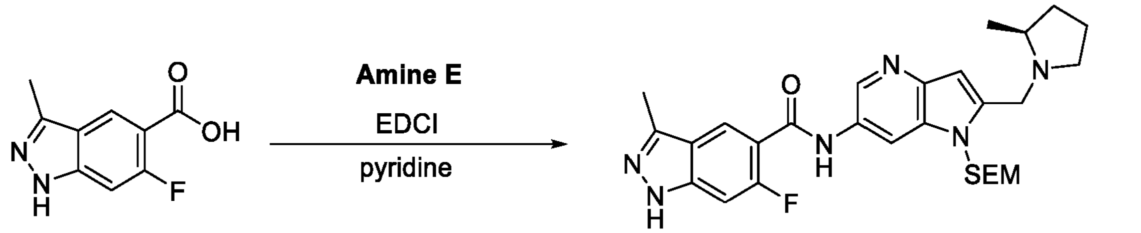


Acid CE

Into a 8-mL vial purged and maintained with an inert atmosphere of nitrogen, was placed 6-fluoro-3-methyl-1H-indazole-5-carboxylic acid (70.0 mg, 0.36 mmol, 1.0 equiv), pyridine (3.00 mL), 2-[[(2S)-2-methylpyrrolidin-1-yl] methyl]-1H-pyrrolo[3,2-b]pyridin-6-amine (83.0 mg, 0.361 mmol, 1.00 equiv), EDCI (103.67 mg, 0.541 mmol, 1.50 equiv). The resulting solution was stirred for 12 hr at room temperature. The resulting mixture was concentrated under vacuum. The resulting solution was diluted with 20 mL of $H_2O$. The resulting solution was extracted with 3x10 mL of ethyl acetate and the organic layers combined. The resulting mixture was washed with 2x20 mL of brine. The mixture was dried over anhydrous sodium sulfate and concentrated under vacuum. This resulted in 100 mg (51.68%) of 6-fluoro-3-methyl-N-(2-[[(2S)-2-methylpyrrolidin-1-yl]methyl]-1-[[2-(trimethylsilyl)ethoxy]methyl]pyrrolo[3,2-b]pyridin-6-yl)-1H-indazole-5-carboxamide as an orange solid. **LCMS:** $[M+H]^+$=537.

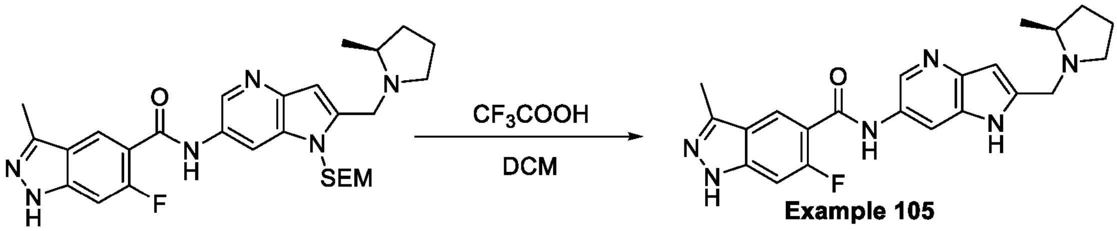


Example 105

Into a 50-mL round-bottom flask, was placed 6-fluoro-3-methyl-N-(2-[[(2S)-2-methylpyrrolidin-1-yl] methyl]-1-[[2-(trimethylsilyl)ethoxy]methyl]pyrrolo[3,2-b]pyridin-6-yl)-1H-indazole-5-carboxamide (100.0 mg, 0.186 mmol, 1.00 equiv), DCM (3.00 mL), $CF_3COOH$ (3.00 mL). The resulting solution was stirred for 16 hr at room temperature. The resulting mixture was concentrated under vacuum. The resulting solution was diluted with 4 mL of DMF. The pH value of the solution was adjusted to 8 with NH3/H2O. The crude product (70 mg) was purified by Prep-HPLC with the following conditions: Column, XBridge Shield RP18 OBD Column, 5um,19*150mm; mobile phase, Water(0.05%$NH_3H_2O$) and ACN (26% PhaseB up to 39% in 7 min); Detector, UV 254 nm. This resulted in 16.6 mg (21.92%) of 6-fluoro-3-methyl-N-(2-[[(2S)-2-methylpyrrolidin-1-yl]methyl]-1H-pyrrolo[3,2-b]pyridin-6-yl)-1H-indazole-5-carboxamide as a solid. **LCMS:** $[M+H]^+$=407. **$^1$H-NMR:** (300 MHz, Methanol-$d_4$, ppm) δ 8.48-8.40 (m, 2H), 8.25 (d, *J* = 6.7 Hz, 1H), 7.32 (d, *J* = 11.1 Hz, 1H), 6.53 (s, 1H), 4.19 (d, *J* = 14.0 Hz, 1H), 3.59 (d, *J* = 14.0 Hz, 1H), 3.07 (s, 1H), 2.62-2.51 (m, 4H), 2.40 (m, 1H), 2.12-1.97 (m, 1H), 1.76 (m, 2H), 1.61-1.42 (m, 1H), 1.24 (d, *J* = 6.1 Hz, 3H). **F-NMR:** (300 MHz, Methanol-$d_4$, ppm) δ -118.207.

The following examples in **Table N** were prepared in a similar fashion to that shown above for **Example 105** using **Amine E** and the appropriate reagents and conditions.

Table N

| Ex. | Acid | Structure | LCMS | $^1$H-NMR |
|---|---|---|---|---|
| 106 | CF | 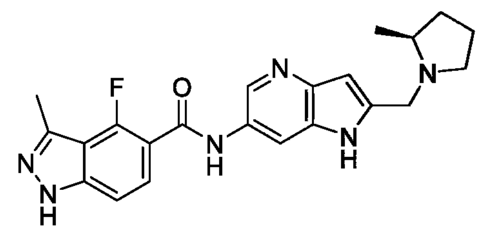 | 407 | (300 MHz, Methanol-$d_4$, ppm)δ 8.42 (d, $J$ = 16.9 Hz, 2H), 7.74 (t, $J$ = 7.6 Hz, 1H), 7.35 (d, $J$ = 8.7 Hz, 1H), 6.50 (s, 1H), 4.15 (d, $J$ = 13.9 Hz, 1H), 3.55 (d, $J$ = 13.9 Hz, 1H), 3.08 - 2.98 (m, 1H), 2.71 (s, 3H), 2.53 (q, $J$ = 7.1 Hz, 1H), 2.35 (q, $J$ = 9.1 Hz, 1H), 2.01 (dt, $J$ = 13.0, 7.9 Hz, 1H), 1.74 (d, $J$ = 11.7 Hz, 2H), 1.48 (t, $J$ = 10.1 Hz, 1H), 1.21 (d, $J$ = 6.1 Hz, 3H). |

Preparation of Example 18 (reference example for comparative purposes)

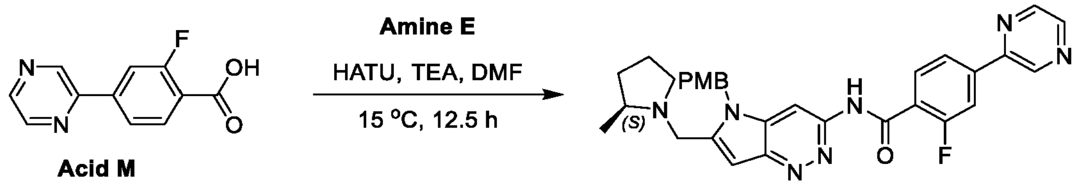


**Acid M**

A mixture of 2-fluoro-4-pyrazin-2-yl-benzoic acid (46.56 mg, 213.41 umol, 1.5 *eq*), HATU (81.14 mg, 213.41 umol, 1.5 *eq*) and TEA (43.19 mg, 426.81 umol, 59.41 uL, 3 *eq*) in DMF (3 mL) was degassed and purged with $N_2$ for 3 times, after 30 min, 5-[(4-methoxyphenyl)methyl]-6-[[(2S)-2-methylpyrrolidin-1-yl]methyl]pyrrolo[3,2-c]pyridazin-3-amine (50 mg, 142.27 umol, 1 *eq*) was added into the mixture, and stirred at 15 °C for 12 h. The reaction mixture was concentrated under reduced pressure to remove solvent. The residue was purified by prep-HPLC (TFA condition). 2-fluoro-N-[5-[(4-methoxyphenyl) methyl]-6-[[(2S)-2-methylpyrrolidin-1-yl]methyl]pyrrolo[3,2-c]pyridazin-3-yl]-4-pyrazin-2-yl-benzamide (15 mg, 20.28 umol, 14.26% yield, 90% purity, TFA) was obtained as a yellow solid.

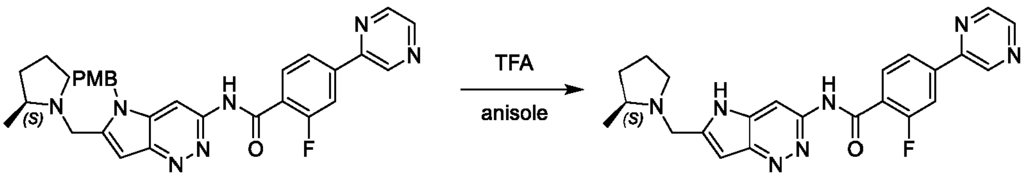


**Example 18**

To 2-fluoro-N-[5-[(4-methoxyphenyl)methyl]-6-[[(2S)-2-methylpyrrolidin-1-yl]methyl] pyrrolo[3,2-c]pyridazin-3-yl]-4-pyrazin-2-yl-benzamide (10 mg, 18.13 umol, 1 *eq*) in TFA (0.5 mL) was added ANISOLE (196.04 ug, 1.81 umol, 1.97 uL, 0.1 *eq*), and then the mixture was stirred at 130 °C for 6 hr under microwave. The reaction mixture (combined with ET16082-1478) was concentrated under reduced pressure to remove solvent. The residue was purified by prep-HPLC. Compound (S)-2-fluoro-N-(6-((2-methylpyrrolidin-1-yl)methyl)-5H-pyrrolo[3,2-c]pyridazin-3-yl)-4-(pyrazin-2-yl)benzamide (1.4 mg, 100% purity, HCl) was obtained as a white solid. LCMS: $[M+H]^+$=432. **$^1$H-NMR:** (400 MHz ,DMSO-$d_6$,ppm) δ 9.28 (s, 1H), 8.78 (s, 1H), 8.68-8.60 (m, 2H), 8.20-8.13 (m, 2H), 8.08 (t, $J$ = 7.83Hz, 1H), 7.40 (s, 1H), 4.99 (d, $J$ = 14.33Hz, 1H), 4.64 (br d, $J$ = 14.33Hz, 1H), 3.79-3.63 (m, 2H), 3.42 (br d, $J$ = 10.58Hz, 1H), 2.44 (br d, $J$ = 7.06Hz, 1H), 2.24-2.13(m, 1H), 2.11 (br s, 1H), 1.94-1.78 (m, 1H), 1.59 (d, $J$ = 6.62Hz, 3H)

The following examples in **Table O** were prepared in a similar fashion to that shown above for **Example 18** using **Amine F** and the appropriate reagents and conditions.

Table O (example 19 is a reference example for comparative purposes)

| Ex. | Acid | Structure | LCMS | $^1$H-NMR |
|---|---|---|---|---|
| 19 | I | 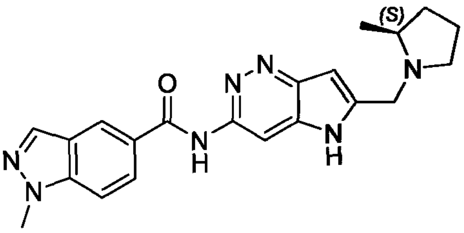 | 390 | (300 MHz ,DMSO-$d_6$,ppm): δ 11.56 (s, 1H), 11.16 (s, 1H), 8.64 (s, 1H), 8.32 (d, $J$ = 1.0 Hz, 1H), 8.24 (d, $J$ = 0.9 Hz, 1H), 8.13 (dd, $J$ = 8.8, 1.7 Hz, 1H), 7.76 (d, $J$ = 8.9 Hz, 1H), 6.65 (s, 1H), 4.11 (s, 4H), 3.49 (d, $J$ = 14.4 Hz, 1H), 2.91 (s, 1H), 2.22 (m, 1H), 1.94 (d, $J$ = 7.0 Hz, 1H), 1.66 (d, $J$ = 8.1 Hz, 2H), 1.39 (s, 1H), 1.12 (d, $J$ = 6.0 Hz, 3H). |

Preparation of Example 45

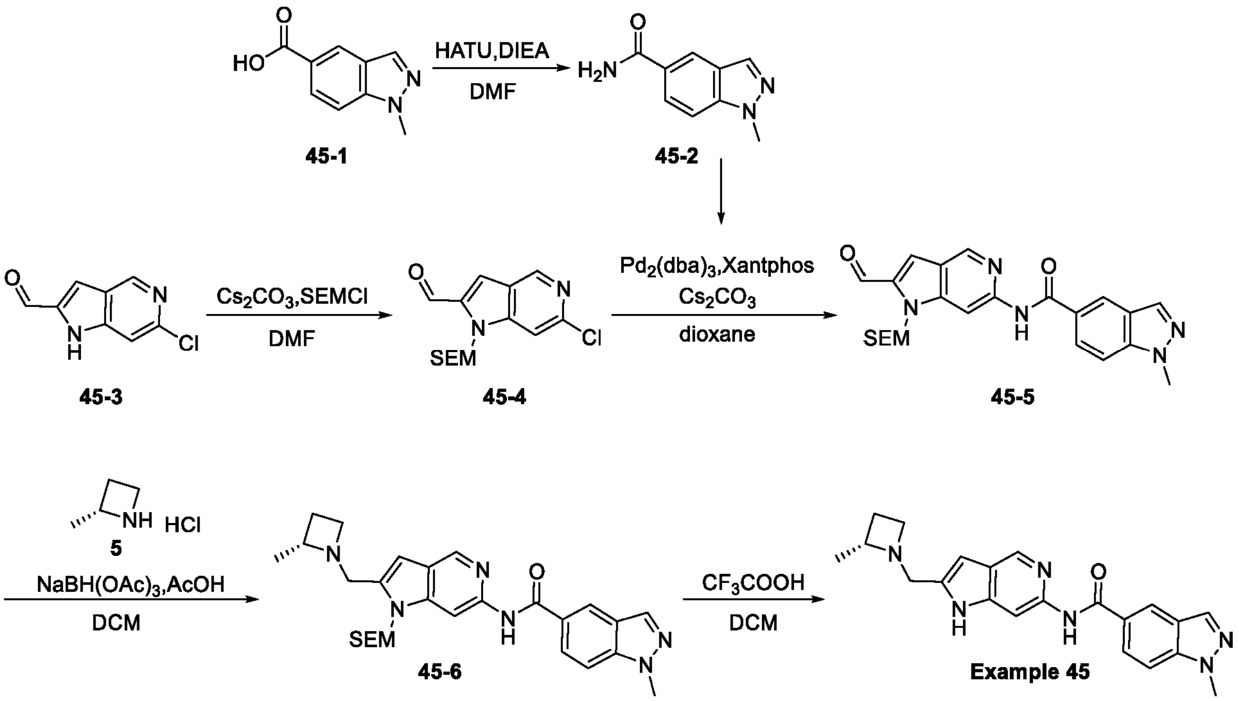


45-1, 45-2, 45-3, 45-4, 45-5, 5, 45-6, Example 45

1-methylindazole-5-carboxamide (45-2)

Into a 100-mL 3-necked round-bottom flask, was placed 1-methylindazole-5-carboxylic acid (1.80 g, 10.22 mmol, 1.00 equiv), DMF (36.0 mL, 465.183 mmol, 45.53 equiv), HATU (5.83 g, 15.326 mmol, 1.50 equiv), DIEA (3.96 g, 30.65 mmol, 3.00 equiv). The resulting solution was stirred for 0.5 hr at room temperature. Then the $NH_4Cl$ (1.09 g, 20.434 mmol, 2.00 equiv) was placed into the flask. The resulting solution was allowed to react, with stirring, for an additional 12 hr at room temperature. The resulting solution was diluted with 50 mL of $H_2O$. The resulting solution was extracted with 3x20 mL of ethyl acetate and the organic layers combined. The resulting mixture was washed with 4 x20 mL of Brine. The mixture was dried over anhydrous sodium sulfate and concentrated under vacuum. This resulted in 1.6 g (89.4%) of 1-methylindazole-5-carboxamide (**45**-2) as a yellow solid. **LCMS:** $[M+H]^+$=176.

6-chloro-1-[[2-(trimethylsilyl)ethoxy]methyl]pyrrolo[3,2-c] pyridine-2-carbaldehyde (45-4)

Into a 250-mL 3-necked round-bottom flask purged and maintained with an inert atmosphere of nitrogen, was placed 6-chloro-1H-pyrrolo[3,2-c]pyridine-2-carbaldehyde **(45-3,** 2.60 g, 14.397 mmol, 1.00 equiv), DMF (78.00 mL), $Cs_2CO_3$ (14.07 g, 43.191 mmol, 3.00 equiv). The resulting solution was stirred at 0 °C in an ice/salt bath. The SEMCl (3.12 g, 18.714 mmol, 1.30 equiv) was placed into the flask. The resulting solution was allowed to react, with stirring, for an

additional 4 hr at room temperature. The resulting solution was diluted with 70 mL of Ethyl Acetate. The solids were filtered out. The resulting solution was extracted with 3 x 50 mL of ethyl acetate and the organic layers combined. The resulting mixture was washed with 4 x 50 mL of Brine. The mixture was dried over anhydrous sodium sulfate and concentrated under vacuum. This resulted in 2.5 g (55.9%) of 6-chloro-1-[[2-(trimethylsilyl)ethoxy]methyl]pyrrolo[3,2-c] pyridine-2-carbaldehyde **(45-2)** as a light brown solid. **LCMS:** $[M+H]^+$=311.

N-(2-formyl-1-[[2-(trimethylsilyl)ethoxy]methyl]pyrrolo[3,2-c] pyridin-6-yl)-1-methylindazole-5-carboxamide (45-5)

Into a 100-mL 3-necked round-bottom flask purged and maintained with an inert atmosphere of nitrogen, was placed 6-chloro-1-[[2-(trimethylsilyl)ethoxy]methyl]pyrrolo[3,2-c]pyridine-2-carbaldehyde **(45-4,** 2.40 g, 7.721 mmol, 1.00 equiv), dioxane (36.00 mL), 1-methylindazole-5-carboxamide **(45-2,** 1487.87 mg, 0.000 mmol, 1.10 equiv), cesium carbonate (7570.06 mg, 23.163 mmol, 3.00 equiv), $Pd_2(dba)_3$ (707.01 mg, 0.772 mmol, 0.10 equiv), Xantphos (893.48 mg, 1.544 mmol, 0.20 equiv). The resulting solution was stirred for 5 hr at 110 °C in an oil bath. The resulting mixture was concentrated under vacuum. The resulting solution was diluted with 50 mL of $H_2O$. The resulting solution was extracted with 3x30 mL of ethyl acetate and the organic layers combined and dried over anhydrous sodium sulfate and concentrated under vacuum. The residue was applied onto a silica gel column with ethyl acetate/petroleum ether (1:1). This resulted in 1.8 g of N-(2-formyl-1-[[2-(trimethylsilyl)ethoxy]methyl]pyrrolo[3,2-c] pyridin-6-yl)-1-methylindazole-5-carboxamide **(45-5)** as a light yellow solid. **LCMS:** $[M+H]^+$=450.

1-methyl-N-(2-[[(2R)-2-methylazetidin-1-yl]methyl]-1-[[2-(trimethylsilyl)ethoxy]methyl]pyrrolo[3,2-c]pyridin-6-yl)indazole-5-carboxamide (45-6)

Into a 8-mL vial, was placed N-(2-formyl-1-[[2-(trimethylsilyl)ethoxy]methyl]pyrrolo[3,2-c]pyridin-6-yl) -1-methylindazole-5-carboxamide **(45-5,** 150 mg, 0.334 mmol, 1.00 equiv), DCM (3.00 mL, 47.190 mmol, 141.44 equiv), (2R)-2-methylazetidine hydrochloride (43.07 mg, 0.401 mmol, 1.20 equiv), acetic acid (2.00 mg, 0.033 mmol, 0.10 equiv). The resulting solution was stirred for 1 hr at room temperature. The $NaBH(OAc)_3$ (106.07 mg, 0.501 mmol, 1.50 equiv) was added the vial, and the resulting solution was stirred for 11 hr at room temperature. The resulting solution was diluted with 15 mL of $H_2O$. The resulting solution was extracted with 3x10 mL of dichloromethane and the organic layers combined. The resulting mixture was washed with 2 x 20 mL of Brine. The mixture was dried over anhydrous sodium sulfate and concentrated under vacuum. This resulted in 160 mg (68.05%) of 1-methyl-N-(2-[[(2R)-2-methylazetidin-1-yl]methyl]-1-[[2-(trimethylsilyl)ethoxy]methyl]pyrrolo[3,2-c]pyridin-6-yl)indazole-5-carboxamide **(45-6)** as a yellow solid. **LCMS:** $[M+H]^+$=505.

1-methyl-N-(2-[[(2R)-2-methylazetidin-1-yl]methyl]-1H-pyrrolo[3,2-c]pyridin-6-yl)indazole -5-carboxamide (Example 45)

Into a 8-mL vial, was placed 1-methyl-N-(2-[[(2R)-2-methylazetidin-1-yl]methyl]-1-[[2-(trimethylsilyl) ethoxy] methyl]pyrrolo[3,2-c]pyridin-6-yl)indazole-5-carboxamide (160.00 mg, 0.317 mmol, 1.00 equiv), DCM (1.60 mL), $CF_3COOH$ (1.60 mL). The resulting solution was stirred for 12 hr at room temperature. The resulting mixture was concentrated under vacuum. The pH value of the solution was adjusted to 8 with $Et_2NH$. The crude product (100 mg) was purified by Prep-HPLC with the following conditions: Column, XBridge Prep C18 OBD Column, 5um,19 x 150mm; mobile phase, Water(0.05%$NH_3H_2O$) and ACN (27% PhaseB up to 45% in 7 min); Detector, UV 254 nm. This resulted in 39.7 mg (33.44%) of 1-methyl-N-(2-[[(2R)-2-methylazetidin-1-yl]methyl]-1H-pyrrolo[3,2-c]pyridin-6-yl)indazole -5-carboxamide **(Example 45)** as a white solid. **LCMS:** $[M+H]^+$=375. **$^1$H-NMR:** (300 MHz, Methanol-$d_4$, ppm) $\delta$ 8.59-8.47 (m, 2H), 8.25-8.17 (m, 2H), 8.08 (dd, $J$ = 8.9, 1.7 Hz, 1H), 7.71 (m, 1H), 6.51 (d, $J$ = 1.0 Hz, 1H), 4.15 (s, 3H), 3.84 (d, $J$ = 13.6 Hz, 1H), 3.72 (d, $J$ = 13.7 Hz, 1H), 3.50 - 3.33 (m, 2H), 3.12-2.98 (m, 1H), 2.23-2.08 (m, 1H), 1.85 (m, 1H), 1.13 (d, $J$ = 6.2 Hz, 3H).

Preparation of Example 46

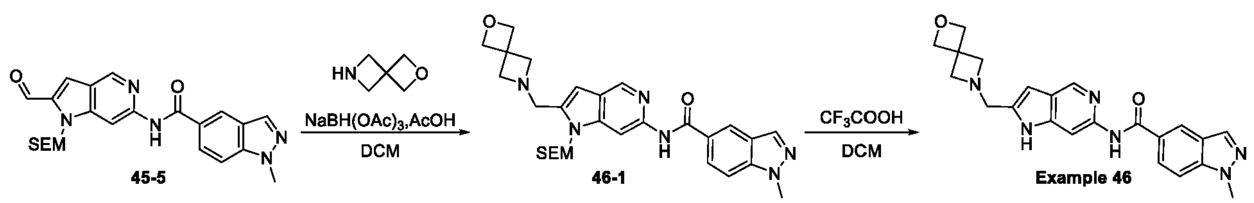


45-5 46-1 Example 46

Prepared in the same fashion as Example 45 but modified as shown above. **LCMS:** $[M+H]^+$=403. **$^1$H-NMR:** (300 MHz, Methanol-$d_4$, ppm) δ 8.53 (dd, *J* = 13.7, 1.3 Hz, 2H), 8.24-8.17 (m, 2H), 8.08 (dd, *J* = 8.9, 1.7 Hz, 1H), 7.71 (d, *J* = 8.9 Hz, 1H), 6.48 (s, 1H), 4.80 (s, 4H), 4.15 (s, 3H), 3.75 (s, 2H), 3.53 (s, 4H).

Preparation of Example 104

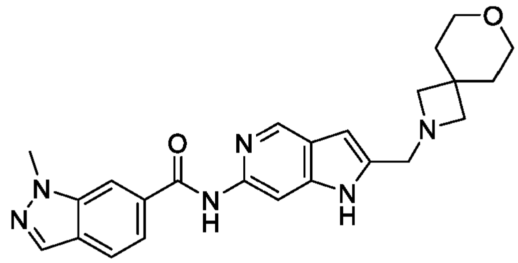

Prepare from N-(2-formyl-1-((2-(trimethylsilyl)ethoxy)methyl)-1H-pyrrolo[3,2-c]pyridin-6-yl)-1-methyl-1H-indazole-6-carboxamide, which is prepared according to Example 45 from 6-chloro-1H-pyrrolo[3,2-c]pyridine-2-carbaldehyde and the corresponding carboxamide) and 7-oxa-2-azaspiro[3.5]nonane. **LCMS:** $[M+H]^+$=431. **$^1$H-NMR:** (300 MHz, Methanol-$d_4$, ppm) δ 8.57 (s, 1H), 8.26 (d, *J* = 10.3 Hz, 2H), 8.12 (s, 1H), 7.92 (d, *J* = 8.5 Hz, 1H), 7.78 (d, *J* = 8.5 Hz, 1H), 6.52 (s, 1H), 4.19 (s, 3H), 3.86 (s, 2H), 3.62 (t, *J* = 4.8 Hz, 4H), 3.26 (s, 4H), 1.79 (t, *J* = 5.2 Hz, 4H).

Preparation of Example 73

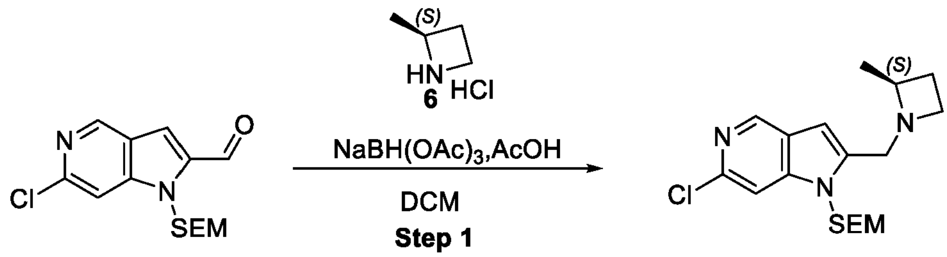


Step 1. Into a 20-mL vial purged and maintained with an inert atmosphere of nitrogen, was placed 6-chloro-1-[[2-(trimethylsilyl)ethoxy]methyl]pyrrolo[3,2-c]pyridine-2-carbaldehyde (350.00 mg, 1.126 mmol, 1.00 equiv), DCM (7.00 mL), (2S)-2-methylazetidine hydrochloride (145.35 mg, 1.351 mmol, 1.20 equiv), AcOH (6.76 mg, 0.113 mmol, 0.10 equiv). The resulting solution was stirred for 1 hr at room temperature. Then $NaBH(OAc)_3$ (357.95 mg, 1.689 mmol, 1.50 equiv) was placed into the vial. The resulting solution was stirred for 12 hr at room temperature. The resulting solution was diluted with 20 mL of $H_2O$. The resulting solution was extracted with 3x20 mL of dichloromethane and the organic layers combined and dried over anhydrous sodium sulfate and concentrated under vacuum. The residue was applied onto a silica gel column with ethyl acetate/hexane (1:5). This resulted in 270 mg (65.52%) of (2S)-1-[(6-chloro-1-[[2-(trimethylsilyl) ethoxymethyl]pyrrolo[3,2-c]pyridin-2-yl)methyl] -2-methylazetidine as brown oil. **LCMS:** $[M+H]^+$=366.

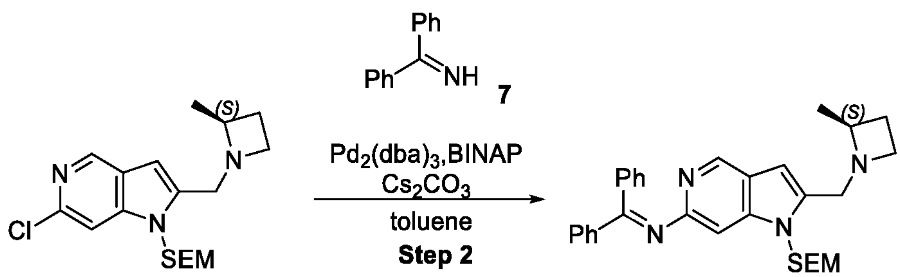


Step 2. Into a 40-mL vial purged and maintained with an inert atmosphere of nitrogen, was placed (2S)-1-[(6-chloro-1-[[2-(trimethylsilyl)ethoxy]methyl]pyrrolo[3,2-c]pyridin-2-yl)methyl]-2-methylazetidine (270.00 mg, 0.738 mmol,

1.00 equiv), Toluene (15.00 mL), benzenemethanimine-phenyl (267.41 mg, 1.475 mmol, 2.00 equiv), $Pd_2(dba)_3$ (67.56 mg, 0.074 mmol, 0.10 equiv), $Cs_2CO_3$ (721.12 mg, 2.213 mmol, 3.00 equiv). The resulting solution was stirred for 5 hr at 110 °C in an oil bath. The resulting mixture was concentrated under vacuum. The residue was applied onto a silica gel column with ethyl acetate/petroleum ether (1:1). This resulted in 180 mg (47.77%) of N-(2-[[(2S)-2-methylazetidin-1-yl]methyl]-1-[[2-(trimethylsilyl)ethoxy]methyl]pyrrolo[3,2-c]pyridin-6-yl)-1,1-diphenylmethanimine as brown oil. **LCMS:** $[M+H]^+$=511.

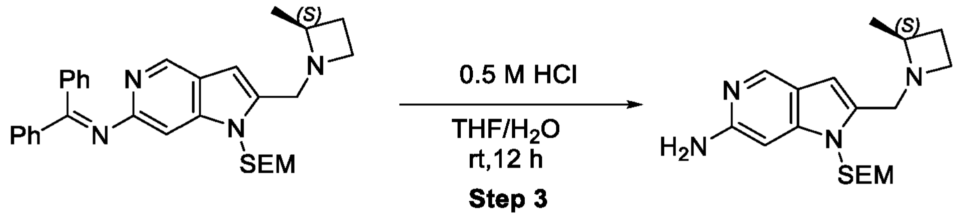


Step 3. Into a 100-mL round-bottom flask, was placed N-(2-[[(2S)-2-methylazetidin-1-yl]methyl]-1-[[2-(trimethylsilyl)ethoxy] methyl]pyrrolo[3,2-c]pyridin-6-yl)-1,1-diphenylmethanimine (180.00 mg, 0.352 mmol, 1.00 equiv), THF (4.00 mL), $H_2O$ (2.00 mL), HCl(0.5M) (2.00 mL). The resulting solution was stirred for 12 hr at room temperature. The resulting mixture was concentrated under vacuum. The resulting solution was diluted with 10 mL of $H_2O$. The resulting solution was extracted with 2x10 mL of ethyl acetate and the aqueous layers combined. The pH value of the solution was adjusted to 8 with $NaHCO_3$. The solids were collected by filtration. This resulted in 100 mg (54.81%) of 2-[[(2S)-2-methylazetidin-1-yl]methyl]-1-[[2-(trimethylsilyl)ethoxy]methyl]pyrrolo[3,2-c]pyridin-6-amine as a yellow solid. **LCMS:** $[M+H]^+$=347.

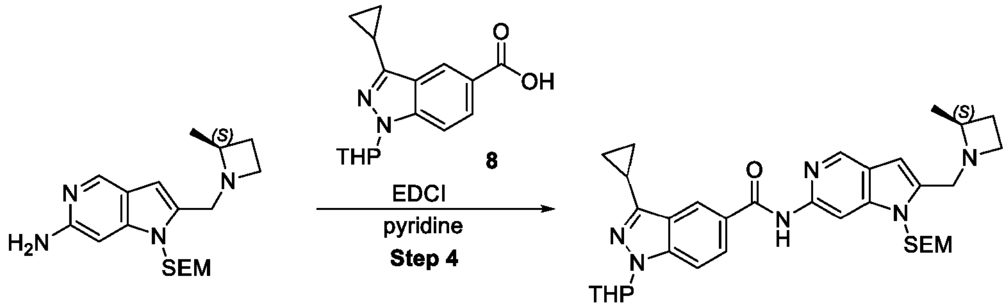


Step 4. Into a 8-mL vial, was placed 2-[[(2S)-2-methylazetidin-1-yl]methyl]-1-[[2-(trimethylsilyl)ethoxy]methyl]pyrrolo[3,2-c]pyridin-6-amine (100.00 mg, 0.289 mmol, 1.00 equiv), Pyridine (2.50 mL), 3-cyclopropyl-1-(oxan-2-yl)indazole-5-carboxylic acid (82.62 mg, 0.289 mmol, 1.00 equiv), EDCI (82.98 mg, 0.434 mmol, 1.50 equiv). The resulting solution was stirred for 12 hr at room temperature. The resulting mixture was concentrated under vacuum. The resulting solution was diluted with 10 mL of $H_2O$. The resulting solution was extracted with 3x10 mL of ethyl acetate and the organic layers combined. The resulting mixture was washed with 2x10 mL of brine. The mixture was dried over anhydrous sodium sulfate and concentrated under vacuum. This resulted in 150 mg (62.35%) of 3-cyclopropyl-N-(2-[[(2S)-2-methylazetidin-1-yl]methyl]-1-[[2-(trimethylsilyl)ethoxy]methyl]pyrrolo[3,2-c]pyridin-6-yl)-1-(oxan-2-yl)indazole-5-carboxamide as brown oil. **LCMS:** $[M+H]^+$=615.

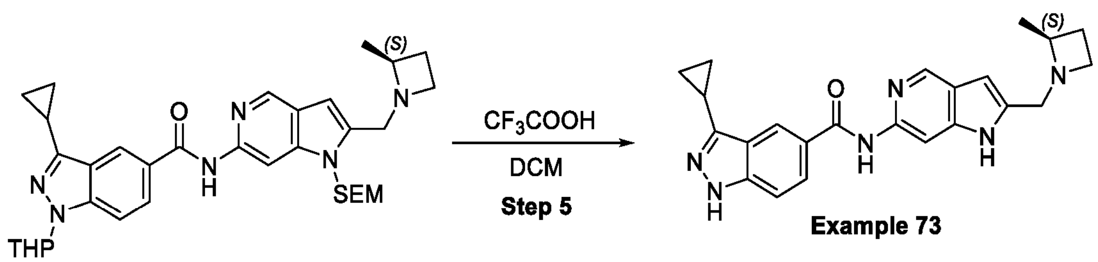

**Example 73**

Step 5. Into a 100-mL round-bottom flask, was placed 3-cyclopropyl-N-(2-[[(2S)-2-methylazetidin-1-yl]methyl]-1-[[2-(trimethylsilyl)ethoxy]methyl]pyrrolo[3,2-c]pyridin-6-yl)-1-(oxan-2-yl)indazole-5-carboxamide (150.00 mg, 0.244 mmol, 1.00 equiv), DCM (3.00 mL), $CF_3COOH$ (3.00 mL). The resulting solution was stirred for 12 hr at room temperature. The resulting mixture was concentrated under vacuum. The resulting solution was diluted with 3 mL of DMF. The pH value of the solution was adjusted to 8 with $NH_3H_2O$. The crude product (100 mg) was purified by Prep-HPLC with

the following conditions: Column, XBridge Prep C18 OBD Column, 5um,19x150mm; mobile phase, Water(0.05% $NH_3H_2O$) and ACN (18% PhaseB up to 39% in 7 min); Detector, UV 254 nm. This resulted in 23.3 mg (23.85%) of 3-cyclopropyl-N-(2-[[(2S)-2-methylazetidin-1-yl]methyl]-1H-pyrrolo[3,2-c]pyridin-6-yl)-1H-indazole-5-carboxamide as a white solid. **LCMS:** $[M+H]^+$=401. **$^1$H-NMR:** (300 MHz, Methanol-$d_4$, ppm) δ 8.55 (t, *J* = 1.2 Hz, 2H), 8.23 (d, *J* = 1.0 Hz, 1H), 8.03 (dd, *J* = 8.8, 1.7 Hz, 1H), 7.58 (dd, *J* = 8.8, 0.8 Hz, 1H), 6.51 (d, *J* = 0.9 Hz, 1H), 3.85 (d, *J* = 13.6 Hz, 1H), 3.72 (d, *J* = 13.7 Hz, 1H), 3.53-3.33 (m, 2H), 3.13-2.98 (m, 1H), 2.45-2.30 (m, 1H), 2.23-2.08 (m, 1H), 1.85 (m, 1H), 1.17-1.08 (m, 7H).

Preparation of Example 107

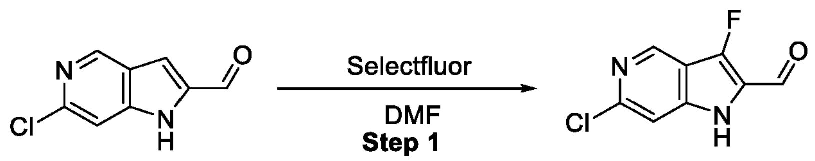


Step 1. Into a 250 mL vial were added 6-chloro-1H-pyrrolo[3,2-c]pyridine-2-carbaldehyde (1.0 g, 5.54mmol, 1.00 equiv), DMF (50.00 mL) and Selectfluor (3.00 g, 8.468 mmol, 1.53 equiv) at room temperature. The resulting mixture was stirred for overnight at room temperature under. The resulting mixture was diluted with EtOAc (250 mL). Then 250 mL of $NaHCO_3$(sat. aq) was added. The resulting mixture was separated and the aqueous layer was extracted with EtOAc (2 x 50 mL). The combined organic layers were dried over anhydrous $Na_2SO_4$. After filtration, the filtrate was concentrated under reduced pressure. The residue was purified by silica gel column chromatography, eluted with PE/EtOAc (1:1) to afford 6-chloro-3-fluoro-1H-pyrrolo[3,2-c]pyridine-2-carbaldehyde (100 mg, 9.0%) as a yellow solid. **LCMS:** $[M+1]^+$=199.

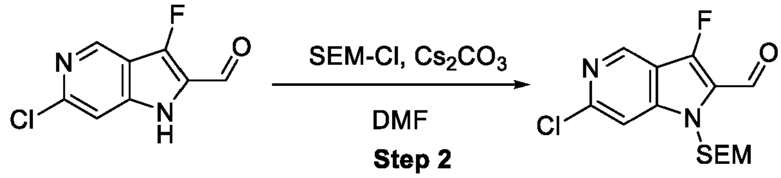


Step 2. Into a 20 mL vial were added 6-chloro-3-fluoro-1H-pyrrolo[3,2-c]pyridine-2-carbaldehyde (100.0mg, 0.504 mmol, 1.00 equiv), DMF (10.00 mL), $Cs_2CO_3$ (328.15 mg, 1.008 mmol, 2.00 equiv) and [2-(chloromethoxy)ethyl] trimethylsilane (125.93 mg, 0.756 mmol, 1.50 equiv) at room temperature. The resulting mixture was stirred for 4 h at room temperature under nitrogen atmosphere. The reaction was quenched with $NaHCO_3$(sat.) at room temperature. The resulting mixture was extracted with EtOAc (3 x 40 mL). The combined organic layers were dried over anhydrous $Na_2SO_4$. After filtration, the filtrate was concentrated under reduced pressure. The residue was purified by reverse flash chromatography with the following conditions: column, C18 silica gel; mobile phase, A: water (0.5% TFA); B: ACN, 40% to 85% B gradient in 30 min; detector, UV 254 nm. This resulted in 6-chloro-3-fluoro-1-[[2-(trimethylsilyl)ethoxy] methyl]pyrrolo[3,2-c]pyridine-2-carbaldehyde (90 mg, 54.35%) as a yellow oil. **LCMS:** $[M+1]^+$=329.

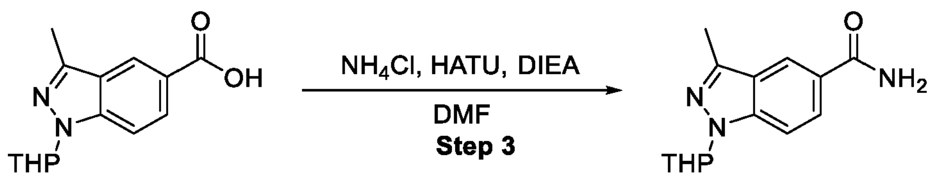


Step 3. Into a 8 mL vial were added 3-methyl-1-(oxan-2-yl)indazole-5-carboxylic acid (100.0 mg, 0.384 mmol, 1.00 equiv), $NH_4Cl$ (61.7 mg, 1.15 mmol, 3.00 equiv), DMF (2.00 mL), DIEA (248.26 mg, 1.92 mmol, 5 equiv) and HATU (219.12 mg, 0.58 mmol, 1.5 equiv) at room temperature. The resulting mixture was stirred for overnight at room temperature. The resulting mixture was diluted with EtOAc (30 mL). The resulting mixture was washed with 2x10 mL of brine. The resulting mixture was concentrated under reduced pressure. The residue was purified by Prep-TLC (PE/EtOAc 1:2) to afford 3-methyl-1-(oxan-2-yl)indazole-5-carboxamide (80 mg, 80.3%) as a light brown solid. **LCMS:** $[M+1]^+$=260.

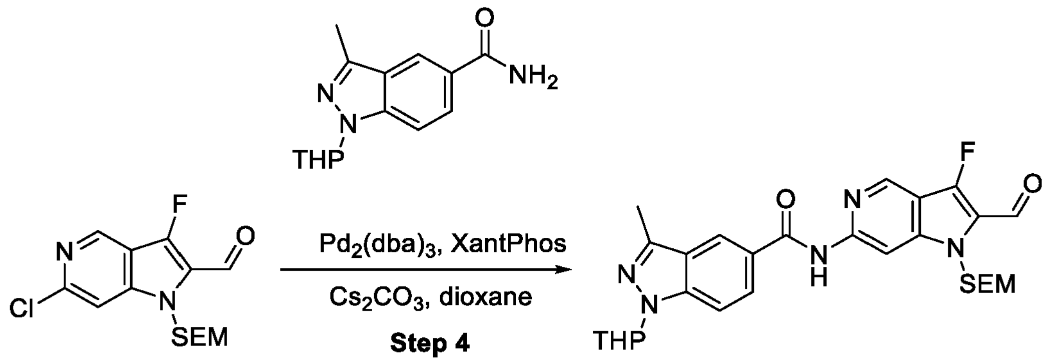


Step 4. Into a 8 mL vial were added 6-chloro-3-fluoro-1-[[2-(trimethylsilyl)ethoxy]methyl]pyrrolo[3,2-c]pyridine-2-carbaldehyde (90.0 mg, 0.274 mmol, 1.00 equiv), 3-methyl-1-(oxan-2-yl)indazole-5-carboxamide(80.00 mg, 0.309 mmol, 1.13 equiv), dioxane (3.00 mL), $Pd_2(dba)_3$ (25.06 mg, 0.027 mmol, 0.10 equiv), XantPhos (31.67 mg, 0.055 mmol, 0.20 equiv) and $Cs_2CO_3$ (267.52 mg, 0.822 mmol, 3.00 equiv) at room temperature. Nitrogen was bubbled slowly into the solution. The resulting mixture was stirred for 6 h at 100 °C under nitrogen atmosphere. The resulting mixture was diluted with EtOAc (50 mL). The resulting mixture was filtered, and the filter cake was washed with EtOAc (1x10 mL). The filtrate was concentrated under reduced pressure. The residue was purified by silica gel column chromatography, eluted with PE/EtOAc (3:1) to afford N-(3-fluoro-2-formyl-1-[[2-(trimethylsilyl)ethoxy]methyl]pyrrolo[3,2-c]pyridin-6-yl)-3-methyl-1-(oxan-2-yl)indazole-5-carboxamide (75 mg, 38.7%) as a yellow solid. **LCMS:** $[M+1]^+$=552.

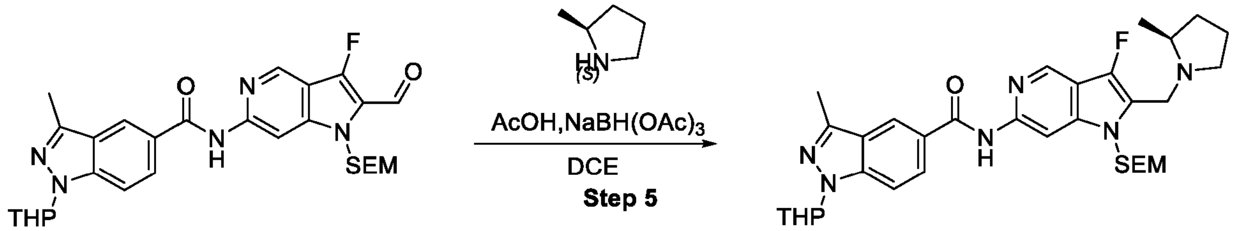


Step 5. Into a 8 mL vial were added N-(3-fluoro-2-formyl-1-[[2-(trimethylsilyl)ethoxy]methyl]pyrrolo[3,2-c]pyridin-6-yl)-3-methyl-1-(oxan-2-yl)indazole-5-carboxamide (75.0 mg, 0.106 mmol, 1.00 equiv, 78%), DCE (4.00 mL), AcOH (6.37 mg, 0.106 mmol, 1.00 equiv) and (2S)-2-methylpyrrolidine (18.06 mg, 0.212 mmol, 2.00 equiv) at room temperature. The resulting mixture was stirred for 30 min at room temperature. To the above mixture was added $NaBH(OAc)_3$ (67.42 mg, 0.318 mmol, 3.00 equiv). The resulting mixture was stirred for 16h at room temperature. The reaction was quenched with $NaHCO_3$(sat.) at room temperature. The resulting mixture was extracted with DCM (3 x 20 mL). The combined organic layers were washed with brine (1x130 mL), dried over anhydrous $Na_2SO_4$. After filtration, the filtrate was concentrated under reduced pressure. This resulted in N-(3-fluoro-2-[[(2S)-2-methylpyrrolidin-1-yl]methyl]-1-[[2-(trimethylsilyl)ethoxy]methyl]pyrrolo[3,2-c]pyridin-6-yl)-3-methyl-1-(oxan-2-yl)indazole-5-carboxamide (60 mg, 64%) as a yellow oil. The crude product was used in the next step directly without further purification. **LCMS:** $[M+1]^+$=621.

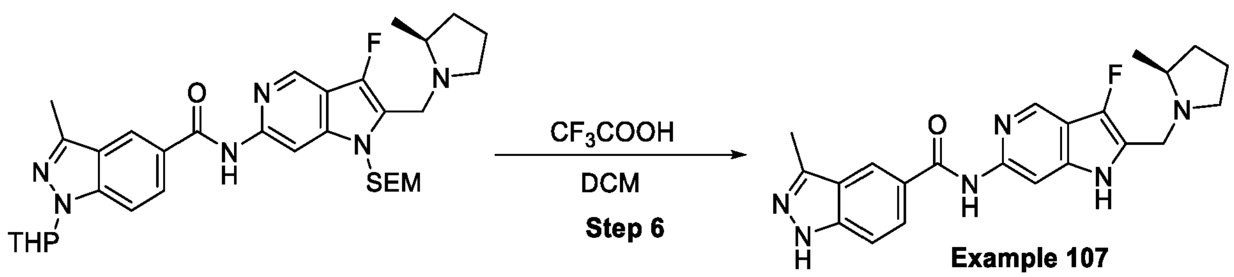


**Example 107**

Step 6. Into a 8 mL vial were added N-(3-fluoro-2-[[(2S)-2-methylpyrrolidin-1-yl]methyl]-1-[[2-(trimethylsilyl)ethoxy]methyl]pyrrolo[3,2-c]pyridin-6-yl)-3-methyl-1-(oxan-2-yl)indazole-5-carboxamide (60.00 mg, 0.068 mmol, 1.00 equiv, 70%), DCM (2.00 mL) and TFA (2.00 mL) at room temperature. The resulting mixture was stirred for overnight at room temperature. The resulting mixture was concentrated under vacuum. The residue was dissolved in DMF (5 mL). The mixture was basified to pH 10 with ammonium hydroxide. The crude product was purified by Prep-HPLC with the following conditions (Column: X-Bridge Shield RP18 OBD Column, 5um, 19*150mm; Mobile Phase A: Water(0.05%NH3H2O),

Mobile Phase B: ACN; Flow rate: 20 mL/min; Gradient: 33% B to 50% B in 7 min, 50% B; Wave Length: 220 nm) to afford N-(3-fluoro-2-[[(2S)-2-methylpyrrolidin-1-yl]methyl]-1H-pyrrolo[3,2-c]pyridin-6-yl)-3-methyl-1H-indazole-5-carboxamide (17.2 mg, 62.55%) as a white solid. **LCMS:** $[M+1]^+$=407. **$^1$H-NMR:** (400 MHz, DMSO-$d_6$, ppm)δ 12.87 (s, 1H), 11.25 (s, 1H), 10.53 (s, 1H), 8.65 (s, 1H), 8.60 (s, 1H), 8.25 (s, 1H), 8.01 (d, J = 8.8 Hz, 1H), 7.52 (d, J = 8.8 Hz, 1H), 3.96 (d, J = 14 Hz, 1H), 3.50 (d, J = 14 Hz, 1H), 2.87 (s, 1H), 2.57 (s, 3H), 2.43 (d, J = 6.8 Hz, 1H), 2.24 (q, J = 8.8 Hz, 1H), 2.00 - 1.86 (m, 1H), 1.64-1.62 (m, 2H), 1.36 (s, 1H), 1.12 (d, J = 6 Hz, 3H). **$^{19}$F-NMR** (PH-PUK): (376 MHz, DMSO-$d_6$, ppm)δ -176.66. ANAL_SFC: 100% ee.

Preparation of Example 108

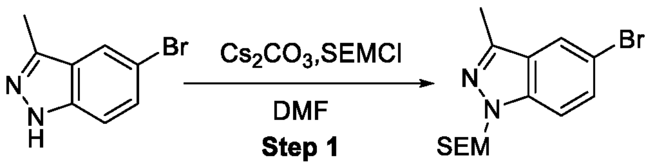


Step 1. Into a 100-mL 3-necked round-bottom flask purged and maintained with an inert atmosphere of nitrogen, was placed 5-bromo-3-methyl-1H-indazole (1.20 g, 5.68 mmol, 1.00 equiv), DMF (15.0 mL), $Cs_2CO_3$ (4.63 g, 0.015 mmol, 2.50 equiv), SEMCl (1.23 g, 7.38 mmol, 1.30 equiv). The resulting solution was stirred for 10 hr at room temperature. The resulting solution was diluted with 50 mL of ice/water. The resulting solution was extracted with 3x20 mL of ethyl acetate and the organic layers combined. The resulting mixture was washed with 3x20 mL of brine. The mixture was dried over anhydrous sodium sulfate and concentrated under vacuum. The residue was applied onto a silica gel column with ethyl acetate/hexane (1:5). This resulted in 1 g (51.53%) of 5-bromo-3-methyl-1-[[2-(trimethylsilyl)ethoxy]methyl]indazole as light yellow oil. **LCMS:** $[M+H]^+$=341.

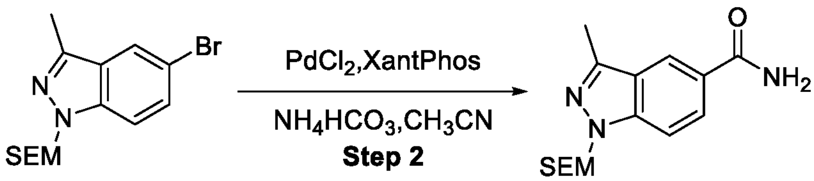


Step 2. Into a 50-mL pressure tank reactor, was placed 5-bromo-3-methyl-1-[[2-(trimethylsilyl)ethoxy]methyl] indazole (1.00 g, 2.930 mmol, 1.00 equiv), $CH_3CN$ (20.00 mL), $PdCl_2$ (51.95 mg, 0.293 mmol, 0.10 equiv), XantPhos (339.04 mg, 0.586 mmol, 0.20 equiv), $NH_4HCO_3$ (2.32 g, 29.3mmol, 10.0 equiv), CO (10 atm). The resulting solution was stirred for 12 hr at 120 °C in an oil bath. The resulting mixture was concentrated under vacuum. The resulting solution was diluted with 20 mL of $H_2O$. The resulting solution was extracted with 3x20 mL of ethyl acetate and the organic layers combined. The resulting mixture was washed with 2x20 mL of brine. The mixture was dried over anhydrous sodium sulfate and concentrated under vacuum. The residue was applied onto a silica gel column with ethyl acetate/petroleum ether (1/1). This resulted in 270 mg (30%) of 3-methyl-1-[[2-(trimethylsilyl)ethoxy]methyl]indazole-5-carboxamide as a yellow semi-solid. **LCMS:** $[M+H]^+$=30.6.

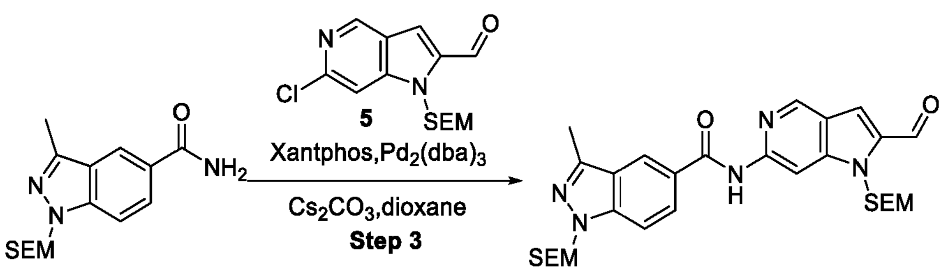


Step 3. Into a 40-mL vial purged and maintained with an inert atmosphere of nitrogen, was placed 3-methyl-1-[[2-(trimethylsilyl)ethoxy]methyl]indazole-5-carboxamide (270.0 mg, 0.884 mmol, 1.00 equiv), dioxane (10.0 mL), 6-chloro-1-[[2-(trimethylsilyl)ethoxy]methyl]pyrrolo[3,2-c]pyridine-2-carbaldehyde (274.77 mg, 0.884 mmol, 1.00 equiv), Xantphos (102.29 mg, 0.177 mmol, 0.20 equiv), $Pd_2(dba)_3$ (80.94 mg, 0.088 mmol, 0.10 equiv), $Cs_2CO_3$ (864.0

mg, 2.65 mmol, 3.00 equiv). The resulting solution was stirred for 12 hr at 100 °C in an oil bath. The resulting mixture was concentrated under vacuum. The resulting solution was diluted with 20 mL of $H_2O$. The resulting solution was extracted with 3x20 mL of ethyl acetate and the organic layers combined. The resulting mixture was washed with 2x20 mL of brine. The mixture was dried over anhydrous sodium sulfate and concentrated under vacuum. The residue was applied onto a silica gel column with ethyl acetate/petroleum ether (1/1). This resulted in 300 mg of N-(2-formyl-1-[[2-(trimethylsilyl) ethoxy]methyl]pyrrolo[3,2-c]pyridin-6-yl)-3-methyl-1-[[2-(trimethylsilyl) ethoxy]methyl]indazole-5-carboxamide as yellow oil. **LCMS:** $[M+H]^+$=580.

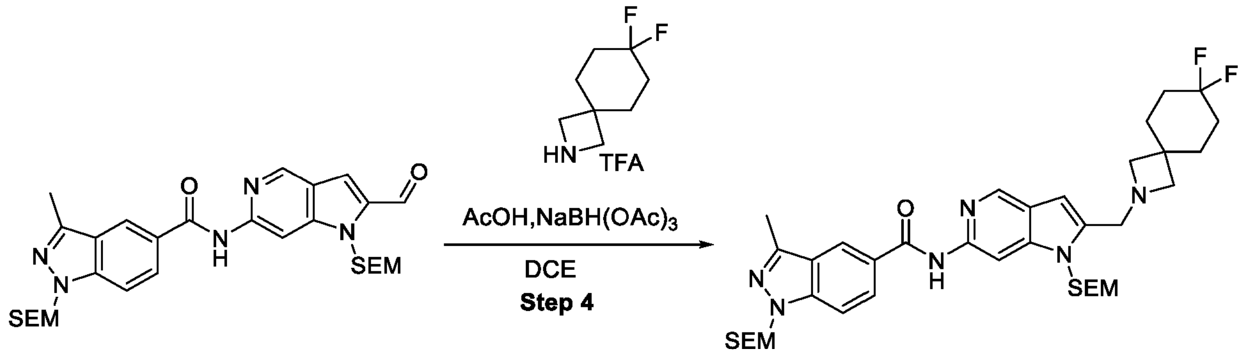


Step 4. Into a 8-mL vial purged and maintained with an inert atmosphere of nitrogen, was placed N-(2-formyl-1-[[2-(trimethylsilyl)ethoxy]methyl]pyrrolo[3,2-c]pyridin-6-yl)-3-methyl-1-[[2-(trimethylsilyl)ethoxy]methyl]indazole-5-carboxamide (150.0mg, 0.259 mmol, 1.00 equiv), DCE (3.00 mL), 7,7-difluoro-2-azaspiro[3.5]nonane; trifluoroacetaldehyde (134.11 mg, 0.518 mmol, 2.00 equiv), AcOH (1.55 mg, 0.026 mmol, 0.10 equiv). The resulting solution was stirred for 30 min at room temperature. Then the $NaBH(OAc)_3$ (109.7 mg, 0.517 mmol, 2.00 equiv) was placed into the vial. The resulting solution was allowed to react, with stirring, for an additional 16 hr at room temperature. The resulting solution was diluted with 20 mL of $NaHCO_3$(5%). The resulting solution was extracted with 3x10 mL of ethyl acetate and the organic layers combined. The resulting mixture was washed with 2x mL of brine. The mixture was dried over anhydrous sodium sulfate and concentrated under vacuum. This resulted in 120 mg (63.98%) of N-[2-([7,7-difluoro-2-azaspiro[3.5]nonan-2-yl]methyl)-1-[[2-(trimethylsilyl)ethoxy]methyl]pyrrolo[3,2-c]pyridin-6-yl]-3-methyl-1-[[2-(trimethylsilyl)ethoxy]methyl]indazole-5-carboxamide as yellow oil. **LCMS:** $[M+H]^+$=725.

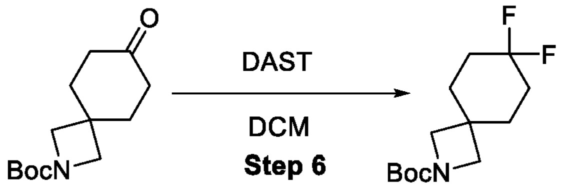


Step 6. Into a 50-mL 3-necked round-bottom flask purged and maintained with an inert atmosphere of nitrogen, was placed tert-butyl 7-oxo-2-azaspiro[3.5]nonane-2-carboxylate (320.0 mg, 1.337 mmol, 1.00 equiv), DCM (25.0 mL), DAST (1.08 g, 6.700 mmol, 5.01 equiv). The resulting solution was stirred for 20 hr at room temperature in a water/ice bath. The resulting solution was diluted with 20 mL of $NaHCO_3$. The resulting solution was extracted with 3x20 mL of dichloromethane and the organic layers combined. The resulting mixture was washed with 2x20 mL of brine. The mixture was dried over anhydrous sodium sulfate and concentrated under vacuum. This resulted in 320 mg (91.6%) of tert-butyl 7,7-difluoro-2-azaspiro[3.5]nonane-2-carboxylate as a solid. **$^1$H-NMR:** (400 MHz, DMSO-d6, ppm) δ 3.54-3.42 (m, 1H), 3.33-3.23 (m, 3H), 2.23 (d, $J$ = 4.6 Hz, 1H), 1.87 (m, 3H), 1.75 (m, 2H), 1.38 (m, 11H). $^{19}$F-NMR: (400 MHz, DMSO-d6, ppm) δ -101.481.

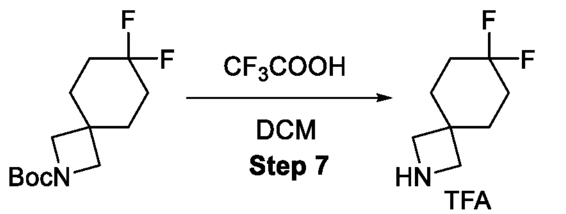


Step 7. Into a 50-mL round-bottom flask, was placed tert-butyl 7,7-difluoro-2-azaspiro[3.5]nonane-2-carboxylate

(320.0 mg, 1 equiv), DCM (5.0 mL), $CF_3COOH$ (5.0 mL). The resulting solution was stirred for 10 hr at room temperature. The resulting mixture was concentrated under vacuum. The crude product was purified by slurry from $Et_2O$. This resulted in 150 mg (47 %) of 7,7-difluoro-2-azaspiro[3.5]nonane; trifluoroacetaldehyde as a white solid. **$^1$H-NMR:** (300 MHz, DMSO-d6, ppm) δ 8.76 (s, 2H), 3.73-3.65(m, 4H), 2.25 (d, *J* = 18.0 Hz, 1H), 1.94-1.84 (m, 7H).

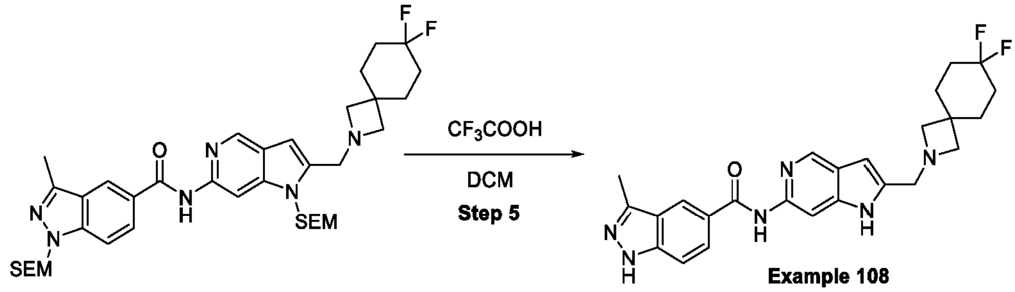


Step 5. Into a 50-mL round-bottom flask, was placed N-[2-([7,7-difluoro-2-azaspiro[3.5]nonan-2-yl] methyl)-1-[[2-(trimethylsilyl)ethoxy]methyl]pyrrolo[3,2-c]pyridin-6-yl]-3-methyl-1-[[2-(trimethylsilyl)ethoxy]methyl]indazole-5-carboxamide (120.00 mg), DCM (3.00 mL), $CF_3COOH$ (3.00 mL). The resulting solution was stirred for 12 hr at room temperature. The resulting mixture was concentrated under vacuum. The resulting solution was diluted with 4 mL of DMF. The pH value of the solution was adjusted to 8 with $NH_3H_2O$. The crude product (80 mg) was purified by Prep-HPLC with the following conditions: Column, XBridge Shield RP18 OBD Column, 5um,19*150mm; mobile phase, Water(0.05% $NH_3H_2O$) and ACN (20% PhaseB up to 45% in 7 min); Detector, UV 254 nm. This resulted in 16.7 mg of N-[2-([7,7-difluoro-2-azaspiro[3 .5]nonan-2-yl]methyl)-1H-pyrrolo[3,2-c]pyridin-6-yl]-3-methyl-1H-indazole-5-carboxamide as a white solid. **LCMS:** $[M+H]^+$=465. **$^1$H-NMR:** (300 MHz, Methanol-$d_4$, ppm) δ 8.54 (m, 1H), 8.48 (m, 1H), 8.20 (s, 1H), 8.04-8.00 (m, 1H), 7.60 (d, *J* = 8.7 Hz, 1H), 6.48 (s, 1H), 3.83 (s, 2H), 3.22 (s, 4H), 2.67 (s, 3H), 1.89-1.82 (m, 8H). $^{19}$F-NMR: (300 MHz, Methanol-$d_4$, ppm) δ -99.161.

Preparation of Example 109

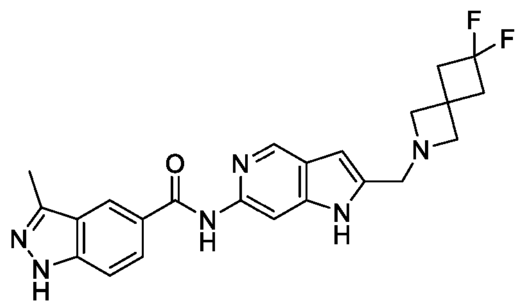

Prepared according to Example 108 using N-(2-formyl-1-[[2-(trimethylsilyl)ethoxy]methyl] pyrrolo[3,2-c]pyridin-6-yl)-3-methyl-1-[[2-(trimethylsilyl)ethoxy]methyl]indazole-5-carboxamide and 6,6-difluoro-2-azaspiro[3.3]heptane. **LCMS:** $[M+H]^+$=437. **$^1$H-NMR:** (300 MHz, Methanol-$d_4$, ppm) δ 8.53 (m, 1H), 8.48 (m, 1H), 8.19 (s, 1H), 8.05 (dd, *J* = 8.8, 1.8 Hz, 1H), 7.60 (d, *J* = 8.9 Hz, 1H), 6.47 (s, 1H), 3.78 (d, *J* = 2.2 Hz, 2H), 3.42 (d, *J* = 2.3 Hz, 4H), 2.74-2.70 (m, 4H), 2.66-2.64 (m, 3H). $^{19}$F-NMR: (300 MHz, Methanol-$d_4$, ppm) δ -93.610.

FRET Assays

Compounds of the invention were tested in a TR-FRET ENL Screening Assay. TR-FRET (time-resolved fluorescence energy transfer) can be used to quantify ENL YEATS domain binding to a crotonylated histone peptide (H3K9cr, aa1-20). Streptavidin-Europium (Eu) chelate binds the biotinylated peptide, while Anti-6xHIS ULight™ binds 6xHIS-ENL. When Eu chelate is excited at 320 nm, fluorescence resonance energy transfer (FRET) occurs if Eu and ULight are made proximal by ENL binding to the acyl-peptide. ULight emission (FRET) is measured at 665 nm and normalized to the Eu emission at 615 nm to reduce variability between wells.

FRET Assay - Protocol 1.

Compounds of the invention were dissolved in DMSO at a concentration of 3mM with subsequent dilutions in assay buffer (50mM HEPES PH7.0, 150mM NaCl, 0.05% BSA, 0.2% Pluronic F-127) such that the assay contained 1% DMSO. In a white 384 shallow well Microplate (Proxiplate-384 Plus, PerkinElmer, 6008280), 150nL of compound or vehicle (1% DMSO in assay buffer) for the high control (HC) wells and 5uL of 30nM ENL Protein (6xHIS ENL YEATS Domain, EpiCypher, 15-0069) were combined and incubated 15 minutes at RT. Low control (LC) wells received 5uL of assay buffer instead of ENL protein. Then 5 μL of 15nM H3K9cr peptide (H3 aa1-20, biotinylated; EpiCypher, 12-0099) in assay buffer was added and incubated 30 minutes at RT. Finally a 5 μL mix of 45nM Anti-6HIS ULight (PerkinElmer, TRF0105) and 1.5nM Streptavidin-Europium Chelate (PerkinElmer, AD0060) were added and incubated for a further 30 minutes at RT. The TR-FRET signal (665 nm signal / 615 nm signal X 10,000) was measured using a PerkinElmer 2104 EnVision (Xenon Flash Lamp excitation, 320 nm ± 37.5 nm excitation filter, 407 nm cut off dichroic mirror, 615 nm ± 4.25 (Europium) nm and 665 nm ± 3.75 nM (ULight) emission filters). Compound concentration response curves were performed in duplicate over the concentration range of 0.15nM-30uM. The response at each compound concentration minus the LC value was converted to percent inhibition of the vehicle control group response (HC-LC). The relationship between the % inhibition and the compound concentration was analyzed using a four parameter logistic equation to estimate lower and upper asymptotes, the compound concentration producing 50% inhibition (IC50 value) and the slope at the mid-point location.

FRET Assay - Protocol 2.

Compounds of the invention were dissolved in DMSO and diluted to 500 μM in assay buffer (50 mM Tris-HCl pH 8.0, 150 mM NaCl, 0.5% Casein, and 0.1% NP-40). Compound dilutions were prepared in assay buffer supplemented with 5% DMSO. In a white 384-well Optiplate (PerkinElmer, 6007299), 4 μL of compound and 4 uL of 625 nM human ENL YEATS (accession Q03111; aa1-150; EpiCypher, 15-0069) were combined and incubated 15 minutes at 23°C. Then 4 μL of 25 nM H3K9cr peptide (H3 aa1-20, biotinylated; EpiCypher, 12-0099) in assay buffer was added and incubated 30 minutes at 23°C. In previous studies (not shown), titrations of each binding partner from 1000 - 1 nM (1:2 dilutions) determined the optimal concentrations for assay development. An 8 μL mix of 37.5 nM Anti-6HIS ULight (PerkinElmer, TRF0105) and 1.25 nM Streptavidin-Europium Chelate (PerkinElmer, AD0060) were added and incubated for 60 minutes at 23°C. TR-FRET signal (665 nm signal / 615 nm signal X 10,000) was measured using a PerkinElmer 2104 EnVision (Xenon Flash Lamp excitation, 320 nm ± 37.5 nm excitation filter, 407 nm cutoff dichroic mirror, 615 nm ± 4.25 (Europium) nm and 665 nm ± 3.75 nM (ULight) emission filters). Each $IC_{50}$ apparent was determined by a 10-point data curve (in duplicate) to identify upper and lower plateaus, with values calculated for compounds inhibiting ≥50% of signal. When necessary (to avoid computation errors in GraphPad Prism), bottom signal constraints were applied equaling the average of the lowest signal (max inhibition). The results indicate that the compounds can block the interaction of a group of epigenetic proteins with acylated histones and can be used as inhibitors for these proteins in broad biological contexts.

Table P (examples 18 and 19 are reference examples for comparative purposes)

| Ex. | ENL FRET (uM) |
|---|---|
| 1 | 0.143 |
| 2 | 0.465 |
| 3 | 0.884 |
| 4 | 0.569 |
| 5 | 0.125 |
| 6 | 0.133 |
| 7 | 0.298 |
| 8 | 0.245 |
| 9 | 0.283 |
| 10 | 1.782 |
| 14 | 1.416 |
| 15 | 0.263 |
| 16 | 1.163 |

(continued)

| Ex. | ENL FRET (uM) |
|---|---|
| 17 | 0.441 |
| 18† | 1.5 |
| 19 | 0.200 |
| 20 | 0.166 |
| 21 | 0.250 |
| 22 | 0.139 |
| 23 | 0.208 |
| 24 | 1.091 |
| 25 | 0.395 |
| 26 | 0.435 |
| 27 | 0.258 |
| 28 | 0.416 |
| 29 | 0.527 |
| 30 | 0.763 |
| 31 | 0.864 |
| 32 | 1.402 |
| 33 | 0.418 |
| 34 | 0.227 |
| 35 | 0.657 |
| 36 | 0.669 |
| 37 | 0.239 |
| 38 | 0.489 |
| 39 | 0.368 |
| 40 | 2.522 |
| 41 | 0.410 |
| 42 | 0.161 |
| 43 | 0.699 |
| 44 | 0.760 |
| 45 | 2.178 |
| 46 | 0.306 |
| 47 | 0.144 |
| 48 | 0.140 |
| 49 | 0.151 |
| 50 | 0.158 |
| 51 | 0.187 |
| 52 | 0.154 |
| 53 | 0.417 |
| 54 | 0.212 |
| 55 | 0.116 |

(continued)

| Ex. | ENL FRET (uM) |
|---|---|
| 56 | 0.078 |
| 57 | 0.272 |
| 58 | 0.295 |
| 59 | 0.513 |
| 60 | 0.330 |
| 61 | 0.325 |
| 62 | 0.193 |
| 63 | 1.965 |
| 64 | 1.352 |
| 65 | 0.137 |
| 66 | 0.085 |
| 67 | 0.125 |
| 68 | 0.622 |
| 69 | 0.412 |
| 71 | 1.042 |
| 72 | 0.364 |
| 73 | 0.238 |
| 74 | 1.090 |
| 75 | 2.342 |
| 76 | 1.586 |
| 77 | 0.963 |
| 78 | 0.145 |
| 79 | 0.511 |
| 80 | 1.896 |
| 81 | 1.198 |
| 82 | 0.033 |
| 83 | 0.168 |
| 84 | 0.177 |
| 85 | 0.176 |
| 86 | 0.209 |
| 87 | 0.330 |
| 88 | 0.141 |
| 89 | 0.499 |
| 90 | 0.126 |
| 91 | 0.707 |
| 92 | 0.210 |
| 93 | 0.097 |
| 94 | 0.118 |
| 95 | 0.160 |

(continued)

| Ex. | ENL FRET (uM) |
|---|---|
| 97 | 0.572 |
| 98 | 0.330 |
| 99 | 0.186 |
| 100 | 0.214 |
| 101 | 0.126 |
| 102 | 0.276 |
| 104 | 0.210 |
| 105 | 0.270 |
| 106 | 0.170 |
| 107 | 0.094 |
| 109 | 0.152 |
| † Examples analyzed via FRET Assay - Protocol 2. | |

Cell Assay

Cell-based assays were used to assess the ability of test compounds to reduce cell viability in both MV4:11 (MLL-AF4 MLL) and K562cells, which were cultured in Iscove's Modified Dulbecco's medium (Gibco, 12440061) containing 10% FBS. The assays were conducted over 12 days and the cells being split on days 4 and 8. Compound concentration response curves were performed in duplicate over the concentration range of 0.15nM-30uM. On day 0, the compounds or vehicle were plated in a 300nL directly into 96 well cell culture plates (Corning, 3599) with 5000 cells/ well in a volume of 100uL. Blank wells received cell culture medium. Plates were incubated for 4 days at 37°C with 5% $CO_2$. On days 4 and day 8 the cells were split and incubated for a further 4 days whilst an aliquot of cells were taken for the CTG readout. For the cell splitting, 270nL of compounds or DMSO was added to a new 96 well cell culture plate to which 90uL of medium plus 10uL of cells from the original assay plate (after mixing) or 100uL of medium (Blank wells) was added. This was repeated on day 8.

Cell viability was assessed using the CellTiter-Glo® homogeneous luminescent assay kit (Promega, G9243), according to the manufacturer's instructions. This quantifies ATP, which indicates the presence of metabolically active cells. On days 4, 8 and 12, 20ul of the remaining cell suspension was aspirated into 384-well plate (Corning 3570) to which an equal volume CellTiter-Glo reagent was. Plates were incubated for 10 minute incubation at RT prior to recording the luminescence signal using EnVision plate reader (PE, 2104). The resulting data were analyzed as follows:

$$Inhibition\ (\%) = 100\% \times (Lum_{vehicle} - Lum_{sample})/(Lum_{vehicle} - Lum_{blank})$$

where vehicle are cells treated with 0.3% DMSO, Blank is culture medium. $IC_{50}$ determinations were calculated by fitting the curve using XLfit (v5.3.1.3): Y = Bottom + (Top - Bottom)/(1 + 10^((LogIC50 - X)*HillSlope)).

Table Q

| Ex. | IC50 (uM) in MV4-11 cells |
|---|---|
| 1 | 0.057 |
| 5 | 0.022 |
| 6 | 0.061 |
| 7 | 0.173 |
| 21 | 0.301 |
| 22 | 0.127 |
| 23 | 0.076 |
| 24 | 1.459 |

(continued)

| Ex. | IC50 (uM) in MV4-11 cells |
|---|---|
| 32 | 0.455 |
| 34 | 0.122 |
| 42 | 0.109 |
| 46 | 1.217 |
| 47 | 0.128 |
| 48 | 0.057 |
| 50 | 0.100 |
| 51 | 0.077 |
| 52 | 0.184 |
| 55 | 0.058 |
| 56 | 0.044 |
| 57 | 0.314 |
| 60 | 0.182 |
| 66 | 0.094 |
| 67 | 0.149 |
| 73 | 0.684 |
| 82 | 0.454 |
| 85 | 0.183 |
| 86 | 0.482 |
| 88 | 0.623 |
| 92 | 0.134 |
| 93 | 0.181 |
| 94 | 0.365 |
| 95 | 0.704 |
| 97 | 0.321 |
| 98 | 1.056 |
| 99 | 0.468 |
| 104 | 0.220 |

## Claims

**1.** A compound selected from

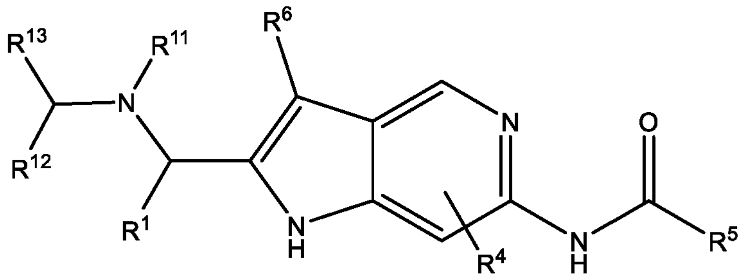

and

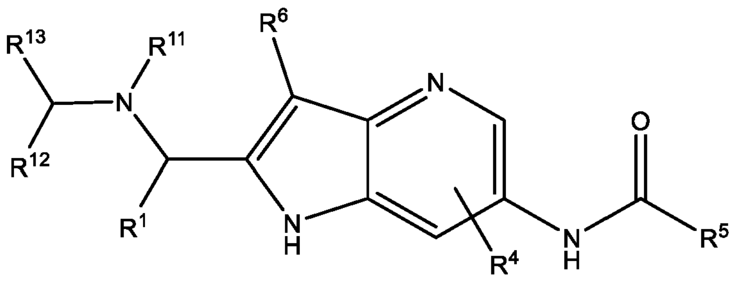

wherein:

$R^1$ is chosen from: H, $(C_{1-6})$alkyl, aryl$(C_{1-6})$alkyl, $(C_{3-12})$cycloalkyl$(C_{1-6})$alkyl, heterocyclyl, heterocyclyl$(C_{1-6})$alkyl, $(C_{1-6})$alkylamino$(C_{1-6})$alkyl, heterocyclylamino$(C_{1-6})$alkyl, heterocyclyl$(C_{1-6})$alkylamino$(C_{1-6})$alkyl, $(C_{3-12})$cycloalkylamino$(C_{1-6})$alkyl, $(C_{3-12})$cycloalkyl$(C_{1-6})$alkylamino$(C_{1-6})$alkyl, arylamino$(C_{1-6})$alkyl, and aryl$(C_{1-6})$alkylamino$(C_{1-}C_6)$alkyl;
$R^4$ is chosen from H, $CH_3$, and Cl;
$R^5$ is chosen from 5- or 6- member carbocycle or heterocycle; bicyclic 5:6 carbocycle or heterocycle and bicyclic 6:6 carbocycle or heterocycle, not attached at a nitrogen of said heterocycle, wherein said carbocycle or heterocycle may be optionally substituted with one or more groups chosen from $(C_{1-8})$hydrocarbyl, $(C_{1-10})$alkoxy, halogen, $(C_{1-6})$haloalkyl, $-SO_2(C_{1-6})$alkyl, $-SO_2NH(C_{0-3}H_{1-7})$, $-CONH(C_{0-3}H_{1-7})$, $-SO_2NH(C_{1-6})$alkoxy, -CN, $CH_2CN$, $CH_2NH_2$, $-NH_2$, $NR^{14}$ where $R^{14}$ is independently chosen from hydrogen, $(C_{1-6})$fluoroalkyl, and $(C_{1-3})$alkoxy, $-CH_2OH$, benzyloxy, $-C(=NH)-NH_2$, -A-Het and additionally, when $R^5$ is a 5- or 6-member heterocycle, bicyclic 5:6 heterocycle, quinoline, isoquinolin, quinazolin, or benzo[b][1,4]oxazine, =O; wherein A is chosen from direct bond, $-(C_{1-6})$alkyl-, $-(C_{1-10})$alkoxy-, $-CO(C_{1-6})$alkyl-, $-SO_2(C_{1-6})$alkyl-, $-SO_2NH(C_{1-6})$alkyl-, and $-CONH(C_{1-6})$alkyl-; and Het is heterocyclyl optionally substituted with $(C_{1-6})$hydrocarbyl, $(C_{1-10})$alkoxy, $(C_{1-10})$alkoxy(C=O)-, hydroxy, =O, or halogen;
$R^6$ is selected from: H, Me, F, and Cl;
$R^{11}$, $R^{12}$ and $R^{13}$ are chosen from the following three groups:

(a) $R^{11}$ is H or $CH_3$;
$R^{12}$ is chosen from H, $(C_1$-$C_6)$hydrocarbyl, hydroxy$(C_1$-$C_6)$hydrocarbyl, and 5- or 6-membered monocyclic heterocyclyl, wherein said heterocycle may be optionally substituted with $(C_1$-$C_6)$hydrocarbyl, hydroxyl, or hydroxy$(C_1$-$C_6)$hydrocarbyl; and $R^{13}$ is hydrogen or methyl; or
(b) $R^{11}$ and $R^{12}$ taken together form an optionally substituted nitrogenous heterocycle attached via nitrogen, said nitrogenous heterocycle chosen from (a) a monocyclic aliphatic nitrogenous heterocycle, (b) a 5:5 or 5:6 bicyclic aliphatic nitrogenous heterocycle, (c) a spirobicyclic aliphatic nitrogenous heterocycle, and (d) an 8-azabicyclo[3.2.1]octane, wherein said optional substituents are independently chosen from $(C_{1-10})$hydrocarbyl, halo$(C_{1-10})$hydrocarbyl, halo$(C_{1-10})$hydrocarbyloxy, $-(C_{1-10})$alkoxy, COOH, $-SO_2(C_{1-6})$alkyl, =O, =S, =NH, and additionally, when $R^{11}$ and $R^{12}$ taken together for a spirobicyclic aliphatic nitrogenous heterocycle, halogen; and $R^{13}$ is chosen from H, $(C_{1-10})$hydrocarbyl, halo$(C_{1-10})$hydrocarbyl, halo$(C_{1-10})$hydrocarbyloxy, $-(C_{1-10})$alkoxy, $-SO_2(C_{1-6})$alkyl, =O, =S, and =NH; or
(c) $R^{11}$ is H; and

$R^{12}$ and $R^{13}$ taken together form a 3- to 7- membered aliphatic carbocycle, wherein said carbocycle may be optionally substituted with $(C_1$-$C_6)$hydrocarbyl, hydroxyl, or hydroxy$(C_1$-$C_6)$hydrocarbyl,
wherein said heterocycle or heterocyclyl moiety contains a heteroatom selected from the group consisting of N, O, or S; and wherein the N or S atom may be optionally oxidized and the N atom optionally may be quaternized.

**2.** A compound according to claim 1 wherein $R^{11}$ and $R^{12}$ taken together form an optionally substituted nitrogenous heterocycle, chosen from (a) a monocyclic aliphatic nitrogenous heterocycle, (b) a 5:5 or 5:6 bicyclic aliphatic nitrogenous heterocycle, (c) a spirobicyclic aliphatic nitrogenous heterocycle, and (d) an 8-azabicyclo[3.2.1]octane, wherein said optional substituents are independently chosen from $(C_{1-10})$hydrocarbyl, halo$(C_{1-10})$hydrocarbyl, halo$(C_{1-10})$hydrocarbyloxy, $-(C_{1-10})$alkoxy, COOH, $-SO_2(C_{1-6})$alkyl, =O, =S, =NH, and additionally, when $R^{11}$ and $R^{12}$ taken together form a spirobicyclic aliphatic nitrogenous heterocycle, halogen.

**3.** A compound according to claim 1 wherein

$R^{11}$ is H; and
$R^{12}$ and $R^{13}$ taken together form a 3- to 7- membered aliphatic carbocycle, wherein said carbocycle may be optionally substituted with $(C_1-C_6)$hydrocarbyl, hydroxyl, or hydroxy$(C_1-C_6)$hydrocarbyl.

**4.** A compound according to claim 1 wherein

$R^{11}$ is H or $CH_3$;
$R^{12}$ is chosen from H, $(C_1-C_6)$hydrocarbyl, hydroxy$(C_1-C_6)$hydrocarbyl, and 5- or 6-membered monocyclic heterocyclyl, wherein said heterocycle may be optionally substituted with $(C_1-C_6)$hydrocarbyl, hydroxyl, or hydroxy$(C_1-C_6)$hydrocarbyl; and
$R^{13}$ is hydrogen or methyl.

**5.** A compound of claim 1 wherein $R^5$ is chosen from bicyclic 5:6 carbocycle or heterocycle and bicyclic 6:6 carbocycle or heterocycle, not attached at a nitrogen of said heterocycle, wherein said carbocycle or heterocycle may be optionally substituted with a group chosen from $(C_{1-6})$alkyl, $(C_{1-6})$alkoxy, halogen, $(C_{1-6})$haloalkyl, $CH_2CN$, $CH_2NH_2$,

$-NH_2$, $NR^{14}$ where $R^{14}$ is independently chosen from hydrogen, $(C_{1-6})$fluoroalkyl, and $(C_{1-3})$alkoxy, $-CH_2OH$, and -A-Het, and additionally, when $R^5$ is a 5- or 6- member heterocycle, bicyclic 5:6 heterocycle, quinoline, isoquinolin, quinazolin, or benzo[*b*][1,4]oxazine, =O; wherein A is chosen from direct bond, $-(C_{1-6})$alkyl-, $-(C_{1-10})$alkoxy-, $-CO(C_{1-6})$alkyl-,
$-SO_2(C_{1-6})$alkyl-, $-SO_2NH(C_{1-6})$alkyl-, and $-CONH(C_{1-6})$alkyl-; and
Het is heterocyclyl optionally substituted with $(C_{1-6})$hydrocarbyl, $(C_{1-10})$alkoxy, $(C_{1-10})$alkoxy(C=O)-, hydroxy, =O, or halogen.

**6.** A compound according to claim 2 wherein the optionally substituted nitrogenous heterocycle is selected from

a monocyclic aliphatic nitrogenous heterocycle optionally substituted with one or more groups chosen from $(C_{1-10})$hydrocarbyl, halo$(C_{1-10})$hydrocarbyl, halo$(C_{1-10})$hydrocarbyloxy, - $(C_{1-10})$alkoxy, COOH, $-SO_2(C_{1-6})$alkyl, =O, =S, and =NH, preferably substituted with methyl or =O;
a 5:5 or 5:6 bicyclic aliphatic nitrogenous heterocycle optionally substituted with one or more groups chosen from $(C_{1-10})$hydrocarbyl, halo$(C_{1-10})$hydrocarbyl, halo$(C_{1-10})$hydrocarbyloxy, - $(C_{1-10})$alkoxy, COOH, $-SO_2(C_{1-6})$alkyl, =O, =S, and =NH;
and
a spirobicyclic aliphatic nitrogenous heterocycle, preferably chosen from the group consisting of azaspirohexanes, heptanes and octanes and oxaazaspirohexanes, heptanes and octanes.

**7.** A compound according to any of claims 1-6 wherein $R^5$ is chosen from phenyl, indazole, pyridine, imidazolopyridine, pyrazolopyrimidine, imidazolopyrazine, triazolopyridine, and benzoxazine, or reduced forms thereof, wherein said carbocycle or heterocycle may be optionally substituted.

**8.** A compound according to any of claims 1-6, wherein $R^5$ is chosen from optionally substituted pyridine, indazole, pyrazine, pyrazole, thiazole, and pyrimidine, substituted with an optionally substituted heterocycle, -CN or $-SO_2NHCH_3$.

**9.** An *in vitro* method of suppressing oncogene expression in a cell comprising exposing said cell to a compound according to any of claims 1-6.

**10.** A compound according to any of claims 1-6 for use in a method for treating a patient with leukemia, wherein the method optionally comprises administering a BET inhibitor.

**11.** A compound selected from

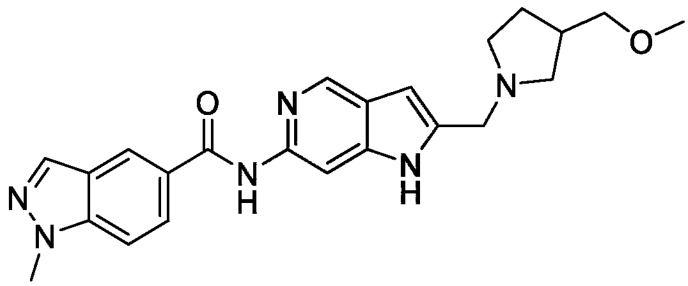

(Example 9)

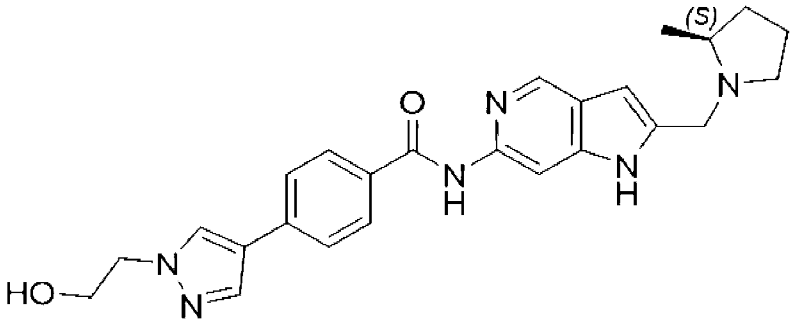

(Example 20)

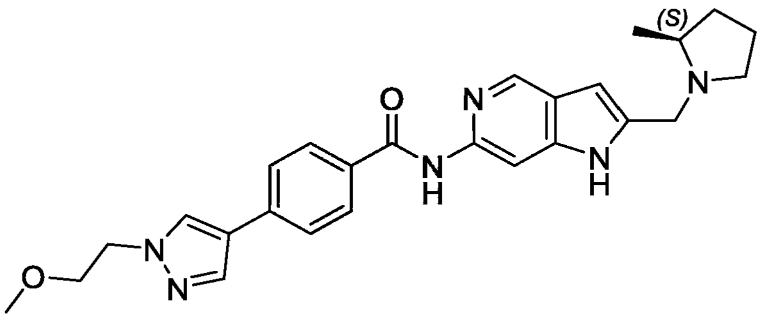

(Example 51)

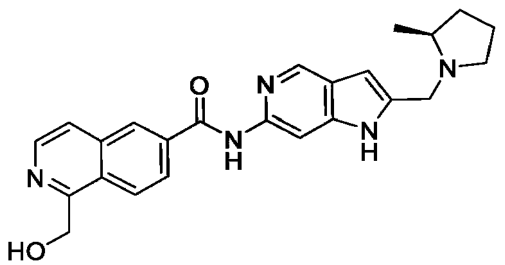

(Example 66)

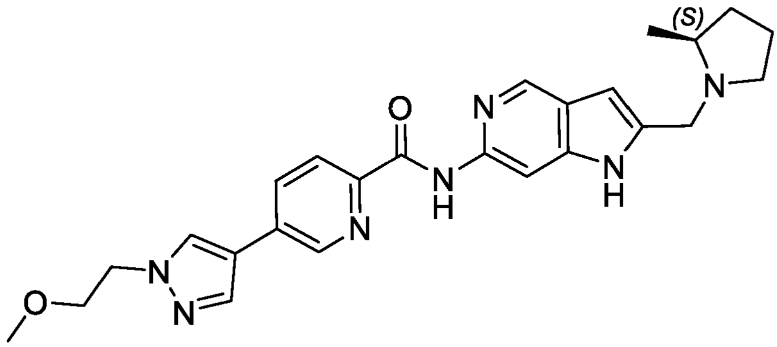

(Example 71

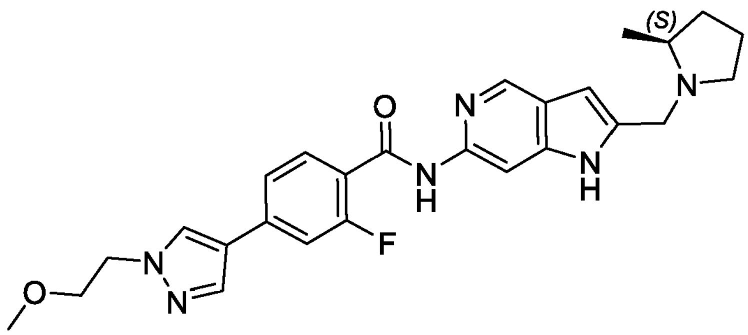

(Example 72)

**12.** A compound according to claim 11 for use in a method for treating a patient with leukemia, wherein the method optionally comprises administering a BET inhibitor.

**Patentansprüche**

**1.** Verbindung, ausgewählt aus

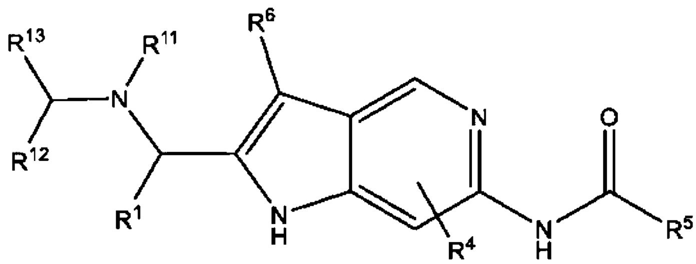

und

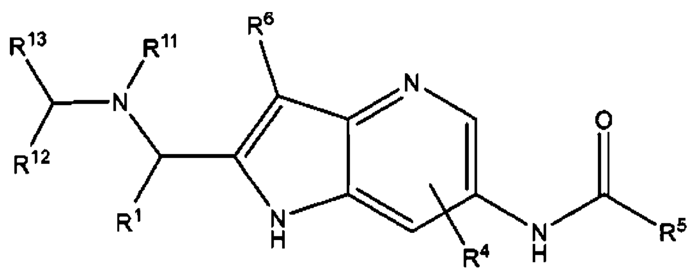

wobei:

$R^1$ ausgewählt ist aus: H, $(C_{1-6})$-Alkyl, Aryl-$(C_{1-6})$-alkyl, $(C_{3-12})$-Cycloalkyl-$(C_{1-6})$-alkyl, Heterocyclyl, Heterocyclyl-$(C_{1-6})$-alkyl, $(C_{1-6})$-Alkylamino-$(C_{1-6})$-alkyl, Heterocyclylamino-$(C_{1-6})$-alkyl, Heterocyclyl-$(C_{1-6})$-alkylamino-$(C_{1-6})$-alkyl, $(C_{3-12})$-Cycloalkylamino-$(C_{1-6})$-alkyl, $(C_{3-12})$-Cycloalkyl-$(C_{1-6})$-alkylamino-$(C_{1-6})$-alkyl, Arylamino-$(C_{1-6})$-alkyl und Aryl-$(C_{1-6})$-alkylamino-$(C_1$-$C_6)$-alkyl;
$R^4$ aus H, $CH_3$ und Cl ausgewählt ist;
$R^5$ aus einem 5- oder 6-gliedrigen Carbocylus oder Heterocyclus; bicyclischen 5:6-Carbocylus oder - Heterocyclus und bicyclischen 6:6-Carbocylus oder - Heterocyclus, der nicht an einen Stickstoff des Heterocyclus gebunden ist, ausgewählt ist, wobei der Carbocylus oder Heterocyclus gegebenenfalls durch eine oder mehrere Gruppen, die aus $(C_{1-8})$-Hydrocarbyl, $(C_{1-10})$-Alkoxy, Halogen, $(C_{1-6})$-Halogenalkyl, -$SO_2$-$(C_{1-6})$-Alkyl, -$SO_2NH(C_{0-3}H_{1-7})$, -CONH $(C_{0-3}H_{1-7})$, -$SO_2NH$-$(C_{1-6})$-Alkoxy, -CN, $CH_2CN$, $CH_2NH_2$, -$NH_2$, $NR^{14}$, wobei $R^{14}$ unabhängig aus Wasserstoff, $(C_{1-6})$-Fluoralkyl und $(C_{1-3})$-Alkoxy ausgewählt ist, -$CH_2OH$, Benzyloxy, -C(=NH)-$NH_2$, -A-Het und zusätzlich dann, wenn $R^5$ für einen 5- oder 6-gliedrigen Heterocyclus, bicyclischen 5:6-Heterocyclus, Chinolin, Isochinolin, Chinazolin oder Benzo[b][1,4]oxazin steht, =O ausgewählt sind, substituiert sein kann; wobei A aus einer direkten Bindung, -$(C_{1-6})$-Alkyl-, -$(C_{1-10})$-Alkoxy-, -CO-$(C_{1-6})$-Alkyl-, -$SO_2$-$(C_{1-6})$-Alkyl-, -$SO_2NH$-$(C_{1-6})$-Alkyl- und -CONH-$(C_{1-6})$-Alkyl- ausgewählt ist und Het für Heterocyclyl steht, das gegebenenfalls durch $(C_{1-6})$-Hydrocarbyl, $(C_{1-10})$-Alkoxy, $(C_{1-10})$-Alkoxy- (C=O)-, Hydroxy, =O oder Halogen substituiert ist;
$R^6$ ausgewählt ist aus: H, Me, F und Cl;
$R^{11}$, $R^{12}$ und $R^{13}$ aus den folgenden drei Gruppen ausgewählt sind:

(a) $R^{11}$ steht für H oder $CH_3$;

$R^{12}$ ist aus H, $(C_1$-$C_6)$-Hydrocarbyl, Hydroxy- $(C_1$-$C_6)$-hydrocarbyl und 5- oder 6-gliedrigem monocyclischem Heterocyclyl ausgewählt, wobei der Heterocyclus gegebenenfalls durch $(C_1$-$C_6)$-Hydrocarbyl, Hydroxyl oder Hydroxy-$(C_1$-$C_6)$-hydrocarbyl substituiert ist; und
$R^{13}$ steht für Wasserstoff oder Methyl; oder

(b) $R^{11}$ und $R^{12}$ bilden zusammengenommen einen gegebenenfalls substituierten stickstoffhaltigen Heterocyclus, der über Stickstoff gebunden ist, wobei der stickstoffhaltige Heterocyclus aus (a) einem monocyclischen aliphatischen stickstoffhaltigen Heterocyclus, (b) einem 5:5- oder 5:6-bicyclischen aliphatischen stickstoffhaltigen Heterocyclus, (c) einem spirobicyclischen aliphatisch stickstoffhaltigen Heterocyclus und (d) einem 8-Azabicyclo[3.2.1]octan ausgewählt ist, wobei die fakultativen Substituenten unabhängig aus $(C_{1-10})$-Hydrocarbyl, Halogen-$(C_{1-10})$-hydrocarbyl, Halogen-$(C_{1-10})$-hydrocarbyloxy, -$(C_{1-10})$-Alkoxy, COOH, -$SO_2$-$(C_{1-6})$-Alkyl, =O, =S, =NH und zusätzlich dann, wenn $R^{11}$ und $R^{12}$ zusammengenommen einen spirobicyclischen aliphatischen stickstoffhaltigen Heterocyclus bilden, Halogen ausgewählt sind; und $R^{13}$ ist aus H, $(C_{1-10})$-Hydrocarbyl, Halogen-$(C_{1-10})$-hydrocarbyl, Halogen-$(C_{1-10})$-hydrocarbyloxy, -$(C_{1-10})$-Alkoxy, -$SO_2(C_{1-6})$-Alkyl, =O, =S und =NH ausgewählt; oder
(c) $R^{11}$ steht für H; und

$R^{12}$ und $R^{13}$ bilden zusammengenommen einen 3- bis 7-gliedrigen aliphatischen Carbocyclus, der gegebenenfalls durch $(C_1$-$C_6)$-Hydrocarbyl, Hydroxyl oder Hydroxy-$(C_1$-$C_6)$-hydrocarbyl substituiert sein kann,
wobei der Heterocyclus bzw. die Heterocyclylgruppierung ein aus der Gruppe bestehend aus N, O oder S ausgewähltes Heteroatom enthält und wobei das N- oder S-Atom gegebenenfalls oxidiert sein kann und das N-Atom gegebenenfalls quaternisiert sein kann.

**2.** Verbindung nach Anspruch 1, wobei $R^{11}$ und $R^{12}$ zusammengenommen einen gegebenenfalls substituierten stickstoffhaltigen Heterocyclus bilden, der aus (a) einem monocyclischen aliphatischen stickstoffhaltigen Heterocyclus, (b) einem 5:5- oder 5:6-bicyclischen aliphatischen stickstoffhaltigen Heterocyclus, (c) einem spirobicyclischen aliphatisch stickstoffhaltigen Heterocyclus und (d) einem 8-Azabicyclo[3.2.1]octan ausgewählt ist, wobei die fakultativen Substituenten unabhängig aus $(C_{1-10})$-Hydrocarbyl, Halogen-$(C_{1-10})$-hydrocarbyl, Halogen-$(C_{1-10})$-hydrocarbyloxy, -$(C_{1-10})$-Alkoxy, COOH, -$SO_2$-$(C_{1-6})$-Alkyl, =O, =S, =NH und zusätzlich dann, wenn $R^{11}$ und $R^{12}$ zusammengenommen einen spirobicyclischen aliphatischen stickstoffhaltigen Heterocyclus bilden, Halogen ausgewählt sind.

**3.** Verbindung nach Anspruch 1, wobei

$R^{11}$ für H steht; und

$R^{12}$ und $R^{13}$ zusammengenommen einen 3- bis 7-gliedrigen aliphatischen Carbocyclus bilden, der gegebenenfalls durch ($C_1$-$C_6$)-Hydrocarbyl, Hydroxyl oder Hydroxy-($C_1$-$C_6$)-hydrocarbyl substituiert sein kann.

**4.** Verbindung nach Anspruch 1, wobei

$R^{11}$ für H oder $CH_3$ steht;
$R^{12}$ ist aus H, ($C_1$-$C_6$)-Hydrocarbyl, Hydroxy-($C_1$-$C_6$)-hydrocarbyl und 5- oder 6-gliedrigem monocyclischem Heterocyclyl ausgewählt, wobei der Heterocyclus gegebenenfalls durch ($C_1$-$C_6$)-Hydrocarbyl, Hydroxyl oder Hydroxy-($C_1$-$C_6$)-hydrocarbyl substituiert ist; und
$R^{13}$ für Wasserstoff oder Methyl steht.

**5.** Verbindung nach Anspruch 1, wobei $R^5$ aus einem bicyclischen 5:6-Carbocylus oder -Heterocyclus und bicyclischen 6:6-Carbocylus oder -Heterocyclus, der nicht an einen Stickstoff des Heterocyclus gebunden ist, ausgewählt ist, wobei der Carbocyclus oder Heterocyclus gegebenenfalls durch eine Gruppe, die aus ($C_{1-6}$)-Alkyl, ($C_{1-6}$)-Alkoxy, Halogen, ($C_{1-6}$)-Halogenalkyl, $CH_2CN$, $CH_2NH_2$, $-NH_2$, $NR^{14}$, wobei $R^{14}$ unabhängig aus Wasserstoff, ($C_{1-6}$)-Fluoralkyl und ($C_{1-3}$)-Alkoxy ausgewählt ist, $-CH_2OH$ und - A-Het und zusätzlich dann, wenn $R^5$ für einen 5- oder 6-gliedrigen Heterocyclus, bicyclischen 5:6-Heterocyclus, Chinolin, Isochinolin, Chinazolin oder Benzo[b] [1,4]oxazin steht, =O ausgewählt ist, substituiert sein kann; wobei A aus einer direkten Bindung, -($C_{1-6}$)-Alkyl-, -($C_{1-10}$)-Alkoxy-, -CO-($C_{1-6}$)-Alkyl-, $-SO_2$-($C_{1-6}$)-Alkyl-, $-SO_2NH$-($C_{1-6}$)-Alkyl- und -CONH-($C_{1-6}$)-Alkyl- ausgewählt ist und Het für Heterocyclyl steht, das gegebenenfalls durch ($C_{1-6}$)-Hydrocarbyl, ($C_{1-10}$)-Alkoxy, ($C_{1-10}$)-Alkoxy-(C=O)-, Hydroxy, =O oder Halogen substituiert ist.

**6.** Verbindung nach Anspruch 2, wobei der gegebenenfalls substituierte stickstoffhaltige Heterocyclus ausgewählt ist aus

einem monocyclischen aliphatischen stickstoffhaltigen Heterocyclus, der gegebenenfalls durch eine oder mehrere Gruppen, die aus ($C_{1-10}$)-Hydrocarbyl, Halogen-($C_{1-10}$)-hydrocarbyl, Halogen-($C_{1-10}$)-hydrocarbyloxy, -($C_{1-10}$)-Alkoxy, COOH, $-SO_2$-($C_{1-6}$)-Alkyl, =O, =S und =NH ausgewählt sind, substituiert ist und vorzugsweise durch Methyl oder =O substituiert ist;
einem 5:5- oder 5:6-bicyclischen aliphatischen stickstoffhaltigen Heterocyclus, der gegebenenfalls durch eine oder mehrere Gruppen, die aus ($C_{1-10}$)-Hydrocarbyl, Halogen-($C_{1-10}$)-hydrocarbyl, Halogen-($C_{1-10}$)-hydrocarbyloxy, -($C_{1-10}$)-Alkoxy, COOH, $-SO_2$-($C_{1-6}$)-Alkyl, =O, =S und =NH ausgewählt sind, substituiert ist;
und
einem spirobicyclischen aliphatischen stickstoffhaltigen Heterocyclus, der vorzugsweise aus der Gruppe bestehend aus Azaspirohexanen, -heptanen und -octanen und Oxaazaspirohexanen, -heptanen und -octanen ausgewählt ist.

**7.** Verbindung nach einem der Ansprüche 1-6, wobei $R^5$ aus Phenyl, Indazol, Pyridin, Imidazolopyridin, Pyrazolopyrimidin, Imidazolopyrazin, Triazolopyridin und Benzoxazin oder reduzierten Formen davon ausgewählt ist, wobei der Carbocyclus bzw. Heterocyclus gegebenenfalls substituiert sein kann.

**8.** Verbindung nach einem der Ansprüche 1-6, wobei $R^5$ aus gegebenenfalls substituiertem Pyridin, Indazol, Pyrazin, Pyrazol, Thiazol und Pyrimidin, das durch einen gegebenenfalls substituierten Heterocyclus, -CN oder - $SO_2NHCH_3$ substituiert ist, ausgewählt ist.

**9.** In-vitro-Verfahren zur Unterdrückung der onkogenen Expression in einer Zelle, bei dem man die Zelle einer Verbindung nach einem der Ansprüche 1 bis 6 aussetzt.

**10.** Verbindung nach einem der Ansprüche 1-6 zur Verwendung bei einem Verfahren zur Behandlung eines Patienten mit Leukämie, wobei das Verfahren gegebenenfalls die Verabreichung eines BET-Inhibitors umfasst.

**11.** Verbindung, ausgewählt aus

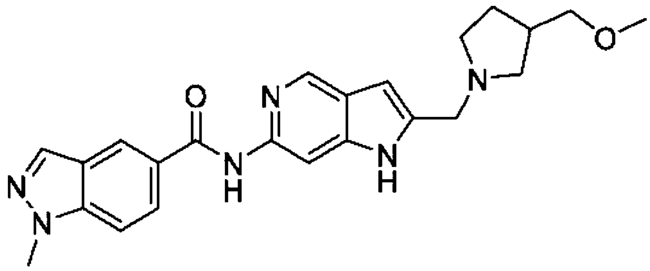

(Beispiel 9)

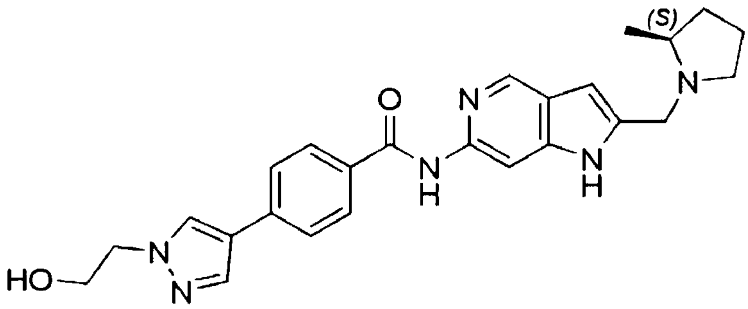

(Beispiel 20)

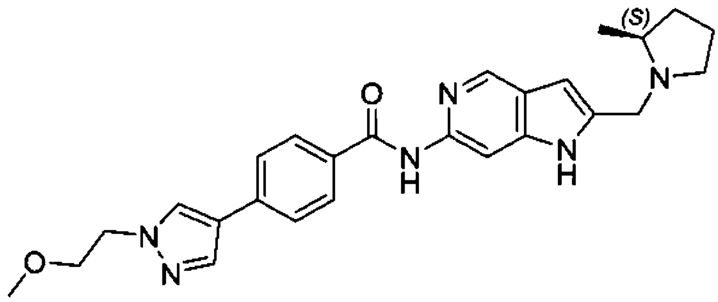

(Beispiel 51)

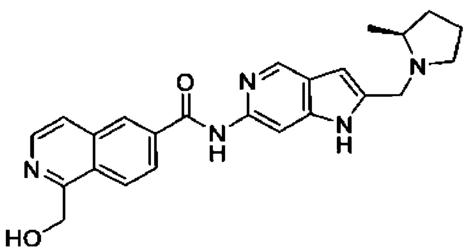

(Beispiel 66)

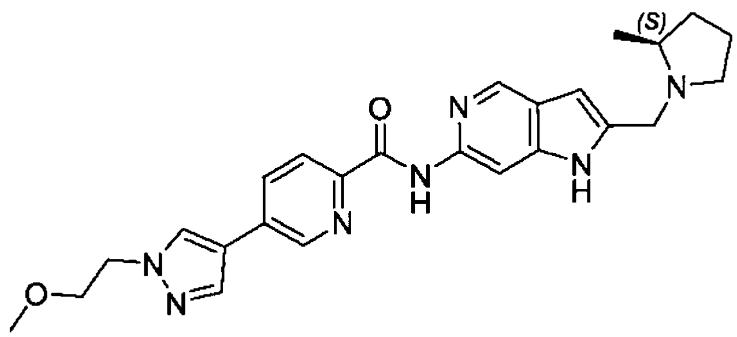

(Beispiel 71)

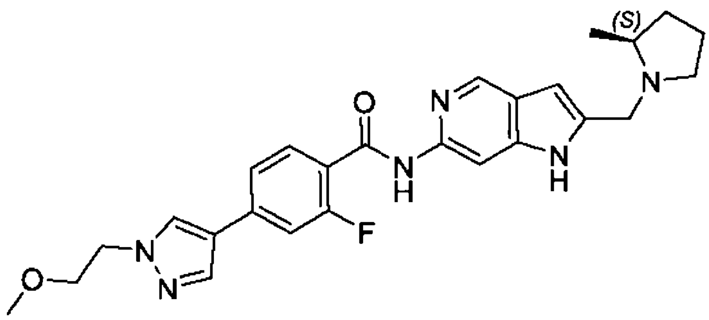

(Beispiel 72)

12. Verbindung nach Anspruch 11 zur Verwendung bei einem Verfahren zur Behandlung eines Patienten mit Leukämie, wobei das Verfahren gegebenenfalls die Verabreichung eines BET-Inhibitors umfasst.

**Revendications**

1. Composé choisi parmi

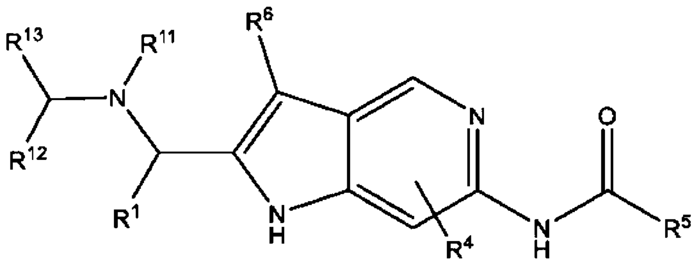

et

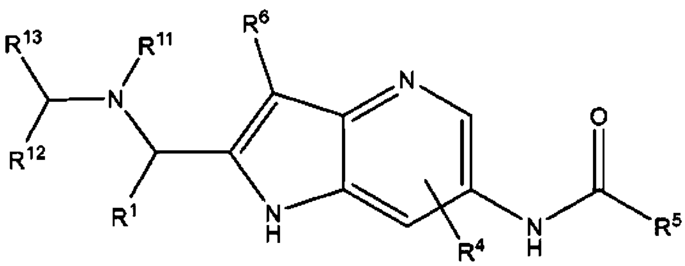

$R^1$ étant choisi parmi : H, $(C_{1-6})$alkyle, aryl$(C_{1-6})$alkyle, $(C_{3-12})$cycloalkyl$(C_{1-6})$alkyle, hétérocyclyle, hétérocyclyl$(C_{1-6})$alkyle, $(C_{1-6})$alkylamino$(C_{1-6})$alkyle, hétérocyclylamino$(C_{1-6})$alkyle, hétérocyclyl$(C_{1-6})$alkylamino$(C_{1-6})$

alkyle, $(C_{3-12})$cycloalkylamino$(C_{1-6})$alkyle, $(C_{3-12})$cycloalkyl$(C_{1-6})$alkylamino$(C_{1-6})$alkyle, arylamino$(C_{1-6})$alkyle, et aryl$(C_{1-6})$alkylamino$(C_1$-$C_6)$alkyle ;

$R^4$ étant choisi parmi H, $CH_3$, et Cl ;

$R^5$ étant choisi parmi carbocycle ou hétérocycle à 5 ou 6 chaînons ; carbocycle ou hétérocycle bicyclique 5:6 et carbocycle ou hétérocycle bicyclique 6:6, non fixé au niveau d'un azote dudit hétérocycle, ledit carbocycle ou hétérocycle pouvant éventuellement être substitué par un ou plusieurs groupes choisis parmi $(C_{1-8})$hydrocarbyle, $(C_{1-10})$alcoxy, halogène, $(C_{1-6})$halogénoalkyle, $-SO_2(C_{1-6})$alkyle, $-SO_2NH(C_{0-3}H_{1-7})$, $-CONH(C_{0-3}H_{1-7})$, $-SO_2NH(C_{1-6})$alcoxy, -CN, $CH_2CN$, $CH_2NH_2$, $-NH_2$, $NR^{14}$, $R^{14}$ étant indépendamment choisi parmi hydrogène, $(C_{1-6})$fluoroalkyle, et $(C_{1-3})$alcoxy, $-CH_2OH$, benzyloxy, - C(=NH)-$NH_2$, -A-Het et de plus, lorsque $R^5$ est un hétérocycle à 5 ou 6 chaînons, un hétérocycle bicyclique 5:6, quinoléine, isoquinoléine, quinazoline, ou benzo[b][1,4]oxazine, =O ; A étant choisi parmi une liaison directe, - $(C_{1-6})$alkyle-, - $(C_{1-10})$alcoxy-, $-CO(C_{1-6})$alkyle-, $-SO_2(C_{1-6})$alkyle-, $-SO_2NH(C_{1-6})$alkyle-, et - $CONH(C_{1-6})$alkyle- ; et Het étant hétérocyclyle éventuellement substitué par $(C_{1-6})$hydrocarbyle, $(C_{1-10})$alcoxy, $(C_{1-10})$alcoxy (C=O) -, hydroxy, =O, ou halogène ; $R^6$ étant choisi parmi : H, Me, F, et Cl ;

$R^{11}$, $R^{12}$ et $R^{13}$ étant choisis parmi les trois groupes suivants :

(a) $R^{11}$ étant H ou $CH_3$ ;

$R^{12}$ étant choisi parmi H, $(C_1$-$C_6)$hydrocarbyle, hydroxy$(C_1$-$C_6)$hydrocarbyle, et hétérocyclyle monocyclique à 5 ou 6 chaînons, ledit hétérocycle pouvant être éventuellement substitué par $(C_1$-$C_6)$hydrocarbyle, hydroxyle, ou hydroxy$(C_1$-$C_6)$hydrocarbyle ; et

$R^{13}$ étant hydrogène ou méthyle ; ou

(b) $R^{11}$ et $R^{12}$ pris ensemble formant un hétérocycle azoté éventuellement substitué fixé via un azote, ledit hétérocycle azoté étant choisi parmi (a) un hétérocycle azoté aliphatique monocyclique, (b) un hétérocycle azoté aliphatique bicyclique 5:5 ou 5:6, (c) un hétérocycle azoté aliphatique spirobicyclique, et (d) un 8-azabicyclo[3.2.1]octane, lesdits substituants éventuels étant indépendamment choisis parmi $(C_{1-10})$hydrocarbyle, halogéno $(C_{1-10})$hydrocarbyle, halogéno $(C_{1-10})$hydrocarbyloxy, $-(C_{1-10})$alcoxy, COOH, $-SO_2(C_{1-6})$alkyle, =O, =S, =NH, et de plus, lorsque $R^{11}$ et $R^{12}$ pris ensemble forment un hétérocycle azoté aliphatique spirobicyclique, halogène ; et

$R^{13}$ étant choisi parmi H, $(C_{1-10})$hydrocarbyle, halogéno$(C_{1-10})$hydrocarbyle, halogéno $(C_{1-10})$hydrocarbyloxy, $-(C_{1-10})$alcoxy, $-SO_2(C_{1-6})$alkyle, =O, =S, et =NH ; ou

(c) $R^{11}$ étant H ; et

$R^{12}$ et $R^{13}$ pris ensemble formant un carbocycle aliphatique à 3 à 7 chaînons, ledit carbocycle pouvant être éventuellement substitué par $(C_1$-$C_6)$hydrocarbyle, hydroxyle, ou hydroxy$(C_1$-$C_6)$hydrocarbyle, ledit groupement hétérocycle ou hétérocyclyle contenant un hétéroatome choisi dans le groupe constitué par N, O, ou S ; et l'atome de N ou S pouvant éventuellement être oxydé et l'atome de N pouvant éventuellement être quaternisé.

**2.** Composé selon la revendication 1, $R^{11}$ et $R^{12}$ pris ensemble formant un hétérocycle azoté éventuellement substitué, choisi parmi (a) un hétérocycle azoté aliphatique monocyclique, (b) un hétérocycle azoté aliphatique bicyclique 5:5 ou 5:6, (c) un hétérocycle azoté aliphatique spirobicyclique, et (d) un 8-azabicyclo[3.2.1]octane, lesdits substituants éventuels étant indépendamment choisis parmi $(C_{1-10})$hydrocarbyle, halogéno $(C_{1-10})$hydrocarbyle, halogéno$(C_{1-10})$hydrocarbyloxy, $-(C_{1-10})$alcoxy, COOH, $-SO_2(C_{1-6})$alkyle, =O, =S, =NH, et de plus, lorsque $R^{11}$ et $R^{12}$ pris ensemble forment un hétérocycle azoté aliphatique spirobicyclique, halogène.

**3.** Composé selon la revendication 1,

$R^{11}$ étant H ; et

$R^{12}$ et $R^{13}$ pris ensemble formant un carbocycle aliphatique à 3 à 7 chaînons, ledit carbocycle pouvant être éventuellement substitué par $(C_1$-$C_6)$hydrocarbyle, hydroxyle, ou hydroxy$(C_1$-$C_6)$hydrocarbyle.

**4.** Composé selon la revendication 1,

$R^{11}$ étant H ou $CH_3$ ;

$R^{12}$ étant choisi parmi H, $(C_1$-$C_6)$hydrocarbyle, hydroxy$(C_1$-$C_6)$hydrocarbyle, et hétérocyclyle monocyclique à 5 ou 6 chaînons, ledit hétérocycle pouvant être éventuellement substitué par $(C_1$-$C_6)$hydrocarbyle, hydroxyle, ou

hydroxy($C_1$-$C_6$)hydrocarbyle ; et
$R^{13}$ étant hydrogène ou méthyle.

**5.** Composé selon la revendication 1, $R^5$ étant choisi parmi carbocycle ou hétérocycle bicyclique 5:6 et carbocycle ou hétérocycle bicyclique 6:6, non fixé au niveau d'un azote dudit hétérocycle, ledit carbocycle ou hétérocycle pouvant être éventuellement substitué par un groupe choisi parmi ($C_{1-6}$)alkyle, ($C_{1-6}$)alcoxy, halogène, ($C_{1-6}$)halogénoalkyle, $CH_2CN$, $CH_2NH_2$, $-NH_2$, $NR^{14}$, $R^{14}$ étant indépendamment choisi parmi hydrogène, ($C_{1-6}$)fluoroalkyle, et ($C_{1-3}$) alcoxy, $-CH_2OH$, et -A-Het, et de plus, lorsque $R^5$ est un hétérocycle à 5 ou 6 chaînons, hétérocycle bicyclique 5:6, quinoléine, isoquinoléine, quinazoline, ou benzo[b] [1,4]oxazine, =O ; A étant choisi parmi une liaison directe, -($C_{1-6}$) alkyle-, -($C_{1-10}$)alcoxy-, -CO($C_{1-6}$)alkyle-, $-SO_2$($C_{1-6}$)alkyle-, $-SO_2NH$($C_{1-6}$)alkyle-, et -CONH($C_{1-6}$)alkyle- ; et Het étant hétérocyclyle éventuellement substitué par ($C_{1-6}$)hydrocarbyle, ($C_{1-10}$)alcoxy, ($C_{1-10}$)alcoxy(C=O)-, hydroxy, =O, ou halogène.

**6.** Composé selon la revendication 2, l'hétérocycle azoté éventuellement substitué étant choisi parmi un hétérocycle azoté aliphatique monocyclique éventuellement substitué par un ou plusieurs groupes choisis parmi ($C_{1-10}$)hydrocarbyle, halogéno ($C_{1-10}$)hydrocarbyle, halogéno ($C_{1-10}$)hydrocarbyloxy, -($C_{1-10}$)alcoxy, COOH, $-SO_2$($C_{1-6}$)alkyle, =O, =S, et =NH, préférablement substitué par méthyle ou =O ;

un hétérocycle azoté aliphatique bicyclique 5:5 ou 5:6 éventuellement substitué par un ou plusieurs groupes choisis parmi ($C_{1-10}$)hydrocarbyle, halogéno($C_{1-10}$)hydrocarbyle, halogéno($C_{1-10}$)hydrocarbyloxy, -($C_{1-10}$)alcoxy, COOH, $-SO_2$($C_{1-6}$)alkyle, =O, =S, et =NH ;
et
un hétérocycle azoté aliphatique spirobicyclique, préférablement choisis dans le groupe constitué par azaspirohexanes, heptanes et octanes et oxaazaspirohexanes, heptanes et octanes.

**7.** Composé selon l'une quelconque des revendications 1 à 6, $R^5$ étant choisi parmi phényle, indazole, pyridine, imidazolopyridine, pyrazolopyrimidine, imidazolopyrazine, triazolopyridine, et benzoxazine, ou des formes réduites correspondantes, ledit carbocycle ou hétérocycle pouvant éventuellement être substitué.

**8.** Composé selon l'une quelconque des revendications 1 à 6, $R^5$ étant choisi parmi pyridine, indazole, pyrazine, pyrazole, thiazole, et pyrimidine éventuellement substitué(e), substitué(e) par un hétérocycle éventuellement substitué, -CN ou $-SO_2NHCH_3$.

**9.** Procédé *in vitro* de suppression de l'expression d'oncogène dans une cellule, comprenant l'exposition de ladite cellule à un composé selon l'une quelconque des revendications 1 à 6.

**10.** Composé selon l'une quelconque des revendications 1 à 6 pour une utilisation dans un procédé de traitement d'un patient atteint de leucémie, le procédé comprenant facultativement l'administration d'un inhibiteur de BET.

**11.** Composé choisi parmi

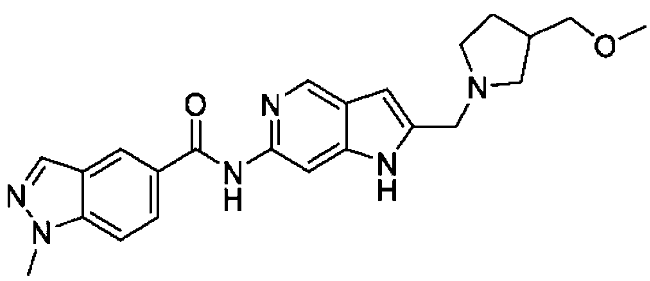

(Exemple 9)

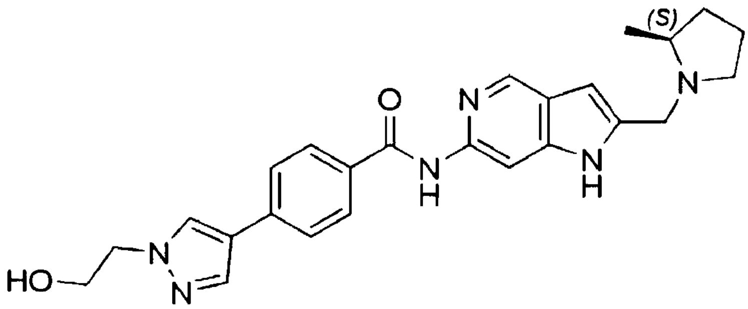

(Exemple 20)

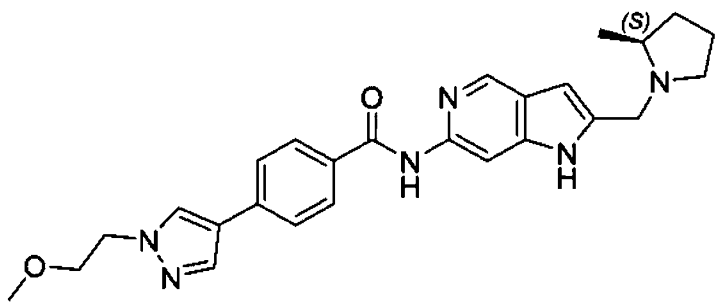

(Exemple 51)

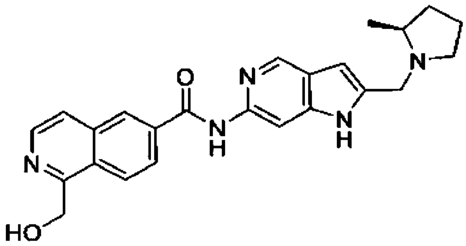

(Exemple 66)

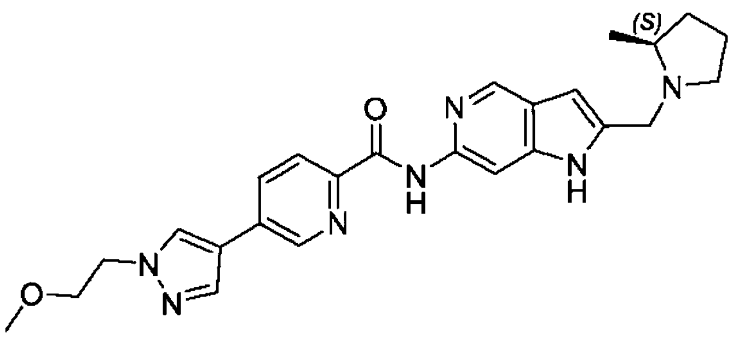

(Exemple 71)

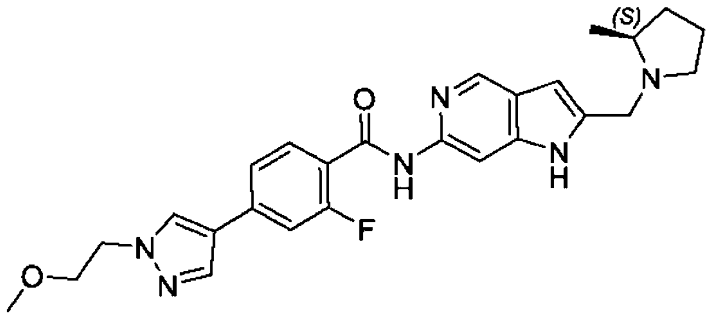

(Exemple 72)

**12.** Composé selon la revendication 11, pour une utilisation dans un procédé de traitement d'un patient atteint de leucémie, le procédé comprenant facultativement l'administration d'un inhibiteur de BET.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2013018073 A1


### Non-patent literature cited in the description


- **WAN et al.** *Nature*, 2017, vol. 543, 265-269
- *CHEMICAL ABSTRACTS*, 202590-98-5
- *CHEMICAL ABSTRACTS*, 2140289-17-2
- *CHEMICAL ABSTRACTS*, 1445993-26-9
- *CHEMICAL ABSTRACTS*, 1300031-49-5
- *CHEMICAL ABSTRACTS*, 1349719-98-7
- *CHEMICAL ABSTRACTS*, 1380087-89-7
- *CHEMICAL ABSTRACTS*, 1260907-17-2
- *CHEMICAL ABSTRACTS*, 1446144-04-2
- *CHEMICAL ABSTRACTS*, 1403764-72-6
- *CHEMICAL ABSTRACTS*, 1268524-70-4
- **CHRISTOTT THOMAS et al.** Discovery of a Selective Inhibitor for the YEATS Domains of ENL/AF9. *SLAS Discovery: Advancing Life Sciences R&D*, 2019, vol. 24 (2), ISSN 2472-5552, 133-141, https://doi.org/10.1177/2472555218809904
- **HEIDENREICH DAVID et al.** Structure-Based Approach toward Identification of Inhibitory Fragments for Eleven-Nineteen-Leukemia Protein (ENL). *J Med Chem.*, 13 December 2018, vol. 61 (23), 10929-10934
- **ANSEL, HOWARD C. et al.** Ansel's Pharmaceutical Dosage Forms and Drug Delivery Systems. Lippincott, Williams & Wilkins, 2004
- **GENNARO, ALFONSO R. et al.** Remington: The Science and Practice of Pharmacy. Lippincott, Williams & Wilkins, 2000
- **ROWE, RAYMOND C**. Handbook of Pharmaceutical Excipients. Pharmaceutical Press, 2005
- **ANSEL, HOWARD C et al.** Ansel's Pharmaceutical Dosage Forms and Drug Delivery Systems. Lippincott, Williams & Wilkins, 2004
- **ALFONSO R. et al.** Remington: The Science and Practice of Pharmacy. Lippincott, Williams & Wilkins, 2000
- **P. G. M. WUTS** ; **T. W. GREENE**. Greene's Protecting Groups in Organic Synthesis. Wiley, 2006
- **T. W. GREENE** ; **P. G. M. WUTS**. Greene's Protective Groups in Organic Synthesis. John Wiley and Sons, 2006
- *CHEMICAL ABSTRACTS*, 214848-62-1
- *CHEMICAL ABSTRACTS*, 1416713-96-6